(19) Europäisches Patentamt
European Patent Office
Office européen des brevets

(11) EP 1 230 224 B1

(12) **EUROPÄISCHE PATENTSCHRIFT**

(45) Veröffentlichungstag und Bekanntmachung des Hinweises auf die Patenterteilung:
**29.12.2004 Patentblatt 2004/53**

(21) Anmeldenummer: **00969149.4**

(22) Anmeldetag: **27.10.2000**

(51) Int Cl.7: **C07D 239/49**, A61K 31/505, A61P 31/04

(86) Internationale Anmeldenummer:
**PCT/CH2000/000575**

(87) Internationale Veröffentlichungsnummer:
**WO 2001/032633 (10.05.2001 Gazette 2001/19)**

# (54) SUBSTITUIERTE 5-BENZYL-2,4-DIAMINOPYRIMIDINE

SUBSTITUTED 5-BENZYL-2,4-DIAMINOPYRIMIDINES

5-BENZYL-2,4-DIAMINOPYRIMIDINES SUBSTITUEES

(84) Benannte Vertragsstaaten:
**AT BE CH CY DE DK ES FI FR GB GR IE IT LI LU MC NL PT SE**

(30) Priorität: **04.11.1999 CH 202199**

(43) Veröffentlichungstag der Anmeldung:
**14.08.2002 Patentblatt 2002/33**

(73) Patentinhaber: **Basilea Pharmaceutica AG
4005 Basel (CH)**

(72) Erfinder:
- **GUERRY, Philippe
CH-4102 Binningen (CH)**
- **MOHR, Peter
CH-4054 Basel (CH)**
- **MULLER, Marc
F-68300 St. Louis (FR)**
- **MUELLER, Werner
CH-4147 Aesch (CH)**
- **PFLIEGER, Philippe
F-68130 Schwoben (FR)**

(74) Vertreter: **Zimmermann, Hans, Dr. et al
A. Braun Braun Héritier Eschmann AG
Holbeinstrasse 36-38
4051 Basel (CH)**

(56) Entgegenhaltungen:
**WO-A-96/16046** **US-A- 4 515 948**

- **HANS H. LOCHER ET AL.: "Antibacterial activities of epiroprim, a new dihydrofolate redictase inhibitor, alone and in combination with dapsone" ANTIMICROBIAL AGENTS AND CHEMOTHERAPY, Bd. 40, Nr. 6, 1996, Seiten 1376-1381, XP002156588**
- **C. D. SELASSIE ET AL.: "On the optimization of hydrophobic and hydrophilic substituent intgeractions of 2,4-diamino-5-(substituted-benzyl)pyrimidi nes with dihydropholate reductase" JOURNAL OF MEDICINAL CHEMISTRY, Bd. 34, Nr. 1, 1991, Seiten 46-54, XP002156589**

EP 1 230 224 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue substituierte 5-Benzyl-2,4-diaminopyrimidine der Formel

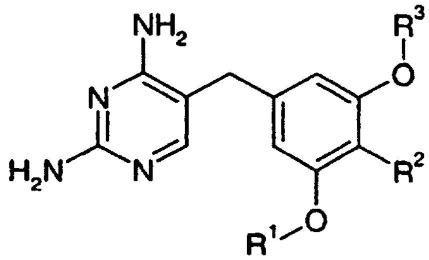

A

in der $R^1$ C2-C3 Alkyl und $R^2$ über eines seiner C-Atome gebundenes Heterocyclyl, Phenyl oder Naphthyl, $R^3$ C2-C6 Alkyl, Alkenyl, Cycloalkyl, Cycloalkylalkyl, Heterocyclylalkyl, Alkylsulfonyl, Cycloalkylsulfonyl, Cycloalkylalkylsulfamoyl, Heterocyclylsulfonyl, Heterocyclylalkylsulfonyl oder Dialkylsulfamoyl darstellt;

wobei Alkyl, Cycloalkyl und Alkenyl allein oder in Zusammensetzungen bis zu 6 Kohlenstoffatome tragen können, Heterocyclyl allein oder in Zusammensetzungen bis zu 6 Ringglieder tragen können und die Gruppen $R^2$ und $R^3$ substituiert sein können,

Säureadditionssalze dieser Verbindungen, sowie die Herstellung und Verwendung solcher Verbindungen als therapeutisch wirksame Pharmazeutika.

Substituierte 5-Benzyl-2,4-diaminopyrimidine werden bei der Bekämpfung bzw. Verhütung von Infektionskrankheiten verwendet, wie zum Beispiel in EP-A 0 793 656 beschrieben.

Die obigen Verbindungen sind neu und besitzen wertvolle antibiotische Eigenschaften. Sie können bei der Bekämpfung bzw. Verhütung von Infektionskrankheiten verwendet werden. Sie zeigen insbesondere eine ausgeprägte antibakterielle Wirkung, auch gegen multiresistente, Gram-positive Stämme, wie Streptococcus pneumoniae, Moraxella catarrhalis und Staphylococcus aureus (einschliesslich Methicillin-resistente Stämme), sowie gegen opportunistische Erreger, wie z.B. Pneumocystis carinii. Diese Verbindungen können auch in Kombination mit bekannten antibakteriell wirksamen Substanzen verabreicht werden und zeigen dann synergistische Effekte. Typische Kombinationspartner sind z.B. Sulfonamide, welche den Verbindungen der Formel A bzw. deren Salzen in verschiedenen Verhältnissen beigemischt werden können.

Gegenstand der vorliegenden Erfindung sind insbesondere Verbindungen der Formel A

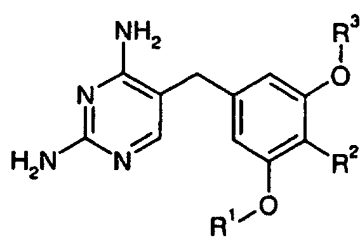

A

in der

- $R^1$ C2-C3 Alkyl ist;
- $R^2$ Phenyl; Naphthyl oder über eines seiner C-Atome gebundenes Heterocyclyl bedeutet, wobei Phenyl, Naphthyl oder Heterocydyl einfach oder mehrfach substituiert sein können; und
- $R^3$ C2-C6 Alkyl, C2-C6 Alkenyl, Cycloalkyl, Cycloalkylalkyl, Heterocyclylalkyl, Alkylsulfonyl, Phenylsulfonyl, Cycloalkylsulfonyl, Cycloalkylalkylsulfonyl, Cycloalkylalkylsulfamoyl, Heterocyclylsulfonyl, Heterocyclylalkylsulfonyl oder Dialkylsulfamoyl darstellt, wobei diese Gruppen unsubstituiert oder substituiert sein können;

und deren pharmazeutisch annehmbaren Säureadditionssalze.

Weiter sind Gegenstand der vorliegenden Erfindung die Anwendung der Verbindungen der Formel A und deren Säureadditionssalze als therapeutische Wirkstoffe, sowie Arzneimittel auf der Basis dieser Stoffe, ggfs. in Kombination mit Sulfonamiden, und deren Herstellung; die Verwendung dieser Stoffe als Heilmittel und zur Herstellung von

antibakeriell wirksamen Arzneimitteln; sowie die Herstellung der Verbindungen der Formel A und ihrer pharmazeutisch annehmbaren Salze und Zwischenprodukte zu deren Herstellung.

Eine Untergruppe der Verbindungen der Formel A ist diejenige, worin die Heterocyclyl- und Phenylgruppen $R^2$ unsubstituiert oder einfach oder mehrfach durch Halogen, Cyan, Alkyl, Alkoxy, Hydroxy, Nitro, Amino, Alkylamino, Dialkylamino, Alkanoylamino, Formyl, Alkanoyloxy, Cyanalkyl, Cyanalkoxy, Hydroxyalkyl, Alkoxyalkyl, Hydroxyalkoxy, Hydroxyalkylamino, Alkoxyalkoxy, Carbamoylalkoxy, Alkylaminoalkyl, Dialkylaminoalkyl, Halogenalkylaminoalkyl,N-Alkyl-N-hatogenalkyl-aminoalkyl, Alkylsulfanyl (Alkylthio), Alkylsulfinyl, Alkylsulfonyl, Alkylsulfonyloxy, Alkylsulfonylamino, $R^7$, $R^7$-alkyl, $R^7$-alkoxy, $R^7$- carbonylalkoxyalkyl oder $R^7$-alkanoylamino substituiert sind, oder auch durch zwei benachbarte, zusammen einen ankondensierten 5- oder 6-gliedrigen Heterocyclus bildende Substituenten substituiert sind; wobei $R^7$ ggfs. substituiertes Heterocyclyl darstellt und Alkyl allein oder in Zusammensetzungen bis zu 6 Kohlenstoffatome tragen kann.

Eine weitere Untergruppe der Verbindungen der Formel A ist diejenige, worin $R^2$ Naphthyl darstellt, das unsubstituiert oder einfach oder mehrfach durch Hydroxy substituiert ist.

Ferner stellen die Verbindungen der Formel A eine Untergruppe dar, worin die Heterocyclylgruppen $R^7$ unsubstituiert oder durch Halogen, Alkyl, Alkoxyalkyl, Hydroxyalkyl, Alkanoyl, Alkanoylaminoalkyl oder Oxo substituiert sind; wobei Alkyl, Alkoxy und Alkanoyl allein oder in Zusammensetzungen bis zu 6 Kohlenstoffatome tragen können.

Eine weitere Untergruppe der Verbindungen der Formel A ist diejenige, worin $R^3$ C2-C6 Alkyl, C2-C6 Alkenyl, Alkylsulfonyl oder Dialkylsulfamoyl bedeutet und unsubstituiert oder durch Halogen, Cyan, Hydroxy oder Alkoxy mit bis zu 6 Kohlenstoffatomen substituiert ist.

Noch eine Untergruppe obiger Verbindungen ist diejenige, worin $R^3$ Cycloalkyl, Cycloalkylalkyl, Heterocyclylalkyl, Cycloalkylsulfonyl, Cycloalkylalkylsulfonyl, Heterocyclylsulfonyl, Heterocyclylalkylsulfonyl, Phenylsulfonyl oder Cycloalkylalkylsulfamoyl bedeutet und diese Gruppen unsubstituiert oder durch Alkyl, Alkoxy, Hydroxyalkyl, Alkoxyalkyl, Cyan, Cyanalkyl oder Cyanalkenyl substituiert sind; wobei Alkyl, Alkoxy und Alkenyl allein oder in Zusammensetzungen bis zu 6 Kohlenstoffatome tragen können.

Nachstehend folgt eine nähere Erläuterung der oben definierten Reste. Setzt sich ein Rest aus zwei oder mehreren der nachstehenden Reste zusammen (z.B. "Cycloalkylalkyl" aus "Cycloalkyl" und "Alkyl"; "Halogenalkylaminoalkyl" aus "Halogen", "Alkyl" und "Alkyl" usw.), gelten die nachstehenden Erläuterungen sinngemäss:

"Halogen" umfasst im Rahmen der vorliegenden Erfindung Fluor, Chlor, Brom und Iod; bevorzugt sind Fluor oder Chlor.

"Alkyl" bzw. "Alkoxy" bedeuten im Rahmen der vorliegenden Erfindung geradkettige oder verzweigte Kohlenwasserstoffgruppen mit höchstens 6, vorzugsweise höchstens 4 Kohlenstoffatomen, falls nicht anders definiert, wie z. B. Methyl, Ethyl, n-Propyl, Isopropyl, Butyl, Isobutyl, sec-Butyl, tert.-Butyl bzw. Methoxy, Ethoxy, n-Propyloxy, Isopropyloxy, Butyloxy, Isobutyloxy, sec-Butyloxy, tert.-Butyloxy.

"Alkenyl" bedeutet eine ungesättigte Kohlenwasserstoffgruppe und trägt höchstens 6, vorzugsweise bis zu 4 Kohlenstoffatome, wie z.B. Vinyl, 2-Propenyl, 2,4-Butadienyl.

"Cycloalkyl" bedeutet einen cyclischen Kohlenwasserstoff mit 3 bis 6 Kohlenstoffatomen wie zB. in Cyclopropyl, Cyclobutyl, Cyclopentyl und Cyclohexyl.

"Alkanoyloxy" bzw. "Alkanoylamino" bedeuten Alkyl-COO- bzw. Alkyl-CONH-Gruppen mit höchstens 6, vorzugsweise bis zu 4 Kohlenstoffen worin Alkyl die oben erläuterte Bedeutung hat.

"Heterocyclyl" bzw. "Heterocyclus" bedeuten im Rahmen der vorliegenden Erfindung ungesättigte oder gesättigte, unsubstituierte oder substituierte 5- oder 6-gliedrige heterocyclische Ringe mit mindestens einem Heteroatom aus Stickstoff, Sauerstoff und Schwefel. Beispiele hierfür sind Pyridyl, Pyrazinyl, Pyridazinyl, Pyrimidyl, Piperidyl, Piperazinyl, Piperimidyl, Pyrrolidinyl, Pyrazolyl, Pyrazolidinyl, Triazinyl, Imidazolyl, Thiazolyl, 1,2,3-Thiadiazolyl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl, 1,2,3-Triazolyl, 1,2,4-Triazolyl, 1H-Tetrazolyl, 2H-Tetrazolyl; Thienyl, Furyl, 3H-1,2,3-Oxathiazolyl, 1,2,3-Oxadiazolyl, 1,2,5-Oxadithioiyl, Isoxazolyl, Isothiazolyl, 4H-1,2,4-Oxadiazinyl, 1,2,5-Oxathiazinyl, 1,2,3,5-Oxathiadiazinyl, Oxazolidinyl, Tetrahydrofuranyl, Tetrahydrothienyl, Morpholino, Thiomorphino, Pyranyl, Tetrahydropyranyl, Dioxanyl usw. Unter diesen Heterocyclen sind bevorzugt: Pyridyl, Piperidyl, Pyirolidinyl, Pyrazolidinyl, Thiazolyl, Thienyl, Furyl, Oxazolidinyl, Tetrahydrofuranyl, Morpholinyl, Thiomorpholinyl und Tetrahydropyranyl. Diese Heterocyclen können unsubstituiert oder durch Halogen, Hydroxy, Alkyl, Alkoxy, Hydroxyalkyl, Alkoxyalkyl, Cyan, Cyanalkyl oder Cyanalkenyl substituiert sein.

Zwei benachbarte Substituenten an einem Phenylring oder Heterocyclus können zusammen einen ankondensierten, 5- oder 6-gliedrigen Heterocyclus bilden, Beispiele solcher Ringe sind 1H-Indolyl, 1H-Indazolyl, 3,4-Dihydro-2H-benzo[1,4]oxazinyl, Benzo[1,3]dioxolyl, 2,3-Dihydro-benzo[1,4]dioxinyl, Chinolinyl oder 1,2,3,4-Tetrahydrochinolinyl.

Die Verbindungen der Formel A bilden pharmazeutisch annehmbare Säureadditionssalze mit organischen und anorganischen Säuren. Beispiele von Säureadditionssalzen von Verbindungen der Formel A sind Salze mit Mineralsäuren, beispielsweise Halogenwasserstoffsäuren, wie Salzsäure, Bromwasserstoff und Iodwasserstoff, Schwefelsäure, Salpetersäure, Phosphorsäure und dergleichen, Salze mit organischen Sulfonsäuren, beispielsweise mit Alkyl- und Arylsulfonsäuren, wie Methansulfonsäure, p-Toluolsulfonsäure, Benzolsulfonsäure und dergleichen, sowie Salze mit organischen Carbonsäuren, beispielsweise mit Essigsäure, Weinsäure, Maleinsäure, Zitronensäure, Benzoesäure, Salicylsäure, Ascorbinsäure und dergleichen.

Bevorzugte Untergruppen der erfindungsgemässen 5-Benzyl-2,4-diaminopyrimidine der Formel A mit besonders wertvollen antibiotischen Eigenschaften sind die folgenden Untergruppen A1, A2 und A3:

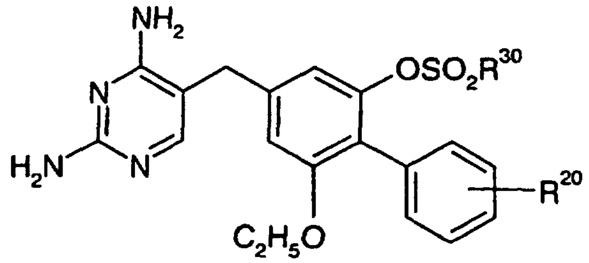

A1

in der

$R^{20}$ C1-C6 Alkyl, C1-C6 Alkoxy, Amino, C1-C6 Alkylamino, Fluor oder Chlor und

$R^{30}$ C1-C6 Alkyl, C3-C6 Cycloalkyl; di-(C1-C6 Alkyl)amino, N-(C3-C6 Cycloalkyl)-N-(C1-C6 Alkyl)amino oder einen 5- oder 6-gliedrigen, gesättigten, am Stickstoff verknüpften N-Heterocyclus darstellt,

sowie Säureadditionssalze dieser Verbindungen.

$R^{20}$ ist bevorzugt Methyl, Methoxy, Amino, Methylamino oder Fluor; $R^{30}$ ist bevorzugt Isopropyl, sec-Butyl, Cyclobutyl, Dimethylamino, N-Cyclopropyl-N-methyl-amino oder Morpholino.

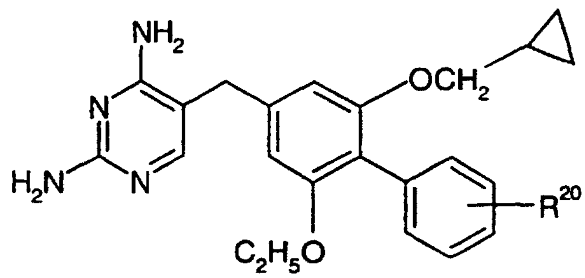

A2

in der $R^{20}$ C1-C6 Alkyl, C1-C6 Alkoxy, Amino, C1-C6 Alkylamino, Fluor oder Chlor bedeutet, sowie Säureadditionssalze dieser Verbindungen.

$R^{20}$ ist bevorzugt Methyl, Methoxy, Amino, Methylamino oder Fluor.

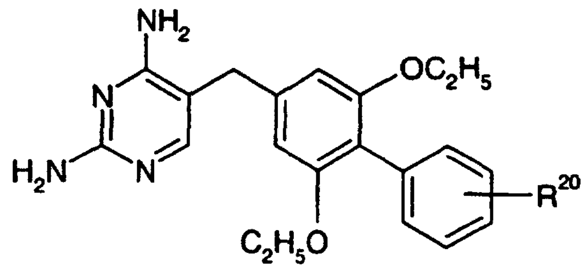

A3

in der $R^{20}$ C1-C6 Alkyl, C1-C6 Alkoxy, Amino, C1-C6 Alkylamino, Fluor oder Chlor bedeutet, sowie Säureadditionssalze dieser Verbindungen.

$R^{20}$ ist bevorzugt Methyl, Methoxy, Amino, Methylamino oder Fluor.

Bevorzugte Verbindungen mit besonders wertvollen antibiotischen Eigenschaften sind:

Butan-2-sulfonsäure-4'-amino-4-(2,4-diamino-pyrimidin-5-ylmethyl)-6-ethoxybiphenyl-2-yl-ester,
Cyclobutan-sulfensäure-4'-amino-4-(2,4-diamino-pyrimidin-5-ylmethyl)-6-ethoxy-3'-methyl-biphenyl-2-yl-ester,
Morpholino-4-sulfonsäure-4-(2,4-diamino-pyrimidin-5-ylmethyl)-6-ethoxy-4'methylamino-biphenyl-2-yl-ester,
Dimethyl-sulfaminsäure-3'-amino-4-(2,4-diamino-pyrimidin-5-ylmethyl)-6-ethoxy-4'-methyl-biphenyl-2-yl-ester,
Propan-2-sulfonsäure-4'-amino-4-(2,4-diamino-pyrimidin-5-ylmethyl)-6-ethoxybiphenyl-2-yl-ester,
Butan-2-sulfonsäure-3'-amino-4-(2,4-diamino-pyrimidin-5-ylmethyl)-6-ethoxybiphenyl-2-yl-ester,
N-Cyclopropyl-N-methyl-sulfaminsäure-4'-amino-4-(2,4-diamino-pyrimidin-5-ylmethyl)-6-ethoxy-biphenyl-2-yl-ester,
Propan-2-sulfonsäure-3'-amino-4-(2,4-diamino-pyrimidin-5-ylmethyl)-6-ethoxybiphenyl-2-yl-ester,
Propan-2-sulfonsäure-4'-amino-4-(2,4-diamino-pyrimidin-5-ylmethyl)-6-ethoxy-3'-methyl-biphenyl-2-yl-ester,
5-(3'-Amino-6-cyclopropylmethoxy-2-ethoxy-biphenyl-4-ylmethyl)-pyrimidin-2,4-diamin,
5-(3'-Amino-2,6-diethoxy-biphenyl-4-ylmethyl)-pyrimidin-2,4-diamin sowie Säureadditionssalze dieser Verbindungen.

Die Verbindungen der Formel A und ihre pharmazeutisch annehmbaren Salze können erfindungsgemäss hergestellt werden, indem man

a) eine Verbindungen der allgemeinen Formel

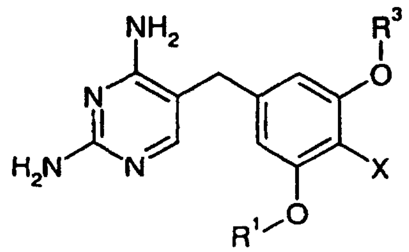

B

mit einer Verbindung der allgemeinen Formel

$$R^2Y \qquad C$$

umsetzt,

in denen $R^1$, $R^2$ und $R^3$ die obige Bedeutung haben, wobei darin ggfs. vorhandene phenolische Hydroxygruppen und Amino/Alkylaminogruppen geschützt sind, eines der Symbole X und Y eine Abgangsgruppe und das andere eine mit dieser Abgangsgruppe austretende Gruppe darstellt,
vorhandene Schutzgruppen abspaltet und gewünschtenfalls aromatische Substituenten an $R^2/R^3$ derivatisiert, oder dass man

b) eine Verbindung der allgemeinen Formel

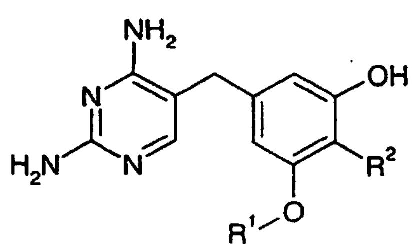

D

mit einer Verbindung der allgemeinen Formel

$$R^3Z \qquad E$$

in Gegenwart einer Base umsetzt,

in denen $R^1$, $R^2$ und $R^3$ die obige Bedeutung haben, wobei darin ggfs. vorhandene phenolische Hydroxygruppen und Amino/Alkylaminogruppen geschützt sind und Z eine Abgangsgruppe darstellt,

vorhandene Schutzgruppen abspaltet und gewünschtenfalls aromatische Substituenten an $R^2$/$R^3$ derivatisiert, oder dass man

c) zur Herstellung von Verbindungen der Formel A, worin $R^2$ und/oder $R^3$ eine Sulfonylgruppe $-SO_2-$ enthält, eine entsprechende Verbindung, worin $R^2$ und/oder $R^3$ eine entsprechende Sulfanylgruppe -S- oder Sulfinylgruppe -SO- enthält, einer Oxidation unterwirft, oder dass man

d) eine Verbindung der Formel A in ein pharmazeutisch annehmbares Säureadditionssalz überführt.

In der Umsetzung der Verbindungen der Formeln B und C gemäss Verfahrensvariante a) des erfindungsgemässen Verfahrens versteht man unter austretenden Gruppen Abgangsgruppen X bzw. Y, welche miteinander reagieren und somit, unter Bildung eines austretenden Nebenproduktes, beide "austreten". Es stehen dem Fachmann diesbezüglich viele Möglichkeiten offen; als Beispiele seien die folgenden Ausführungsformen genannt:

X bedeutet beispielsweise Brom, Iod, Methylsulfonyloxy, Trifluormethylsulfonyloxy, Phenylsulfonyloxy, p-Tosylsulfonyloxy; und

Y ist $(OH)_2B$-.

Diese Umsetzung mit einer Aryl/Heterocyclylboronsäure C, ebenfalls als "Suzuki-Kopplung" bekannt, erfolgt vorzugsweise in einem inerten organischen Lösungsmittel, wie z.B. Dioxan, Tetrahydrofuran, Dimethylformamid oder Dimethoxyethan, bei einer Temperatur zwischen etwa 20°C und dem Siedepunkt des Reaktionsgemisches. Vorzugsweise wird eine Base, wie Alkalimetallcarbonat, z.B. Kaliumcarbonat, zugesetzt sowie ein Katalysator, vorzugsweise ein Palladiumkomplex, wie Tetrakis-triphenylphosphin-palladium.

Eine Variante der Suzuki-Kopplung besteht in der in situ Generierung des Reagenzes $R^2-B(OH)_2$, indem man die Verbindung der Formel B mit geschütztem Tetrahydroxydiboron (z.B. Bis(pinakolato)diboron) und einer Verbindung der Formel $R^2Y^1$, worin $Y^1$ eine austretende Gruppe, wie Brom, Iod, Methylsulfonyloxy, Trifluormethylsulfonyloxy, Phenylsulfonyloxy oder p-Tosylsulfonyloxy, umsetzt. Die Reaktionsbedingungen sind sonst die gleichen.

Es kann in der obigen Reaktion als Reaktionspartner der Formel C eine Aryl/Heterocyclyl-Metallverbindung mit Y = $Sn(\text{nieder-Alkyl})_3$, z.B. $-Sn(CH_3)_3$ oder - $Sn(n\text{-Butyl})_3$ ("Stille Reaktion"); -MgHal ("Grignard-Kopplung"); oder -ZnHal mit Hal = Brom oder Iod eingesetzt werden. In dieser Reaktion wird keine Base verwendet, jedoch vorzugsweise der oben beschriebene Katalysator. Es kann auch von Vorteil sein, ein inertes Salz, insbesondere Lithiumchlorid, zuzugeben.

Die oben erwähnte Reaktion kann ebenfalls mit vertauschten Substituenten X und Y durchgeführt werden, z.B. mit X = $-Sn(CH_3)_3$, -MgHal oder -ZnHal und Y = Brom, Iod, Methylsulfonyloxy, Trifluormethylsulfonyloxy, Phenylsulfonyloxy, p-Tosylsulfonyloxy. Die Reaktionsbedingungen sind im wesentlichen die gleichen.

Die bei der Verfahrensvariante b) des erfindungsgemässen Verfahrens eingesetzte Verbindung der Formel E enthält eine Abgangsgruppe Z; diese stellt vorzugsweise Chlor, Brom, Iod, Methylsulfonyloxy, Trifluormethylsulfonyloxy, Phenylsulfonyloxy oder p-Tosylsulfonyloxy dar, vorzugsweise Chlor oder Brom. Die bei der Umsetzung von Verbindungen D und E verwendete Base ist vorzugsweise ein Alkalimetall-nieder-alkoxid, insbesondere Kalium-tert.-butoxid, kann aber auch Alkalimetallcarbonat oder -hydrid, z.B. Natrium- oder Kaliumcarbonat, Natrium- oder Kaliumhydrid, sein. Die Umsetzung erfolgt vorzugsweise in einem inerten organischen Lösungsmittel, wie z.B. Dimethylformamid, Dimethylacetamid, Tetrahydrofuran oder Dioxan oder einem Gemisch dieser Lösungsmittel bei einer Temperatur zwischen etwa -80°C und +150°C, vorzugsweise 0-80°C. In den Reaktionsteilnehmern der Verfahrensvarianten a) und b) vorhandene phenolische Hydroxygruppen werden vorzugsweise auf einer Vorproduktstufe durch Benzylsubstitution geschützt. Dies erfolgt durch Behandeln mit dem entsprechenden Benzylhalogenid. Die Abspaltung zum Hydroxy-Endprodukt erfolgt katalytisch über Palladiumkohle, z.B. in Aethanol/Essigsäure bei Raumtemperatur. Weitere Möglichkeiten der Schützung von phenolischen Hydroxygruppen ist die Methoxymethylsubstitution. Einführung erfolgt durch Umsetzung mit einem Methoxymethylhalogenid, z.B. mit dem Chlorid, die Abspaltung erfolgt z.B. mit Chlorwasserstoff in Tetrahydrofuran bei etwa 0°C bis 60°C.

Die phenolischen Hydroxygruppen können auch unter Verwendung von Silylgruppen, z.B. Trimethylsilyl, t-Butyldimethylsilyl geschützt werden. Diese werden vorteilhaft eingeführt durch Behandeln mit dem entsprechenden Silylchlorid. Die Abspaltung kann durch Einwirken eines Fluorids erfolgen, vorzugsweise Alkalimetallfluorid oder Tetrabutylammoniumfluorid, in einem organischen Lösungsmittel, z.B. Dimethylformamid oder Acetonitril, bei etwa 0°C bis 50°C.

Die phenolischen Hydroxygruppen können ebenfalls durch niedere Alkanoylgruppen, z.B. Acetyl, geschützt

werden. Eingeführt wird z.B. durch Behandeln mit einem niederen Alkanoylhalogenid oder -anhydrid, z.B. dem Chlorid, in Gegenwart einer Base, wie Natriumhydroxid, DBU (1,5-Diazabicyclo[4.3.0]non-5-en) oder Diisopropylethylamin. Die Abspaltung erfolgt unter milden alkalischen Bedingungen (pH etwa 7-8), z.B. mit Natriumhydroxid oder -carbonat, bei etwa 0° bis 50°C.

In den Reaktionsteilnehmern der Verfahrensvarianten a) und b) vorhandene Aminogruppen bzw. Alkylaminogruppen sind zweckmässig durch Trifluoracetyl, tert-Butoxycarbonyl oder Benzyloxycarbonyl geschützt. Einführung der Schutzgruppen erfolgt mit dem entsprechenden Anhydrid oder Halogenid in einer Base, wie Pyridin, in einem inerten Lösungsmittel, wie Methylenchlorid, bei etwa -20°C bis Raumtemperatur. Die Abspaltung der Schutzgruppen erfolgt vielfach spontan bei der Kopplung oder auch durch Behandlung mit verdünnter wässriger Natronlauge und Ethanol bei etwa Raumtemperatur bis zum Siedepunkt des Reaktionsgemisches.

Aromatische Substituenten an $R^2/R^3$ können weiter derivatisiert werden. Beispiele hierfür sind:

1) $-NO_2 \rightarrow -NH_2$
Diese Reduktion erfolgt vorteilhaft durch katalytische Hydrierung mit Palladiumkohle, einer niederen Alkancarbonsäure und einem niederen Alkanol, z.B. Essigsäure/Methanol bei etwa 20°C bis zum Siedepunkt des Reaktionsgemisches (vgl. Beispiel 2).

2) $-CHO \rightarrow -CH_2-R$, worin R Alkylamino, Halogenalkylamino, Dialkylamino oder N-Heterocyclyl darstellt.
Diese reduktive Kopplung erfolgt z.B. durch Umsetzung des Aldehyds mit dem entsprechenden Amin und einem Alkalimetallborhydrid oder Alkalimetallcyanborhydrid, z.B. die Natriumverbindung, in einem niederen Alkanol, wie Methanol, bei etwa 0°C bis Raumtemperatur (vgl. Beispiel 4v).

3) Alkylamino $\rightarrow$ Dialkylamino
Durch reduktive Alkylierung mit Formaldehyd in der gleichen Weise wie für 2 oben führt man eine Methylgruppe ein. Andere niedere Alkylaldehyde führen zum entsprechenden Ergebnis (vgl. Beispiele 4ac und 4ad).

4)

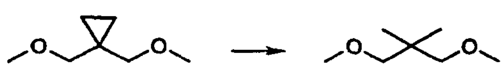

Die Cyclopropylgruppe wird geöffnet durch katalytische Reduktion mit Platindioxid in einer niederen Alkancarbonsäure/einem niederen Alkanol, z.B. Essigsäure/Methanol, bei etwa 20 bis 100°C (vgl. Beispiel 8a).

5)

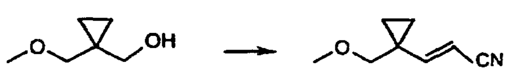

Die Umwandlung erfolgt durch Oxidation der Hydroxygruppe zum Aldehyd mit z.B. Mangandioxid, gefolgt durch Wittig-Synthese mit Triphenylphosphoranylidenacetonitril in Methylenchlorid und/oder Dimethylformamid bei etwa 20°C bis zum Siedepunkt des Reaktionsgemisches (vgl. Beispiel 8d).

6)

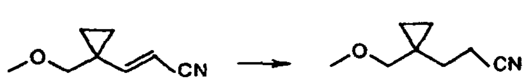

Diese Reduktion erfolgt mit einem Alkalimetallborhydrid oder Alkalimetallcyanborhydrid, z.B. die Natriumverbindung, in einem niederen Alkanol, wie Isopropanol, bei etwa -20°C bis zum Siedepunkt des Reaktionsgemisches (vgl. Beispiele 9c, 47 und 48).

7) Subsitution von aromatischem Amino und phenolischem Hydroxy, z.B. $NH_2 \rightarrow NHSO_2$Alkyl; OH $\rightarrow OSO_2$Alkyl; OH $\rightarrow$ Carbamoylalkoxy; OH $\rightarrow$ Cyanalkoxy; OH $\rightarrow$ Alkanoyloxy; OH $\rightarrow$ Alkoxyalkoxy; OH $\rightarrow$ Hydroxyalkoxy; OH $\rightarrow$ Heterocyclylalkoxy.

Diese Reaktionen erfolgen mit dem entsprechenden Halogenid, z.B. dem Chlorid, Bromid oder Iodid, mit dem Azid oder mit dem Säureanhydrid in einem inerten organischen Lösungsmittel, wie Methylenchlorid, Chloroform, Dimethylformamid, Dimethylacetamid, Tetrahydrofuran oder Dioxan bei etwa -50°C bis etwa 80°C. Zur Bildung von Hydroxyalkoxy wird mit einem geschützten Hydroxyalkylderivat, z.B. mit einem Tetrahydropyranyloxyalkylchlorid, Trimethylsilyloxyalkyliodid oder t-Butyl-dimethylsilyloxyalkyliodid, umgesetzt, und die Schutzgruppe anschliessend sauer abgespalten, z.B. mit Mineralsäure, z.B. methanolischer Salzsäure, bei Raumtemperatur. (Vgl. Beispiele 44, 45, 46, 50, 55, 56, 58, 59 und 61).

8) Sättigung von Aromaten, z.B.

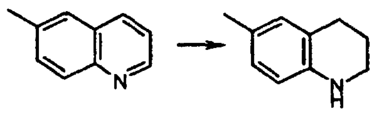

Diese Reaktion erfolgt mit einem Alkalimetallborhydrid, z.B. der Natriumverbindung. Die Chinolingruppe geht dabei in Gegenwart eines Nickel(II)chlorid-Katalysators bei Raumtemperatur in die 1,2,3,4-Tetrahydrochinolingruppe über.

Zur Herstellung von Endprodukten mit einer Sulfonylgruppe $-SO_2-$, gemäss obiger Verfahrensvariante c) kann man die entsprechende Sulfanyl- oder Sulfinyl-Verbindung herstellen und anschliessend oxidieren. Beispielsweise wird ein Alkylsulfanylsubstituent am $R^2$ (Alkylthio) durch Oxidation in einem inerten organischen Lösungsmittel, wie Methylenchlorid, mit $H_2O_2$ oder mit m-Chlorperbenzoesäure und Trifluoressigsäure, gefolgt von Behandlung mit Thiosulfat ($Na_2S_2O_3$) bei 0°C bis Raumtemperatur, in die entsprechende Alkylsulfonyl-Verbindung übergeführt.

Die Herstellung der Säureadditionssalze der Verbindungen der Formel A gemäss Variante d) kann in an sich bekannter Weise erfolgen, z.B. durch Addition einer organischen oder anorganischen Säure. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die nachstehenden Reaktionsschemata 1 und 2 veranschaulichen die Herstellung der Endprodukte:

Reaktionsschema 1

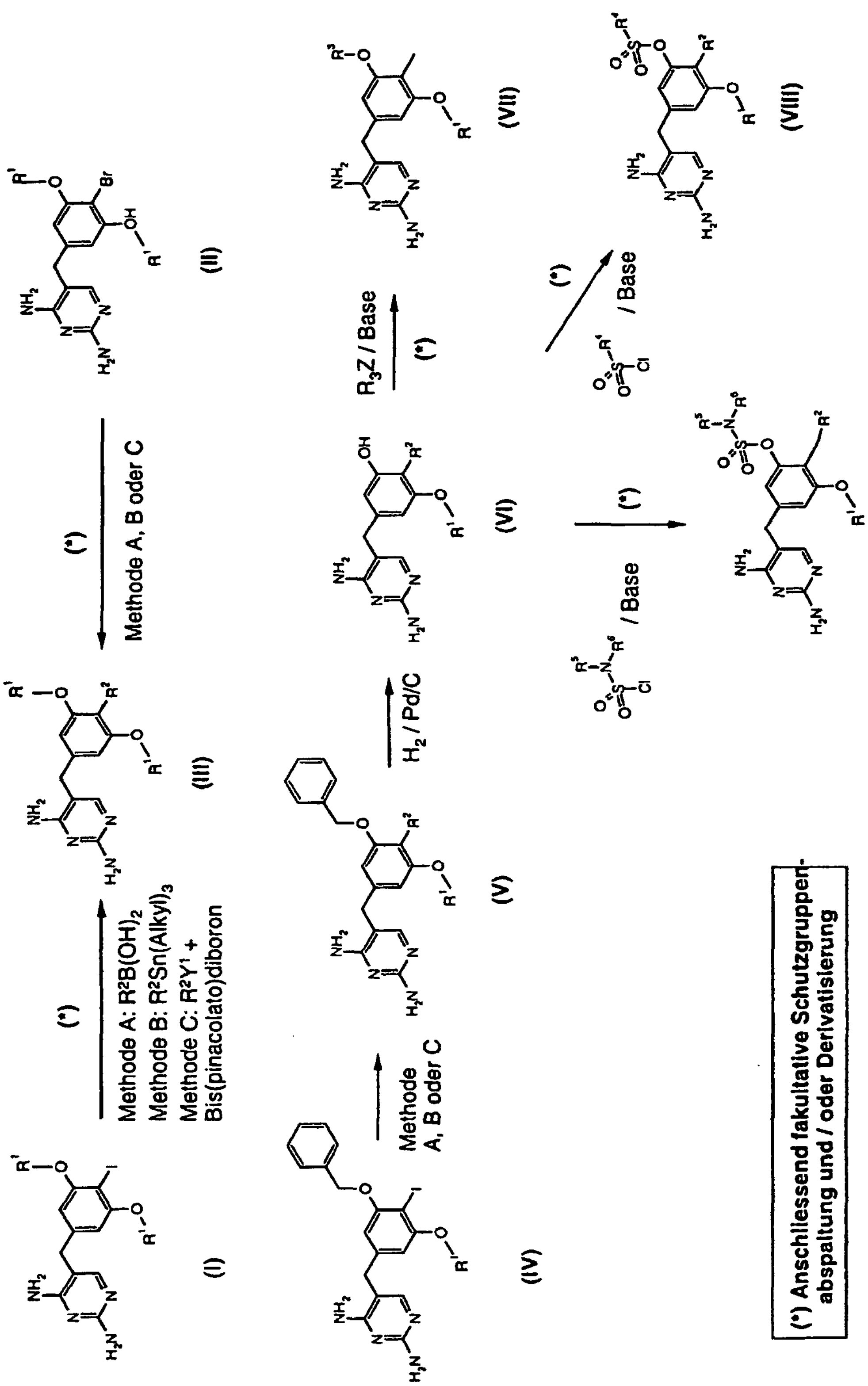

Reaktionsschema 2

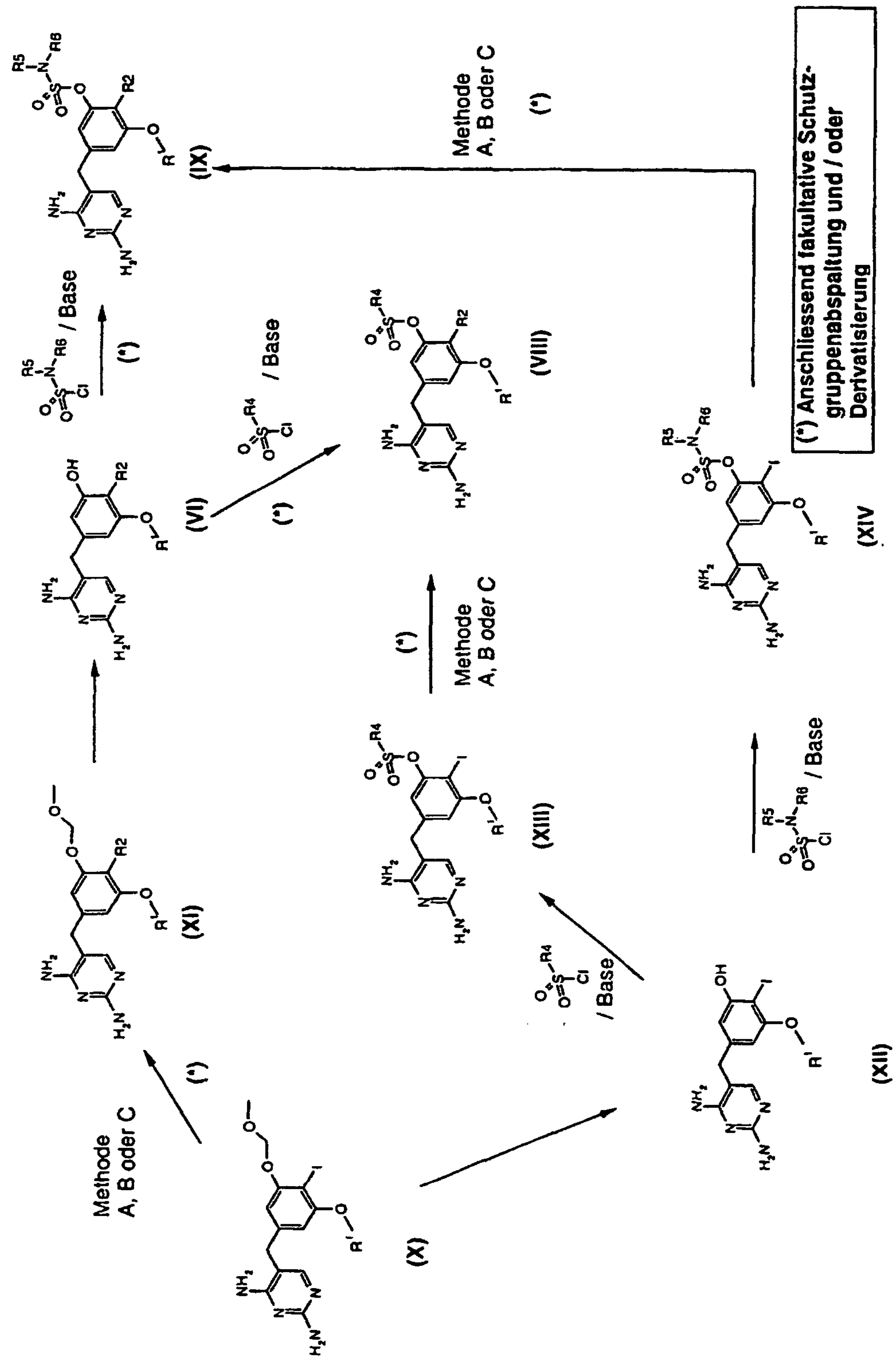


(X) (XI) (VI) (IX) (VIII) (XII) (XIII) (XIV)

In den Reaktionsschemata 1 und 2 bedeuten:

$Y^1$: eine Abgangsgruppe, z.B. Brom, Iod, Methylsulfonyloxy, Trifluormethylsulfonyloxy, Phenylsulfonyloxy oder p-Tosylsulfonyloxy;

$R^4$: C1-C6 Alkyl, C3-C6 Cycloalkyl; C3-C6 Cycloalkylalkyl oder Heterocyclyl-C1-C6 Alkyl;

| | |
|---|---|
| $R^5$, $R^6$: | C1-C6 Alkyl oder mit dem benachbarten Stickstoffatom einen gesättigten, 5- oder 6-gliedrigen N-Heterocyclus. |
| $R^1$, $R^2$ und $R^3$ | haben die obige Bedeutung. |

I → III; II → III; IV → V; X → XI; XIII → VIII; XIV → IX

Die Ausgangsverbindung II mit R' = Ethyl ist bekannt (Proc. Meet., 1980, 177-189), die Ausgangsverbindungen I, IV und X sind Analoga hiervon, welche gemäss Beispiel 1, 6 und 25 hergestellt werden können. Die Reaktionsbedingungen sind dieselben wie sie oben für die Verfahrensalternative a) angegeben sind.

V → VI

Die Abspaltung der Benzylschutzgruppe in Verbindungen V erfolgt katalytisch mit Wasserstoff und Palladiumkohle in einem niederen Alkanol, wie Ethanol, und einer niederen Alkancarbonsäure, wie konzentrierter Essigsäure, bei etwa 0-50°C, vorzugsweise bei Raumtemperatur.

X → XII; XI → VI

Die Abspaltung der Methoxymethylschutzgruppe in Verbindungen X erfolgt durch saure Hydrolyse in wässriger Mineralsäure, z.B. wässriger Salzsäure, bei Raumtemperatur bis zum Siedepunkt des Reaktionsgemisches.

VI → VII; VI → IX; VI → VIII; XII → XIII

Diese Kondensationsreaktionen werden in der gleichen Weise, wie oben für die Verfahrensalternative b) angegeben, durchgeführt.

Wie bereits erwähnt besitzen die Verbindungen der Formel A und ihre pharmazeutisch annehmbaren Salze wertvolle antibakterielle Eigenschaften. Sie sind gegen eine Vielzahl von pathogenen Mikroorganismen wirksam, wie z.B. Staphylococcus aureus, Streptococcus pneumoniae einschliesslich resistente Stämme, und verdanken ihre Wirkung der Hemmung der bakteriellen Dihydrofolat-Reduktase (DHFR).

Die Hemmung dieses Enzyms wurde als Mass für die antibakterielle Wirkung genommen. Sie wird mit der Method von BaCanari und Joyner (Biochemistry 20, 1710 (1981)) bestimmt; vgl. auch P.G. Hartman et al., FEB 242, 157-160 (1988).

Die $IC_{50}$-Werte (Konzentration, bei der das Enzym zu 50% gehemmt wird) werden graphisch ermittelt.

Die nachfolgenden Tabellen 1 und 2 enthalten für repräsentative Vertreter der durch die Formel A definierten Verbindungsklasse die im obigen Versuch ermittelten Hemmkonzentrationen. Angegeben sind:

| | |
|---|---|
| Spalte 1: | MIC SP1/1; µg/ml (Streptococcus pneumoniae 1/1, Trimethoprim- und Penicillin-resistent, Serotyp 6; klinisches Isolat, bei -80°C gelagert). Lit.: H. Locher et al., Can. J. Infect. Dis. 6: Suppl. C, p 469C. |
| Spalte 2: | MIC Sa101; µg/ml (Staphylococcus aureus 101, MRSA*)- und Trimethoprim-resistent; klinisches Isolat, bei -80°C gelagert). Lit.: A. Burdeska et al., FEBS 266: 159-162, 1999; G. Dale et al., J. Mol. Biol. 266: 23-30, 1997. |
| Spalte 3: | DHFR SP1/1; µM) - die $IC_{50}$-Werte in µM gegen die gereinigte DHFR des obigen Stammes Sp1/1 von StreptocoCus pneumoniae. |
| Spalte 4: | DHFR Sa 1; µM - die $IC_{50}$-Werte in µM gegen die gereinigte DHFR des Stammes 157/4696 (Trimethoprim-hochresistent; klinisches Isolat) von Staphylococcus aureus. Lit.: A. Burdeska et al., FEBS 266: 159-162, 1999; G. Dale et al., J. Mol. Biol. 266: 23-30, 1997. |
| *) MRSA = | Methicillin Resistant Staphylococcus Aureus |

Tabelle 1

| Aktivität der bevorzugten Verbindungen | | | | |
|---|---|---|---|---|
| Beispiel No. | MIC Sp1/1 µg/ml | MIC Sa101 µg/ml | DHFR Sp1/1 µM | DHFR Sa1 µM |
| 35e | 0,5 | 1 | 0,0046 | 0,062 |
| 40c | 0,5 | 1 | 0,019 | 0,037 |

Tabelle 1 (fortgesetzt)

| Aktivität der bevorzugten Verbindungen | | | | |
|---|---|---|---|---|
| Beispiel No. | MIC Sp1/1 µg/ml | MIC Sa101 µg/ml | DHFR Sp1/1 µM | DHFR Sa1 µM |
| 26c | 0,5 | 1 | 0,0032 | 0,056 |
| 31a | 1 | 1 | 0,0019 | 0,11 |
| 35a | 0,5 | 2 | 0,0037 | 0,11 |
| 18 | 1 | 2 | 0,0056 | 0,039 |
| 14g | 1 | 1 | 0,0037 | 0,03 |
| 22e | 1 | 2 | 0,013 | 0,15 |
| 14c | 1 | 1 | 0,02 | 0,061 |
| 33e | 1 | 1 | 0,019 | 0,2 |
| 7a | 1 | 8 | 0,0021 | 0,51 |
| 2a | 4 | 8 | 0.03 | 0,75 |
| Trimethoprim | >32 | 32 | 3,1 | 19 |
| Epiroprim | 4 | 16 | 0,19 | 2 |

Tabelle 2

| Beispiel | MIC Sp1/1 µg/ml | MIC Sa101 µg/ml | DHFR Sp1/1 µM | DHFR Sa1 µM |
|---|---|---|---|---|
| 02-aa | 4 | 8 | 0,06 | 2,5 |
| 02-ab | 2 | 4 | 0,038 | 0,88 |
| 02-ac | 2 | 8 | 0,065 | 0,65 |
| 02-ad | 4 | 8 | 0,085 | 2 |
| 02-ae | 8 | 4 | 0,15 | 1,6 |
| 02-af | 8 | 8 | 0,085 | 1,8 |
| 02-ag | 8 | 8 | 0,13 | 2,2 |
| 02-d | 1 | 8 | 0,035 | 1,4 |
| 02-e | 2 | 4 | 0,022 | 1 |
| 02-f | 4 | 4 | 0,028 | 1,7 |
| 02-g | 2 | 8 | 0,012 | 0,65 |
| 02-h | 4 | 8 | 0,038 | 1,2 |
| 02-i | 2 | 8 | 0,1 | 1,6 |
| 02-j | 4 | 8 | 0,072 | 1,7 |
| 02-1 | 4 | 8 | 0,16 | 8 |
| 02-m | 4 | 8 | 0,058 | 0,7 |
| 02-n | 4 | 8 | 0,11 | 1,4 |
| 02-o | 4 | 4 | 0,1 | 2,2 |
| 02-p | 4 | 8 | 0,1 | 2,2 |
| 02-q | 8 | 4 | 0,079 | 1,1 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 02-r | 4 | 4 | 0,07 | 2,5 |
| 02-s | 8 | 4 | 0,083 | 2,2 |
| 02-t | 4 | 8 | 0,081 | 1,1 |
| 02-u | 4 | 8 | 0,18 | 4 |
| 02-v | 4 | 8 | 0,11 | 10 |
| 02-w | 4 | 8 | 0,28 | 2,2 |
| 02-x | 2 | 8 | 0,075 | 2,3 |
| 02-y | 4 | 8 | 0,15 | 0,8 |
| 02-z | 8 | 8 | 0,06 | 1,6 |
| 03-a | 2 | 8 | 0,031 | 1,8 |
| 03-b | 2 | 8 | 0,019 | 1,6 |
| 03-c | 2 | 8 | 0,011 | 2 |
| 03-d | 8 | 8 | 0,34 | 6 |
| 03-e | 4 | 8 | 0,08 | 2,1 |
| 04-a | 4 | 8 | 0,082 | 2,7 |
| 04-aa | 8 | 8 | 0,33 | 1,8 |
| 04-ab | 4 | 8 | 0,12 | 1,4 |
| 04-ac | 4 | 8 | 0,059 | 0,45 |
| 04-ad | 4 | 8 | 0,17 | 2,7 |
| 04-ae | 8 | 8 | 0,019 | 1,4 |
| 04-ag | 8 | 8 | 0,35 | 1,8 |
| 04-ah | 8 | 8 | 0,18 | 1,8 |
| 04-b | 2 | 8 | 0,036 | 1,7 |
| 04-c | 4 | 8 | 0,04 | 0,7 |
| 04-d | 4 | 8 | 0,065 | 2,1 |
| 04-e | 4 | 8 | 0,03 | 1,8 |
| 04-f | 2 | 4 | 0,055 | 1,5 |
| 04-g | 4 | 8 | 0,04 | 1,1 |
| 04-h | 2 | 4 | 0.042 | 1 |
| 04-i | 4 | 8 | 0,043 | 1,2 |
| 04-j | 4 | 8 | 0,048 | 0,85 |
| 04-k | 4 | 8 | 0,095 | 10 |
| 04-l | 4 | 8 | 0,082 | 1,9 |
| 04-m | 8 | 8 | 0,072 | 1,8 |
| 04-n | 2 | 8 | 0,068 | 1,4 |
| 04-o | 8 | 8 | 0,06 | 1,3 |
| 04-p | 2 | 8 | | |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 04-q | 4 | 8 | 0,069 | 1,9 |
| 04-r | 4 | 8 | 0,085 | 3,2 |
| 04-s | 4 | 8 | 0,061 | 1,9 |
| 04-t | 2 | 8 | 0,031 | 1,2 |
| 04-u | 4 | 8 | 0,065 | 1,9 |
| 04-v | 4 | 8 | 0,15 | 1,3 |
| 04-w | 8 | 8 | 0,082 | 1,2 |
| 04-x | 8 | 8 | 0,08 | 7,9 |
| 04-y | 8 | 8 | 0,092 | 0,89 |
| 04-z | 4 | 8 | 0,058 | 3,8 |
| 05-a | 2 | 8 | 0,046 | 6,1 |
| 05-b | 4 | 16 | 0,04 | 1,5 |
| 05-c | 4 | 8 | 0,03 | 2,6 |
| 05-d | 2 | 4 | 0,07 | 3,1 |
| 07-b | 2 | 8 | 0,036 | 0,95 |
| 07-c | 4 | 8 | 0,048 | 2,2 |
| 07-d | 1 | 4 | 0,0082 | 0,68 |
| 07-e | 1 | 4 | 0,028 | 0,64 |
| 07-f | 2 | 8 | 0,055 | 0,75 |
| 07-g | 8 | 8 | 0,03 | 0,65 |
| 07-h | 2 | 8 | 0,027 | 2,2 |
| 07-i | 4 | 4 | 0,028 | 0,9 |
| 07-j | 1 | 8 | 0,029 | 1,3 |
| 08-a | 2 | 8 | 0,018 | 1,7 |
| 08-b | 2 | 8 | 0,038 | 0,95 |
| 08-c | 4 | 8 | 0,024 | 1,6 |
| 08-d | 4 | 8 | 0,016 | 0,62 |
| 08-e | 1 | 8 | 0,0056 | 0,95 |
| 08-f | 4 | 8 | 0,049 | 0,95 |
| 08-g | 4 | 8 | 0,052 | 1,3 |
| 08-h | 2 | 4 | 0,011 | 1,2 |
| 08-j | 4 | 2 | 0,04 | 1 |
| 08-j | 2 | 2 | 0,026 | 1,6 |
| 08-k | 2 | 4 | 0,043 | 2,1 |
| 08-l | 8 | 4 | 0,058 | 2,2 |
| 08-m | 4 | 8 | 0,065 | 0,86 |
| 08-n | 2 | 4 | 0,015 | 1,2 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 08-o | 2 | 4 | 0,051 | 1,4 |
| 08-p | 4 | 8 | 0,02 | 0,8 |
| 08-q | 8 | 8 | 0,028 | 1,4 |
| 08-r | 8 | 8 | 0,037 | 1,2 |
| 08-s | 2 | 4 | 0,031 | 3,7 |
| 08-t | 2 | 8 | 0,025 | 2,4 |
| 08-u | 4 | 8 | 0,15 | 1,8 |
| 08-v | 2 | 8 | 0,082 | 1,5 |
| 08-w | 4 | 8 | 0,021 | 0,92 |
| 08-x | 4 | 8 | 0,025 | 1,4 |
| 08-y | 4 | 8 | 0,095 | 2,7 |
| 09-a | 4 | 8 | 0,09 | 3,1 |
| 09-b | 2 | 8 | | 1,9 |
| 09-c | 1 | 4 | 0,016 | 0,9 |
| 09-d | 2 | 4 | 0,03 | 1,5 |
| 09-e | 4 | 4 | 0,045 | 1 |
| 09-f | 8 | 8 | 0,14 | 1,9 |
| 09-g | 4 | 4 | 0,05 | 1,6 |
| 10-a | 8 | 8 | 0,034 | 1,4 |
| 10-b | 2 | 8 | 0,04 | 2,4 |
| 10-c | 4 | 8 | 0,011 | 5 |
| 10-d | 8 | 4 | 0,042 | 1,7 |
| 10-e | 8 | 8 | 0,05 | 1,8 |
| 10-f | 8 | 8 | 0,085 | 1,8 |
| 11-a | 1 | 8 | 0,018 | 1,8 |
| 11-b | 1 | 8 | 0,0098 | 0,9 |
| 12- | 2 | 8 | 0,014 | 1,8 |
| 13- | 2 | 8 | 0,016 | 1,4 |
| 14-a | 4 | 8 | 0,023 | 0,35 |
| 14-b | 2 | 2 | 0,019 | 0,14 |
| 14-d | 1 | 2 | 0,016 | 0,082 |
| 14-e | 1 | 8 | 0,012 | 0,41 |
| 14-f | 1 | 4 | 0,0018 | 0,32 |
| 14-h | 0,5 | 2 | 0,0048 | 0,32 |
| 14-i | 0,5 | 8 | 0,0054 | 0,22 |
| 15-a | 4 | 8 | 0,032 | 0,55 |
| 15-b | 2 | 4 | 0,023 | 0,18 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 15-c | 1 | 1 | 0,012 | 0,064 |
| 15-d | 1 | 2 | 0,018 | 0,062 |
| 15-e | 1 | 8 | 0,012 | 0,094 |
| 15-f | 0,5 | 4 | 0,0067 | 0,38 |
| 15-g | 0,5 | 2 | 0,025 | 0,039 |
| 15-h | 0,25 | 4 | 0,0041 | 0,92 |
| 15-i | 4 | 2 | 0,022 | 0,081 |
| 15-j | 1 | 8 | 0,0017 | 0,55 |
| 16-a | 2 | 4 | 0,021 | 0,31 |
| 16-b | 2 | 2 | 0,039 | 0,1 |
| 16-c | 2 | 2 | 0,018 | 0,26 |
| 17-a | 2 | 4 | 0,038 | 0,6 |
| 17-b | 2 | 4 | 0,038 | 0,37 |
| 17-c | 2 | 2 | 0,033 | 0,18 |
| 17-d | 1 | 4 | 0,014 | 0,15 |
| 19-a | 1 | 1 | 0,0062 | 0,085 |
| 19-b | 0,5 | 1 | 0,0024 | 0,13 |
| 19-c | 1 | 2 | 0,019 | 0,066 |
| 19-d | 2 | 2 | 0,0083 | 0,055 |
| 19-e | 1 | 2 | 0,0039 | 0,038 |
| 19-f | 1 | 1 | 0,0062 | 0,082 |
| 19-g | 1 | 1 | 0,0068 | 0,056 |
| 19-h | 1 | 2 | 0,00055 | 0,16 |
| 20-a | 1 | 2 | 0,0075 | 0,15 |
| 20-b | 0,5 | 1 | 0,0018 | 0,14 |
| 20-c | 1 | 4 | 0,0081 | 0,18 |
| 20-d | 1 | 8 | 0,0058 | 0,095 |
| 20-e | 2 | 2 | 0,014 | 0,16 |
| 20-f | 1 | 1 | 0,0098 | 0,14 |
| 20-g | 1 | 2 | 0,0072 | 0,16 |
| 20-h | 1 | 2 | 0,0092 | 0,35 |
| 21- | 2 | 2 | 0,052 | 0,13 |
| 22-a | 1 | 2 | 0,0031 | 0,048 |
| 22-b | 1 | 1 | 0,0023 | 0,038 |
| 22-c | 1 | 1 | 0,0033 | 0,14 |
| 22-d | 1 | 2 | 0,0061 | 0,045 |
| 22-f | 1 | 2 | 0,014 | 0,15 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 23- | 1 | 8 | 0,008 | 2,6 |
| 24- | 1 | 4 | 0,016 | 0,19 |
| 26-a | 1 | 1 | 0,0021 | 0,13 |
| 26-b | 1 | 1 | 0,0019 | 0,17 |
| 26-d | 1 | 1 | 0,0051 | 0,14 |
| 26-e | 1 | 1 | 0,0023 | 0,13 |
| 26-f | 1 | 2 | 0,0088 | 0,23 |
| 27-a | 1 | 2 | 0,022 | 0,22 |
| 27-b | 1 | 2 | 0,014 | 0,19 |
| 27-c | 1 | 1 | 0,0026 | 0,18 |
| 28-a | 1 | 2 | 0,0058 | 0,32 |
| 28-b | 1 | 2 | 0,0068 | 0,32 |
| 28-c | 1 | 2 | 0,018 | 0,24 |
| 29-a | 2 | 1 | 0,0063 | 0,22 |
| 29-b | 2 | 2 | 0,014 | 0,095 |
| 29-c | 2 | 2 | 0,0052 | 0,17 |
| 30-a | 2 | 2 | 0,0045 | 0,088 |
| 30-b | 2 | 2 | 0,014 | 0,037 |
| 30-c | 2 | 2 | 0,016 | 0,14 |
| 31-b | 1 | 2 | 0,0044 | 0,13 |
| 32-a | 1 | 4 | 0,0046 | 0,12 |
| 32-b | 1 | 4 | 0,0042 | 0,16 |
| 32-c | 1 | 4 | 0,0061 | 0,13 |
| 32-d | 1 | 2 | 0,0059 | 0,14 |
| 32-e | 1 | 4 | 0,0068 | 0,28 |
| 32-f | 1 | 4 | 0,03 | 0,42 |
| 32-g | 4 | 8 | 0,075 | 0,068 |
| 33-a | 1 | 1 | 0,012 | 0,065 |
| 33-b | 1 | 2 | 0,014 | 0,26 |
| 33-c | 2 | 2 | 0,014 | 0,1 |
| 33-d | 2 | 2 | 0,0091 | 0,039 |
| 33-f | 8 | 2 | 0,089 | 0,042 |
| 33-g | 8 | 4 | 0,076 | 0,98 |
| 34-a | 1 | 2 | 0,0092 | 0,27 |
| 34-b | 1 | 2 | 0,011 | 0,21 |
| 34-c | 2 | 4 | 0,0034 | 0,19 |
| 34-d | 1 | 2 | 0,0024 | 0,14 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 34-e | 4 | 4 | 0,015 | 0,14 |
| 34-f | 1 | 2 | 0,0095 | 0,097 |
| 35-b | 0,5 | 2 | 0,009 | 0,13 |
| 35-c | 1 | 2 | 0,0046 | 0,043 |
| 35-d | 1 | 2 | 0,0043 | 0,095 |
| 35-f | 0,5 | 4 | 0,0085 | 0,12 |
| 36-a | 0,25 | 2 | 0,0033 | 0,032 |
| 36-b | 0,5 | 8 | 0,0058 | 0,23 |
| 36-c | 0,5 | 2 | 0,0048 | 0,24 |
| 36-d | 0,5 | 2 | 0,00068 | 0,066 |
| 36-e | 0,5 | 4 | 0,0021 | 0,25 |
| 37-a | 0,25 | 4 | 0,0045 | 0,55 |
| 37-b | 0,25 | 4 | 0,0017 | 0,26 |
| 37-c | 0,5 | 4 | 0,0018 | 0,58 |
| 37-d | 0,25 | 8 | 0,0015 | 0,29 |
| 38-a | 1 | 2 | 0,0052 | 0,16 |
| 38-b | 1 | 2 | 0,0065 | 0,22 |
| 38-c | 0,5 | 2 | 0,0058 | 0,19 |
| 38-d | 1 | 4 | 0,0032 | 0,24 |
| 39-a | 1 | 4 | 0,0042 | 0,39 |
| 39-b | 0,5 | 4 | 0,0049 | 0,25 |
| 39-c | 1 | 8 | 0,014 | 1 |
| 39-d | 2 | 8 | 0,0026 | 0,41 |
| 40-a | 1 | 2 | 0,0065 | 0,052 |
| 40-b | 1 | 2 | 0,027 | 0,31 |
| 40-d | 2 | 2 | 0,017 | 0,13 |
| 40-e | 2 | 2 | 0,079 | 0,16 |
| 41-a | 0,5 | 2 | 0,0003 | 0,055 |
| 41-b | 0,5 | 4 | 0,0062 | 0,25 |
| 41-c | 0,5 | 4 | 0,0042 | 0,043 |
| 42-a | 2 | 8 | 0,0057 | 0,25 |
| 42-b | 2 | 8 | 0,0076 | 0,51 |
| 42-c | 1 | 8 | 0,0063 | 0,3 |
| 43-a | 4 | 2 | 0,0067 | 0,32 |
| 43-b | 1 | 1 | 0,0098 | 0,26 |
| 43-c | 2 | 2 | 0,024 | 0,16 |
| 43-d | 1 | 1 | 0,042 | 0,18 |

Tabelle 2 (fortgesetzt)

| Beispiel | MIC Sp1/1 µg/ml | MIC Sa101 µg/ml | DHFR Sp1/1 µM | DHFR Sa1 µM |
|---|---|---|---|---|
| 44- | 2 | 8 | 0,018 | 0,68 |
| 45- | 4 | 8 | 0,13 | 2,4 |
| 46- | 2 | 8 | 0,055 | 0,75 |
| 47- | 2 | 8 | 0,01 | 1,2 |
| 48- | 2 | 4 | 0,03 | 0,75 |
| 49- | 8 | 4 | 0,046 | 0,82 |
| 50- | 4 | 8 | 0,032 | 10 |
| 51- | 2 | 8 | 0,043 | 8,5 |
| 52- | 1 | 8 | 0,02 | 5,8 |
| 53- | 4 | 8 | 0,028 | 1,8 |
| 54- | 2 | 8 | 0,019 | 3,1 |
| 55- | 4 | 8 | 0,048 | 1,7 |
| 56- | 2 | 8 | 0,032 | 0,8 |
| 57- | 4 | 8 | 0,03 | 3,6 |
| 58- | 2 | 2 | 0,032 | 0,055 |
| 59- | 2 | 8 | 0,018 | 1,8 |
| 60- | 8 | 4 | 0,088 | 0,28 |
| 61-a | 0,5 | 2 | 0,0018 | 0,55 |
| 61-b | 1 | 2 | 0,011 | 0,21 |
| 62- | 2 | 2 | 0,0079 | 0,33 |
| 63- | 1 | 4 | 0,0018 | 0,38 |
| 64-a | 4 | 8 | 0,052 | 1,5 |
| 64-b | 2 | 2 | 0,027 | 0,3 |
| 65- | 2 | 2 | 0,024 | 0,16 |
| 66- | 4 | 2 | 0,052 | 0,17 |
| Trimethoprim | >32 | 32 | 3,1 | 19 |
| Epiroprim | 4 | 16 | 0,19 | 2 |

Die erfindungsgemässen Produkte können als Heilmittel, z.B. in Form pharmazeutischer Präparate zur enteralen oder parenteralen Applikation, Verwendung finden. Die erfindungsgemässen Produkte können beispielsweise peroral, z.B. in Form von Tabletten, Lacktabletten, Dragees, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die erfindungsgemässen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als solche Trägermaterialien eignen sich sowohl anorganische als auch organische Trägermaterialien. So kann man für Tabletten, Lacktabletten, Dragees und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und Antioxydantien in Frage.

Für die parenterale Applikation werden die Verbindungen der Formel I bzw. ihre Salze vorzugsweise als Lyophilisat oder Trockenpulver zur Verdünnung mit üblichen Trägern, wie Wasser oder isotonischer Kochsalzlösung, zur Verfügung gestellt.

Wie bereits erwähnt, sind die Verbindungen der Formel I und ihre Salze antibakteriell wirksam. Sie hemmen die bakterielle Dihydrofolat-Reduktase und potenzieren die antibakterielle Wirkung von Sulfonamiden, wie z.B. Sulfisoxazol, Sulfadimethoxin, Sulfamethoxazol, 4-Sulfanilamido-5,6-dimethoxy-pyrimidin, 2-Sulfanilamido-4,5-dimethylpyrimidin oder Sulfachinoxalin, Sulfadiazin, Sulfamonomethoxin, 2-Sulfanilamido-4,5-dimethyl-isoxazol und anderen Inhibitoren für Enzyme, die an der Folsäurebiosynthese beteiligt sind, wie z.B. Pteridinderivate.

Für solche Kombinationen einer oder mehrerer der erfindungsgemässen Verbindungen A mit Sulfonamiden kommt in der Humanmedizin orale, rektale und parenterale Applikation in Frage. Das Verhältnis von Verbindung der Formel A zu Sulfonamid kann innerhalb eines weiten Bereiches variieren; es beträgt z.B. zwischen 1:40 (Gewichtsteile) und 1:1 (Gewichtsteile); bevorzugte Verhältnisse sind 1:10 bis 1:2.

So kann z.B. eine Tablette 80 mg einer erfindungsgemässen Verbindung der Formel A und 400 mg Sulfamethoxazol, eine Kindertablette 20 mg einer erfindungsgemässen Verbindung der Formel A und 100 mg Sulfamethoxazol; Sirup (pro 5 ml) 40 mg Verbindung der Formel A und 200 mg Sulfamethoxazol enthalten.

Für den Erwachsenen kommt eine Tagesdosis von etwa 0,2 g bis etwa 2 g einer erfindungsgemässen Verbindung der Formel A in Betracht.

Die Verbindungen der Formel A zeichnen sich durch eine hohe antibakterielle Wirksamkeit bzw. einen ausgeprägten synergistischen Effekt in Kombination mit Sulfonamiden und gute Verträglichkeit aus.

Die nachstehenden Beispiele erläutern die Erfindung. Die Temperaturen sind in Celsiusgraden angegeben.

Beispiel I

Herstellung von Verbindung (I), worin $R^1$ Ethyl ist (Schlüsselzwischenprodukt - Schema 1): 5-(3,5-Diethoxy-4-iod-benzyl)-pyrimidin-2,4-diamin

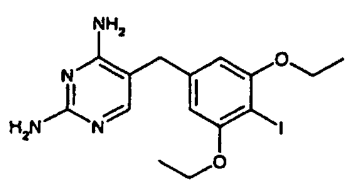

Diese Verbindung kann durch folgende Reaktionssequenz (Stufen a)-g)) erhalten werden:

Stufe a) 3,5-Dihydroxy-4-iod-benzoesäure:

3,5 Dihydroxybenzoesäure (500 g; 3,24 Mol) wird unter Rühren und Argonbegasung in Methanol (2000 ml) gelöst. Nach Abkühlen auf 1°C wird bei 1-6°C innerhalb 90 Minuten eine Lösung von N-Jodsuccinimid (730 g; 3,24 Mol) in Methanol (2000 ml) zugetropft. Nach einer Stunde wird die Reaktionslösung auf Eiswasser (1500 ml) gegossen und mit einer 5 %igen Natriumthiosulfatlösung in Wasser (500 ml) bis zur Entfärbung versetzt. Das Gemisch wird mit tert-Butylmethylether (4050 ml) extrahiert. Anschliessend wird die organische Phase zweimal mit gesättigter wässriger Kochsalzlösung (2000 ml) gewaschen und die wässrigen Phasen mit tert-Butylmethylether (2 x 3240 ml) nachextrahiert. Die vereinigten organischen Phasen werden mit Natriumsulfat (160 g) getrocknet, filtriert und eingeengt. Der Rückstand wird aus Wasser kristallisiert. Ausbeute : 816 g (90 %) als hellbeige Kristalle. Smp. 245-248°C
MS: 280 (M)

Stufe b) 3,5-Dihydroxy-4-iod-benzoesäure-methyl ester:

3,5-Dihydroxy-4-iod-benzoesäure (810 g; 2,89 Mol) wird unter Rühren und Argonbegasung in Methanol (3800 ml) gelöst, vorsichtig mit Schwefelsäure (57,5 ml) versetzt und auf 60°C erwärmt. Nach 3 Stunden bei dieser Temperatur wird erneut Schwefelsäure (12 ml) zugegeben und 45 Minuten weitergerührt. Nach Entfernen des Heizbades wird Wasser (5000 ml) vorsichtig zugegeben und auf 0°C gekühlt. Die ausgefallenen Kristalle werden anschliessend abgenutscht, mit Eiswasser (2 x 500 ml) gewaschen und am Hochvakuum getrocknet. Ausbeute: 772 g (90 %) als weisse Kristalle.
Smp. 222-223°C
MS: 294 (M); 263 (M-$OCH_3$)

Stufe c) 3,5-Diethoxy-4-iod-benzoesäure-methyl ester:

3,5-Dihydroxy-4-iod-benzoesäure-methyl ester (283 g; 1,30 Mol) wird in Aceton (6170 ml) gelöst und mit Kaliumcarbonat (362 g; 2,62 Mol) sowie Diethylsulfat (402 g; 2,61 Mol) versetzt. Anschliessend wird 3 Stunden unter Rückfluss gerührt (nach 2,5 Stunden wird mit Aceton (1000 ml) verdünnt). Nach 3 Stunden wird erneut Diethylsulfat (20,4 g; 0,13 Mol) zugegeben. Nach insgesamt 5,5 Stunden wird das Reaktionsgemisch heiss filtriert. Der gallertartige Rückstand wird mit Aceton (40°C, 2 x 1000 ml und 1500 ml) gewaschen. Die vereinigten Aceton-Phasen werden bis auf 1000 ml eingeengt und bei 0°C über Nacht stehengelassen. Die ausgefallenen Kristalle werden abgenutscht, mit Aceton (-20°C, 250 ml) gewaschen und getrocknet (Hochvakuum). Ausbeute: 437 g (96 %) als weisse Kristalle.
MS: 350 (M)

Stufe d) (3,5-Diethoxy-4-iod-phenyl)-methanol:

3,5-Diethoxy-4-iod-benzoesäure-methyl ester (403 g; 1,15 Mol) wird bei 35°C in Tetrahydrofuran (2300 ml) gelöst und anschliessend auf +8°C abgekühlt. Di-isobutylaluminiumhydrid 1,2 M in Toluol (2400 ml; 2,85 Mol) wird innerhalb 190 Minuten unter Eisbadkühlung zwischen 9 und 11°C zugetropft. Nach 30 Minuten bei +10°C wird das Reaktionsgemisch auf Eis (2820 g) gegossen und unter starkem Rühren mit wässriger 3N Salsäure (4030 ml) versetzt. Anschliessend wird Toluol (2820 ml) zugegeben und heftig gerührt. Nach Abtrennung wird die organische Phase nacheinander mit 0.3 N wässriger Salzsäure (4030 ml) und wässriger Kochsalzlösung (¼-gesättigt, 4030 ml) gewaschen. Die wässrigen Phasen werden mit Toluol (2015 ml) nachextrahiert. Die vereinigten organischen Phasen werden anschliessend über Natriumsulfat (400 g) getrocknet, abgenutscht und eingeengt.
Ausbeute: 396 g einer weissen festen Masse, die ohne weitere Reinigung in die nächste Stufe eingesetzt wird.
MS: 322 (M)

Stufe e) 3, 5-Diethoxy-4-iod-benzaldehyd:

(3,5-Diethoxy-4-iod-phenyl)-methanol (393 g; maximal 1,15 Mol) wird unter Rühren und Argonbegasung in Methylenchlorid (3930 ml) gelöst und mit Mangandioxid (593 g; 6,9 Mol) versetzt. Nach 15 Stunden unter Rückfluss wird die warme Lösung über ein 2,5 l Druckfilter filtriert und mit Methylenchlorid (1000 ml; 35°C) nachgewaschen. Die vereinigten organischen Phasen werden eingeengt und mit n-Hexan (1650 ml) 2 Stunden uner Rückflussbedingungen erhitzt, auf 0°C abgekühlt, abgenutscht und am Hochvakuum getrocknet.
Ausbeute: 324 g schwach gelbliche Kristalle (88 % über 2 Stufen).
MS: 320 (M)

Stufe f) 2-(3,5-Diethoxy-4-iod-benzyl)-3-phenylamino-acrylnitril

Kalium-tert-butylat (120,7 g; 1,07 Mol) wird unter Argon und Rühren in tert-Butanol (790 ml) heiss gelöst. Die Lösung wird dann auf 40°C abgekühlt und innerhalb 25 Minuten zu einer Lösung bestehend aus 3,5-Diethoxy-4-iod-benzaldehyd (286 g; 0,893 Mol) und 3-Anilinopropionitril (158 g; 1,08 Mol) in Dimethylsulfoxid (490 ml) bei 38-40°C (20 Minuten) zugetropft. Anschliessend wird das Reaktionsgemisch 2 Stunden auf 58-60°C erwärmt. Nach dieser Zeit werden 980 ml Lösungsmittel abdestilliert. Die warme Lösung wird mit Wasser (2650 ml) verdünnt, auf Raumtemperatur gekühlt, abfiltriert, mit Wasser (2 x 1000 ml) gewaschen und getrocknet. Die hellgelbe, kristalline Masse (369,5 g) wird mit Diisopropylether (2200 ml) bei 50°C verrührt, abfiltriert, mit Diisopropylether (2 x 500 ml) gewaschen und am Hochvakuum getrocknet.
Ausbeute: 323 g schwach beige Kristalle (67 %)
MS (ISP): 449,2 $(M+H)^+$; 466,1 $(M+NH_4)^+$; 471,0 $(M+Na)^+$

Stufe g) 5-(3,5-Diethoxy-4-iod-benzyl)-pyrimidin-2,4-diamin

Guanidin-HCl (192,1 g; 2,01 Mol) wird in Ethanol (2420 ml) gelöst und mit Kalium-tert-butylat 245,6 g; 2,19 Mol) versetzt. Die Temperatur des Reaktionsgemisches wird durch leichte Kühlung eine Stunde unterhalb 38°C gehalten. Anschliessend wird 2-(3,5-Diethoxy-4-iod-benzyl)-3-phenylamino-acrylnitril (270 g; 0,60 Mol) zugegeben und das Reaktionsgemisch 20 Stunden auf 67-69°C erwärmt. Die warme Reaktionslösung wird dann mit Wasser (1000 ml) verdünnt, auf 3°C abgekühlt und abfiltriert. Die Kristalle werden nacheinander mit Ethanol (2 x 500 ml, -20°C), Wasser (2 x 500 ml), Ethanol (500 ml, -20°C) und n-Pentan (500 ml) gewaschen und am Hochvakuum getrocknet.
Ausbeute: 228 g hellbeige Kristalle (92 %)
MS (ISP): 415,1 $(M+H)^+$

Beispiel 2

Herstellung von Verbindungen der Formel III Schema 1 nach Methode A (Suzuki-Kopplung mit $R^2$-$B(OH)_2$)

(2a) 5-(3'-Amino-2,6-diethoxy-biphenyl-4-ylmethyl)-pyrimidin-2,4-diamin

Tetrakis-triphenylphosphin-palladium (555 mg; 0,48 mmol) wird unter Argonbegasung in Dimethoxyethan (7 ml) suspendiert und mit 5-(3,5-Diethoxy-4-iodbenzyl)-pyrimidin-2,4-diamin (Beispiel 1) (4,14 g; 10 mmol) in Dimethoxyethan (60 ml) versetzt. Nach 15-minutigem Rühren bei Raumtemperatur wird 3-Amino-phenylboronsäure-Monohydrat (2,37 g; 15 mmol) in Ethanol (17 ml) zugegeben, weitere 10 Minuten bei Raumtemperatur gerührt, wässrige 2M Natriumcarbonat-Lösung (44 ml) hinzugefügt, und anschliessend 4 Stunden am Rückfluss gekocht. Das Reaktionsgemisch wird eingeengt, der Rückstand mit Wasser verrührt und abgenutscht. Der Nutschkuchen wird mit MeOH (230 ml) verrührt und abgenutscht. Das Filtrat wird eingeengt und über Kieselgel (225 g) mit Methylenchlorid / Methanol / Ammoniak (19/1/0.05, später 90/10/1) chromatographiert. Die reinen Fraktionen werden vereinigt, eingeengt und am Hochvakuum getrocknet.
Ausbeute: 3,2 g (85 %) 5-(3'-Amino-2,6-diethoxy-biphenyl-4-ylmethyl)-pyrimidin-2,4-diamin als farbloses Pulver.
MS (ISP): 380,3 $(M+H)^+$
NMR $^1H$: (250 MHz, $\delta$, TMS, DMSO): 1.13 (t; J=6.9; 6H); 3.57 (s; 2H); 3.87 (q; J=6.9; 4H); 4.86 (s; 2H); 5.71 (s; 2H); 6.12 (s; 2H); 6.3-6.4 (m; 3H); 6.56 (s; 2H); 6.9 (m; 1H); 7.56 (s; 1H).

In Analogie hierzu werden hergestellt:

(2b) 5-(2,6-diethoxy-biphenyl-4-ylmethyl)-pyrimidin-2,4-diamin

Ausgehend von 5-(3,5-Diethoxy-4-iod-benzyl)-pyrimidin-2,4-diamin (Beispiel 1) (414 mg; 1 mmol) und Phenylboronsäure (488 mg; 4 mmol) werden 264 mg (72 %) 5-(2,6-diethoxy-biphenyl-4-ylmethyl)-pyrimidin-2,4-diamin als farbloses Pulver erhalten.
Smp: 187-188°C
MS (ISP): 365,2 $(M+H)^+$

(2c) 5-(3,5-Diethoxy-4-naphthalen-2-yl-benzyl)-pyrimidin-2,4-diamin

Ausgehend von 5-(3,5-Diethoxy-4-iod-benzyl)-pyrimidin-2,4-diamin (Beispiel 1) (414 mg; 1 mmol) und Naphthalen-2-yl-boronsäure (690 mg; 4 mmol) werden 327 mg (79 %) 5-(2,6-Diethoxy-biphenyl-4-ylmethyl)-pyrimidin-2,4-diamin als farbloses Pulver erhalten.
Smp: 189-191°C
MS (ISP): 365,2 $(M+H)^+$

(2d) 5-(3,5-Diethoxy-4-(1H-indol-5-yl)-benzyl)-pyrimidin-2,4-diamin

Ausgehend von 5-(3,5-Diethoxy-4-iod-benzyl)-pyrimidin-2,4-diamin (Beispiel 1) (207 mg; 0,5 mmol) und 5-(1H-Indolyl)-boronsäure (161 mg; 1 mmol) werden 103 mg (51 %) 5-(3,5-Diethoxy-4-(1H-indol-5-yl)-benzyl)-pyrimidin-2,4-diamin als farbloses Pulver erhalten.
Smp: 103-105 °C
MS (ISP): 404,4 $(M+H)^+$

(2e) 5-[3,5-Diethoxy-4-(1H-indol-6-yl)-benzyl]-pyrimidin-2,4-diamin

Ausgehend von 5-(3,5-Diethoxy-4-iod-benzyl)-pyrimidin-2,4-diamin (Beispiel 1) (207 mg; 0,5 mmol) und 6-(1H-Indolyl)-boronsäure (161 mg; 1 mmol) werden 120 mg (60 %) 5-(3,5-Diethoxy-4-(1H-indol-6-yl)-benzyl)-pyrimidin-2,4-diamin als farbloses Pulver erhalten.
Smp: 176-178 °C
MS (ISP): 404,4 $(M+H)^+$

(2f) 6-[4-(2,4-Diamino-pyrimidin-5-ylmethyl)-2,6-diethoxy-phenyl]-naphthalen-2-ol

Ausgehend von 5-(3,5-Diethoxy-4-iod-benzyl)-pyrimidin-2,4-diamin (Beispiel 1) (828 mg; 2,0 mmol) und 6-Hydroxy-2-naphthalen-boronsäure (2,42 g; 8,0 mmol) werden 732 mg (85 %) 6-[4-(2,4-Diamino-pyrimidin-5-ylmethyl)-2,6-diethoxy-phenyl]-naphthalen-2-ol als beiges Pulver erhalten.
Smp: 250-251 °C
MS (ISP): 431,3 $(M+H)^+$

(2g) 5-[3,5-Diethoxy-4-(1H-indazol-5-yl)-benzyl]-pyrimidin-2,4-diamin

Ausgehend von 5-(3,5-Diethoxy-4-iod-benzyl)-pyrimidin-2,4-diamin (Beispiel 1) (230 mg; 0,55 mmol) und 5-(1H-Indazol)-boronsäure (89 mg; 0,55 mmol) werden 57 mg (26 %) 5-[3,5-Diethoxy-4-(1H-indazol-5-yl)-benzyl]-pyrimidin-2,4-diamin als farbloses Pulver erhalten.
Smp: 174 °C
MS (ISP): 405,3 $(M+H)^+$

Die in Beispiel 2g) eingesetzte 5-(1H-Indazol)-boronsäure wird wie folgt aus 5-Bromindazol hergestellt:

5-Bromindazol (638 mg; 3,24 mmol) wird in Diethylether (10 ml) vorgelegt und bei -20°C (Kühlbad) mit einer 1,6 M n-Butyllithium Lösung in n-Hexan (4,05 ml; 6,48 mmol) innerhalb 15 Minuten versetzt. Das Kühlbad wird entfernt, das Reaktionsgemisch eine Stunde bei Raumtemperatur nachgerührt, anschliessend mit Diethylether (10 ml) verdünnt und 90 Minuten bei Raumtemperatur und 15 Minuten bei 30°C weitergerührt. Das Reaktionsgemisch wird dann auf eine Lösung bestehend aus Trimethylborat (0,36 ml; 3,24 mmol) in Diethylether (5 ml) bei -70°C gegossen. Man lässt das Reaktionsgemisch auf Raumtemperatur erwärmen und rührt 3 Stunden bei Rautemperatur nach. Anschliessend wird das Reaktionsgemisch mit Diethylether (20 ml) versetzt und 12 Stunden bei Raumtemperatur weitergerührt. Danach wird das Reaktionsgemisch auf wässrige 1 N Salzsäure gegossen , mit Diethylether extrahiert, über Magnesiumsulfat getrocknet, abgenutscht und eingengt. Der Rückstand wird über Kieselgel (100 g) mit Methylenchlorid / Methanol (97,5/2,5 bis 90/10) chromatographiert.
Ausbeute: 100 mg (19 %) 5-(1H-Indazol)-boronsäure als beiges Pulver.
MS (ISN): 161,3$(M-H)^-$

(2h) 5-[4-(3,4-Dihydro-2H-benzo[1,4]oxazin-6-yl)-3,5-diethoxy-benzyl]-pyrimidin-2,4-diamin

Ausgehend von 5-(3,5-Diethoxy-4-iod-benzyl)-pyrimidin-2,4-diamin (Beispiel 1) (414 mg; 1 mmol) und 2H-1,4-Benzoxazin-3,4-dihydro-6-boronsäure (179 mg; 1 mmol) werden 251 mg (60 %) 5-[4-(3,4-Dihydro-2H-benzo[1,4] oxazin-6-yl)-3,5-diethoxybenzyl]-pyrimidin-2,4-diamin als braune Kristalle erhalten.
MS (ISP): 422.3$(M+H)^+$

Die in Beispiel (2h) eingesetzte 2H-1,4-Benzoxazin-3,4-dihydro-6-boronsäure wird wie folgt aus 2H-3,4-dihydro-6-brom-1,4-benzoxazin hergestellt:

2H-3,4-dihydro-6-brom-1,4-benzoxazin (600 mg; 2.8 mmol) wird in absolutem Tetrahydrofuran (30 ml) gelöst und mit Natriumhydrid (202 mg; 55%; 4.2 mmol) bei 0°C versetzt. Nach einstündigem Rühren bei dieser Temperatur, wird tert-Butyl-dimethyl-chlorsilan (465 mg; 3.08 mmol) zugegeben und das Ganze eine halbe Stunde bei Raumtemperatur weitergerührt. Das Reaktionsgemisch wird dann auf -78°C gekühlt, langsam mit tert-Butyllithium (5.61 ml einer 1.5 M Lösung; 5.6 mmol) versetzt und eine Viertelstunde bei -78°C (Kühlbad) weitergerührt. Anschliessend wird Trimethoxyborat (0.625 ml; 5.6 mmol) zugegeben und das Kühlbad entfernt. Sobald das Reaktionsgemisch Raumtemperatur erreicht hat, wird wässrige Salzsäure (10 ml einer 2M Lösung; 20 mmol) zugegeben und das Ganze eine Stunde bei Raumtemperatur weitergerührt. Das Reaktionsgemisch wird dann auf Eiswasser gegossen und mit Ethylacetat zweimal extrahiert. Die vereinigten organischen Phasen werden auf Natriumsulfat getrocknet, abgenutscht und das Lösungsmittel abgedampft. Der Rückstand wird aus einem 1/1 n-Hexan/Ethylacetat-Gemisch umkristallisiert.
Ausbeute: 163 mg (32 %) 2H-1,4-Benzoxazin-3,4-dihydro-6-boronsäure als braunes Pulver.
NMR (1H, 250 MHz in DMSO): ppm: 3,1 (breites singulet, 2H); 4,0 (breites singulet, 2H); 5,47 (breites singulet, 1H); 6,47 (d, 1H); 6,85 (d, 1H); 6,93 (s, 1H)

(2i) 5-(2,6-Diethoxy-4'-methansulfonyl-biphenyl-4-ylmethyl)-pyrimidin-2,4-diamin

5-(2,6-Diethoxy-4'-methylsulfanyl-biphenyl-4-ylmethyl)-pyrimidin-2,4-diamin (616 mg; 1,5 mmol) wird in Methylenchlorid (12 ml) suspendiert und mit Trifluoressigsäure (0,276 ml; 3,6 mmol) versetzt. Nach Abkühlung auf 0°C wird m-Chlorperbenzoesäure (456 mg; 2,25 mmol) zugegeben. Nach 40 Minuten wird eine 10%ige wässrige $Na_2S_2O_3$-lösung (15 ml) zugegeben und bei Raumtemperatur 15 Minuten gerührt. Anschliessend wird mit Methylenchlorid (60 ml) extrahiert, mit einer wässrigen gesättigten Natriumbicarbonatlösung gewaschen, über Magnesiumsulfat getrocknet, abgenutscht und eingeengt . Das Rohprodukt wird über Kieselgel (100 g) mit Methylenchlorid/Methanol/$NH_4OH$ (konz.) (19/1/0.05) chromatographiert.
Ausbeute: 172 mg (26 %) 5-(2,6-Diethoxy-4'-methansulfonyl-biphenyl-4-ylmethyl)-pyrimidin-2,4-diamin als farbloses Pulver.
MS: 442 (M)

Die obige Ausgangsverbindung 5-(2,6-Diethoxy-4'-methylsulfanyl-biphenyl-4-ylmethyl)-pyrimidin-2,4-diamin wird in Analogie zu Beispiel (2a), hergestellt:

Ausgehend von 5-(3,5-Diethoxy-4-iod-benzyl)-pyrimidin-2,4-diamin (Beispiel 1) (1,243 g; 3,0 mmol) und 4-(Methylthio)-phenylboronsäure (0,780 g; 4,64 mmol) werden nach Kristallisation aus Diethylether 1,09 g (89 %) 5-(2,6-Diethoxy-4'-methylsulfanylbiphenyl-4-ylmethyl)-pyrimidin-2,4-diamin als beiges Pulver erhalten.
MS: 410 (M)

(2j) (RS)-5-(2,6-Diethoxy-4'-methansulfinyl-biphenyl-4-ylmethyl)-pyrimidin-2,4-diamin

Wird gleich wie Beispiel (2i) hergestellt (Nebenprodukt der Reaktion). Ausgehend von 5-(2,6-Diethoxy-4'-methylsulfanyl-biphenyl-4-ylmethyl)-pyrimidin-2,4-diamin (616 mg; 1,5 mmol) werden 444 mg (67 %) (RS)-5-(2,6-Dicthoxy-4'methanesulfinyl-biphenyl-4-ylmethyl)-pyrimidin-2,4-diamin als farbloses Pulver erhalten.
MS: 426 (M); 411 ($M-CH_3$)

(2k) 5-(3,5-Diethoxy-4-thiophen-3-yl-benzyl)-pyrimidin-2,4-diamin

Ausgehend von 5-(3,5-Diethoxy-4-iod-benzyl)-pyrimidin-2,4-diamin (Beispiel 1) (414 mg; 1,0 mmol) und 3-Thiophen-borsäure (191 mg; 1,5 mmol), werden nach Umkristallisation aus Methanol, 146 mg (40 %) 5-(3,5-Diethoxy-4-thiophen-3-yl-benzyl)-pyrimidin-2,4-diamin als beiges Pulver erhalten.
MS (ISP): 371,2 $(M+H)^+$

(2l) 5-(2,6-Diethoxy-3',4'-dimethoxy-biphenyl-4-ylmethyl)-pyrimidin-2,4-diamin

Ausgehend von 5-(3,5-Diethoxy-4-iod-benzyl)-pyrimidin-2,4-diamin (Beispiel 1) (414 mg; 1,0 mmol) und 3,4-Dimethoxy-phenyl-borsäure (363 mg; 2,0 mmol) werden nach Umkristallisation aus Wasser und Methanol 328 mg (77 %) 5-(3,5-Diethoxy-4-thiophen-3-yl-benzyl)-pyrimidin-2,4-diamin als gelbliches Pulver erhalten.
MS (ISP): 425,3 $(M+H)^+$

(2m) 5-(3,5-Diethoxy-4-thiophen-2-yl-benzyl)-pyrimidin-2,4-diamin

Ausgehend von 5-(3,5-Diethoxy-4-iod-benzyl)-pyrimidin-2,4-diamin (Beispiel 1) (207 mg; 0,5 mmol) und 2-Thiophen-boronsäure (108 mg; 0,96 mmol) werden nach Kristallisation aus Methanol 110 mg (59 %) 5-(3,5-Diethoxy-4-thiophen-2-yl-benzyl)-pyrimidin-2,4-diamin als beiges Pulver erhalten.
Smp: 202-203 °C
MS (ISP): 371,1 $(M+H)^+$

(2n) 5-(2,6-Diethoxy-4'-methoxy-3'-methyl-biphenyl-4-ylmethyl)-pyrimidin-2,4-diamin

Ausgehend von 5-(3,5-Diethoxy-4-iod-benzyl)-pyrimidin-2,4-diamin (Beispiel 1) (207 mg; 0,5 mmol) und 4-Methoxy-3-methyl-phenyl-boronsäure (125 mg; 0,75 mmol) werden 172 mg (84 %) 5-(2,6-Diethoxy-4'-methoxy-3'-methyl-biphenyl-4-ylmethyl)-pyrimidin-2,4-diamin als farbloses Pulver erhalten.
MS (ISP): 409,3 $(M+H)^+$

(2o) 5-(3,5-Diethoxy-4-furan-2-yl-benzyl)-pyrimidin-2,4-diamin

Ausgehend von 5-(3,5-Diethoxy-4-iod-benzyl)-pyrimidin-2,4-diamin (Beispiel 1) (100 mg; 0,24 mmol) und

2-Furan-boronsäure (108 mg; 0,96 mmol) werden nach Kristallisation aus Methanol 54 mg (63 %) 5-(3,5-Diethoxy-4-furan-2-yl-benzyl)-pyrimidin-2,4-diamin als beiges Pulver erhalten.
Smp: 196-198 °C

(2p) 5-(3,5-Diethoxy-4-pyridin-3-yl-benzyl)-pyrimidin-2,4-diamin

Ausgehend von 5-(3,5-Diethoxy-4-iod-benzyl)-pyrimidin-2,4-diamin (Beispiel 1) (300 mg; 0,72 mmol) 3-Pyridin-boronsäure (221 mg; 1,8 mmol) und Tetrakis-triphenylphosphin-palladium (42 mg; 0,036 mmol) in Dimethylformamid (15 ml), Ethanol (3 ml) und einer 2M wässrigen Kaliumphosphat-Lösung (2 ml) werden nach Kristallisation aus Methanol 163 mg (62 %) 5-(3,5-Diethoxy-4-pyridin-3-yl-benzyl)-pyrimidin-2,4-diamin als farbloses Pulver erhalten.
MS (ISP): 366,3 $(M+H)^+$

(2q) 5-(2,6-Diethoxy-4'-methyl-biphenyl-4-ylmethyl)-pyrimidin-2,4-diamin

Ausgehend von 5-(3,5-Diethoxy-4-iod-benzyl)-pyrimidin-2,4-diamin (Beispiel 1) (300 mg; 0.72 mmol) und 4-Methyl-phenylboronsäure (147 mg; 1.08 mmol) werden 193mg (71 %) 5-(2,6-Diethoxy-4'-methylbiphenyl-4-ylmethyl)-pyrimidin-2,4-diamin als farbloses Pulver erhalten.
MS (ISP): 379,3 $(M+H)^+$.
Smp. 182-185°C

(2r) 5-(4-Benzo[1,3]dioxol-5-yl-3,5-diethoxy-benzyl)-pyrimidin-2,4-diamin

Ausgehend von 5-(3,5-Diethoxy-4-iod-benzyl)-pyrimidin-2,4-diamin (Beispiel 1) (207 mg; 0,5 mmol), 1,3-Benzodioxol-5-boronsäure (166 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach Kristallisation aus Methanol 129 mg (63 %) 5-(4-Benzo[1,3]dioxol-5-yl-3,5-diethoxybenzyl)-pyrimidin-2,4-diamin als farbloses Pulver erhalten.
MS (ISP): 409,3 $(M+H)^+$

(2s) 4'-(2,4-Diamino-pyrimidin-5-ylmethyl)-2',6'-diethoxy-3-methyl-biphenyl-4-ol

Ausgehend von 5-(3,5-Diethoxy-4-iod-benzyl)-pyrimidin-2,4-diamin (Beispiel 1) (207 mg; 0,5 mmol), 4-Benzyloxy-3-methyl-phenylboronsäure (242 mg; 4,64 mmol) und Tetrakis-triphenylphosphin-palladium (28 mg; 0,024 mmol) in Dimethylformamid (3 ml), Ethanol (0,84 ml) und 2M wässriger Kaliumphosphat-Lösung (2,2 ml) werden nach Verrühren mit 20 ml n-Hexan/Diethylether (3/1) 220 mg (91 %) 5-(4'-Benzyloxy-2,6-diethoxy-3'-methyl-biphenyl-4-ylmethyl)-pyrimidin-2,4-diamin als farbloses Pulver erhalten.
MS(ISP): 485,4 $(M+H)^+$

5-(4'-Benzyloxy-2,6-diethoxy-3'-methyl-biphenyl-4-ylmethyl)-pyrimidin-2,4-diamin (214 mg; 0,44 mmol) wird in Methanol (20 ml) und in konzentrierter Essigsäure (2 ml) gelöst und über 10 % Pd/C (250 mg) hydriert. Der Katalysator wird abgenutscht, mit Metanol nachgewaschen und eingedampft. Der Rückstand wird mit Diethylether (25 ml) verrührt, abgenutscht, mit Diethylether gewaschen und am Hochvakuum getrocknet. Das farblose Pulver (129 mg) wird anschliessend in Wasser (2 ml) suspendiert, der pH-Wert auf 10 eingestellt mittels Zugabe einer konzentrierten wässrigen Ammoniaklösung, 15 Minuten bei Raumtemperatur verrührt, abgenutscht, gut mit Wasser gewaschen und am Hochvakuum getrocknet.
Ausbeute: 111 mg (64 %) 4'-(2,4-Diamino-pyrimidin-5-ylmethyl)-2',6'-diethoxy-3-methyl-biphenyl-4-ol als farbloses Pulver.
MS (ISP): 395,2 $(M+H)^+$

Die eingesetzte 4-Benzyloxy-3-methyl-phenylboronsäure wird ausgehend von 4-Brom-2-methyl-phenol in zwei Stufen (a-b) erhalten:

Stufe a) 4-Brom-1-benzyloxy-2-methyl-benzol

4-Brom-2-methyl-phenol (1,0 g; 5 mmol) wird in Dimethylformamid (25 ml; über Molekularsieb getrocknet) gelöst und mit Kalium-tert-butylat (0,8 g; 7,0 mmol) versetzt. Nach einstündigem Rühren bei Raumtemperatur wird mit einem Eisbad auf 0°C abgekühlt und innerhalb 45 Minuten mit einer Lösung von Benzylchlorid (0,7 ml; 6,0 mmol) in Dimethylformamid (15 ml; über Molekularsieb getrocknet) versetzt. Es wird eine Stunde bei 0°C und zwei Stunden bei Raumtemperatur weitergerührt. Nach dieser Zeit wird das Lösungsmittel abgedampft, der Rückstand mit Wasser versetzt, mit Ethylacetat extrahiert, mit Wasser gewaschen und über Magnesiumsulfat getrocknet, abgenutscht und eingeengt. Der erhaltene Rückstand wird am Hochvakuum bis zur Gewichtskonstanz getrocknet. Ausbeute: 1,5 g 4-Brom-1-benzyloxy-2-methyl-benzol als beiges Pulver, das ohne Reinigung im nächsten Schritt eingesetzt wird.
MS: 276 (M)

Stufe b) 4-Benzyloxy-3-methyl-phenylboronsäure

4-Brom-1-benzyloxy-2-methyl-benzol (6,9 g; 25 mmol) wird in Tetrahydrofuran (37,5 ml; über Molekularsieb getrocknet) gelöst und auf -78°C abgekühlt. Eine 1,6 M n-Butyllithium-lösung in n-Hexan (17 ml; 27,5 mmol) wird innerhalb 30 Minuten so zugetropft, dass die Temperatur nicht über -70°C ansteigt. Nach weiteren 10 Minuten bei dieser Temperatur wird Trimethylborat (8,3 ml; 75 mmol) tropfenweise innerhalb 25 Minuten unter -70°C zugetropft. Man lässt das Reaktionsgemisch langsam über Nacht auf Raumtemperatur erwärmen. Anschliessend wird auf 0°C gekühlt und eine wässrige 1 N Salzsäure-lösung zugetropft. Das Reaktionsgemisch wird mit Wasser und Ethylacetat verdünnt, die Phasen getrennt, und die wässrige Phase mit Ethylacetat nachextrahiert. Die vereinigten organischen Phasen werden nacheinander mit Wasser und einer gesättigten wässrigen Kochsalzlösung gewaschen, über Magnesiumsulfat getrocknet, abgenutscht und eingeengt.
Ausbeute: 3,95 g (65 %) 4-Benzyloxy-3-methyl-phenylboronsäure als weisses Pulver.
MS(ISN): 301,2 $(M-H)^-$

<u>(2t) 4'-(2,4-Diamino-pyrimidin-5-ylmethyl)-2',6'-diethoxy-biphenyl-4-ol</u>

Ausgehend von 5-(3,5-Diethoxy-4-iod-benzyl)-pyrimidin-2,4-diamin (<u>Beispiel 1</u>) (11,677 g ; 28,19 mmol) und 4-Methoxymethoxy-phenylboronsäure (7,934 g; 43,6 mmol) werden analog Beispiel (2a) nach Kristallisation aus Methanol 10,07 g (84 %) 5-(2,6-Diethoxy-4'-methoxymethoxy-biphenyl-4-ylmethyl)-pyrimidin-2,4-diamin als gräuliches Pulver erhalten.
MS(ISP): 425,5 $(M+H)^+$

5-(2,6-Diethoxy-4'-methoxymethoxy-biphenyl-4-ylmethyl)-pyrimidin-2,4-diamin (10,06 g; 23,7 mmol) wird in Methanol (410 ml) gelöst und mit einer 3N Salzsäure-Lösung in Methanol (119,1 ml; 357,3 mmol) versetzt. Das Reaktionsgemisch wird 30 Minuten bei einer Temperatur von 56°C gerührt und eingeengt. Der graue Rückstand wird anschliessend mit Wasser (400 ml) verrührt , der pH durch zugabe einer wässrigen gesättigten Ammoniaklösung (ca. 8 ml) auf 9 gestellt und das Ganze 15 Minuten gerührt. Die gräuliche Suspension wird abgenutscht und am Hochvakuum getrocknet. Es werden 8,85 g (98 %) 4'-(2,4-Diamino-pyrimidin-5-ylmethyl)-2',6'-diethoxy-biphenyl-4-ol als gräuliches Pulver erhalten.
MS (ISP): 381,3 $(M+H)^+$

<u>(2u) 4'-(2,4-Diamino-pyrimidin-5-ylmethyl)-2',6'-diethoxy-biphenyl-3-carbonitril</u>

Ausgehend von 5-(3,5-Diethoxy-4-iod-benzyl)-pyrimidin-2,4-diamin (<u>Beispiel 1</u>) (207 mg; 0,5 mmol), 3-Cyanphenyl-boronsäure(147 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach Chromatographie über Kieselgel (100 g) mit Methylenchlorid/Methanol/$NH_4OH$ konz. ( 19/1/0,05) und aufeinanderfolgender Kristallisation aus n-Hexan/Diethylether 3/1 (20 ml) und Methanol (1 ml), 52 mg (27 %) 4'-(2,4-Diamino-pyrimidin-5-ylmethyl)-2',6'diethoxy-biphenyl-3-carbonitril als beiges Pulver erhalten.
MS (ISP): 390,2 $(M+H)^+$

<u>(2v) 5-(4'-Dimethylamino-2,6-diethoxy-biphenyl-4-ylmethyl)-pyrimidin-2,4-diamin</u>

Ausgehend von 5-(3,5-Diethoxy-4-iod-benzyl)-pyrimidin-2,4-diamin (<u>Beispiel 1</u>) (207 mg; 0,5 mmol), 4-Dimethylamino-phenylboronsäure(165 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach Kristallisation aus n-Hexan/Diethylether 3/1 (20 ml) 143 mg (70 %) 5-(4'-Dimethylamino-2,6-diethoxy-biphenyl-4-ylmethyl)-pyrimidin-2,4-diamin als beiges Pulver erhalten.
MS (ISP): 408,3 $(M+H)^+$

<u>(2w) 5-(2,6-Diethoxy-4'-morpholin-4-ylmethyl-biphenyl-4-ylmethyl)-pyrimidin-2,4-diamin</u>

Ausgehend von 5-(3,5-Diethoxy-4-iod-benzyl)-pyrimidin-2,4-diamin (<u>Beispiel 1</u>) (300 mg; 0.72 mmol) und 4'-Morpholin-4-ylmethyl-phenylboronsäure (260 mg; 1.08 mmol) werden 185mg (56 %) 5-(2,6-Diethoxy-4'-morpholin-4-ylmethyl-biphenyl-4-ylmethyl)-pyrimidin-2,4-diamin als farbloses Pulver erhalten.

MS (ISP): 464,3 $(M+H)^+$.
Smp. > 250°C

(2x) 5-[4-(2,3-Dihydro-benzo[1,4]dioxin-6-yl)-3,5-diethoxy-benzyl]-pyrimidin-2,4-diamin

Ausgehend von 5-(3,5-Diethoxy-4-iod-benzyl)-pyrimidin-2,4-diamin (Beispiel 1) (207 mg; 0,5 mmol), 3,4-Ethylendioxy-phenylboronsäure(180 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach aufeinanderfolgender Kristallisation aus n-Hexan/Diethylether 3/1 (20 ml) und Methanol [124 mg (59 %)] 5-[4-(2,3-Dihydro-benzo[1,4]dioxin-6-yl)-3,5-diethoxy-benzyl]-pyrimidin-2,4-diamin als farbloses Pulver erhalten.
MS (ISP): 423,3 $(M+H)^+$

(2y) %) 5-(3'-Dimethylamino-2,6-diethoxy-biphenyl-4-ylmethyl)-pyrimidin-2,4-diamin

Ausgehend von 5-(3,5-Diethoxy-4-iod-benzyl)-pyrimidin-2,4-diamin (Beispiel 1) (207 mg; 0,5 mmol), 3-Dimethylamino-phenylboronsäure(165 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach aufeinanderfolgender Kristallisation aus n-Hexan/Diethylether 3/1 (20 ml) und Methanol 151 mg (74 %) 5-(3'-Dimethylamino-2,6-diethoxy-biphenyl-4-ylmethyl)-pyrimidin-2,4-diamin als farbloses Pulver erhalten.
MS (ISP): 408,4 $(M+H)^+$

(2z) 5-(2,6-Diethoxy-3'-nitro-biphenyl-4-ylmethyl)-pyrimidin-2,4-diamin

Ausgehend von 5-(3,5-Diethoxy-4-iod-benzyl)-pyrimidin-2,4-diamin (Beispiel 1) (207 mg; 0,5 mmol), 3-Nitrophenylboronsäure(167 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach aufeinanderfolgender Kristallisation aus n-Hexan/Diethylether 3/1 (20 ml) und Methylenchlorid 146 mg (71 %) 5-(2,6-Diethoxy-3'-nitro-biphenyl-4-ylmethyl)-pyrimidin-2,4-diamin als farbloses Pulver erhalten.
MS (ISP): 410,3 $(M+H)^+$

(2aa) 5-(2,6-Diethoxy-4'-methoxy-3'-methoxymethyl-biphenyl-4-ylmethyl)-pyrimidin-2,4-diamin

Ausgehend von 5-(3,5-Diethoxy-4-iod-benzyl)-pyrimidin-2,4-diamin (Beispiel 1) (207 mg; 0,5 mmol), 4-Methoxy-3-methoxymethyl-phenylboronsäure( 196 mg; 1,0 mmol) und Tetrakis-triphenylphosphin-palladium (28 mg; 0,024 mmol) in Dimethylformamid (3 ml), Ethanol (0,84 ml) und einer 2M wässrigen Kaliumphosphat-Lösung (2,2 ml) werden nach aufeinanderfolgender Kristallisation aus n-Hexan/Diethylether 3/1 (30 ml) 200 mg (91 %) 5-(2,6-Diethoxy-4'-methoxy-3'-methoxymethyl-biphenyl-4-ylmethyl)-pyrimidin-2,4-diamin als farbloses Pulver erhalten.
MS (ISP): 439,4 $(M+H)^+$

Die eingesetzte 4-Methoxy-3-methoxymethyl-phenylboronsäure wird ausgehend von 5-Brom-2-hydroxy-benzylalkohol in zwei Stufen(a-b) erhalten:

Stufe a) 4-Brom-1-methoxy-2-methoxymethyl-benzol

5-Brom-2-hydroxy-benzylalkohol (4,4 g; 20 mmol) wird in Tetrahydrofuran (200 ml; über Molekularsieb getrocknet) gelöst, mit einer 60% Natriumhydrid-Suspension in Öl (3,2 g; 80 mmol) versetzt und eine Stunde bei Raumtemperatur gerührt. Anschliessend wird Methyliodid (3,75 ml; 60 mmol) zugegeben und das Reaktionsgemisch während 22 Stunden bei 60°C gerührt. Nach Abkühlung auf Raumtemperatur wird das Reaktionsgemisch mit Wasser verdünnt und mit Ethylacetat extrahiert. Die vereinigten organischen Phasen werden nacheinander mit Wasser und einer gesättigten wässrigen Kochsalzlösung gewaschen, über Magnesiumsulfat getrocknet, abgenutscht und eingeengt. Das erhaltene gelbe Öl wird über Kieselgel (100 g) mit n-Hexan/Essigester 4/1 chromatographiert.
Es werden 3,04 g (66 %) 4-Brom-1-methoxy-2-methoxymethyl-benzol als farbloses Öl erhalten.
MS: 232 (M)

Stufe b) 4-Benzyloxy-3-methyl-phenylboronsäure

Wird in Analogie zu 4-Benzyloxy-3-methyl-phenylboronsäure (In Beispiel (2s) Stufe b) beschrieben) hergestellt.

Ausgehend von 4-Brom-1-methoxy-2-methoxymethyl-benzol (3,77 g; 16,3 mmol) werden 1,55 g (49 %)

4-Benzyloxy-3-methyl-phenylboronsäure als farbloses Pulver erhalten.
MS(ISN): 195,3 $(M-H)^-$

(2ab) [5-[4-(2,4-Diamino-pyrimidin-5-ylmethyl)-2,6-diethoxy-phenyl]-thiophen-2-yl]-methanol

Ausgehend von 5-(3,5-Diethoxy-4-iod-benzyl)-pyrimidin-2,4-diamin (Beispiel 1) (828 mg; 2,0 mmol) und (5-Hydroxymethyl-thiophen-2-yl)-boronsäure (632 mg; 4,0 mmol), werden nach Kristallisation aus Methanol 55 mg (7 %)[5-[4-(2,4-Diaminopyrimidin-5-ylmethyl)-2,6-diethoxy-phenyl]-thiophen-2-yl]-methanol als beiges Pulver erhalten.
Smp: 225-228 °C
MS (ISP): 401,3 $(M+H)^+$
(5-Hydroxymethyl-thiophen-2-yl)-boronsäure wird wie folgt, ausgehend von 2-Hydroxymethyl-thiophen, hergestellt:
2-Hydroxymethyl-thiophen (2,28 g; 20 mmol) wird in Tetrahydrofuran (40 ml, über Molekularsieb getrocknet) gelöst und auf -78°C unter Argonbegasung gekühlt. Eine 1,6 M n-Butyllithium-Lösung in n-Hexan (25 ml; 40 mmol) wird langsam zugetropft. Anschliessend lässt man das Reaktionsgemisch auf -30°C aufwärmen, kühlt erneut auf -78°C und tropft innerhalb 15 Minuten zwischen -78 und -70°C eine Lösung von Trimethylborat (6,69 ml; 60 mmol) in Tetrahydrofuran (20 ml) zu. Nach 2-stündigem Rühren bei -78°C lässt man auf Raumtemperatur erwärmen und giesst auf Wasser (50 ml), stellt das pH durch Zugabe einer 2 N wässrigen Salzsäurelösung (28 ml) auf 3 ein, extrahiert mit Diethylether, wäscht mit einer gesättigten wässrigen Kochsalzlösung, trocknet mit Natriumsulfat, nutscht ab und engt ein.
Ausbeute: 1,1 g (36 %) (5-Hydroxymethyl-thiophen-2-yl)-boronsäure als braunes Öl.
MS: 158 (M)

(2ac) 5-[3,5-Diethoxy-4-(5-morpholin-4-ylmethyl-thiophen-2-yl)-benzyl]-pyrimidin-2,4-diamin

Ausgehend von 5-(3,5-Diethoxy-4-iod-benzyl)-pyrimidin-2,4-diamin (Beispiel 1) (1,24 g; 3,0 mmol) und (5-Morpholin-4-ylmethyl-thiophen-2-yl)-boronsäure (1,02 g; 4,5 mmol) werden nach Kristallisation aus Methanol/Methylenchlorid (2/1) 280 mg (20 %)5-[3,5-Diethoxy-4-(5-morpholin-4-ylmethyl-thiophen-2-yl)-benzyl]-pyrimidin-2,4-diamin als farbloses Pulver erhalten.
Smp: 213-214 °C
MS (ISP): 470,2 $(M+H)^+$
(5-Morpholin-4-ylmethyl-thiophen-2-yl)-boronsäure wird in Analogie zu (5-Hydroxymethyl-thiophen-2-yl)-boronsäure (in Beispiel (2ab) beschrieben) ausgehend von 4-Thiophen-2-ylmethyl-morpholin (1,83 g; 10 mmol) hergestellt.
Ausbeute: 900 mg (40 %) (5-Morpholin-4-ylmethyl-thiophen-2-yl)-boronsäure als beiges Pulver.
Smp: 100°C (Zersetzung)
MS (ISN): 226,2 $(M-H)^-$
Das hierfür eingesetzte 4-Thiophen-2-ylmethyl-morpholin wird wie folgt, ausgehend von 2-Chlormethyl-thiophen, hergestellt:
2-Chlormethyl-thiophen (2,11 g; 16 mmol), Morpholin (1,39 ml; 16 mmol) und Natriumcarbonat (wasserfrei; 1,76 g; 16 mmol) werden in Toluol (3,2 ml, über Molekularsieb getrocknet) 3 Tage unter Rückfluss gekocht. Anschliessend wird das Reaktionsgemisch auf Raumtemperatur abgekühlt, mit Wasser verdünnt, mit Diethylether extrahiert, zweimal mit einer gesättigten wässrigen Kochsalzlösung gewaschen, über Natriumsulfat getrocknet, abgenutscht und eingeengt.
Ausbeute: 2,7 g (92 %) 4-Thiophen-2-ylmethyl-morpholin als braune Flüssigkeit.
MS: 183 (M)

(2ad1) 5-(2,6-Diethoxy-4'-methyl-3'-nitro-biphenyl-4-ylmethyl)-pyrimidin-2,4-diamin

Ausgehend von 5-(3,5-Diethoxy-4-iod-benzyl)-pyrimidin-2,4-diamin (Beispiel 1) (828 mg; 2 mmol) und 3-Nitro-4-methyl-phenylboronsäure (724 mg; 4 mmol), Tetrakistriphenyl-phosphin-palladium (83.3 mg; 0.07 mmol) und wässriger 2M Kalium-phosphat-Lösung (5.5 ml; 11.0 mmol) in Dimethylformamid werden in Analogie zu Beispiel 2a 800 mg (94 %) 5-(2,6-Diethoxy-4'-methyl-3'-nitro-biphenyl-4-ylmethyl)-pyrimidin-2,4-diamin als beiges Pulver erhalten.
MS (ISP): 424,2 $(M+H)^+$

(2ad) 5-(3'-Amino-2,6-diethoxy-4'-methyl-biphenyl-4-ylmethyl)-pyrimidin-2,4-diamin

5-(2,6-Diethoxy-4'-methyl-3'-nitro-biphenyl-4-ylmethyl)-pyrimidin-2,4-diamin (105 mg; 0,25 mmol) wird in Essigsäure konz. (6 ml), Wasser (0,3 ml) und Ethanol (1,5 ml) gelöst und über Pd/C 10% (85 mg) hydriert. Anschliessend

wird der Katalysator abgenutscht, gut mit Ethanol nachgewaschen und das Lösungsmittel abgedampft. Der Rückstand wird in Wasser (20 ml) aufgenommen und das pH mit $NH_4OH$ konz. auf ca. 9-10 eingestellt, wobei das Produkt ausfällt. Nach 30-minütigem Rühren wird das ausgefallene Produkt abgenutscht, mit Wasser gewaschen und am Hochvakuum getrocknet.
Ausbeute: 77 mg (78 %) 5-(3'-Amino-2,6-diethoxy-4'-methyl-biphenyl-4-ylmethyl)-pyrimidin-2,4-diamin als braunes Pulver.
MS (ISP): 394,3 $(M+H)^+$

(2ae) 4'-(2,4-Diamino-pyrimidin-5-ylmethyl)-2',6'-diethoxy-3-fluor-biphenyl-4-ol

Ausgehend von 5-(3,5-Diethoxy-4-iod-benzyl)-pyrimidin-2,4-diamin (Beispiel 1) (300 mg; 0,72 mmol), 4-Benzyloxy-3-Fluor-phenylboronsäure(445 mg; 1,81 mmol) und Tetrakis-triphenylphosphin-palladium (50 mg; 0,043 mmol) in Dimethylformamid (20 ml) und einer 2M wässrigen Kaliumphosphat-Lösung (2,1 ml) werden in Analogie zu Beispiel 2a nach Chromatographie über Kieselgel mit Methylenchlorid/Methanol/$NH_4OH$ konz. (90/10/1), 110 mg (30 %) 5-(4'-Benzyloxy-2,6-diethoxy-3'-fluor-biphenyl-4-ylmethyl)-pyrimidin-2,4-diamin als farbloses Pulver erhalten.
MS (ISP): 489,3 $(M+H)^+$

5-(4'-Benzyloxy-2,6-diethoxy-3'-fluor-biphenyl-4-ylmethyl)-pyrimidin-2,4-diamin (110 mg; 0,23 mmol) wird in Ethanol (3 ml), konz. Essigsäure (9 ml) und Wasser (1 ml) über Pd/C 10% (80 mg) hydriert. Der Katalysator wird abgenutscht und die Lösung eingedampft. Der Rückstand wird in wenig Wasser aufgenommen, das pH mit $NH_4OH$ konz. auf 9-10 gestellt und der ausgefallene Feststoff abgenutscht, mit Wasser gewaschen und am Hochvakuum getrocknet.
Ausbeute: 52 mg (58 %) 4'-(2,4-Diamino-pyrimidin-5-ylmethyl)-2',6'-diethoxy-3-fluorbiphenyl-4-ol als farbloses Pulver.
MS (ISP): 399,3 $(M+H)^+$

(2af) 5-(2,6-Diethoxy-3'-methylsulfonyl-biphenyl-4-ylmethyl)-pyrimidin-2,4-diamin

Ausgehend von 5-(3,5-Diethoxy-4-iod-benzyl)-pyrimidin-2,4-diamin (Beispiel 1) (178 mg; 0,43 mmol), 3-Methylsulfonyl-phenylboronsäure(140 mg; 0,86 mmol) und Tetrakis-triphenylphosphin-palladium (69 mg; 0,060 mmol) in Dimethoxyethan (4 ml), Ethanol (3 ml) und Natriumcarbonat (387 mg; 3,6 mmol) in Wasser (4,5 ml) werden in Analogie zu Beispiel 2a nach Kristallisation aus Methanol, 110 mg (63 %) 5-(2,6-Diethoxy-3'-methylsulfanyl-biphenyl-4-ylmethyl)-pyrimidin-2,4-diamin als gräuliches Pulver erhalten.
MS (ISP): 411,2 $(M+H)^+$

Ausgehend von 5-(2,6-Diethoxy-3'-methylsulfanyl-biphenyl-4-ylmethyl)-pyrimidin-2,4-diamin (205 mg; 0,5 mmol) werden in Analogie zu Beispiel (2i) 73 mg (33 %) 5-(2,6-Diethoxy-3'-methylsulfonyl-biphenyl-4-ylmethyl)-pyrimidin-2,4-diamin als farbloses Pulver erhalten.
MS (ISP): 443,2 $(M+H)^+$

(2ag) 4'-(2,4-Diamino-pyrimidin-5-ylmethyl)-2',6'-diethoxy-biphenyl-4-carbaldehyd

Ausgehend von 5-(3,5-Diethoxy-4-iod-benzyl)-pyrimidin-2,4-diamin (Beispiel 1) (621 mg; 0.15 mmol) und 4-Formyl-phenylboronsäure (300 mg; 2.0 mmol) werden 570 mg (72 %) 4'-(2,4-Diamino-pyrimidin-5-ylmethyl)-2',6'-diethoxy-biphenyl-4-carbaldehyd als farbloses Pulver erhalten.
MS (ISP): 393,2 $(M+H)^+$
Smp: 178-182 °C

Beispiel 3

Herstellung von Verbindungen der Formel III Schema 1 nach Methode B (Stille-kopplung mit Organostannan)

(3a) 2-[5-[4-(2,4-Diamino-pyrimidin-5-ylmethyl)-2,6-diethoxy-phenyl]-thiophen-2-ylmethoxy]-1-morpholin-4-yl-ethanon

5-(3,5-Diethoxy-4-iod-benzyl)-pyrimidin-2,4-diamin (Beispiel 1) (828 mg; 2 mmol); Tris-(dibenzylidenaceton)-dipalladium-chloroform-Komplex ($Pd_2(dba)_3.CHCl_3$) (83 mg; 0,08 mmol), Kupferiodid (38 mg; 0,2 mmol) und Triphenylarsen ($Ph_3As$) (245 mg; 0,8 mmol) werden in 1-Methyl-2-pyrrolidon (10 ml) auf 120°C unter Argon erhitzt. Innerhalb 1 Stunde wird eine Lösung von 1-Morpholin-4-yl-2-(5-trimethylstannanylthiophen-2-ylmethoxy)-ethanon (970 mg; 2,4 mmol) in 1-Methyl-2-pyrrolidon (NMP) (5 ml) bei 120 °C zugetropft. Anschliessend wird das Reaktionsgemisch abfiltriert, mit Methanol gut gewaschen und eingeengt. Der Rückstand wird über Kieselgel mit Methylenchlorid/Methanol/Ammoniak (95/5/0.5) chromatographiert und anschliessend aus Methanol kristallisiert.
Ausbeute: 69 mg (23 %) 2-[5-[4-(2,4-Diamino-pyrimidin-5-ylmethyl)-2,6-diethoxyphenyl]-thiophen-2-ylmethoxy]-1-morpholin-4-yl-ethanon als farbloses Pulver.
MS (ISP): 528,3 $(M+H)^+$

Das eingesetzte 1-Morpholin-4-yl-2-(5-trimethylstannanyl-thiophen-2-ylmethoxy)-ethanon wird wie folgt hergestellt (Stufen a-b):

Stufe a) (5-Trimethylstannanyl-thiophen-2-yl)-methanol

2-Thiophen-methanol (4,8 ml; 50 mmol) wird unter Argon in Tetrahydrofuran über Molekularsieb gelöst. Das Reaktionsgemisch wird auf -78°C abgekühlt und mit einer 1,6 M Lösung von n-Butyl-lithium in Tetrahydrofuran (62,5 ml; 100 mmol) tropfenweise so versetzt, dass die Temperatur -70°C nicht übersteigt. Es wird anschliessend eine Stunde bei -78°C und eine halbe Stunde bei Raumtemperatur nachgerührt. Das Reaktionsgemisch wird dann auf eine gesättigte wässrige Kochsalzlösung (50 ml) gegossen und mit Ether (3 x 50 ml) extrahiert. Die vereinigten organischen Phasen werden mit einer gesättigten wässrigen Kochsalzlösung (2 x 30 ml) gewaschen, über Natriumsulfat getrocknet, abgenutscht und eingeengt. Der Rückstand wird mittels Kugelrohrdestillation bei 122-124°C / 0,6 mbar gereinigt.
Ausbeute: 9,6 g (69 %) eines gelben Öles.

Stufe b) 1-Morpholin-4-yl-2-(5-trimethyistannanyl-thiophen-2-ylmethoxy)-ethanon

(5-Trimethylstannanyl-thiophen-2-yl)-methanol (6,51 g; 23,5 mmol) und Natriumiodid (3,46 g; 23,2 mmol) werden unter Argon bei Raumtemperatur in Tetrahydrofuran (100 ml) gelöst und auf -78°C abgekühlt. Es wird dann eine 1,6 M n-Butyllithium-Lösung in n-Hexan (14,7 ml; 23,5 mmol) innerhalb 10 Minuten zugetropft und weitere 60 Minuten bei -78°C gerührt. Es werden dann nacheinander Hexamethylphosphoramid (8,33 ml, 46,48 mmol) und 4-(Chloracetyl) morpholin (4,58 g; 28 mmol) zugetropft. Das Reaktionsgemisch wird anschliessend 18 Stunden bei Raumtemperatur nachgerührt. Nach dieser Zeit wird Diisopropylamin (4 ml) zugetropft und eine Stunde bei Raumtemperatur gerührt. Das Reaktionsgemisch wird dann auf eine wässrige gesättigte Ammoniumchloridlösung (150 ml) gegossen und mit Diethylether (3 x 200 ml) extrahiert. Die vereinigten organischen Phasen werden mit einer gesättigten wässrigen Kochsalzlösung (2 x 80 ml) gewaschen, über Natriumsulfat getrocknet, abgenutscht und eingeengt. Der Rückstand wird über Kieselgel mit n-Hexan/Ethylacetat 1/1 chromatographiert.
Ausbeute: 1,03 g (9 %) 1-Morpholin-4-yl-2-(5-trimethylstannanyl-thiophen-2-ylmethoxy)-ethanon als gelbliches Öl.
MS: 390 (M-⁻CH3)

In Analogie hierzu werden hergestellt [Beispiele 3(b) und 3(c)]:

<u>(3b) 2-[5-[4-(2,4-Diamino-pyrimidin-5-ylmethyl)-2,6-diethoxy-phenyl]-thiophen-2-ylmethoxy]-1-thiomorpholin-4-yl-ethanon</u>

Ausgehend von 207 mg; 0,5 mmol 5-(3,5-Diethoxy-4-iod-benzyl)-pyrimidin-2,4-diamin (Beispiel 1) (207 mg; 0,5 mmol) und 1-Thiomorpholin-4-yl-2-(5-trimethylstannanyl-thiophen-2-ylmethoxy)-ethanon (3 x 210 mg; 3 x 0,5 mmol) werden 24 mg (9 %) 2-[5-[4-(2,4-Diamino-pyrimidin-5-ylmethyl)-2,6-diethoxy-phenyl]-thiophen-2-ylmethoxy]-1-thiomorpholin-4-yl-ethanon als beiges Pulver erhalten.
Smp: >180°C (Zersetzung)
MS (ISP): 544,2 $(M+H)^+$

Das eingesetzte 1-Thiomorpholin-4-yl-2-(5-trimethylstannanyl-thiophen-2-ylmethoxy)-ethanon wird in Analogie zu Beispiel (3a) (Stufe b)) ausgehend von (5-Trimethylstannanyl-thiophen-2-yl)-methanol (7,66 g; 27,67 mmol; in Beispiel (3a), Stufe a) beschrieben), Natriumiodid (4,07 g; 27,34 mmol) und 2-Chlor-1-thiomorpholin-4-yl-ethanon (5,92 g; 32,94 mmol) hergestellt.
Ausbeute: 1,6 g (12 %) 1-Thiomorpholin-4-yl-2-(5-trimethylstannanyl-thiephen-2-ylmethoxy)-ethanon als gelbes Öl.
MS: 421 (M); 406 $(M-CH_3)$

<u>(3c) 2-[5-[4-(2,4-Diamino-pyrimidin-5-ylmethyl)-2,6-diethoxy-phenyl]-thiophen-2-ylmethoxy]-1-piperidin-1-yl-ethanon</u>

Ausgehend von 5-(3,5-Diethoxy-4-iod-benzyl)-pyrimidin-2,4-diamin (Beispiel 1) (828 mg; 2,0 mmol) und 1-Piperidin-1-yl-2-(5-trimethylstannanyl-thiophen-2-ylmethoxy)-ethanon (3 x 1,6g; 3 x 4,0 mmol) werden 85 mg (5 %) 2-[5-[4-(2,4-Diaminopyrimidin-5-ylmethyl)-2,6-diethoxy-phenyl]-thiophen-2-ylmethoxy]-1-piperidin-1-ylethanon als farbloses Pulver erhalten.
Smp: 155-160°C
MS (ISP): 526,3 $(M+H)^+$

Das eingesetzte 1-Piperidin-1-yl-2-(5-trimethylstannanyl-thiophen-2-ylmethoxy)-ethanon wird in Analogie zu Beispiel (3a) (Stufe b)) ausgehend von (5-Trimethyltannanylthiophen-2-yl)-methanol (6,98 g; 25,2 mmol; in Beispiel (3a) Stufe a) beschrieben), Natriumiodid (3,71 g; 24,9 mmol) und 1-Chloracetyl-piperidin (4,85 g; 30 mmol) hergestellt. Ausbeute: 4,4 g (37 %) 1-Piperidin-1-yl-2-(5-trimethylstannanyl-thiophen-2-ylmethoxy)-ethanon als farbloses Öl.
MS: 403 (M); 388 ($M-CH_3$)

(3d) 5-[4-(2,6-Dimethyl-pyridin-4-yl)-3,5-diethoxy-benzyl]-pyrimidin-2,4-diamin

5-(3,5-Diethoxy-4-iod-benzyl)-pyrimidin-2,4-diamin (Beispiel 1) (414 mg; 1 mmol), 2,6-Dimethyl-4-trimethyltin-pyridine (404 mg; 1,5 mmol), Bis(Triphenylphosphin)palladiumdichlorid (20 mg) und einige Kristalle 2,6-Di-tert-butyl-4-methylphenol werden in Dimethylformamid (5 ml) gelöst und 24 Stunden bei 140°C unter Argonbegasung gerührt. Das Reaktionsgemisch wird eingeengt und der erhaltene Rückstand mit wenig Wasser versetzt, das pH durch Zugabe von $NH_4OH$ konz. auf 8 eingestellt und das Ganze abgenutscht. Die Mutterlauge wird eingeengt und über Kieselgel (30 g) mit Methylenchlorid/Methanol/$NH_4OH$ konz. 19/1/0,05 chromatographiert. Es werden 26 mg (6,6 %) 5-[4-(2,6-Dimethyl-pyridin-4-yl)-3,5-diethoxy-benzyl]-pyrimidin-2,4-diamin als gelbes Pulver erhalten.
MS(ISP): 394,3 $(M+H)^+$

In Analogie zu Beispiel (3d) wird hergestellt:

(3e) 5-[3,5-Diethoxy-4-(6-methylsulfanyl-pyridin-3-yl)-benzyl]-pyrimidin-2,4-diamin

Ausgehend von 5-(3,5-Diethoxy-4-iod-benzyl)-pyrimidin-2,4-diamin (Beispiel 1) (2,07 ; 5,0 mmol) und 2-Methylsulfanyl-5-trimethylstannanyl-pyridin (2,12 g; 7,5 mmol), werden 420 mg (21 %) 5-[3,5-Diethoxy-4-(6-methylsulfanyl-pyridin-3-yl)-benzyl]-pyrimidin-2,4-diamin als leicht gelbes Pulver erhalten.
MS (ISP): 412,3 $(M+H)^+$

Beispiel 4

Herstellung von Verbindungen der Formel III, Schema 1, nach Methode C (Suzuki-Kopplung mit *in situ* Generierung von $R^2-B(OH)_2$)

(4a) 4'-(2,4-Diamino-pyrimidin-5-ylmethyl)-2',6'-diethoxy-biphenyl-4-carbonitril

4-Brom-benzonitril (186 mg; 1 mmol), Bis(pinacolato)diboron (279 mg; 1,1 mmol), Kalium-acetat (294 mg; 3 mmol) und 1,1'-Bis(Diphenylphosphino)ferrocendichlorpalladium (II) ($PdCl_2$(dppf)) (46 mg; 0,06 mmol) werden unter Argonbegasung in Dimethylformamid (6 ml; über Molekularsieb getrocknet) bei 80°C Badtemperatur während 3 Stunden gerührt. Nach Abkühlen auf Raumtemperatur werden Dimethylformamid (20 ml; über Molekularsieb getrocknet), Tetrakis-triphenylphosphin-palladium (90 mg; 0,078 mmol), 5-(3,5-Diethoxy-4-iod-benzyl)-pyrimidin-2,4-diamin (Beispiel 1) (331 mg; 0,8 mmol) und eine 2M wässrige Kaliumphosphat-Lösung (4 ml) zugegeben. Das Reaktionsgemisch wird ca. 15 Stunden bei 80°C gerührt, auf Raumtemperatur abgekühlt und eingeengt. Der erhaltene Rückstand wird über Kieselgel (100 g) mit Methylenchlorid/Methanol/$NH_4OH$ (19/1/0,05) chromatographiert. Es werden 61 mg (20 %) 4'-(2,4-Diamino-pyrimidin-5-ylmethyl)-2',6'-diethoxy-biphenyl-4-carbonitril als beiges Pulver erhalten.
MS(ISP): 390,1 $(M+H)^+$

In Analogie hierzu werden hergestellt (Beispiele 4(b)-(4u), 4(x), 4(z), 4(ab), (4ae), 4(f), wobei allfällig von Beispiel 4(a) abweichende Mengen der Reaktionsteilnehmer spezifisch angegeben werden):

(4b) N-[4'-(2,4-Diamino-pyrimidin-5-ylmethyl)-2',6'-diethoxy-biphenyl-4-yl]-methansulfonamid

Ausgehend von N-(4-Brom-phenyl)-methansulfonamid (250 mg; 1 mmol) und 5-(3,5-Diethoxy-4-iod-benzyl)-pyrimidin-2,4-diamin (Beispiel 1) (331 mg; 0,8 mmol) werden 133 mg (36 %) N-[4'-(2,4-Diamino-pyrimidin-5-ylmethyl)-2',6'-diethoxybiphenyl-4-yl]-methansulfonamid als leicht beiges Pulver erhalten.
MS (ISP): 458,2 $(M+H)^+$

(4c) 5-(4'-Amino-2,6-diethoxy-biphenyl-4-ylmethyl)-pyrimidin-2,4-diamin

Ausgehend von N-(4-Brom-phenyl)-2,2,2-trifluor-acetamid (536 mg; 2 mmol) und 5-(3,5-Diethoxy-4-iod-benzyl)-pyrimidin-2,4-diamin (Beispiel 1) (662 mg; 1,6 mmol) werden nach Behandlung während einer Nacht mit 1N Natronlauge (0,55 ml; 0,55 mmol) und Ethanol (15 ml) bei 70°C, 89 mg (15 %) 5-(4'-Amino-2,6-diethoxy-biphenyl-4-ylmethyl)-pyrimidin-2,4-diamin als hellbraunes Pulver erhalten.

MS (ISP): 380,4 $(M+H)^+$

(4d) 5-[3,5-Diethoxy-4-(6-morpholin-4-ylmethyl-pyridin-3-yl)-benzyl]-pyrimidin-2,4-diamin

Ausgehend von 4-(5-Brom-pyridin-2-ylmethyl)-morpholin (280 mg; 1.08 mmol), Bis(pinacolato)diboron (305 mg; 1,2mmol), Kaliumacetat (321 mg; 3.27 mmol) und 1,1'-Bis(Diphenylphosphino)ferrocendichlorpalladium (II) ($PdCl_2$ (dppf)) (46 mg; 0,06 mmol)und von 5-(3,5-Diethoxy-4-iod-benzyl)-pyrimidin-2,4-diamin (Beispiel 1) (361mg; 0.872mmol) werden 43 mg (10 %) 5-[3,5-Diethoxy-4-(6-morpholin-4-ylmethyl-pyridin-3-yl)-benzyl]-pyrimidin-2,4-diamin als farbloses Pulver erhalten.
MS (ISP): 465,3 $(M+H)^+$.

(4e) N-[4'-(2,4-Diamino-pyrimidin-5-ylmethyl)-2',6'-diethoxy-3-methyl-biphenyl-4-yl]-acetamid

Ausgehend von N-[4-Brom-2-methyl]-acetanilid (274 mg; 1.2 mmol), Bis(pinacolato) diboron (336 mg; 1,32 mmol), Kaliumacetat (354 mg; 3.6 mmol) und 1,1'-Bis(Diphenylphosphino)ferrocendichlor-palladium (II) ($PdCl_2$(dppf)) (35 mg; 0,05 mmol) in Dimethylformamid, und danach 5-(3,5-Diethoxy-4-iod-benzyl)-pyrimidin-2,4-diamin (Beispiel 1) (414 mg; 1 mmol), Tetrakis-triphenylphosphin-palladium (83.3 mg; 0.07 mmol), wässriger 2M Kaliumphosphat Lösung (3.36 ml; 6.7 mmol) in Dimethylformamid werden 115 mg (22 %) N-[4'-(2,4-Diamino-pyrimidin-5-ylmethyl)-2',6'diethoxy-3-methyl-biphenyl-4-yl]-acetamid als braune Kristalle erhalten.
MS (ISP): 436.3$(M+H)^+$

(4f) 5-(4'-Amino-2,6-diethoxy-3'-methyl-biphenyl-4-ylmethyl)-pyrimidin-2,4-diamin

Ausgehend von N-(4-Brom-2-methyl-phenyl)-2,2,2-trifluoracetamid (670 mg; 2.5 mmol), Bis(pinacolato)diboron (698 mg; 2.75 mmol), Kaliumacetat (736mg; 7.5 mmol) und 1,1'-Bis(Diphenylphosphino)ferrocendichlorpalladium (II) ($PdCl_2$(dppf)) (73 mg; 0,1 mmol) in Dimethylformamid, danach 5-(3,5-Diethoxy-4-iod-benzyl)-pyrimidin-2,4-diamin (Beispiel 1) (828 mg; 2 mmol), Tetrakis-triphenylphosphin-palladium (173.3 mg; 0.15 mmol), wässriger 2M Kaliumphosphat-Lösung (7 ml; 14 mmol) in Dimethylformamid werden 56 mg (10 %) 5-(4'-Amino-2,6-diethoxy-3'-methyl-biphenyl-4-ylmethyl)-pyrimidin-2,4-diamin als hellbraune Kristalle erhalten.
MS (ISP): 394.3$(M+H)^+$
N-(4-Brom-2-methyl-phenyl)-2,2,2-trifluoracetamid wurde aus 4-Brom-2-methyl-anilin hergestellt in Analogie zu Beispiel (41) (nachstehend).

(4g) N-[4'-(2,4-Diamino-pyrimidin-5-ylmethyl)-2',6'-diethoxy-biphenyl-4-yl]-2-morpholin-4-yl-acetamid

Ausgehend von N-(4-Brom-phenyl)-4-morpholin-acetamid (299 mg; 1 mmol) und 5-(3,5-Diethoxy-4-iod-benzyl)-pyrimidin-2,4-diamin (Beispiel 1) (331 mg; 0,8 mmol) werden 70 mg (17 %) N-[4'-(2,4-Diamino-pyrimidin-5-ylmethyl)-2',6'-diethoxybiphenyl-4-yl]-2-morpholin-4-yl-acetamid als leicht beiges Pulver erhalten.
MS (ISP): 507,4 $(M+H)^+$

(4h) 4-Amino-4'-(2,4-diamino-pyrimidin-5-ylmethyl)-2',6'-diethoxy-biphenyl-3-carbonitril

Ausgehend von 2-Amino-5-benzonitril (940 mg; 3,85 mmol) und 5-(3,5-Diethoxy-4-iod-benzyl)-pyrimidin-2,4-diamin (Beispiel 1) (1,27 g; 3,06 mmol) werden 845 mg (68 %) 4-Amino-4'-(2,4-diamino-pyrimidin-5-ylmethyl)-2',6'-diethoxy-biphenyl-3-carbonitril als grünes Pulver erhalten.
MS (ISP): 405,4 $(M+H)^+$

(4i) 5-(3'-Amino-2,6-diethoxy-4'-methoxy-biphenyl-4-ylmethyl)-pyrimidin-2,4-diamin

Ausgehend von N-(3-Brom-6-methoxy-phenyl)-2,2,2-trifluoracetamid (450 mg; 1.5 mmol), Bis(pinacolato)diboron (380 mg; 1.5 mmol), Kaliumacetat (442 mg; 3 mmol) und 1,1'-Bis(Diphenylphosphino)ferrocendichlorpalladium (II) ($PdCl_2$(dppf)) (72 mg; 0,06 mmol) in Dimethylformamid (10 ml), danach 5-(3,5-Diethoxy-4-iod-benzyl)-pyrimidin-2,4-diamin (Beispiel 1) (415 mg; 1 mmol), Tetrakis-triphenylphosphin-palladium (87mg; 0.1 mmol) und wässriger 2M Kaliumphosphat-Lösung (3 ml; 6 mmol) in Dimethylformamid (30 ml) werden 225 mg (68 %) 5-(3'-Amino-2,6-diethoxy-4'-methoxy-biphenyl-4-ylmethyl)-pyrimidin-2,4-diamin als hellbraune Kristalle erhalten.
MS (ISP): 410.4 $(M+H)^+$
N-(3-Brom-6-methoxy-phenyl)-2,2,2-triHuoracetamid wird aus 3-Brom-6-methoxy-anilin hergestellt in Analogie zu Beispiel (41) (nachstehend).

(4j) 5-(3'-Amino-2,6-diethoxy-4'-morpholino-4-ylmethyl-biphenyl)-pyrimidin-2,4-diamin

Ausgehend von N-(5-Brom-2-methylmorpholin-phenyl)-2,2,2-trifluoracetamid (550 mg; 1.5 mmol), Bis(pinacolato)diboron (380 mg; 1.5 mmol), Kaliumacetat (442 mg; 3 mmol) und 1,1'-Bis(Diphenylphosphino)ferrocendichlorpalladium (II) ($PdCl_2$(dppf)) (72 mg; 0,06 mmol) in Dimethylformamid (10 ml), danach 5-(3,5-Diethoxy-4-iodbenzyl)-pyrimidin-2,4-diamin (Beispiel 1) (415 mg; 1 mmol), Tetrakis-triphenylphosphin-palladium (87 mg; 0.1 mmol) und wässriger 2M Kaliumphosphat-Lösung (3 ml; 6 mmol) in Dimethylformamid (30 ml), werden 375 mg (78 %) 5-(3'-Amino-2,6-diethoxy-4'-methylmorpholino-4-ylmethyl-biphenyl)-pyrimidin-2,4-diamin als beiger Feststoff erhalten.
MS (ISP): 479.4 $(M+H)^+$

N-(5-Brom-2-methylmorpholin-phenyl)-2,2,2-trifluoracetamid wird aus 5-Brom-2-methylmorpholin-anilin hergestellt, in Analogie zu Beispiel (4l).

(4k) 5-[3,5-Diethoxy-4-(2-morpholin-4-yl-pyrimidin-5-yl)-benzyl]-pyrimidin-2,4-diamin

Ausgehend von 4-(5-Brom-2-pyrimidin-yl)-morpholin (244 mg; 1 mmol), Bis(pinacolato)diboron (279 mg; 1,1mmol), Kaliumacetat (294 mg; 3mmol) und 1,1'-Bis(diphenylphosphino)ferrocendichlorpalladium (II) ($PdCl_2$(dppf)) (24 mg; 0,035 mmol) und von 5-(3,5-Diethoxy-4-iod-benzyl)-pyrimidin-2,4-diamin (Beispiel 1) (373mg; 0.9 mmol) werden 49mg (12 %) 5-[3,5-Diethoacy-4-(2-morpholin-4-yl-pyrimidin-5-yl)-benzyl]-pyrimidin-2,4-diamin als farbloses Pulver erhalten.
MS (ISP): 452,3 $(M+H)^+$
Smp. 196-199°C.

(4l) 5-(4'-Amino-2,6-diethoxy-3'-methoxy-biphenyl-4-ylmethyl)-pyrimidin-2,4-diamin

Ausgehend von N-(4-Iod-2-methoxy-phenyl)-2,2,2-trifluoracetamid (700 mg; 1.5 mmol), Bis(pinacolato)diboron (380 mg; 1.5 mmol), Kaliumacetat (442 mg; 3 mmol) und 1,1'-Bis(diphenylphosphino)ferrocendichlorpalladium (II) ($PdCl_2$(dppf)) (72 mg; 0,06 mmol) in Dimethylformamid (10 ml), danach 5-(3,5-Diethony-4-iod-benzyl)-pyrimidin-2,4-diamin (Beispiel 1) (415 mg; 1 mmol), Tetrakis-triphenylphosphin-palladium (87mg; 0.1 mmol) und wässriger 2M Kaliumphosphat-Lösung (3 ml; 6 mmol) in Dimethylformamid (30 ml) werden 250 mg (61 %) 5-(4'-Amino-2,6-diethoxy-3'-methoxy-biphenyl-4-ylmethyl)-pyrimidin-2,4-diamin als beiger Feststoff erhalten.
MS (ISP): 410.4 $(M+H)^+$
N-(4-Iod-2-methoxy-phenyl)-2,2,2-trifluoracetamid wird wie folgt hergestellt:

2-Methoxy-anilin (1 g; 8.12 mmol) wird in einem 2/1 Methanol/Methylenchlorid-Gemisch gelöst. Calciumcarbonat (1.5 g) und Benzyl-trimethyl-ammoniumdichlorat (3.1 g; 8.93 mmol) werden bei Raumtemperatur zugegeben. Nach 2-tägigem Rühren wird das Lösungsmittel abgedampft und der Rückstand über Kieselgel mit n-Hexan/Ethylacetat (8/2) chromatographiert . Die reinen Fraktionen werden vereinigt und das Lösungsmittel abgedampft. Es werden 1,76 g (87 %) 4-Iod-3-methoxy-anilin als braunes Oel erhalten. Die gesamte Menge wird mit Methylenchlorid (30 ml) verdünnt, mit Pyridin (1.6 ml; 19.9 mmol) und Trifluoressigsäureanhydrid (1.12 ml; 7.96 mmol) versetzt und eine Stunde bei Raumtemperatur gerührt. Die Reaktionslösung wird auf wässrige Salzsäure (1M; 20 ml) gegossen und zweimal mit Ethylacetat (20 ml) extrahiert. Die vereinigten organischen Phasen werden über Natriumsulfat getrocknet, filtriert und eingeengt. Der Rückstand wird über Kieselgel mit n-Hexan/Ethylacetat (7/3) chromatographiert . Die reinen Fraktionen werden vereinigt und das Lösungsmittel abgedampft.
Ausbeute: 2.10 g (92%) als weisser Feststoff.
MS: 345 (M)

(4m) 5-(3,5-Diethoxy-4-chinolin-6-yl-benzyl)-pyrimidin-2,4-diamin

Ausgehend von Trifluormethansulfonsäure- 6-chinolinyl ester(424 mg; 1.53 mmol), Bis(pinacolato)diboron (279 mg; 1,1 mmol), Kaliumacetat (450 mg; 4.6 mmol), 1,1'-Bis(diphenylphosphino)ferrocendichlorpalladium (II) ($PdCl_2$(dppf)) (18 mg; 0,025 mmol) und von 5-(3,5-Diethoxy-4-iod-benzyl)-pyrimidin-2,4-diamin (Beispiel 1) (414 mg; 1 mmol) werden 66 mg (12 %) 5-(3,5-Diethoxy-4-quinolin-6-yl-benzyl)-pyrimidin-2,4-diamin als farbloses Pulver erhalten.
MS (ISP): 416.2 $(M+H)^+$
Smp: 232-236°C.

(4n) 5-(3'-Amino-2,6-diethoxy-4'-fluor-biphenyl-4-ylmethyl)-pyrimidin-2,4-diamin

Ausgehend von N-(3-Brom-4-fluor-phenyl)-2,2,2-trifluoracetamid (200 mg; 0.7 mmol), Bis(pinacolato)diboron (266 mg; 1.05 mmol), Kaliumacetat (206 mg; 2.1 mmol) und 1,1'-Bis(diphenylphosphino)ferrocendichlorpalladium (II)

($PdCl_2$(dppf)) (31 mg; 0,04 mmol) in Dimethylformamid (10 ml), danach 5-(3,5-Diethoxy-4-iod-benzyl)-pyrimidin-2,4-diamin (Beispiel 1) (232 mg; 0.56 mmol), Tetra-(triphenyl-phosphin)-palladium (48mg; 0.04 mmol) und wässriger 2M Kaliumphosphat-Lösung (2.1 ml; 4.2 mmol) in Dimethylformamid (30 ml) werden 102 mg (37 %) 5-(3'-Amino-2,6-diethoxy-4'-fluor-biphenyl-4-ylmethyl)-pyrimidin-2,4-diamin als hellbrauner Feststoff erhalten.
MS (ISP): 398.3$(M+H)^+$

Das eingesetzte N-(3-Brom-4-fluor-phenyl)-2,2,2-trifluoracetamid wird aus 3-Brom-4-fluor-anilin hergestellt, in Analogie zu Beispiel (4l).

(4o) 4'-(2,4-Diamino-pyrimidin-5-ylmethyl)-2',6'-diethoxy-biphenyl-4-sulfonsäureamid

Ausgehend von 4-Brombenzolsulfonamid (236 mg; 1 mmol) und 5-(3,5-Diethoxy-4-iod-benzyl)-pyrimidin-2,4-diamin (Beispiel 1) (331 mg; 0,8 mmol) werden 46 mg (13 %) 4'-(2,4-Diamino-pyrimidin-5-ylmethyl)-2',6'-diethoxy-biphenyl-4-sulfonsäureamid als graues Pulver erhalten.
MS (ISP): 444,2 $(M+H)^+$

(4p) N-[4'-(2,4-Diamino-pyrimidin-5-ylmethyl)-2',6'-diethoxy-biphenyl-4-yl]-acetamid

Ausgehend von 4-Bromacetanilid(214 mg; 1 mmol) und 5-(3,5-Diethoxy-4-iodbenzyl)-pyrimidin-2,4-diamin (Beispiel 1) (331 mg; 0,8 mmol) werden 136 mg (40 %) N-[4'-(2,4-Diamino-pyrimidin-5-ylmethyl)-2',6'-diethoxy-biphenyl-4-yl]-acetamid als graues Pulver erhalten.
MS (ISP): 422,2 $(M+H)^+$

(4q) 5-[4-(6-Amino-pyridin-3-yl)-3,5-diethoxy-benzyl]-pyrimidin-2,4-diamin

Bis-(triphenylphosphin)-palladium(II)dichlorid (42 mg; 0,036 mMol) wird unter Argonbegasung in Dioxan (2 ml) suspendiert und mit 5-Brom-2-(2,2,5,5-tetramethyl-[1,2,5]azadisilolidin-1-yl)-pyridine (0.63g; 2 mMol) in Dioxan (6 ml) und mit Triethylamin (0.607g; 6 mMol) versetzt. Nach 15-minütigem Rühren bei Raumtemperatur wird eine 1M Lösung von 4,4,5,5-Tetramethyl-1,3,2-dioxaborolan (Bis(pinacolato)-diboron) in Dioxan (3 ml; 3mMol) zugegeben und das Reaktionsgemisch 6 Stunden am Rückfluss erhitzt. Die Lösungsmittel werden am Rollverdampfer eingedampft und der Rückstand im Hochvakuum während 2 Stunden getrocknet. Man erhält 720 mg rohes 2-(2,2,5,5-Tetramethyl [1,2,5]azadisilolidin-1-yl)-5-(4,4,5,5-tetramethyl[1,3,2]dioxaborolan-2-yl)-pyridin. Das rohe Zwischenprodukt wird unter Argonbegasung in 6ml Dimethylformamid gelöst und die entstandene Lösung mit Tetrakis-triphenylphosphinpalladium (50 mg; 0,043 mMol), 5-(3,5-Diethoxy-4-iod-benzyl)-pyrimidin-2,4-diamin (Beispiel 1) (414 mg; 1 mMol) und mit 2M Kaliumphosphat-Lösung (6 ml) versetzt. Das Reaktionsgemisch wird anschliessend 5 Stunden am Rückfluss gekocht. Das Reaktionsgemisch wird mit Wasser (50 ml) verrührt und dreimal mit jeweils 25 ml Methylenchlorid extrahiert. Die vereinigten organischen Phasen werden über Magnesiumsulfat getrocknet und eingeengt. Der Rückstand wird über Kieselgel (50 g) mit Methylenchlorid / Methanol (17/3) chromatographiert. Die reinen Fraktionen werden vereinigt, eingeengt und am Hochvakuum getrocknet.
Ausbeute: 0.32 g (84 %) 5-[4-(6-Amino-pyridin-3-yl)-3,5-diethoxy-benzyl]-pyrimidin-2,4-diamin als beiges Pulver.
MS (EI): 380 $(M^+)$. Smp. 215-218°C

(4r) 5-(4'-Amino-2,6-diethoxy-3'-fluor-biphenyl-4-ylmethyl)-pyrimidin-2,4-diamin

Ausgehend von N-(4-Brom-3-fluor-phenyl)-2,2,2-trifluoracetamid (700 mg; 2.45 mmol), Bis(pinacolato) diboron (932 mg; 3.67 mmol), Kaliumacetat (721 mg; 7.34 mmol) und 1,1'-Bis(Diphenylphosphino)ferrocendichlorpalladium (II) ($PdCl_2$(dppf)) (107 mg; 0,15 mmol) in Dimethylformamid (16 ml), danach 5-(3,5-Diethoxy-4-iod-benzyl)-pyrimidin-2,4-diamin (Beispiel 1) (760 mg; 1.84 mmol), Tetrakis-triphenylphosphin-palladium (170 mg; 0.15 mmol) und wässriger 2M Kaliumphosphat-Lösung (7.4 ml; 14.8 mmol) in Dimethylformamid (48 ml) werden 307 mg (42%) 5-(4'-Amino-2,6-diethoxy-3'-fluor-biphenyl-4-ylmethyl)-pyrimidin-2,4-diamin als grauer Feststoff erhalten.
MS (ISP): 398.3$(M+H)^+$

Das eingesetzte N-(4-Brom-3-fluor-phenyl)-2,2,2-trifluoracetamid wird aus 4-Brom-3-fluor-anilin hergestellt, in Analogie zu Beispiel (4l).

(4s) (RS)-3-[4'-(2,4-Diamino-pyrimidin-5-ylmethyl)-2',6'-diethoxy-biphenyl-4-yl]-5-methoxymethyl-oxazolidin-2-on

Ausgehend von (RS)-3-(4-Iodphenyl)-5-(methoxymethyl)-oxazolidin-2-on (333 mg; 1 mmol) und 5-(3,5-Diethoxy-4-iod-benzyl)-pyrimidin-2,4-diamin (Beispiel 1) (331 mg; 0,8 mmol) werden 194 mg (49 %) (RS)-3-[4'-(2,4-Diamino-pyrimidin-5-ylmethyl)-2',6'-diethoxy-biphenyl-4-yl]-5-methoxymethyl-oxazolidin-2-on als beiges

Pulver erhalten.
MS (ISP): 494,3 $(M+H)^+$

(4t) 5-(5'-Amino-2,6-diethoxy-3'-fluor-biphenyl-4-ylmethyl)-pyrimidin-2,4-diamin

Ausgehend von N-(5-Iod-3-fluor-phenyl)-2,2,2-trifluoracetamid (600 mg; 1.8 mmol), Bis(pinacolato)diboron (595 mg; 2.34 mmol), Kaliumacetat (530 mg; 5.41 mol) und 1,1'-Bis(diphenylphosphino)ferrocendichlorpalladium (II) ($PdCl_2$(dppf)) (80 mg; 0,11 mmol) in Dimethylformamid (10 ml) wird das Reaktionsgemisch auf Wasser gegossen und mit Ethylacetat extrahiert. Die vereinigten organischen Phasen werden mit einer gesättigten wässrigen Kochsalzlösung gewaschen, über Magnesiumsulfat getrocknet, abgenutscht, eingeengt und direkt in den nächsten Schritt eingesetzt. Nach Umsetzung mit 5-(3,5-Diethoxy-4-iod-benzyl)-pyrimidin-2,4-diamin (Beispiel 1) (523 mg; 1.26 mmol), Tetrakis-triphenylphosphin-palladium (125 mg; 0.11 mmol) und wässriger 2M Natriumcarbonat-Lösung (18 ml; 36 mmol) in einem 5/1 Dimethoxyethan/Ethanol-Gemisch (30 ml) werden 321 mg (45 %) 5-(5'-Amino-2,6-diethoxy-3'-fluor-biphenyl-4-ylmethyl)-pyrimidin-2,4-diamin als brauner Feststoff erhalten.
MS (ISP): 398.4$(M+H)^+$

Das eingesetzte N-(5-Iod-3-fluor-phenyl)-2,2,2-trifluoracetamid wird wie folgt hergestellt:

3-Iod-5-fluor-1-nitrobenzol (5.2 g; 19.48 mmol) wird in einem 4/3 Wasser/Tetrahydrofuran-Gemisch gelöst. Pulverisiertes Eisen (8.7 g; 156 mmol), gefolgt von Essigsäure (4.5 ml) werden bei Raumtemperatur zugegeben und die entstandene Suspension bei 40°C über Nacht gerührt. Das Reaktionsgemisch wird über Diatomeenerde filtriert und auf Wasser gegossen. Das Gemisch wird mit Essigester extrahiert, die vereinigten organischen Phasen über Natriumsulfat getrocknet, filtriert und eingeengt. Der Rückstand wird über Kieselgel mit n-Hexan/Essigester (3/1) chromatographiert. Die reinen Fraktionen werden vereinigt und das Lösungsmittel abgedampft. Der Rückstand wird mit Methylenchlorid (30 ml) verdünnt, mit Pyridin (4.0 ml; 49.5 mmol) und Trifluoressigsäureanhydrid (3.44 ml; 24.7mmol) versetzt und eine Stunde bei Raumtemperatur gerührt. Die Reaktionslösung wird aufwässrige Salzsäure (1M; 50 ml) gegossen und zweimal mit Ethylacetat (50 ml) extrahiert. Die vereinigten organischen Phasen werden über Natriumsulfat getrocknet, filtriert und eingeengt. Der Rückstand wird über Kieselgel mit n-Hexan/Ethylacetat (9/1) chromatographiert . Die reinen Fraktionen werden vereinigt und das Lösungsmittel abgedampft.
Ausbeute: 5.40 g (98%) als weisser Feststoff.
MS: 333 (M)

(4u) 4'-(2,4-Diamino-pyrimidin-5-ylmethyl)-2',6'-diethoxy-biphenyl-3-carbaldehyd

Ausgehend von 3-Brombenzaldehyd (515 mg; 3 mmol), Bis(pinacolato)diboron (1.14 g ; 4.5 mmol), Kaliumacetat (883 mg; 9 mmol), Bis(triphenylphosphin)palladium-(II)dichlorid (142 mg; 0,2 mmol) und von 5-(3,5-Diethoxy-4-iod-benzyl)-pyrimidin-2,4-diamin (Beispiel 1) (931 mg; 2.25 mmol) werden 838 mg (95 %) 4'-(2,4-Diaminopyrimidin-5-ylmethyl)-2',6'-diethoxy-biphenyl-3-carbaldehyd als hellgelbes Pulver erhalten.
MS (ISP): 393.2 $(M+H)^+$.

(4v) 5-{2,6-Diethoxy-4'-[(2,2,2-trifluorethylamino)-methyl]-biphenyl-4-ylmethyl}pyrimidin-2,4-diamin

Eine Lösung von 2,2,2-Trifluorethylamin (0.477ml; 6 mMol) in 4 ml Methanol wird mit Eisessig auf pH 6 eingestellt und mit 4'-(2,4-Diamino-pyrimidin-5-ylmethyl)-2',6'diethoxy-biphenyl-4-carbaldehyd (392 mg; 1 mmol) versetzt. Anschliessend wird das Reaktionsgemisch auf 0°C abgekühlt und mit Natriumcyanborhydrid (38 mg; 0.6 mMol) versetzt. Nach einer halben Stunde bei 0°C wird das Gemisch 3 Std. bei Raumtemperatur gerührt. Nach Eindampfen des Reaktionsgemisches wird der Rückstand in 20 ml Wasser verrührt und mit 1N wässriger Natronlauge auf pH 8 eingestellt. Die ausgefallene Substanz wird abgenutscht und am Hochvakuum getrocknet. Der Rückstand wird in 20 ml Methylenchlorid verrührt und wieder abgenutscht und getrocknet. Man erhält 105 mg (22%) 5-{2,6-Diethoxy-4'-[(2,2,2-trifluor-ethylamino)-methyl]-biphenyl-4-ylmethyl}pyrimidin-2,4-diamin als farbloses Pulver.
MS (ISP): 476.2 $(M+H)^+$
Smp. 146-149°C.

(4w) 1-[4'-(2,4-Diamino-pyrimidin-5-ylmethyl)-2',6'-diethoxy-biphenyl-4-ylmethyl]-pyrazolidin-3-on

3-Pyrazolidinon-hydrochlorid (123 mg; 1mmol) wird in 2.5 ml Wasser gelöst und mit Natrium hydrogencarbonat (84 mg; 1 mmol) versetzt und während 15 Min. gerührt. Diese Lösung wird mit einer Lösung von 4'-(2,4-Diamino-pyrimidin-5-ylmethyl)-2',6'diethoxy-biphenyl-4-carbaldehyd (392 mg; 1 mmol) in 2.5 ml Methanol versetzt und das Reaktionsgemisch 24 Std. bei 80°C erhitzt. Anschliessend wird das Raktionsgemisch auf Raumtemperatur abgekühlt und der Niederschlag abgenutscht, mit 5 ml Wasser gewaschen und getrocknet. Der rohe Rückstand (360 mg) wird

in 10 ml Methanol gelöst, mit Platindioxyd (10 mg; 0.044 mMol) verstetzt und anschliessend bei Raumtemperatur und normalem Wasserstoffdruck hydriert. Nach 48 Std. wird das Reaktionsgemisch filtriert. Das Filtrat wird eingeengt und der Rückstand über Kieselgel (50 g) mit Methylenchlorid /Methanol (4/1) chromatographiert. Die reinen Fraktionen werden vereinig, eingeengt und am Hochvakuum getrocknet.
Ausbeute: 74 mg (21 %) 1-[4'-(2,4-Diamino-pyrimidin-5-ylmethyl)-2',6'-diethoxybiphenyl-4-ylmethyl]-pyrazolidin-3-on als farbloses Pulver.
MS (EI): 463.3 ($M^+$)
Smp. 102-105°C

(4x) 5-[3,5-Diethoxy-4-(2-morpholin-4-ylmethyl-pyrimidin-5-yl)-benzyl]-pyrimidin-2,4-diamin

In Analogie zu Beispiel (4a) ausgehend von 4-(5-Brom-pyrimidin-2-ylmethyl)-morpholin (500 mg; 1.94 mmol), ), Bis(pinacolato)diboron (739 mg; 2.9 mmol), Kaliumacetat (572 mg; 9 mmol), Bis(triphenylphosphin)palladium (II)dichlorid (91 mg; 0,13 mmol) und von 5-(3,5-Diethoxy-4-iod-benzyl)-pyrimidin-2,4-diamin (Beispiel 1) (600 mg; 1.45 mmol) werden 112 mg (17 %) 5-[3,5-Diethoxy-4-(2-morpholin-4-ylmethyl-pyrimidin-5-yl)-benzyl]-pyrimidin-2,4-diamin als hellgelbes Pulver erhalten.
MS (ISP): 466.3 $(M+H)^+$
Smp. 200-206°C (Zers.)

(4y) 5-(2,6-Diethoxy-4'-pyrrolidin-1-ylmethyl-biphenyl-4-ylmethyl)-pyrimidin-2,4-diamin

In Analogie zu Beispiel (4v) erhält man aus 4'-(2,4-Diamino-pyrimidin-5-ylmethyl)-2',6'-diethoxy-biphenyl-4-carbaldehyd (392 mg; 1 mmol) und Pyrrolidin (427 mg; 6 mmol) 124 mg (28%) 5-(2,6-Diethoxy-4'-pyrrolidin-1-ylmethyl-biphenyl-4-ylmethyl)-pyrimidin-2,4-diamin als farbloses Pulver.
MS (ISP): 448.4 $(M+H)^+$
Smp. 144-146°C.

(4z) 5-(2,6-Diethoxy-3'-fluor-4'-morpholin-4-ylmethyl-biphenyl-4-ylmethyl)-pyrimidin-2,4-diamin

In Analogie zu Beispiel (4a) ausgehend von 4-(4-Brom-2-fluor-benzyl)-morpholin (274 mg; 1 mmol), Bis(pinacolato)diboron (380 mg ; 1.5 mmol), Kaliumacetat (294 mg; 3 mmol), Bis(triphenylphosphin)palladium(II)dichlorid (47 mg; 0,07 mmol) und von 5-(3,5-Diethoxy-4-iod-benzyl)-pyrimidin-2,4-diamin (Beispiel 1) (248 mg; 0.6 mmol) werden 166 mg (34 %) 5-(2,6-Diethoxy-3'-fluor-4'-morpholin-4-ylmethyl-biphenyl-4-ylmethyl)-pyrimidin-2,4-diamin als hellgelbes Pulver erhalten.
MS (ISP): 482.4 $(M+H)^+$
Smp. 113-116°C (Zers.)

(4aa) 5-(2,6-Diethoxy-3'-fluor-4'-pyrrolidin-1-ylmethyl-biphenyl-4-ylmethyl)-pyrimidin-2,4-diamin

In Analogie zu Beispiel (4v) erhält man aus 4'-(2,4-Diamino-pyrimidin-5-ylmethyl)-2',6'-diethoxy-3-fluor-biphenyl-4-carbaldehyd (450 mg; 1.1 mmol) und Pyrrolidin (467 mg; 6.6 mmol) 281 mg (55%) 5-(2,6-Diethoxy-3'-fluor-4'-pyrrolidin-1-ylmethylbiphenyl-4-ylmethyl)-pyrimidin-2,4-diamin als farbloses Pulver.
MS (ISP): 466.3 $(M+H)^+$
Smp. 131-134°C.

(4ab) 5-(2,6-Diethoxy-4'-methylamino-biphenyl-4-ylmethyl)-pyrimidin-2,4-diamin

N-(4-Bromphenyl)-2,2,2-trifluor-N-methyl-acetamid (1,32 g; 5,2 mmol), Bis(pinacolato)diboron (1,57 g; 14,1 mmol), Kaliumacetat (1,38 g; 14,1 mmol) und 1,1'-Bis(diphenylphosphino)ferrocendichlorpalladium (II) ($PdCl_2$(dppf)) (228 mg; 0,29 mmol) werden unter Argonbegasung in Dimethoxyethan (47 ml; über Molekularsieb getrocknet) bei 80°C Badtemperatur während 17 Stunden gerührt. Nach Abkühlen auf Raumtemperatur werden erneut Bis(pinacolato) diboron (250 mg; 0,995 mmol) und Bis(diphenylphosphino)ferrocendichlorpalladium (II) ($PdCl_2$(dppf)) (31 mg; 0,039 mmol) zugegeben und erneut unter Argon 1 Stunde bei 80°C Badtemperatur gerührt. Nach Abkühlen auf Raumtemperatur werden Dimethylformamid (95 ml), Tetrakis-triphenylphosphin-palladium (416 mg; 0,36 mmol), 5-(3,5-Diethoxy-4-iod-benzyl)-pyrimidin-2,4-diamin (Beispiel 1) (1,56 g; 3,76 mmol) und eine 2M wässrige Kaliumphosphat-Lösung (18,8 ml) zugegeben. Das Reaktionsgemisch wird 7 Stunden bei 80°C gerührt, auf Raumtemperatur abgekühlt und eingeengt. Der Rückstand wird mit Wasser (300 ml) versetzt und das ausgefallene Produkt abgenutscht. Der klebrige Nutschkuchen wird in Methanol gelöst und abgedampft. Es wird zweimal mit Methanol azeotropiert und am Hochvakuum getrocknet. Das erhaltene Rohprodukt wird zweimal über Kieselgel (125 g) mit Methylenchlorid/Methanol/$NH_4OH$ konz. 19/1/0,05 chromatographiert. Die reinen Fraktionen werden vereinigt, das Lösungsmittel eingeengt, und der Rückstand mit Methanol (10 ml) verrührt, abgenutscht und am Hochvakuum getrocknet. Es werden 570 mg (26 %) 5-(2,6-Diethoxy-4'-methylamino-biphenyl-4-ylmethyl)-pyrimidin-2,4-diamin als leicht gelbes Pulver erhalten.
MS(ISP): 394,4 $(M+H)^+$

(4ac) 5-(2,6-Diethoxy-4'-{[N-(2-fluor-ethyl)-N-methyl-amino]-methyl}-biphenyl-4-ylmethyl)-pyrimidin-2,4-diamin

Aus 5-{2,6-Diethoxy-4'-[(2-fluor-ethylamino)-methyl]-biphenyl-4-ylmethyl}pyrimidin-2,4-diamin (213 mg; 0.48 mmol) erhält man nach Behandlung mit 0.1 ml 35% wässrigem Formaldehyd und Reduktion mit Natriumborhydrid (18 mg; 0.48 mmol) 87mg (39%) 5-(2,6-Diethoxy-4'-{[N-(2-fluor-ethyl)-N-methyl-amino}-methyl]-biphenyl-4-ylmethyl)-pyrimidin-2,4-diamin als farbloses Pulver.
MS (ISP): 454.5 $(M+H)^+$
Smp. 145-148°C .

(4ad) 5-(2,6-Diethoxy-3'-fluor-4'-{[N-(2-fluor-ethyl)-N-methyl-amino]-methyl}biphenyl-4-ylmethyl)-pyrimidin-2,4-diamin

Aus 5-{2,6-Diethoxy-3'-fluor-4'-[(2-fluor-ethylamino)-methyl]-biphenyl-4-ylmethyl}-pyrimidin-2,4-diamin (Beispiel (4ah)) (145 mg; 0.32 mmol) erhält man nach Behandlung mit 0.1 ml einer 35% wässrigen Formaldehyd-Lösung und Reduktion mit Natriumborhydrid (12 mg; 0.32 mmol) 134 mg (89%) 5-(2,6-Diethoxy-3'-fluor-4'-{[N-(2-fluor-ethyl) -N-methyl-amino]-methyl]-biphenyl-4-ylmethyl)-pyrimidin-2,4-diamin als farbloses Pulver.
MS (ISP): 472.3 $(M+H)^+$
Smp. 127-129°C.

(4ae) 5-(5'-Amino-2,6-diethoxy-3'-methyl-biphenyl-4-ylmethyl)-pyrimidin-2,4-diamin

Ausgehend von N-(5-Brom-3-methyl-phenyl)-2,2,2-trifluoracetamid (600 mg; 1.6 mmol), Bis(pinacolato)diboron (608mg; 2.39 mmol), Kaliumacetat (470 mg; 4.79mmol) und (Diphenylphosphino)-dichlorpalladium (II) (67 mg; 0,10 mmol) in Dioxan (10 ml) wird das Reaktionsgemisch auf Wasser gegossen und mit Ethylacetat extrahiert. Die vereinigten organischen Phasen werden mit einer gesättigten wässrigen Kochsalzlösung gewaschen, über Magnesiumsulfat getrocknet, abgenutscht, eingeengt und direkt in den nächsten Schritt eingesetzt. Nach Umsetzung mit 5-(3,5-Diethoxy-4-iod-benzyl)-pyrimidin-2,4-diamin (Beispiel 1) (330 mg; 1.26 mmol), Tetrakis-triphenylphosphin-palladium (55 mg; 0.05 mmol) und wässriger 2M Natriumcarbonat-Lösung (4 ml; 8 mmol) in einem 5/1 Dimethoxyethan/ Ethanol-Gemisch (30 ml) werden 102 mg (32 %) 5-(5'-Amino-2,6-diethoxy-3'-methyl-biphenyl-4-ylmethyl)-pyrimidin-2,4-diamin als gelber Schaum erhalten.
MS (ISP): 394,4$(M+H)^+$

(4af) 4'-(2,4-Diamino-pyrimidin-5-ylmethyl)-2',6'-diethoxy-3-fluor-biphenyl-4-carbaldehyd

In Analogie zu Beispiel (4a) erhält man ausgehend von 4-Brom-2-fluor-benzaldehyd (406 mg; 2 mmol), ), Bis (pinacolato)diboron (762 mg ; 3 mmol), Kaliumacetat (589 mg; 6 mmol), Bis(triphenylphosphin) palladium(II)dichlorid (94 mg; 0,13 mmol) und von 5-(3,5-Diethoxy-4-iod-benzyl)-pyrimidin-2,4-diamin (Beispiel 1) (414 mg; 1 mmol) 132 mg (32 %) 4'-(2,4-Diamino-pyrimidin-5-ylmethyl)-2',6'-diethoxy-3-fluor-biphenyl-4-carbaldehyd als hellgelbes Pulver.
MS (ISP): 411.3 $(M+H)^+$.

4ag) 5-{2,6-Diethoxy-4'-[(2-fluor-ethylamino)-methyl]-biphenyl-4-ylmethyl}-pyrimidin-2,4-diamin

In Analogie zu Beispiel (4v) erhält man aus 4'-(2,4-Diamino-pyrimidin-5-ylmethyl)-2',6'-diethoxy-biphenyl-4-carbaldehyd (392 mg; 1 mmol) und 2-Fluorethylamin-hydrochlorid (597 mg; 6 mmol) 170 mg (38%) 5-{2,6-Diethoxy-4'-[(2-fluor-ethylamino)-methyl]-biphenyl-4-ylmethyl}-pyrimidin-2,4-diamin als farbloses Pulver.
MS (ISP): 440.5 $(M+H)^+$
Smp. 150-153°C .

(4ah) 5-{2,6-Diethoxy-3'-fluor-4'-[(2-fluor-ethylamino)-methyl]-biphenyl-4-ylmethyl}pyrimidin-2,4-diamin

In Analogie zu Beispiel (4v) erhält man aus 4'-(2,4-Diamino-pyrimidin-5-ylmethyl)-2',6'-diethoxy-3-fluor-biphenyl-4-carbaldehyd (410 mg; 1 mmol) und 2-Fluorethylamin-hydrochlorid (597 mg; 6 mMol) 237 mg (52%) 5-{2,6-Diethoxy-3'-fluor-4'-[(2-fluorethylamino)-methyl]-biphenyl-4-ylmethyl}-pyrimidin-2,4-diamin als farbloses Pulver.

MS (ISP): 458.5 $(M+H)^+$
Smp. 151-154°C.

Beispiel 5

Herstellung von Verbindungen der Formel III aus Edukten der Formel (II), Schema 1, Methode A (Suzuki-Kopplung mit $R^2$-$B(OH)_2$)

(5a) 5-(2,6-Diethoxy-3'-methoxy-biphenyl-4-ylmethyl)-pyrimidin-2,4-diamin

Tetrakis-triphenylphosphin-palladium (57 mg; 0,050 mmol) wird unter Argonbegasung in Dimethoxyethan (1 ml) suspendiert und mit 2,4-Diamino-5-(4-brom-3,5-diethoxybenzyl)pyrimidin (367 mg; 1 mmol) in Dimethoxyethan (6 ml) versetzt. Nach viertelstündigem Rühren bei Raumtemperatur wird 3-Methoxy-phenylboronsäure (228 mg; 1,5 mmol) in Ethanol (1,5 ml) zugegeben, weitere 5 Minuten bei Raumtemperatur gerührt, eine wässrige 2M Natriumcarbonat-Lösung (4,2 ml; 8,5 mmol) hinzugefügt und anschliessend 2,5 Stunden am Rückfluss gekocht (Badtemperatur 85°C). Das Reaktionsgemisch wird eingeengt und der Rückstand mit Wasser und etwas Methanol verrührt und abgenutscht. Der Nutschkuchen wird mit Wasser verrührt, abgenutscht und am Hochvakuum getrocknet.
Ausbeute: 340 mg (86 %)5-(2,6-Diethoxy-3'-methoxy-biphenyl-4-ylmethyl)-pyrimidin-2,4-diamin als graues Pulver.
MS (ISP): 395,4 $(M+H)^+$

In Analogie hierzu werden hergestellt:

(5b) 5-(2,6-Diethoxy-4'-methoxy-biphenyl-4-ylmethyl)-pyrimidin-2,4-diamin

Ausgehend von 2,4-Diamino-5-(4-brom-3,5-diethoxybenzyl)pyrimidin (367 mg; 1 mmol) und 4-Methoxy-phenylboronsäure (304 mg; 2,0 mmol) werden nach Chromatographie über Kieselgel (100 g) mit Methylenchlorid/Methanol/$NH_4OH$ konz. (19/1/0,05) 91 mg (23 %) 5-(2,6-Diethoxy-4'-methoxy-biphenyl-4-ylmethyl)-pyrimidin-2,4-diamin als leicht gelbliches Pulver erhalten.
MS (ISP): 395,5 $(M+H)^+$

(5c) N-[4'-(2,4-Diamino-pyrimidin-5-ylmethyl)-2',6'-diethoxy-biphenyl-3-yl]-acetamid

Ausgehend von von 2,4-Diamino-5-(4-brom-3,5-diethoxybenzyl)pyrimidin (367 mg; 1 mmol) und 4-Acetamido-phenylboronsäure (268 mg; 1,5 mmol) werden nach Verrühren mit Wasser und Umkristallisation aus heissem Methanol 158 mg (38 %) N-[4'-(2,4-Diamino-pyrimidin-5-ylmethyl)-2',6'-diethoxy-biphenyl-3-yl]-acetamid in Form eines leicht grauen Pulvers erhalten.
MS (ISP): 422,5 $(M+H)^+$

(5d) 4'-(2,4-Diamino-pyrimidin-5-ylmethyl)-2',6'-diethoxy-biphenyl-3-ol

Ausgehend von 2,4-Diamino-5-(4-brom-3,5-diethoxybenzyl)pyrimidin (735 mg; 2 mmol) und 3-Hydroxy -phenylboronsäure (413 mg; 3,0 mmol) werden nach aufeinanderfolgendem Verrühren mit Wasser und Methylenchlorid 634 mg (83 %) 4'-(2,4-Diamino-pyrimidin-5-ylmethyl)-2',6'-diethoxy-biphenyl-3-ol als beiges Pulver erhalten.
MS (ISP): 381,2 $(M+H)^+$

Beispiel 6

Herstellung der Verbindung der Formel IV, Schema 1, worin $R^1$ Ethoxy bedeutet. 5-(3-Benzyloxy-5-ethoxy-4-iod-benzyl)-pyrimidin-2,4-diamin (Schlüsselzwischenprodukt):

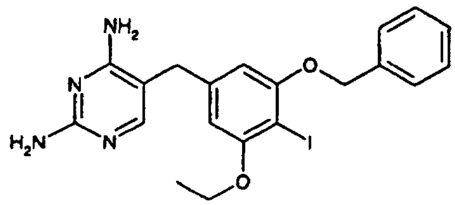

Diese Verbindung wird durch folgende Reaktionssequenz (Stufen a)-f)) hergestellt:

Stufe a) 3-Benzyloxy-5-hydroxy-4-iod-benzesäure-methylester

3,5-Dihydroxy-4-iod-benzoesäure-methylester (Beispiel 1, Stufe b) (1000 g; 3,4 Mol) wird in Dimethylformamid (9l) unter Argon vorgelegt und mit Benzylchlorid (455 ml; 3,95 Mol) versetzt. Das Reaktionsgemisch wird dann auf 0-5°C abgekühlt und mit Kalium-tert-butylat (840 g; 7,48 Mol) in Dimethylformamid (3,65l) zwischen 0-5°C innerhalb 1 Stunde versetzt. Nach einstündigem Rühren bei dieser Temperatur wird das Reaktionsgemisch auf Wasser gegossen und der pH durch Zugabe einer 1N wässrigen Salzsäure-Lösung (ca 41) auf 5 eingestellt. Anschliessend wird mit Ethylacetat (15 l) extrahiert, die organische Phase mit einer Kochsalzlösung (1 kg Kochsalz in 10 l Wasser) gewaschen, über Natriumsulfat (1 kg) getrocknet und filtriert. Die erste wässrige Phase wird mit Ethylacetat (10 l) nachextrahiert, über Natriumsulfat getrocknet und filtriert. Die organischen Phasen werden vereinigt und eingeengt, in Toluol (5 l) aufgenommen und über Kieselgel (8 kg) mit Toluol (160 l) Chromatographiert. Die reinen Fraktionen werden bis auf ein Volumen von ca. 5 l konzentriert und unter Rühren auf 0-5°C abgekühlt. Die ausgefallenen Kristalle werden nach 2-stündigem Rühren abgenutscht, mit 1 l kaltem Toluol nachgewaschen und am Hochvakuum getrocknet.
Ausbeute: 812 g (63 %) 3-Benzyloxy-5-hydroxy-4-iod-benzoesäure-methylester als farblose Kristalle.
Smp: 131 °C

Stufe b) 3-Benryloxy-5-ethoxy-4-iod-benzoesäure-methylester

3-Benzyloxy-5-hydroxy-4-iod-benzoesäure-methylester (801 g; 2,085 Mol) wird in Aceton (8,91) gelöst und mit Kaliumcarbonat (344 g; 2,49 Mol) versetzt. Danach wird Ethyliodid (206 ml; 2,55 Mol) zugegeben und das Reaktionsgemisch unter Rückfluss 16 Stunden gerührt. Nach Abkühlen auf Raumtemperatur werden die Salze abgenutscht und eingeengt. Das erhaltene Rohprodukt wird in Methylenchlorid (5 l) aufgenommen und auf Wasser (5 l) gegossen, die organische Phase abgetrennt und mit Wasser (5 l) nachgewaschen. Die wässrigen Phasen werden mit Methylenchlorid (1 l) nachextrahiert und die vereinigten organischen Phasen eingeeengt und aus Ethylacetat bei 0-5°C kristallisiert. Die Kristalle werden abgenutscht, mit kaltem Ethylacetat (2 x 0,5 l) gewaschen und am Hochvakuum getrocknet.
Ausbeute: 787 g (93 %) 3-Benzyloxy-5-ethoxy-4-iod-benzoesäure-methylester als farbloses Pulver.
Smp: 126°C

Stufe c) (3-Benzyloxy-5-ethoxy-4-iod-phenyl)-methanol

3-Benzyloxy-5-ethoxy-4-iod-benzoesäure-methylester (780 g; 1,892) wird unter Argon in Tetrahydrofuran (4,7l) vorgelegt und auf 0-5°C abgekühlt. Eine 1,2 M Diisobutylaluminiumhydrid Lösung (DIBAH) in Toluol (3,91; 4,68 Mol) wird dann so zugetropft, das die Temperatur nicht über 0-5°C steigt (ca. 75 Minuten). Nach 1 Stunde wird das Kühlbad entfernt und das Reaktionsgemisch unter heftigem Rühren auf eine 1 M wässrige Zitronensäure-Lösung (1732 g Zitronensäure in 9 l Wasser) innerhalb 2 Stunden gegossen. Nach Abtrennung der wässrigen Phase wird die organische Phase mit Wasser (9 l) nachgewaschen und eingedampft. Der Rückstand wird in Methylenchlorid (1,5 l) gelöst und dann durch Abdestillieren langsam mit n-Hexan (3,71) ersetzt. Die Suspension wird dann auf 0°C abgekühlt und nach 1 Stunde bei dieser Temperatur abgenutscht, mit n-Hexan (0,5 l) nachgewaschen und am Hochvakuum getrocknet.
Ausbeute: 716 g (98 %) (3-Benzyloxy-5-ethoxy-4-iod-phenyl)-methanol als farbloses Pulver.

Smp: 85°C

Stufe d) 3-Benzyloxy-5-ethoxy-4-iod-benzaldehyd

(3-Benzyloxy-5-ethoxy-4-iod-phenyl)-methanol (710 g; 1,848 Mol) wird unter Argon in Methylenchlorid (7,11) gelöst und mit Mangan(IV)-oxid (919 g; 10,57 Mol) versetzt. Die Suspension wird 17 Stunden unter Rückfluss gerührt, auf Raumtemperatur abgekühlt, filtriert und mit Methylenchlorid (5 l) nachgewaschen. Das Lösungsmittel wird dann durch Abdestillieren mit n-Hexan (3,51) ersetzt und anschliessend unter Rühren auf 0-5°C gekühlt. Nach 1 Stunde wird die Suspension abgenutscht, die Kristalle mit n-Hexan (2 x 11) gewaschen und am Hochvakuum getrocknet.
Ausbeute: 656 g (93 %) 3-Benzyloxy-5-ethoxy-4-iod-benzaldehyd als hellgelbes Pulver.
Smp: 104°C

Stufe e) (Z/E)-2-(3-Benzyloxy-5-ethoxy-4-iod-benzyl)-3-phenylamino-acrylonitril

3-Benzyloxy-5-ethoxy-4-iod-benzaldehyd (650 g; 1,7 Mol) wird unter Argon in Dimethylsulfoxid (510 ml) und tert-butanol (1300 ml) vorgelegt, mit 3-Anilinopropionitril (300 g; 2,02 Mol) versetzt und mit Ölbad auf 25-30°C erwärmt. Danach wird Kalium-tert-butylat (231 g; 1,99 Mol) in Dimethylsulfoxid (790 ml) innerhalb 15 Minuten zugetropft und anschliessend die entstandene Lösung auf 60°C innerhalb 1 Stunde erwärmt. Nach 45 Minuten bei dieser Temperatur wird der Ansatz eingedampft, das erhaltene Öl in Methylenchlorid (7 l) gelöst und mit einer gesättigten wässrigen Kochsalz-Lösung (7 1) gewaschen. Die Wasserphase wird mit Methylenchlorid (2 1) nachextrahiert und die vereinigten organischen Phasen am Rollverdampfer eingedampft. Beim Aufkonzentrieren wird Methanol (5 l) zugegeben. Die entstandene Suspension wird auf 0-5°C abgekühlt und nach einstündigem Rühren bei dieser Temperatur abgenutscht, mit kaltem Methanol (0,5 l) nachgewaschen und am Hochvakuum getrocknet.
Ausbeute: 409 g eines gelben Pulvers. Aus der Mutterlauge werden nach Filtration über Kieselgel (1400 g) und Eluieren mit Toluol (5 l) nochmals 372 g eines gelben Pulvers erhalten, das nach Kristallisation aus Methanol/Methylenchlorid (gleiche Methode wie oben) nochmals 93,8 g (gelbes Pulver) und anschliessend 84 g (beiges Pulver) Produkt liefert.
Gesamtausbeute: 576,3 g (67 %) (Z/E)-2-(3-Benzyloxy-5-ethoxy-4-iod-benzyl)-3-phenylamino-acrylonitril
MS (EI): 510 (M)

Stufe f) 5-(3-Benzyloxy-5-ethoxy-4-iod-benzyl)-pyrimidin-2,4-diamin

Guanidin-hydrochlorid (364 g; 3,73 Mol) wird unter Argon in Ethanol (4,3 l) gelöst und mit einer Lösung von Kalium-tert-butylat (465 g; 4,01 Mol) in Ethanol (2,81) versetzt. Nach ca 20 Minuten wird (Z/E)-2-(3-Benzyloxy-5-ethoxy-4-iod-benzyl)-3-phenylaminoacrylonitril (580,8 g; 1,11 Mol) zugegeben. Die entstandene Suspension wird 20 Stunden bei 70°C gerührt und mit Wasser (2100 ml) versetzt. Das Reaktionsgemisch wird anschliessend auf 0-5°C gekühlt und 1 Stunde bei dieser Temperatur gerührt. Die Suspension wird abgenutscht und der Nutschkuchen nacheinander mit Ethanol (2,8 l), Wasser (2,81) und n-Hexan (2,8 l) nachgewaschen und getrocknet. Das Rohprodukt wird aus Methanol/Methylenchlorid (8/2) kristallisiert.
Ausbeute: 470 g (88 %)
MS: 476 (M)

Beispiel 7

Herstellung von Verbindungen der Formel VII, Schema 1, aus den entsprechenden Verbindungen der Formel VI durch Alkylierung der freien Hydroxygruppe

(7a) 5-(3'-Amino-6-cyclopropylmethoxy-2-ethoxy-biphenyl-4-ylmethyl)-pyrimidin-2,4-diamin (Methode α: Alkylierung mit Bromid)

3'-Amino-4-(2,4-diamino-pyrimidin-5-ylmethyl)-6-ethoxy-biphenyl-2-ol (351 mg; 1 mmol) wird in Dimethylformamid (22,5 ml; über Molekularsieb getrocknet) unter Argonbegasung gelöst und mit Kalium-tert-butylat (172 mg; 1,5 mmol) bei Raumtemperatur versetzt. Nach einstündigem Rühren wird Brommethyl-cydopropan (0,124 ml; 1,3 mmol) hinzugefügt und 1 Stunde bei einer Badtemperatur von 60°C weitergerührt. Anschliessend wird das Reaktionsgemisch auf Raumtemperatur abgekühlt und eingeengt. Der Rückstand wird in Wasser/Ethylacetat aufgenommen, in einen Scheidetrichter gegossen und ausgeschüttelt. Die Wasserphase wird abgetrennt und einmal mit Ethylacetat nachextrahiert. Die vereinigten organischen Phasen werden mit einer wässrigen gesättigten Kochsalz-Lösung gewaschen, über Magnesiumsulfat getrocknet, abgenutscht und eingeengt. Der Rückstand wird über Kieselgel (21 g) mit Methylenchlorid/Methanol/$NH_4OH$ konz. (19/1/0,05) chromatographiert. Die reinen Fraktionen werden eingengt, mit

Diethylether (10 ml) 1 Stunde verrührt, abgenutscht, mit Diethylether nachgewaschen und am Hochvakuum getrocknet. Ausbeute: 203 mg (52 %) 5-(3'-Amino-6-cyclopropylmethoxy-2-ethoxy-biphenyl-4-ylmethyl)-pyrimidin-2,4-diamin als beiges Pulver.
MS(ISP): 406,4 $(M+H)^+$
NMR $^1H$: (250 MHz, $\delta$, TMS, DMSO): 0.15 (m; 2H); 0.39 (m. 2H); 1.00 (m; 1H); 1.12 (t; J=6.9; 3H); 3.55 (s; 2H); 3.68 (d; J=6.5; 2H); 3.87 (q; J=6.9; 2H); 4.85 (s; 2H); 5.69 (s; 2H); 6.10 (s; 2H); 6.39 (m; 3H); 6.56 (s; 2H); 6.93 (m; 1H); 7.55 (s; 1H).

In Analogie hierzu werden hergestellt:

(7b) {1-[3'-Amino-4-(2,4-diamino-pyrimidin-5-ylmethyl)-6-ethoxy-biphenyl-2-yloxymethyl]-cyclopropyl}-acetonitril (Methode $\alpha$)

Ausgehend von 3'-Amino-4-(2,4-diamino-pyrimidin-5-ylmethyl)-6-ethoxybiphenyl-2-ol (150 mg; 0,427 mmol) und (1-Brommethyl-cyclopropyl)-acetonitril (97 mg; 0,555 mmol) werden 121 mg (64 %) {1-[3'-Amino-4-(2,4-diamino-pyrimidin-5-ylmethyl)-6-ethoxy-biphenyl-2-yloxymethyl]-cyclopropyl}-acetonitril als hellgelbes Pulver erhalten.
MS (ISP): 445,4 $(M+H)^+$

Das eingesetzte (1-Brommethyl-cyclopropyl)-acetonitril wird wie folgt, ausgehend von (1-Hydroxymethyl-cyclopropyl)-acetonitril hergestellt:

(1-Hydroxymethyl-ryclopropyl)-acetonitril (1,29 g; 11,6 mmol) und Triphenylphosphin (3,08 g; 11,8 mmol) werden unter Rühren in Dimethylformamid (8 ml; über Molekularsieb getrocknet) gelöst und auf 0-5°C gekühlt. Anschliessend wird N-BromsuCinimid (2,1 g; 11,8 mmol) in Portionen innerhalb 40 Minuten zugegeben. Es wird dann 1,5 Stunden bei dieser Temperatur weitergerührt, Methanol (0,2 ml) und Wasser (8 ml) zugegeben und bei Raumtemperatur 10 Minuten gerührt. Anschliessend wird das Reaktionsgemisch mit n-Pentan (3 x 40 ml) extrahiert. Die vereinigten organischen Phasen werden mit einer 5 % wässrigen Natriumbicarbonat-Lösung (40 ml) gewaschen und eingeengt. Das erhaltene Öl wird ohne weitere Reinigung eingesetzt.
MS: 173 (M)

(7c) 5-[3'-Amino-6-ethoxy-2-[(1-methoxymethyl-cyclopropyl)-methoxy]-biphenyl-4-ylmethyl]-pyrimidin-2,4-diamin (Methode $\alpha$)

Ausgehend von 3'-Amino-4-(2,4-diamino-pyrimidin-5-ylmethyl)-6-ethoxybiphenyl-2-ol (527 mg; 1,5 mmol) und 1-Brommethyl-1-methoxymethylcyclopropan (350 mg; 1,97 mmol) werden 353 mg (52 %) 5-[3'-Amino-6-ethoxy-2-[(1-methoxymethylcyclopropyl)-methoxy]-biphenyl-4-ylmethyl]-pyrimidin-2,4-diamin als fester oranger Schaum erhalten.
MS (ISP): 450,4 $(M+H)^+$

Das eingesetzte 1-Brommethyl-1-methoxymethylcyclopropan wird wie folgt ausgehend von 1,1-Cyclopropandimethanol (Stufen a)-b)) hergestellt:

Stufe a) 1-Methoxymethyl-cyclopropyl)-methanol

1,1-Cyclopropandimethanol (1,02 g; 10 mmol) wird in Dimethylformamid (20 ml; über Molekularsieb getrocknet) gelöst und bei Raumtemperatur nach Zugabe von Kalium-tert-butylat (1,14 g; 10 mmol) 1 Stunde gerührt. Anschliessend wird das Reaktionsgemisch auf 0-5°C gekühlt und mit einer Lösung von Methyliodid (0,63 ml; 10 mmol) in Dimethylformamid (5 ml; über Molekularsieb getrocknet) tropfenweise versetzt. Nach 50-minütigem Rühren bei dieser Temperatur wird das Reaktionsgemisch auf ein Gemisch einer gesättigten wässrigen $NH_4Cl$-Lösung (100 ml) und Diethylether (400 ml) unter Rühren gegossen. Nach Trennung der Phasen wird die Wasserphase mit Diethylether (400 ml) nachextrahiert, die vereinigten organischen Phasen mit einer gesättigten wässrigen Kochsalz-Lösung (2 x 100 ml) gewaschen, über Magnesiumsulfat getrocknet, abgenutscht und eingeengt. Das erhaltene Rohprodukt wird über Kieselgel (50 g) mit Diethylether chromatographiert.
Ausbeute: 493 mg (39 %) (1-Methoxymethyl-cyclopropyl)-methanol als hellgelbes Öl. NMR (1H, 250 MHz in $CDCl_3$) in ppm: 0,5-0,6 (Multiplet, 4H); 3,37 (Multiplet, 5H); 3,54 (s, 2H)

Stufe b) 1-Brommethyl-1-methoxymethylcyclopropan

Wird analog zu (1-Brommethyl-cyclopropyl)-acetonitril (Beispiel 7b) hergestellt. Ausgehend von (1-Methoxymethyl-cyclopropyl)-methanol (490 mg; 4,2 mmol) werden 341 mg (45 %) 1-Brommethyl-1-methoxymethylcyclopropan als farbloses Öl erhalten. NMR (1H, 250 MHz in $CDCl_3$) in ppm: 0,6-0,8 (Multiplet, 4H); 3,35 (s, 2H); 3,38 (s, 3H); 3,49 (s, 2H)

(7d) 5-(3'-Amino-6-cyclopentyloxy-2-ethoxy-biphenyl-4-ylmethyl)-pyrimidin-2,4-diamin (Methode α)

Ausgehend von 3'-Amino-4-(2,4-diamino-pyrimidin-5-ylmethyl)-6-ethoxybiphenyl-2-ol (176 mg; 0,5 mmol) und Brom-cyclopentan(0,121 ml; 1,21 mmol) werden 92 mg (44 %) 5-(3'-Amino-6-cyclopentyloxy-2-ethoxy-biphenyl-4-ylmethyl)-pyrimidin-2,4-diamin als fester gelber Schaum erhalten.
MS (ISP): 420,3 $(M+H)^+$

(7e) 5-(3'-Amino-6-cyclobutoxy-2-ethoxy-biphenyl-4-ylmethyl)-pyrimidin-2,4-diamin (Methode α)

Ausgehend von 3'-Amino-4-(2,4-diamino-pyrimidin-5-ylmethyl)-6-ethoxybiphenyl-2-ol (176 mg; 0,5 mmol) und Brom-cyclobutan(0,061 ml; 0,65 mmol) werden 71 mg (35 %) 5-(3'-Amino-6-cyclobutoxy-2-ethoxy-biphenyl-4-ylmethyl)-pyrimidin-2,4-diamin als fester gelber Schaum erhalten.
MS (ISP): 406,4 $(M+H)^+$

(7f) (E)-5-[3'-Amino-4-(2,4-diamino-pyrimidin-5-ylmethyl)-6-ethoxy-biphenyl-2-yloxy]-4,4-dimethyl-pent-2-ennitril (Methode α)

Ausgehend von 3'-Amino-4-(2,4-diamino-pyrimidin-5-ylmethyl)-6-ethoxybiphenyl-2-ol (351 mg; 1,0 mmol) und (E)-5-Brom-4,4-dimethyl-pent-2-ennitril (245 mg; 1,3 mmol) werden 42 mg (9 %) (E)- und/oder (Z)-5-[3'-Amino-4-(2,4-diaminopyrimidin-5-ylmethyl)-6-ethoxy-biphenyl-2-yloxy]-4,4-dimethyl-pent-2-ennitril als fester gelber Schaum erhalten.
MS (ISP): 459,5 $(M+H)^+$

Das eingesetzte (E)-5-Brom-4,4-dimethyl-pent-2-ennitril wird wie folgt hergestellt:

1-Brom-2,2-dimethyl-3-propanal (710 mg; 4,3 mmol) und (Triphenylphosphoranyliden)acetonitril (1,4 g; 4,73 mmol) werden in Acetonitril (20 ml) und Dimethylformamid (5 ml) gelöst und 18 Stunden bei Raumtemperatur gerührt. Das Reaktionsgemisch wird eingeengt, mit n-Hexan verrührt, vom ausgefallenen Triphenylphosphinoxid abgenutscht, das Filtrat eingeengt und mittels Kugelrohrdestillation gereinigt.
Ausbeute: 312 mg (39 %) (E)-5-Brom-4,4-dimethyl-pent-2-ennitril als farbloses Öl.
MS: 187 (M); 188 $(M+H)^+$

(7g) 5-[3'-Amino-6-ethoxy-2-(2-pyridin-2-yl-ethoxy)-biphenyl-4-ylmethyl]-pyrimidin-2,4-diamin (Methode β: Alkylierung mit Mesylat)

Wird in Analogie zu Beispiel (7i) nachstehend, hergestellt.

Ausgehend von 3'-Amino-4-(2,4-diamino-pyrimidin-5-ylmethyl)-6-ethoxybiphenyl-2-ol (702 mg; 2,0 mmol), 2-(2-Pyridyl)ethylmesylat (dreimal je 402 mg; 2 mmol) und Kaliumcarbonat (dreimal je 414 mg; 3,0 mmol) in siedendem Acetonitril (80 ml) werden 100 mg (11 %) 5-[3'-Amino-6-ethoxy-2-(2-pyridin-2-yl-ethoxy)-biphenyl-4-ylmethyl]-pyrimidin-2,4-diamin als beiges Pulver erhalten.
MS (ISP)): 457,4 $(M+H)^+$

(7h) (RS)-5-[3'-Amino-6-ethoxy-2-[2-(tetrahydro-pyran-2-yl)-ethoxy]-biphenyl-4-ylmethyl]-pyrimidin-2,4-diamin (Methode α)

Ausgehend von 3'-Amino-4-(2,4-diamino-pyrimidin-5-ylmethyl)-6-ethoxybiphenyl-2-ol (211 mg; 0,6 mmol) und (Tetrahydro-pyran-2-yl)-ethylbromid (151 mg; 0,78 mmol) werden 194 mg (70 %) (RS)-5-[3'-Amino-6-ethoxy-2-[2-(tetrahydro-pyran-2-yl)-ethoxy]-biphenyl-4-ylmethyl]-pyrimidin-2,4-diamin als fester gelber Schaum erhalten.
MS (ISP): 464,3 $(M+H)^+$

(7i) 5-[3'-Amino-6-ethoxy-2-(2,2,2-trifluor-ethoxy)-biphenyl-4-ylmethyl]-pyrimidin-2,4-diamin (Methode β)

3'-Amino-4-(2,4-diamino-pyrimidin-5-ylmethyl)-6-ethoxy-biphenyl-2-ol (527 mg; 1,5 mmol) wird in Acetonitril (60 ml; über Molekularsieb getrocknet) suspendiert, mit Kaliumcarbonat (621 mg; 4,5 mmol) versetzt und auf 100°C Badtemperatur erhitzt. Es wird 2,2,2-Trifluorethylmethansulfonat (0,353 ml; 3,0 mmol) hinzugefügt und 3 Stunden am Rückfluss gekocht. Nach dieser Zeit werden erneut Kaliumcarbonat (414 mg; 3 mmol) und 2,2,2-Trifluorethylmethansulfonat (0,353 ml; 3,0 mmol) zugegeben, und weitere 2 Stunden am Rückfluss gerührt. Das selbe wird noch viermal mit zweistündigem Abstand wiederholt. Anschliessend wird die Suspension abgenutscht und eingeengt. Der Rückstand wird über Kieselgel (120 g) mit Methylenchlorid/Methanol/$NH_4OH$ (1/1 Gemisch von 90/10/1 und 19/1/0,05) chromatographiert.

Ausbeute: 33 mg (5 %) 5-[3'-Amino-6-ethoxy-2-(2,2,2-trifluor-ethoxy)-biphenyl-4-ylmethyl]-pyrimidin-2,4-diamin als farbloses Pulver.
MS (ISP)): 434,4 $(M+H)^+$

Bei diesem Chromatographieverfahren erhält man auch als zweite Komponente das Endprodukt von Beispiel 14a.

(7j) 5-{3'-Amino-2-ethoxy-6-[2-(tetrahydro-pyran-4-yl)-ethoxy]-biphenyl-4-ylmethyl}pyrimidin-2,4-diamin (Methode $\alpha$)

Ausgehend von 3'-Amino-4-(2,4-diamino-pyrimidin-5-ylmethyl)-6-ethoxybiphenyl-2-ol (211 mg; 0,6 mmol) und 4-(2-Bromethyl)-tetrahydropyran(151 mg; 0,78 mmol) werden 166 mg (60 %) 5-{3'-Amino-2-ethoxy-6-[2-(tetrahydropyran-4-yl)-ethoxy]-biphenyl-4-ylmethyl}-pyrimidin-2,4-diamin als fester gelber Schaum erhalten.
MS (ISP): 464,3 $(M+H)^+$

Die Ausgangsverbindung 3'-Amino-4-(2,4-diamino-pyrimidin-5-ylmethyl)-6-ethoxy-biphenyl-2-ol wird aus von 5-(3-Benzyloxy-5-ethoxy-4-iod-benzyl)-pyrimidin-2,4-diamin (Beispiel 6) in 2 Stufen a) und b)) wie in Schema 1, (IV) -> (V) -> (VI) hergestellt:

Stufe a) 5-(3'-Amino-2-benzyloxy-6-ethoxy-biphenyl-4-ylmethyl)-pyrimidin-2,4-diamin, wird in Analogie zu Beispiel (2a) (Suzuki-Kopplung mit Boronsäure) hergestellt.

Ausgehend von 5-(3-Benzyloxy-5-ethoxy-4-iod-benzyl)-pyrimidin-2,4-diamin (Beispiel 6) (25,76 g; 54 mmol) und 3-Amino-phenylboronsäure-monohydrat (12,8 g; 81,1 mmol) werden 21,3 g (82 %) 5-(3'-Amino-2-benzyloxy-6-ethoxy-biphenyl-4-ylmethyl)-pyrimidin-2,4-diamin als farbloser Schaum erhalten.
MS: 441 (M)

Stufe b) 3'-Amino-4-(2,4-diamino-pyrimidin-5-ylmethyl)-6-ethoxy-biphenyl-2-ol

5-(3'-Amino-2-benzyloxy-6-ethoxy-biphenyl-4-ylmethyl)-pyrimidin-2,4-diamin (17,51 g; 39,7 mmol) wird in Ethanol (66 ml) und Essigsäure konz. (198 ml) über Pd/C 10% (8,1 g) 20 Stunden hydriert. Der Katalysator wird abfiltriert und die Mutterlauge eingeengt. Der Rückstand wird in Wasser aufgenommen und das pH durch Zugabe von $NH_4OH$ konz auf 10 eingestellt. Die Suspension wird abgenutscht, die Kristalle gut mit Wasser gewaschen und am Hochvakuum getrocknet.
Ausbeute: 11 g (79 %) 3'-Amino-4-(2,4-diamino-pyrimidin-5-ylmethyl)-6-ethoxybiphenyl-2-ol als hellbraunes Pulver.
MS (ISP): 352,3 $(M+H)^+$

Beispiel 8:

Herstellung von Verbindungen der Formel VII, Schema 1, aus Edukten der Formel (VI) mit anschliessender Abspaltung der Schutzgruppe und fakultativer Derivatisierung

(8a) 4'-(2,4-Diamino-pyrimidin-5-ylmethyl)-6'-ethoxy-2'-(3-methoxy-2,2-dimethylpropoxy)-biphenyl-4-ol

4'-(2,4-Diamino-pyrimidin-5-ylmethyl)-2'-ethoxy-6'-(1-methoxymethylcyclopropylmethoxy)-biphenyl-4-ol (235 mg; 0,52 mmol) wird in Ethanol/Essigsäure konz./Wasser (4/1/0,1) (10 ml) über $PtO_2$ (6 x 35 mg) bei 100°C hydriert. Das Reaktionsgemisch wird auf Raumtemperatur abgekühlt, vom Katalysator abgenutscht und eingeengt. Der Rückstand wird in Wasser verrührt und das pH durch zugabe von $NH_4OH$ konz. auf pH 9 eingestellt, 30 Minuten weitergerührt und abgenutscht. Das graue Rohprodukt wird dann über Kieselgel (20 g) mit Methylenchlorid/Methanol/$NH_4OH$ (19/1/0,05) chromatographiert. Es werden 104 mg (44 %) 4'-(2,4-Diamino-pyrimidin-5-ylmethyl)-6'-ethoxy-2'-(3-methoxy-2,2-dimethyl-propoxy)-biphenyl-4-ol als farbloses Pulver erhalten.
MS (ISP): 453,5 $(M+H)^+$

Das eingesetzte 4'-(2,4-Diamino-pyrimidin-5-ylmethyl)-2'-ethoxy-6'-(1-methoxymethyl-cyclopropylmethoxy)-biphenyl-4-ol wird ausgehend von 4-(2,4-Diaminopyrimidin-5-ylmethyl)-6-ethoxy-4'-methoxymethoxy-biphenyl-2-ol wie folgt durch folgende Sequenz (Stufen a)-b))hergestellt:

Stufe a) 5-[6-Ethoxy-4'-methoxymethoxy-2-(1-methoxymethyl-cyclopropylmethoxy)-biphenyl-4-ylmethyl)-pyrimidin-2,4-diamin

In Analogie zu Beispiel 7 (Alkylierung mit Bromid, Methode $\alpha$):

4-(2,4-Diamino-pyrimidin-5-ylmethyl)-6-ethoxy-4'-methoxymethoxy-biphenyl-2-ol (850 mg; 2,15 mmol) wird

in Dimethylformamid (40 ml; über Molekularsieb getrocknet) unter Argonbegasung gelöst und mit Kalium-tert-butylat (362 mg; 3,22 mmol) bei Raumtemperatur versetzt. Nach einstündigem Rühren wird 1-Brommethyl-1-methoxymethyl-cyclopropan (0,5 g; 2,8 mmol) hinzugefügt und 3 Stunden bei einer Badtemperatur von 80°C gerührt. Anschliessend wird das Reaktionsgemisch auf Raumtemperatur abgekühlt und eingeengt. Der Rückstand wird mit Wasser versetzt und zweimal mit Methylenchlorid extrahiert. Die Wasserphase wird einmal mit Ethylacetat nachextrahiert. Die vereinigten organischen Phasen werden eingeengt und über Kieselgel (120 g) mit Methylenchlorid/Methanol/$NH_4OH$ konz. (19/1/0,05) chromatographiert. Die reinen Fraktionen werden eingeengt, mit Diethylether/n-Hexan (1/1) verrührt, abgenutscht und am Hochvakuum getrocknet.
Ausbeute: 487 mg (46 %) 5-[6-Ethoxy-4'-methoxymethoxy-2-(1-methoxymethylcyclopropylmethoxy)-biphenyl-4-ylmethyl]-pyrimidin-2,4-diamin als beiges Pulver.
MS(ISP): 495,3 $(M+H)^+$

Stufe b) 4'-(2,4-Diamino-pyrimidin-5-ylmethyl)-2'-ethoxy-6'-(1-methoxymethylcyclopropylmethoxy)-biphenyl-4-ol

5-[6-Ethoxy-4'-methoxymethoxy-2-(1-methoxymethyl-cyclopropylmethoxy)-biphenyl-4-ylmethyl]-pyrimidin-2,4-diamin (471 mg; 953 mmol) wird in Methanol (25 ml) und einer 3 M Salzsäure-Lösung in Methanol (5,5 ml; 16,5 mmol) während 30 Minuten bei einer Badtemperatur von 60°C gerührt. Das Reaktionsgemisch wird eingeengt und mit Wasser verrührt. Durch Zugabe von $NH_4OH$ konz. wird das pH auf 9 eingestellt. Nach 2-stündigem Rühren bei Raumtemperatur wird die Suspension abgenutscht, gut mit Wasser gewaschen und aus Ethylacetat umkristallisiert.
Ausbeute: 305 mg (71 %) 4'-(2,4-Diamino-pyrimidin-5-ylmethyl)-2'-ethoxy-6'-(1-methoxymethyl-cyclopropylmethoxy)-biphenyl-4-ol als beiges Pulver.
MS (ISP): 451,4 $(M+H)^+$

In Analogie hierzu werden folgende Verbindungen aus 4-(2,4-Diamino-pyrimidin-5-ylmethyl)-6-ethoxy-4'-methoxymethoxy-biphenyl-2-ol hergestellt:

<u>(8b) (E)-5-[4-(2,4-Diamino-pyrimidin-5-ylmethyl)-6-ethoxy-4'-hydroxy-biphenyl-2-yloxy]-4,4-dimethyl-pent-2-en-nitril</u>

Stufe a) (E)-5-[4-(2,4-Diamino-pyrimidin-5-ylmethyl)-6-ethoxy-4'-methoxymethoxybiphenyl-2-yloxy]-4,4-dimethyl-pent-2-en-nitril

In Analogie zu <u>Beispiel 7</u> (Methode $\alpha$):

Ausgehend von 4-(2,4-Diamino-pyrimidin-5-ylmethyl)-6-ethoxy-4'methoxymethoxy-biphenyl-2-ol (890 mg; 2,25 mmol) und (E)-5-Brom-4,4-dimethyl-pent-2-en-nitril (in <u>Beispiel (7f)</u> beschrieben) (549 mg; 2,92 mmol) werden 271 mg (23 %) (E)-5-[4-(2,4-Diamino-pyrimidin-5-ylmethyl)-6-ethoxy-4'-methoxymethoxy-biphenyl-2-yloxy]-4,4-dimethyl-pent-2-en-nitril als hellbraunes Pulver erhalten.
MS (ISP): 504,3 $(M+H)^+$

Stufe b) (E)-5-[4-(2,4-Diamino-pyrimidin-5-ylmethyl)-6-ethoxy-4'-hydroxy-biphenyl-2-yloxy]-4,4-dimethyl-pent-2-en-nitril

Ausgehend von (E)-5-[4-(2,4-Diamino-pyrimidin-5-ylmethyl)-6-ethoxy-4'methoxymethoxy-biphenyl-2-yloxy]-4,4-dimethyl-pent-2-en-nitril (250 mg; 0,497 mmol) werden 202 mg (88 %) (E)-5-[4-(2,4-Diamino-pyrimidin-5-ylmethyl)-6-ethoxy-4'-hydroxy-biphenyl-2-yloxy]-4,4-dimethyl-pent-2-en-nitril als hellbraunes Pulver erhalten.
MS (ISP): 460,4 $(M+H)^+$

<u>(8c) {1-[4-(2,4-Diamino-pyrimidin-5-ylmethyl)-6-ethoxy-4'-hydroxy-biphenyl-2-yloxymethyl]-cyclopropyl}-acetonitril</u>

Stufe a) [1-[4-(2,4-Diamino-pyrimidin-5-ylmethyl)-6-ethoxy-4'-methoxymethoxybiphenyl-2-yloxymethyl]-cyclopropyl]-acetonitril

In Analogie zu <u>Beispiel 7</u> (Methode $\alpha$):

Ausgehend von 4-(2,4-Diamino-pyrimidin-5-ylmethyl)-6-ethoxy-4'methoxymethoxy-biphenyl-2-ol (594 mg; 1,5 mmol) und (1-Brommethyl-cyclopropyl)-acetonitril(340 mg; 1,95 mmol) werden 558 mg (76 %) [1-[4-(2,4-Diamino-pyrimidin-5-ylmethyl)-6-ethoxy-4'-methoxymethoxy-biphenyl-2-yloxymethyl]-cyclopropyl]-acetonitril als hellbraunes Pulver erhalten.
MS (ISP): 490,3 $(M+H)^+$

Stufe b) {1-[4-(2,4-Diamino-pyrimidin-5-ylmethyl)-6-ethoxy-4'-hydroxy-biphenyl-2-yloxymethyl]-cyclopropyl}-acetonitril

Ausgehend von [1-[4-(2,4-Diamino-pyrimidin-5-ylmethyl)-6-ethoxy-4'methoxymethoxy-biphenyl-2-yloxymethyl]-cyclopropyl]-acetonitril (520 mg; 1,06 mmol) werden 312 mg (66 %) {1-[4-(2,4-Diamino-pyrimidin-5-ylmethyl)-6-ethoxy-4'-hydroxybiphenyl-2-yloxymethyl]-cyclopropyl}-acetonitril als braunes Pulver erhalten.
MS (ISP): 446,3 $(M+H)^+$

(8d) (E)-3-[1-[4-(2,4-Diamino-pyrimidin-5-ylmethyl)-6-ethoxy-4'-hydroxy-biphenyl-2-yloxymethyl]-cyclopropyl]-acrylnitril

(E)-3-[1-[4-(2,4-Diamino-pyrimidin-5-ylmethyl)-6-ethoxy-4'-methoxymethoxybiphenyl-2-yloxymethyl]-cyclopropyl]-acrylnitril (350 mg; 0,698 mmol) wird in Methanol (12 ml) und einer 3 M Salzsäure-Lösung in Methanol (3,4 ml) 30 Minuten bei einer Badtemperatur von 60°C gerührt. Das Methanol wird abgedampft und der Rückstand in Wasser (33 ml) gelöst, das pH durch Zugabe von $NH_4OH$ konz. auf ca. 9 eingestellt, wobei die Substanz ausfällt. Nach 2-stündigem Rühren wird abgenutscht, mit Wasser gewaschen und am Hochvakuum getrocknet. Man erhält 295 mg (92 %) (E)-3-[1-[4-(2,4-Diaminopyrimidin-5-ylmethyl)-6-ethoxy-4'-hydroxy-biphenyl-2-yloxymethyl]-cyclopropyl]-acrylnitril als farbloses Pulver.
MS (ISP): 458,4 $(M+H)^+$

Das eingesetzte (E)-3-[1-[4-(2,4-Diamino-pyrimidin-5-ylmethyl)-6-ethoxy-4'methoxymethoxy-biphenyl-2-yloxymethyl]-cyclopropyl]-acrylnitril wird durch folgende Sequenz (Stufen a)-b)) hergestellt:

Stufe a) {1-[4-(2,4-Diamino-pyrimidin-5-ylmethyl)-6-ethoxy-4'-methoxymethoxybiphenyl-2-yloxymethyl]-cyclopropyl}-methanol

In Analogie zu Beispiel 7 (Methode $\alpha$):

4-(2,4-Diamino-pyrimidin-5-ylmethyl)-6-ethoxy-4'-methoxymethoxy-biphenyl-2-ol (2,5 g; 6,31 mmol) wird in Dimethylformamid (125 ml; über Molekularsieb getrocknet) gelöst und bei Raumtemperatur mit Kalium-tert-butylat (920 mg; 8,2 mmol) versetzt. Es wird dann eine Lösung von [(1-Brommethyl-cyclopropyl)methoxymethyl]-benzol (2,253 g; 8,83 mmol) in Dimethylformamid (3 ml) zugegeben und 100 Minuten bei einer Badtemperatur von 60°C gerührt. Das Reaktionsgemisch wird eingeengt, mit Wasser (100 ml) verdünnt und mit Methylenchlorid (120 ml) extrahiert und nachextrahiert (50 ml). Die vereinigten organischen Phasen werden eingeengt und über Kieselgel (300 g) mit Methylenchlorid/Methanol/$NH_4OH$ konz. (19/1/0,05) chromatographiert. Die vereinigten reinen Fraktionen werden eingeengt, in Essigsäure konz (40 ml) und Ethanol (10 ml) gelöst und über Pd/C 10 % (900 mg) hydriert. Das Reaktionsgemisch wird abgenutscht und eingeengt. Der Rückstand wird mit Wasser (50 ml) verrührt und das pH durch Zugabe von $NH_4OH$ konz. auf ca. 10 eingestellt. Das ausgefallene Produkt wird abgenutscht, mit etwas Wasser gewaschen und am Hochvakuum getrocknet. Es werden 2,24 g (95 %) {1-[4-(2,4-Diamino-pyrimidin-5-ylmethyl)-6-ethoxy-4'-methoxymethoxy-biphenyl-2-yloxymethyl]-cyclopropyl}-methanol als farbloses Pulver erhalten.
MS (ISP): 481,4 $(M+H)^+$

Stufe b) (E)-3-[1-[4-(2,4-Diamino-pyrimidin-5-ylmethyl)-6-ethoxy-4'-methoxymethoxybiphenyl-2-yloxymethyl]-cyclopropyl]-acrylnitril

{1-[4-(2,4-Diamino-pyrimidin-5-ylmethyl)-6-ethoxy-4'-methoxymethoxy-biphenyl-2-yloxymethyl]-cyclopropyl}-methanol (1,6 g; 3,33 mmol) wird in Methylenchlorid (260 ml) und Dimethylformamid (68 ml) gelöst, mit $MnO_2$ (9,9 g) versetzt und 5 Stunden bei einer Badtemperatur von 40°C unter Argon gerührt. Das Reaktionsgemisch wird auf Raumtemperatur abgekühlt, abgenutscht, das Lösungsmittel eingeengt und der Rückstand am Hochvakuum getrocknet. Der Rückstand wird in Acetonitril (40 ml) gelöst, mit Triphenylphosphoranyliden-acetonitril (1,35 g; 4,5 mmol) versetzt und anschliessend 6 Stunden bei Raumtemperatur gerührt. Das Reaktionsgemisch wird eingeengt und der Rückstand über Kieselgel (140 g) mit Methylenchlorid/Methanol/$NH_4OH$ konz. 19/1/0,05 chromatographiert. Die reinen Fraktionen werden vereinigt, eingeengt (210 mg) und erneut über Kieselgel (42 g) mit Methylenchlorid/Methanol/$NH_4OH$ konz. 90/10/1 chromatographiert.
Ausbeute: 148 mg (47 %) (E)-3-[1-[4-(2,4-Diamino-pyrimidin-5-ylmethyl)-6-ethoxy-4'-methoxymethoxy-biphenyl-2-yloxymethyl]-cyclopropyl]-acrylnitril als farbloser fester Schaum.
MS (ISP): 502,3 $(M+H)^+$

(8e) 4'-(2,4-Diamino-pyrimidin-5-ylmethyl)-6'-ethoxy-2'-[2-(tetrahydropyran-4-yl)-ethoxy]-biphenyl-4-ol

Stufe a) 5-{6-Ethoxy-4'-methoxymethoxy-2-[2-(tetrahydropyran-4-yl)-ethoxy]-biphenyl-4-ylmethyl}-pyrimidin-2,4-diamin

In Analogie zu Beispiel 7 (Methode α):
Ausgehend von 4-(2,4-Diamino-pyrimidin-5-ylmethyl)-6-ethoxy-4'methoxymethoxy-biphenyl-2-ol (396 mg; 1 mmol) und 4-(2-Bromethyl)-tetrahydro-pyran (251 mg; 1,3 mmol) werden 520 mg (73 %) 5-{6-Ethoxy-4'-methoxymethoxy-2-[2-(tetrahydropyran-4-yl)-ethoxy]-biphenyl-4-ylmethyl}-pyrimidin-2,4-diamin als gelbes Pulver erhalten, das direkt in die nächste Stufe eingesetzt wird.

Stufe b) 4'-(2,4-Diamino-pyrimidin-5-ylmethyl)-6'-ethoxy-2'-[2-(tetrahydropyran-4-yl)-ethoxy]-biphenyl-4-ol

Ausgehend von 5-{6-Ethoxy-4'-methoxymethoxy-2-[2-(tetrahydropyran-4-yl)-ethoxy]-biphenyl-4-ylmethyl}-pyrimidin-2,4-diamin (520 mg; 1,02 mmol) werden 358 mg (76 %) 4'-(2,4-Diamino-pyrimidin-5-ylmethyl)-6'-ethoxy-2'-[2-(tetrahydropyran-4-yl)-ethoxy]-biphenyl-4-ol als farbloses Pulver erhalten.
MS (ISP): 465,3 $(M+H)^+$

(8f) 1-[4-(2,4-Diamino-pyrimidin-5-ylmethyl)-6-ethoxy-4'-hydroxy-biphenyl-2-yloxymethyl]-cyclopropancarbonitril

Stufe a) 1-[4-(2,4-Diamino-pyrimidin-5-ylmethyl)-6-ethoxy-4'-methoxymethoxybiphenyl-2-yloxymethyl]-cyclopropancarbonitril

In Analogie zu Beispiel 7 (Methode α):
Ausgehend von 4-(2,4-Diamino-pyrimidin-5-ylmethyl)-6-ethoxy-4'methoxymethoxy-biphenyl-2-ol (396 mg; 1 mmol) und 1-(Brommethyl)-2,3-cyclopropancarbonitril(184 mg; 1,15 mmol) werden nach aufeinanderfolgendem Verrühren mit Wasser und Methanol 328 mg (69 %) 1-[4-(2,4-Diamino-pyrimidin-5-ylmethyl)-6-ethoxy-4'-methoxymethoxy-biphenyl-2-yloxymethyl]-cyclopropancarbonitril als beiges Pulver erhalten.
MS (ISP): 476,2 $(M+H)^+$

Stufe b) 1-[4-(2,4-Diamino-pyrimidin-5-ylmethyl)-6-ethoxy-4'-hydroxy-biphenyl-2-yloxymethyl]-cyclopropancarbonitril

Ausgehend von 1-[4-(2,4-Diamino-pyrimidin-5-ylmethyl)-6-ethoxy-4'methoxymethoxy-biphenyl-2-yloxymethyl]-cyclopropancarbonitril (300 mg; 0,63 mmol) werden 100 mg (37 %) 1-[4-(2,4-Diamino-pyrimidin-5-ylmethyl)-6-ethoxy-4'-hydroxybiphenyl-2-yloxymethyl]-cyclopropancarbonitril als farbloses Pulver erhalten.
MS (ISP): 432,4 $(M+H)^+$

(8g) 4'-(2,4-Diamino-pyrimidin-5-ylmethyl)-6'-ethoxy-2'-(2-pyridin-2-yl-ethoxy)-biphenyl-4-ol

Stufe a) 5-[6-Ethoxy-4'-methoxymethoxy-2-(2-pyridin-2-yl-ethoxy)-biphenyl-4-ylmethyl]-pyrimidin-2,4-diamin

In Analogie zu Beispiel 7 (Methode β):
Ausgehend von 4-(2,4-Diamino-pyrimidin-5-ylmethyl)-6-ethoxy-4'methoxymethoxy-biphenyl-2-ol (396 mg; 1 mmol) und 2-(2-Pyridyl)ethyl-mesylat (viermal je 400 mg; 2 mmol) und Kaliumcarbonat (viermal je 414 mg; 3,0 mmol) in siedendem Acetonitril (40 ml) werden 187 mg (37 %) 5-[6-Ethoxy-4'-methoxymethoxy-2-(2-pyridin-2-yl-ethoxy)-biphenyl-4-ylmethyl]-pyrimidin-2,4-diamin als beiges Pulver erhalten.
MS (ISP) ): 502,3 $(M+H)^+$

Stufe b) 4'-(2,4-Diamino-pyrimidin-5-ylmethyl)-6'-ethoxy-2'-(2-pyridin-2-yl-ethoxy)-biphenyl-4-ol

Ausgehend von 5-[6-Ethoxy-4'-methoxymethoxy-2-(2-pyridin-2-yl-ethoxy)-biphenyl-4-ylmethyl]-pyrimidin-2,4-diamin (180 mg; 0,36 mmol) werden 151 mg (92 %) 4'-(2,4-Diamino-pyrimidin-5-ylmethyl)-6'-ethoxy-2'-(2-pyridin-2-yl-ethoxy)-biphenyl-4-ol als farbloses Pulver erhalten.
MS (ISP): 458,4 $(M+H)^+$

(8h) 2'-Cyclopropylmethoxy-4'-(2,4-diamino-pyrimidin-5-ylmethyl)-6'-ethoxy-biphenyl-4-ol

Stufe a) 5-(2-Cyclopropylmethoxy-6-ethoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'methoxymethoxy-biphenyl-2-ol (396 mg; 1,0 mmol) und Brommethyl-cyclopropan (0,114 ml; 1,2 mmol) werden 214 mg (53 %) 5-(2-Cyclopropylmethoxy-6-ethoxy-4'-methoxymethoxy-biphenyl-4-ylmethyl)-pyrimidin-2,4-diamin als gelbliches Pulver erhalten.
MS (ISP): 451,3 $(M+H)^+$

Stufe b) 2'-Cyclopropylmethoxy-4'-(2,4-diamino-pyrimidin-5-ylmethyl)-6'-ethoxybiphenyl-4-ol

Ausgehend von 5-(2-Cyclopropylmethoxy-6-ethoxy-4'-methoxymethoxy-biphenyl-4-ylmethyl)-pyrimidin-2,4-diamin (150 mg; 0,33 mmol) werden 118 mg (84 %)) 2'-Cyclopropylmethoxy-4'-(2,4-diamino-pyrimidin-5-ylmethyl)-6'-ethoxy-biphenyl-4-ol als beiges Pulver erhalten.
MS (ISP): 407,3 $(M+H)^+$

(8i) 2'-Cyclobutylmethoxy-4'-(2,4-diamino-pyrimidin-5-ylmethyl)-6'-ethoxy-biphenyl-4-ol

Stufe a) 5-(2-Cyclobutylmethoxy-6-ethoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'methoxymethoxy-biphenyl-2-ol (396 mg; 1,0 mmol) und Brommethyl-cyclobutan (0,135 ml; 1,2 mmol) werden 192 mg (41 %) 5-(2-Cyclobutylmethoxy-6-ethoxy-4'-methoxymethoxy-biphenyl-4-ylmethyl)-pyrimidin-2,4-diamin als farbloses Pulver erhalten.
MS (ISP): 465,2 $(M+H)^+$

Stufe b) 2'-Cyclobutylmethoxy-4'-(2,4-diamino-pyrimidin-5-ylmethyl)-6'-ethoxybiphenyl-4-ol

Ausgehend von 5-(2-Cyclobutylmethoxy-6-ethoxy-4'-methoxymethoxy-biphenyl-4-ylmethyl)-pyrimidin-2,4-diamin (150 mg; 0,32 mmol) werden 123 mg (90 %)) 2'-Cyclobutylmethoxy-4'-(2,4-diamino-pyrimidin-5-ylmethyl)-6'-ethoxy-biphenyl-4-ol als beiges Pulver erhalten.
MS (ISP): 421,2 $(M+H)^+$

(8j) 2'-Cyclopentyloxy-4'-(2,4-diamino-pyrimidin-5-ylmethyl)-6'-ethoxy-biphenyl-4-ol

Stufe a) 5-(2-Cyclopentyloxy-6-ethoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'-methoxymethoxy-biphenyl-2-ol (396 mg; 1,0 mmol) und Bromcyclopentan(0,128 ml; 1,2 mmol) werden 226 mg (48 %) 5-(2-Cyclopentyloxy-6-ethoxy-4'-methoxymethoxy-biphenyl-4-ylmethyl)-pyrimidin-2,4-diamin als beiges Pulver erhalten. MS (ISP): 465,3 $(M+H)^+$

Stufe b) 2'-Cyclopentyloxy-4'-(2,4-diamino-pyrimidin-5-ylmethyl)-6'-ethoxy-biphenyl-4-ol

Ausgehend von 5-(2-Cyclopentyloxy-6-ethoxy-4'-methoxymethoxy-biphenyl-4-ylmethyl)-pyrimidin-2,4-diamin (150 mg; 0,323 mmol) werden 120 mg (88 %) 2'-Cyclopentyloxy-4'-(2,4-diamino-pyrimidin-5-ylmethyl)-6'-ethoxy-biphenyl-4-ol als beiges Pulver erhalten.
MS (ISP): 421,3 $(M+H)^+$

(8k) 2'-Cyclobutoxy-4'-(2,4-diamino-pyrimidin-5-ylmethyl)-6'-ethoxy-biphenyl-4-ol

Stufe a) 5-(2-Cyclobutoxy-6-ethoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'-methoxymethoxy-biphenyl-2-ol (396 mg; 1,0 mmol) und Bromcyclobutan(0,113 ml; 1,2 mmol) werden 268 mg (60 %) 5-(2-Cyclobutoxy-6-ethoxy-4'-methoxymethoxy-biphenyl-4-ylmethyl)-pyrimidin-2,4-diamin als gelbes Pulver erhalten.

MS (ISP): 451,3 $(M+H)^+$

Stufe b) 2'-Cyclobutoxy-4'-(2,4-diamino-pyrimidin-5-ylmethyl)-6'-ethoxy-biphenyl-4-ol

Ausgehend von 5-(2-Cyclopentyloxy-6-ethoxy-4'-methoxymethoxy-biphenyl-4-ylmethyl)-pyrimidin-2,4-diamin (175 mg; 0,415 mmol) werden 151 mg (86 %) 2'-Cyclobutoxy-4'-(2,4-diamino-pyrimidin-5-ylmethyl)-6'-ethoxy-biphenyl-4-ol als gelbliches Pulver erhalten.
MS (ISP): 407,3 $(M+H)^+$

(8l) 2'-(2-Cyclopropyl-ethoxy)-4'-(2,4-diamino-pyrimidin-5-ylmethyl)-6'-ethoxybiphenyl-4-ol

Stufe a) 5-[2-(2-Cyclopropyl-ethoxy)-6-ethoxy-4'-methoxymethoxy-biphenyl-4-ylmethyl]-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'-methoxymethoxy-biphenyl-2-ol (396 mg; 1,0 mmol) und 2-Bromethylcyclopropan(193 mg; 1,3 mmol) werden 261 mg (56 %) 5-[2-(2-Cyclopropyl-ethoxy)-6-ethoxy-4'-methoxymethoxy-biphenyl-4-ylmethyl]-pyrimidin-2,4-diamin als beiges Pulver erhalten.
MS (ISP): 465,2 $(M+H)^+$ Stufe b) 2'-(2-Cyclopropyl-ethoxy)-4'-(2,4-diamino-pyrimidin-5-ylmethyl)-6'-ethoxybiphenyl-4-ol

Ausgehend von 5-[2-(2-Cyclopropyl-ethoxy)-6-ethoxy-4'-methoxymethoxybiphenyl-4-ylmethyl]-pyrimidin-2,4-diamin (190 mg; 0,409 mmol) werden 152 mg (88 %) 2'-(2-Cyclopropyl-ethoxy)-4'-(2,4-diamino-pyrimidin-5-ylmethyl)-6'-ethoxy-biphenyl-4-ol als beiges Pulver erhalten.
MS (ISP): 421,3 $(M+H)^+$

(8m) 4'-(2,4-Diamino-pyrimidin-5-ylmethyl)-6'-ethoxy-2'-isopropoxy-biphenyl-4-ol

Stufe a) 5-(6-Ethoxy-2-isopropoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'methoxymethoxy-biphenyl-2-ol (396 mg; 1,0 mmol) und 2-Brompropan(0,122 ml; 1,3 mmol) werden 241 mg (55 %) 5-(6-Ethoxy-2-isopropoxy-4'-methoxymethoxy-biphenyl-4-ylmethyl)-pyrimidin-2,4-diamin als beiges Pulver erhalten.
MS (ISP): 439,3 $(M+H)^+$

Stufe b) 4'-(2,4-Diamino-pyrimidin-5-ylmethyl)-6'-ethoxy-2'-isopropoxy-biphenyl-4-ol

Ausgehend von 5-(6-Ethoxy-2-isopropoxy-4'-methoxymethoxy-biphenyl-4-ylmethyl)-pyrimidin-2,4-diamin (200 mg; 0,458 mmol) werden 170 mg (94 %) 4'-(2,4-Diamino-pyrimidin-5-ylmethyl)-6'-ethoxy-2'-isopropoxy-biphenyl-4-ol als beiges Pulver erhalten.
MS (ISP): 395,2 $(M+H)^+$

(8n) 4'-(2,4-Diamino-pyrimidin-5-ylmethyl)-6'-ethoxy-2'-isobutoxy-biphenyl-4-ol

Stufe a) 5-(6-Ethoxy-2-isobutoxy-4'-methoxymethoxy-biphenyl-4-ylmethyl)-pyrimidin-2,4-diamin

In Analogie zu Beispiel 7 (Methode $\alpha$):

Ausgehend von 4-(2,4-Diamino-pyrimidin-5-ylmethyl)-6-ethoxy-4'-methoxymethoxy-biphenyl-2-ol (396 mg; 1,0 mmol) und Isobutylbromid(0,141 ml; 1,3 mmol) werden 278 mg (61 %) 5-(6-Ethoxy-2-isobutoxy-4'-methoxymethoxy-biphenyl-4-ylmethyl)-pyrimidin-2,4-diamin als gelbliches Pulver erhalten.
MS (ISP): 453,4 $(M+H)^+$

Stufe b) 4'-(2,4-Diamino-pyrimidin-5-ylmethyl)-6'-ethoxy-2'-isobutoxy-biphenyl-4-ol

Ausgehend von 5-(6-Ethoxy-2-isobutoxy-4'-methoxymethoxy-biphenyl-4-ylmethyl)-pyrimidin-2,4-diamin (240 mg; 0,530 mmol) werden 184 mg (85 %)4'-(2,4-Diaminopyrimidin-5-ylmethyl)-6'-ethoxy-2'-isobutoxy-biphenyl-4-ol als leicht gelbliches Pulver erhalten.
MS (ISP): 409,3 $(M+H)^+$

(8o) 4'-(2,4-Diamino-pyrimidin-5-ylmethyl)-6'-ethoxy-2'-[(1-methyl-cyclopropyl)-methoxy]-biphenyl-4-ol

Stufe a) 5-[6-Ethoxy-4'-methoxymethoxy-2-[(1-methyl-cyclopropyl)-methoxy]-biphenyl-4-ylmethyl]-pyrimidin-2,4-diamin

In Analogie zu Beispiel 7 (Methode α):

Ausgehend von 4-(2,4-Diamino-pyrimidin-5-ylmethyl)-6-ethoxy-4'-methoxymethoxy-biphenyl-2-ol (396 mg; 1,0 mmol) und 1-Brommethyl-1-methylcyclopropan (208 mg; 1,4 mmol) werden 291 mg (62 %) 5-[6-Ethoxy-4'-methoxymethoxy-2-[(1-methyl-cyclopropyl)-methoxy]-biphenyl-4-ylmethyl]-pyrimidin-2,4-diamin als gelbliches Pulver erhalten.
MS (ISP): 465,2 $(M+H)^+$

Stufe b) 4'-(2,4-Diamino-pyrimidin-5-ylmethyl)-6'-ethoxy-2'-[(1-methyl-cyclopropyl)-methoxy]-biphenyl-4-ol

Ausgehend von 5- [6-Ethoxy-4'-methoxymethoxy-2-[(1-methyl-cyclopropyl)-methoxy]-biphenyl-4-ylmethyl]-pyrimidin-2,4-diamin (280 mg; 0,602 mmol) werden 123 mg (49 %) 4'-(2,4-Diamino-pyrimidin-5-ylmethyl)-6'-ethoxy-2'-[(1-methyl-cyclopropyl)-methoxy]-biphenyl-4-ol als farbloses Pulver erhalten.
MS (ISP): 421,2 $(M+H)^+$

(8p) 4'-(2,4-Diamino-pyrimidin-5-ylmethyl)-6'-ethoxy-2'-(3-hydroxy-2,2-dimethylpropoxy)-biphenyl-4-ol

Stufe a) 5-{2-[2,2-Dimethyl-3-(tetrahydro-pyran-2-yloxy)-propoxy]-6-ethoxy-4'-methoxymethoxy-biphenyl-4-ylmethyl}-pyrimidin-2,4-diamin

4-(2,4-Diamino-pyrimidin-5-ylmethyl)-6-ethoxy-4'-methoxymethoxy-biphenyl-2-ol (1,19; 3,0 mmol) wird in Wasser (15 ml) suspendiert und mit einer 1 M wässrigen KOH-Lösung (3,0 ml; 3 mmol) und Ethanol (15 ml) bei Raumtemperatur versetzt. Die Lösung wird eingeengt, mit Aceton verrührt, abgenutscht und am Hochvakuum getrocknet. Es wird 1,09 g eines gelblichen Pulvers erhalten. Davon werden 400 mg (0,92 mmol) entnommen, in Dimethylformamid (10 ml; über Molekularsieb getrocknet) gelöst und mit 2-(3-Brom-2,2-dimethylpropoxy)tetrahydro-2H-pyran (300 mg; 1,19 mmol) versetzt. Das Reaktionsgemisch wird 20 Stunden auf 150°C erhitzt und anschliessend auf Raumtemperatur abgekühlt und eingeengt. Der Rückstand wird mit Wasser verrührt und mit Methylenchlorid zweimal extrahiert. Die vereinigten organischen Phasen werden über Magnesiumsulfat getrocknet und über Kieselgel (100 g) mit Methytenchlorid/ Methanol/$NH_4OH$ konz. (19/1/0,05) chromatographiert. Es werden 144 mg (18 %) 5-{2-[2,2-Dimethyl-3-(tetrahydro-pyran-2-yloxy)-propoxy]-6-ethoxy-4'-methoxyrnethoxybiphenyl-4-ylmethyl}-pyrimidin-2,4-diamin als öliger Rückstand erhalten.
MS (ISP): 567,3 $(M+H)^+$

Stufe b) 4'-(2,4-Diamino-pyrimidin-5-ylmethyl)-6'-ethoxy-2'-(3-hydroxy-2,2-dimethylpropoxy)-biphenyl-4-ol

Ausgehend von 5-{2-[2,2-Dimethyl-3-(tetrahydro-pyran-2-yloxy)-propoxy]-6-ethoxy-4'-methoxymethoxy-biphenyl-4-ylmethyl}-pyrimidin-2,4-diamin (106 mg; 0,187 mmol) werden analog Beispiel (8a), Stufe (b) 68 mg (80 %) 4'-(2,4-Diamino-pyrimidin-5-ylmethyl)-6'-ethoxy-2'-(3-hydroxy-2,2-dimethyl-propoxy)-biphenyl-4-ol als bräunliches Pulver erhalten.
MS (ISP): 439,2 $(M+H)^+$

(8q) 4'-(2,4-Diamino-pyrimidin-5-ylmethyl)-6'-ethoxy-2'-[2-(4-methyl-thiazol-5-yl)-ethoxy]-biphenyl-4-ol

Stufe a) 5-{6-Ethoxy-4'-methoxymethoxy-2-[2-(4-methyl-thiazol-5-yl)-ethoxy]-biphenyl-4-ylmethyl}-pyrimidin-2,4-diamin

In Analogie zu Beispiel 7 (Methode β):

Ausgehend von 4-(2,4-Diamino-pyrimidin-5-ylmethyl)-6-ethoxy-4'methoxymethoxy-biphenyl-2-ol (594 mg; 1,5 mmol) und (4-Methyl-thiazol-5-yl)ethanolmethansulfonat (viermal je 400 mg; 2 mmol) und Kaliumcarbonat (dreimal je 664 mg; 3,0 mmol) in siedendem Acetonitril (67 ml) werden 629 mg (56 %) 4'-(2,4-Diaminopyrimidin-5-ylmethyl)-6'-ethoxy-2'-[2-(4-methyl-thiazol-5-yl)-ethoxy]-biphenyl-4-ol als gelbes Pulver erhalten.
MS (ISP) ): 522,2 $(M+H)^+$

Stufe b) 4'-(2,4-Diamino-pyrimidin-5-ylmethyl)-6'-ethoxy-2'-[2-(4-methyl-thiazol-5-yl)-ethoxy]-biphenyl-4-ol

Ausgehend von 5-{6-Ethoxy-4'-methoxymethoxy-2-[2-(4-methyl-thiazol-5-yl)-ethoxy]-biphenyl-4-ylmethyl}-pyrimidin-2,4-diamin (608 mg; 1,16 mmol) werden 496 mg (90 %) 4'-(2,4-Diamino-pyrimidin-5-ylmethyl)-6'-ethoxy-2'-[2-(4-methyl-thiazo1-5-yl)-ethoxy]-biphenyl-4-ol als farbloses Pulver erhalten.
MS (ISP): 478,3 $(M+H)^+$

(8r) 5-[6-Ethoxy-2-(3-methoxy-cyclopentyloxy)-4'-hydroxy-biphenyl-4-ylmethyl]-pyrimidin-2,4-diamin

Stufe a) 5-[6-Ethoxy-2-(3-methoxy-cyclopentyloxy)-4'-methoxymethoxy-biphenyl-4-ylmethyl]-pyrimidin-2,4-diamin

In Analogie zu Beispiel 7 (Methode $\alpha$):

Ausgehend von 5-(6-Ethoxy-2-hydroxy-4'-methoxymethoxy-biphenyl-4-ylmethyl)-pyrimidin-2,4-diamin (400 mg; 1.0 mmol), 1-Brom-3-methoxy-cyclopentan, Kalium-tert-butylat (272 mg; 2.42 mmol) in Dimethylformamid werden 155 mg (31%) 5-[6-Ethoxy-2-(3-methoxy-cyclopentyloxy)-4'-methoxymethoxy-biphenyl-4-ylmethyl]-pyrimidin-2,4-diamin als gelber Feststoff erhalten.
MS (ISP): 495.3$(M+H)^+$

Stufe b) 5-[6-Ethoxy-2-(3-methoxy-cyclopentyloxy)-4'-hydroxy-biphenyl-4-ylmethyl]-pyrimidin-2,4-diamin

Ausgehend von 5-[6-Ethoxy-2-(3-methoxy-cyclopentyloxy)-4'-methoxymethoxybiphenyl-4-ylmethyl]-pyrimidin-2,4-diamin (196 mg; 0.4 mmol) werden 98 mg (58%) 5-[6-Ethoxy-2-(3-methoxy-cyclopentyloxy)-4'-hydroxy-biphenyl-4-ylmethyl]-pyrimidin-2,4-diamin als gelber Feststoff erhalten.
MS (ISP): 451.4 $(M+H)^+$

Das eingesetzte 1-Brom-3-methoxy-cyclopentan wird wie folgt hergestellt (Stufen a)-d)):

Stufe a) 1-(tert-Butyldimethyl-silanyloxy)-3-hydroxy-cyclopentan

1,3-Dihydroxycyclopentan (4.5 g; 44.06 mmol) wird in Tetrahydrofuran (40 ml) gelöst und mit Natriumhydrid (50%ig in Oel)(2.11 g; 44.06 mmol) versetzt. Das Gemisch wird bei Raumtemperatur kräftig gerührt (es bildet sich ein weisser "Brei"). Anschliessend gibt man tert-Butyl-dimethyl-chlorsilan (6.64 g; 44.06 mmol) zu und rührt bei Raumtemperatur über Nacht. Das Reaktionsgemisch wird auf eine gesättigte Kaliumhydrogencarbonat-Lösung (100 ml) gegossen. Das Gemisch wird zweimal mit Ether extrahiert und die organische Phase auf Natriumsulfat getrocknet, filtriert und eingeengt.
Der Rückstand wird über Kieselgel mit n-Hexan/Ethylacetat (3/1) chromatographiert . Die reinen Fraktionen werden vereinigt und das Lösungsmittel abgedampft.
Ausbeute: 3,24 g (34 %)1-(tert-Butyldimethyl-silanyloxy)-3-hydroxy-cyclopentan als gelbe Flüssigkeit.
MS: 216 (M)

Stufe b) 1-(tert-Butyldimethyl-silanyloxy)-3-methoxy-cyclopentan:

1-(tert-Butyldimethyl-silanyloxy)-3-hydroxy-cyclopentan (3.24 g; 14.97 mmol) wird in Tetrahydrofuran (30 ml) gelöst und mit Kalium-tert-butylat (3.36 g; 29.94 mmol) versetzt. Man rührt eine halbe Stunde bei Raumtemperatur, gibt dann Methyliodid (1.86 ml; 29.94 mmol) hinzu und rührt weitere zwei Stunden. Anschliessend wird das Reaktionsgemisch auf Wasser (100 ml) gegossen und zweimal mit Ethylacetat extrahiert. Die organische Phase wird auf Natriumsulfat getrocknet, filtriert und eingeengt. Der Rückstand wird über Kieselgel mit n-Hexan/Ethylacetat (97/3) chromatographiert. Die reinen Fraktionen werden vereinigt und das Lösungsmittel abgedampft.
Ausbeute: 3.09 g (89%) 1-(tert-Butyldimethyl-silanyloxy)-3-methoxy-cyclopentan als gelbliche Flüssigkeit.
NMR (1H, 250 Mz in DMSO) in ppm: 0,0 (s, 6H); 0,82 (s, 9H); 1,3-2,0 (m, 6H); 3,11 (s, 3H); 3,81 (m, 1H); 4,3 (m, 1H)

Stufe c) 1-hydroxy-3-methoxy-cyclopentan:

1-(tert-Butyldimethyl-silanyloxy)-3-methoxy-cyclopentan (3.08 g; 13.37 mmol) wird in Tetrahydrofuran (40 ml) gelöst, mit einer 1 M Tetrabutylammoniumfluorid Lösung in Tetrahydrofuran (29.4 ml; 29.4 mmol) versetzt und vier Stunden bei Raumtemperatur gerührt. Das Reaktionsgemisch wird eingeengt. Der Rückstand wird über Kieselgel mit n-Hexan/Ethylacetat (4/6) chromatographiert. Die reinen Fraktionen werden vereinigt und das Lösungsmittel abgedampft.
Ausbeute: 1,65 g (Quantitativ) 1-hydroxy-3-methoxy-cyclopentan als gelbliche Flüssigkeit.

NMR (1H, 250 Mz in DMSO) in ppm: 1,4-2,0 (6H); 3,18 (s, 3H); 3,8-3,9 (m, 1H); 4,2 (m, 1H); 4,5 (d, 1H)

Stufe d) 1-Brom-3-methoxy-cyclopentan

1-Hydroxy-3-methoxy-cyclopentan (1.65 g; 13.37 mmol) wird in Methylenchlorid (40 ml) gelöst und mit Tetrabromkohlenstoff (5.54 g; 16.71 mmol) versetzt. Das Reaktionsgemisch wird auf 0°C abgekühlt und Triphenylphosphin (5.26 g; 20.05 mmol) portionenweise zugegeben. Nach einer halben Stunde bei 0°C und einer halben Stunde bei Raumtemperatur wird das Reaktionsgemisch auf Eiswasser (100 ml) gegossen. Das Gemisch wird zweimal mit Ethylacetat extrahiert und die organische Phase auf Natriumsulfat getrocknet, filtriert und eingeengt. Der Rückstand wird über Kieselgel mit n-Hexan/Ethylacetat (9/1) chromatographiert. Die reinen Fraktionen werden vereinigt und das Lösungsmittel abgedampft.
Ausbeute: 2,76 g (Quantitativ)1-Brom-3-methoxy-cyclopentan als gelbliche Flüssigkeit.
MS: 178 (M)

(8s) 4'-(2,4-Diamino-pyrimidin-5-ylmethyl)-6'-ethoxy-2'-(2,2,2-trifluor-ethoxy)-biphenyl-4-ol

Wird analog Beispiel 7i (Methode β) hergestellt:
Ausgehend von 4-(2,4-Diamino-pyrimidin-5-ylmethyl)-6-ethoxy-4'-methoxymethoxy-biphenyl-2-ol (594 mg; 1,5 mmol) und 2,2,2-Trifluorethylmethansulfonat (0,70 ml; 6 mmol) werden nach Chromatographie über Kieselgel (90 g) mit Methylenchlorid/ Methanol/$NH_4OH$ konz. (1/1 Gemisch aus 90/10/1 und 19/1/0,05) 419 mg (62 %) eines weissen Pulvers erhalten. Dieses Pulver wird in Methanol (20 ml) gelöst, mit einer 3M wässrigen Salzsäure-Lösung versetzt und 20 Minuten bei 60°C gerührt. Die Lösung wird eingeengt, in Wasser aufgenommen, mit $NH_4OH$ konz. auf pH 9 eingestellt und über Kieselgel (100 g) mit Methylenchlorid/ Methanol/$NH_4OH$ konz. (19/1/0,05) chromatographiert. Es werden 74 mg (20 %) 4'-(2,4-Diamino-pyrimidin-5-ylmethyl)-6'-ethoxy-2'-(2,2,2-trifluor-ethoxy)-biphenyl-4-ol als farbloses Pulver erhalten.
MS (ISP): 435,3 $(M+H)^+$
Derselbe Ansatz liefert auch Methansulfonsäure-4-(2,4-diamino-pyrimidin-5-ylmethyl)-6-ethoxy-4'-hydroxy-biphenyl-2-yl-ester (Beispiel 15a)

(8t) (RS)-4'-(2,4-Diamino-pyrimidin-5-ylmethyl)-6'-ethoxy-2'-[2-(tetrahydro-pyran-2-yl)-ethoxy]-biphenyl-4-ol

Stufe a) (RS)-5-[6-Ethoxy-4'-methoxymethoxy-2-[2-(tetrahydro-pyran-2-yl)-ethoxy]-biphenyl-4-ylmethyl]-pyrimidin-2,4-diamin

In Analogie zu Beispiel 7 (Methode α):
Ausgehend von 4-(2,4-Diamino-pyrimidin-5-ylmethyl)-6-ethoxy-4'-methoxymethoxy-biphenyl-2-ol (198 mg; 0,5 mmol) und (Tetrahydro-pyran-2-yl)-ethylbromid (126 mg; 0,65 mmol) werden 197 mg (77 %) (RS)-5-[6-Ethooy-4'-methoxymethoxy-2-[2-(tetrahydro-pyran-2-yl)-ethoxy]-biphenyl-4-ylmethyl]-pyrimidin-2,4-diamin als fester gelber Schaum erhalten.
MS (ISP): 509,0 $(M+H)^+$

Stufe b) (RS)-4'-(2,4-Diamino-pyrimidin-5-ylmethyl)-6'-ethoxy-2'-[2-(tetrahydro-pyran-2-yl)-ethoxy]-biphenyl-4-ol

Ausgehend von (RS)-5-[6-Ethoxy-4'-methoxymethoxy-2-[2-(tetrahydro-pyran-2-yl)-ethoxy]-biphenyl-4-ylmethyl]-pyrimidin-2,4-diamin (194 mg; 0,38 mmol) werden 170 mg (96 %) (RS)-4'-(2,4-Diamino-pyrimidin-5-ylmethyl)-6'-ethoxy-2'-[2-(tetrahydropyran-2-yl)-ethoxy]-biphenyl-4-ol als hellgelbes Pulver erhalten.
MS (ISP): 465,3 $(M+H)^+$

(8u) rac-(E)-{2-[4-(2,4-Diamino-pyrimidin-5-ylmethyl)-6-ethoxy-4'-hydroxy-biphenyl-2-yloxy]-cyclopentyl}-acetonitril

Stufe a) rac-(E)-{2-[4-(2,4-Diamino-pyrimidin-5-ylmethyl)-6-ethoxy-4'-methoxymethoxy-biphenyl-2-yloxy]-cyclopentyl}-acetonitril (Kopplung unter Mitsunobu-Bedingungen)

5-(6-Ethoxy-2-hydroxy-4'-methoxymethoxy-biphenyl-4-ylmethyl)-pyrimidin-2,4-diamin (50 mg; 0.13 mmol) wird in Dimethylformamid (10 ml) gelöst und mit Triphenylphosphin (660 mg; 2,52 mmol), rac-(Z)-(2-Hydroxy-cydopentyl)-acetonitril (70 mg; 0.56 mmol) und Triethylamin (0,5 ml; 3,59 mmol) unter Rühren versetzt. Danach wird DEAD (Diethylazodicarboxylat) (448 mg; 2,57 mmol) hinzugefügt und das Gemisch weitergerührt. Das Reaktionsgemisch wird anschliessend auf Wasser gegossen, mit Ethylacetat extrahiert, über Natriumsulfat getrocknet, abgenutscht und

eingeengt. Das Rohprodukt wird über Kieselgel mit Methylenchlorid/Methanol (19/1) chromatographiert. Ausbeute: 50 mg (79%) rac-(E)-{2-[4-(2,4-Diamino-pyrimidin-5-ylmethyl)-6-ethoxy-4'-methoxymethoxy-biphenyl-2-yloxy]-cyclopentyl}-acetonitril als farbloses Wachs.
MS (ISP): 504.3 $(M+H)^+$

Stufe b) rac-(E)-{2-[4-(2,4-Diamino-pyrimidin-5-ylmethyl)-6-ethoxy-4'-hydroxybiphenyl-2-yloxy]-cyclopentyl}-acetonitril

Ausgehend von rac-(E)-{2-[4-(2,4-Diamino-pyrimidin-5-ylmethyl)-6-ethoxy-4'methoxymethoxy-biphenyl-2-yloxy]-cyclopentyl}-acetonitril werden 88 mg (34%) rac-(E)-{2-[4-(2,4-Diamino-pyrimidin-5-ylmethyl)-6-ethoxy-4'-hydroxy-biphenyl-2-yloxy]-cyclopentyl}-acetonitril als weisser Feststoff erhalten.
MS (ISP): 460.4 $(M+H)^+$

Das eingesetzte rac-(Z)-(2-Hydroxy-cyclopentyl)-acetonitril wird wie folgt hergestellt:

rac-(Z)-2-Hydroxy-cyclopentan-carbonsäure-ethylester (5.06 g; 32 mmol) wird in Dimethylformamid (20 ml) gelöst, bei Raumtemperatur mit Imidazol (5.44 g; 80 mmol) und tert-Butyl-dimethyl-chlorsilan (6.19 g; 35 mmol) versetzt und anschliessend 15 Stunden gerührt. Das Reaktionsgemisch wird auf Wasser (100 ml) gegossen und dreimal mit n-Hexan extrahiert. Die vereinigten organischen Phasen werden auf Natriumsulfat getrocknet, filtriert und eingeengt. Der erhaltene rac-(Z)-2-(tert-Butyl-dimethylsilanyloxy)-cyclopentan-carbonsäure-ethylester (8.7 g; 32 mmol) wird in Tetrahydrofuran (100 ml) gelöst, bei 0°C mit Diisobutylaluminiumhydrid (DIBAH) (1.2 M Lösung in Toluol; 76 ml; 91 mmol) versetzt und drei Stunden gerührt. Methanol (10 ml) wird vorsichtig unter heftigem Rühren zugetropft, gefolgt von einer 2 M Kaliumnatriumtartrat-Lösung (100 ml). Nach einer halben Stunde wird die organische Phase abgetrennt, mit Wasser gewaschen, auf Natriumsulfat getrocknet, filtriert und eingeengt. Das erhaltene rac-(Z)-2(tert-Butyl-dimethyl-silanyloxy)-1-hydroxymethyl-cyclopentan (3.57 g; 15.5 mmol) wird in Methylenchlorid (70 ml) gelöst, mit Dimethylaminopyridin (5.67 g; 46.5 mmol) und Tosylchlorid (8.87 g; 46.5 mmol) versetzt und über Nacht gerührt. Das Reaktionsgemisch wird auf wässrige Salzsäure (1M) (100 ml) gegossen und dreimal mit n-Hexan extrahiert. Die vereinigten organischen Phasen werden über Natriumsulfat getrocknet, filtriert und eingeengt. Der Rückstand wird über Kieselgel mit n-Hexan/Ethylacetat (8/2) chromatographiert . Die reinen Fraktionen werden vereinigt und das Lösungsmittel abgedampft. Das erhaltene Tosylat (5.5 g; 15.5 mmol) wird in Dimethylsulfoxid (35 ml) gelöst, mit Natriumcyanid (1.37 g; 28 mmol) versetzt und 1.5 Stunden auf 90°C erhitzt. Das Reaktionsgemisch wird auf Wasser (100 ml) gegossen und zweimal mit Ethylacetat extrahiert. Die vereinigten organischen Phasen werden über Natriumsulfat getrocknet, filtriert und eingeengt. Der Rückstand wird über Kieselgel mit n-Hexan/Ethylacetat (8/2) chromatographiert . Die reinen Fraktionen werden vereinigt und das Lösungsmittel abgedampft. Das erhaltene rac-(Z)-[2-(tert-Butyl-dimethylsilanyloxy)-cydopentyl]-acetonitril (3.5 g; 14.6 mmol) wird in einem 1/1 Acetonitril/Tetrahydrofuran-Gemisch (40 ml) verdünnt, mit 40%iger Fluorwasserstoffsäure (11.3 ml) versetzt und eine Stunde gerührt. Das Reaktionsgemisch wird auf eine gesättigte Natriumhydrogencarbonat-Lösung (200 ml) gegossen und zweimal mit Ethylacetat extrahiert. Die vereinigten organischen Phasen werden über Natriumsulfat getrocknet, filtriert und eingeengt.
Ausbeute: 2.0 g (76%) rac-(Z)-(2-Hydroxy-cyclopentyl)-acetonitril als farblose Flüssigkeit.
MS: 125 (M)

(8v) rac-(E)-5-[6-Ethoxy-4'-hydroxy-2-(2-methoxymethyl-cyclopentyloxy)-biphenyl-4-ylmethyl]-pyrimidin-2,4-diamin

Stufe a) rac-(E)-5-[6-Ethoxy-4'-methoxymethoxy-2-(2-methoxymethyl-cyclopentyloxy)-biphenyl-4-ylmethyl]-pyrimidin-2,4-diamin

Wird in Analogie zu Beispiel 8u (Stufe a)) (Kopplung unter Mitsunobu-Bedingungen) hergestellt.

Ausgehend von 5-(6-Ethoxy-2-hydroxy-4'-methoxymethoxy-biphenyl-4-ylmethyl)-pyrimidin-2,4-diamin (250 mg; 0.63 mmol), und rac-(Z)-2-Methoxymethylcyclopentanol (492 mg; 2.84 mmol) werden 380 mg (92%) rac-(E)-5-[6-Ethoxy-4'-methoxymethoxy-2-(2-methoxymethyl-cyclopentyloxy)-biphenyl-4-yimethyl]-pyrimidin-2,4-diamin als gelbliches Wachs erhalten.
MS (ISP): 509.5 $(M+H)^+$ Stufe b) rac-(E)-5-[6-Ethoxy-4'-hydroxy-2-(2-methoxymethyl-cyclopentyloxy)-biphenyl-4-ylmethyl]-pyrimidin-2,4-diamin

Ausgehend von rac-(E)-5-[6-Ethoxy-4'-methoxymethoxy-2-(2-methoxymethylcyclopentyloxy)-biphenyl-4-ylmethyl]-pyrimidin-2,4-diamin (370 mg; 0.57 mmol) werden 150 mg (57%) rac-(E)-5-[6-Ethoxy-4'-hydroxy-2-(2-methoxymethyl-cyclopentyloxy)-biphenyl-4-ylmethyl]-pyrimidin-2,4-diamin als weisser Feststoff erhalten.
MS (ISP): 465.3 $(M+H)^+$

Das eingesetzte rac-(Z)-2-Methoxymethyl-cyclopentanol wird wie folgt hergestellt:

rac-(Z)-2(tert-Butyl-dimethyl-silanyloxy)-1-hydroxymethyl-cyclopentan (0.5 g; 2.17 mmol) (Herstellung in Beispiel 8u) wird in Methylenchlorid (20 ml) gelöst. 2,6-di-tert-Butylpyridin (1.0 ml; 4.34 mmol) und Methyl-trifluormethansulfonat (0.736 ml; 6.5 mmol) werden hinzugefügt und das Reaktionsgemisch zwei Stunden unter Rückflussbedingungen erhitzt. Das Reaktionsgemisch wird auf verdünnte wässrige Salzsäure (1M)(30 ml) gegossen und zweimal mit Methylenchlorid extrahiert. Die vereinigten organischen Phasen werden auf Natriumsulfat getrocknet, filtriert und eingeengt. Das erhaltene rac-(Z)-2(tert-Butyl-dimethyl-silanyloxy)-1-methoxymethyl-cyclopentan (0.525 g; 2.15 mmol) wird mit einem 1/1 Tetrahydrofuran/Acetonitril-Gemisch (5 ml) und mit 40%iger wässriger Fluorwasserstoffsäure (2 ml) versetzt und drei Stunden gerührt. Das Reaktionsgemisch wird auf eine gesättigte wässrige Natriumbicarbonat-Lösung (20 ml) gegossen. Das Gemisch wird zweimal mit Ethylacetat extrahiert, und die vereinigten organischen Phasen über Natriumsulfat getrocknet, abgenutscht und eingeengt. Ausbeute: 0.24 g (84%) rac-(Z)-2-Methoxymethyl- cyclopentanol als farbloses Oel.
MS (EI): 112 (M-$H_2O$)

(8w) {3-[4-(2,4-Diamino-pyrimidin-5-ylmethyl)-6-ethoxy-4'-hydroxy-biphenyl-2-yloxy}-cyclopentyl}-acetonitril

Stufe a) 5{3-[4-(2,4-Diamino-pyrimidin-5-ylmethyl)-6-ethoxy-4'-methoxymethoxybiphenyl-2-yloxy]-cyclopentyl]-acetonitril

Wird in Analogie zu Beispiel 8u (Stufe a)(Kopplung unter Mitsunobu-Bedingungen) hergestellt.
Ausgehend von 5-(6-Ethoxy-2-hydroxy-4'-methoxymethoxy-biphenyl-4-ylmethyl)-pyrimidin-2,4-diamin (250 mg; 0.63 mmol) und (3-Hydroxy-cyclopentyl)-acetonitril (190 mg; 1.51 mmol) werden 155 mg (49%) 5{3-[4-(2,4-Diamino-pyrimidin-5-ylmethyl)-6-ethoxy-4'-methoxymethoxy -biphenyl-2-yloxy]-cyclopentyl}-acetonitril als gelblicher Schaum erhalten.
MS (ISP): 504.3 $(M+H)^+$

Stufe b) {3-[4-(2,4-Diamino-pyrimidin-5-ylmethyl)-6-ethoxy-4'-hydroxy-biphenyl-2-yloxy]-cyclopentyl}-acetonitril

Ausgehend von 5{3-[4-(2,4-Diamino-pyrimidin-5-ylmethyl)-6-ethoxy-4'methoxymethoxy -biphenyl-2-yloxy]-cyclopentyl}-acetonitril (150 mg; 0.30 mmol) werden 107 mg (78%) {3-[4-(2,4-Diamino-pyrimidin-5-ylmethyl)-6-ethoxy-4'-hydroxy-biphenyl-2-yloxy]-cyclopentyl}-acetonitril als weisser Feststoff erhalten.
MS (ISP): 460.4 $(M+H)^+$
Das eingesetzte (3-Hydroxy-cyclopentyl)-acetonitril wird wie folgt hergestellt:
3-(Oxo-cyclopentyl)-acetonitril (0.9 g; 8.25 mmol) wird in Methanol (15 ml) gelöst und mit Ceriumchlorid (III) (3.38 g; 9.07 mmol) versetzt. Bei 0°C wird Natriumborhydrid(0.343 g; 9.1 mmol) hinzugefügt, eine Viertelstunde bei 0°C und eine halbe Stunde bei Raumtemperatur gerührt. Das Reaktionsgemisch wird auf Wasser (30 ml) gegossen. Das Gemisch wird zweimal mit Ethylacetat extrahiert und die vereinigten organischen Phasen über Natriumsulfat getrocknet, filtriert und eingeengt. Der Rückstand wird über Kieselgel mit n-Hexan/Ethylacetat (1/1) chromatographiert. Die reinen Fraktionen werden vereinigt und das Lösungsmittel abgedampft.
Ausbeute: 0.752 g (82%) (3-Hydroxy-cyclopentyl)-acetonitril als gelbliche Flüssigkeit.
MS (EI): 125 (M)

(8x) 3-[4-(2,4-Diamino-pyrimidin-5-ylmethyl)-6-ethoxy-4'-hydroxy-biphenyl-2-yloxy]-cyclopentancarbonitril

Stufe a) 3-[4-(2,4-Diamino-pyrimidin-5-ylmethyl)-6-ethoxy-4'-methoxymethoxybiphenyl-2-yloxy]-cyclopentancarbonitril

In Analogie zu Beispiel 7 (Methode α Alkylierung mit Bromid):
Ausgehend von 5-(6-Ethoxy-2-hydroacy-4'-methoxymethoxy-biphenyl-4-ylmethyl)-pyrimidin-2,4-diamin (400 mg; 1.01 mmol) und 1-Brom-3-cyan-cyclopentan (263 mg; 1.51 mmol) werden 331 mg (59%) 3-[4-(2,4-Diamino-pyrimidin-5-ylmethyl)-6-ethoxy-4'-methoxymethoxy-biphenyl-2-yloxy]-cyclopentancarbonitril als weisse Kristalle erhalten.
MS (ISP): 490.3 $(M+H)^+$

Stufe b) 3-[4-(2,4-Diamino-pyrimidin-5-ylmethyl)-6-ethoxy-4'-hydroxy-biphenyl-2-yloxy] -cyclopentancarbonitril

Ausgehend von 3-[4-(2,4-Diamino-pyrimidin-5-ylmethyl)-6-ethoxy-4'methoxymethoxy-biphenyl-2-yloxy]-cyclopentancarbonitril (320 mg; 0.65 mmol) werden 242 mg (83%) 3-[4-(2,4-Diamino-pyrimidin-5-ylmethyl)-6-ethoxy-4'-hydroxy-biphenyl-2-yloxy]-cyclopentancarbonitril als weisse Kristalle erhalten.
MS (ISP): 446.5 $(M+H)^+$
Das eingesetzte 1-Brom-3-cyan-cyclopentan wird wie folgt hergestellt:
1-Hydroxy-3-cyan-rydopentan (1.0 g; 9 mmol) wird in Methylenchlorid (30 ml) gelöst und mit Tetrabromkohlenstoff (3.73 g; 11.25 mmol)versetzt. Bei 0°C wird Triphenylphosphin (3.54 g; 13.5 mmol) hinzugefügt und anschliessend eine Viertelstunde bei 0°C und eine halbe Stunde bei Raumtemperatur gerührt. Das Reaktionsgemisch wird auf Wasser (30 ml) gegossen und zweimal mit Ethylacetat extrahiert. Die vereinigten organischen Phasen werden über Natriumsulfat getrocknet, filtriert und eingeengt. Der Rückstand wird über Kieselgel mit n-Hexan/Ethylacetat (8/2) chromatographiert . Die reinen Fraktionen werden vereinigt und das Lösungsmittel abgedampft.
Ausbeute: 0.757 g (48%) 1-Brom-3-cyan-cyclopentan als gelbliche Flüssigkeit.
MS (EI): 173 (M)

(8y) 5-[6-Ethoxy-2((trans)-2-methoxy-cyclopentyloxy)-4'-hydroxy-biphenyl-4-ylmethyl]-pyrimidin-2,4-diamin

Stufe a) 5-[6-Ethoxy-2((trans)-2-methoxy-cyclopentyloxy)-4'-methoxymethoxy-biphenyl-4-ylmethyl]-pyrimidin-2,4-diamin

Wird in Analogie zu Beispiel 8u (Stufe a)(Kopplung unter Mitsunobu-Bedingungen) hergestellt.
Ausgehend von 4-(2,4-Diamino-pyrimidin-5-ylmethyl)-6-ethoxy-4'-methoxymethoxy-biphenyl-2-ol (390 mg; 0.98 mmol) und (cis)-2-Methoxy-cydopentanol (547 mg; 2.21 mmol) werden 400 mg (82%) 5-[6-Ethoxy-2((trans)-2-methoxy-cyclopentyloxy)-4'-methoxymethoxy-biphenyl-4-ylmethyl]-pyrimidin-2,4-diamin als gelblicher Schaum erhalten.
MS (ISP): 495.3 $(M+H)^+$

Stufe b) 5-[6-Ethoxy-2((trans)-2-methoxy-cyclopentyloxy)-4'-hydroxy-biphenyl-4-ylmethyl]-pyrimidin-2,4-diamin

Ausgehend von 5-[6-Ethoxy-2((trans)-2-methoxy-cyclopentyloxy)-4'methoxymethoxy-biphenyl-4-ylmethyl]-pyrimidin-2,4-diamin (365 mg; 0.74 mmol) werden 260 mg (78%) 5-[6-Ethoxy-2((trans)-2-methoxy-cyclopentyloxy)-4'-hydroxybiphenyl-4-ylmethyl]-pyrimidin-2,4-diamin als weisser Schaum erhalten.
MS (ISP): 451.3 $(M+H)^+$
Das obige 4-(2,4-Diamino-pyrimidin-5-ylmethyl)-6-ethoxy-4'-methoxymethoxybiphenyl-2-ol wird ausgehend von 5-(3-Benzyloxy-5-ethoxy-4-iod-benzyl)-pyrimidin-2,4-diamin (Beispiel 6 in 2 Stufen a) und b)) hergestellt: Schema 1, (IV) -> (V) -> (VI) Stufe a) 5-(2-Benzyloxy-6-ethoxy-4'-methoxymethoxy-biphenyl-4-ylmethyl)-pyrimidin-2,4-diamin
Wird in Analogie zu Beispiel (2a) (Suzuki-Kopplung mit Boronsäure) hergestellt.
Ausgehend von 5-(3-Benryloxy-5-ethoxy-4-iod-benzyl)-pyrimidin-2,4-diamin (Beispiel 6) (20 g; 42 mmol), 4-Methoxymethoxy-phenylboronsäure (15,1 g; 84 mmol), Tetrakis(triphenylphosphin)palladium (2,4 g; 2,1 mmol) und einer 2M $K_3PO_4$-Lösung in Wasser (115,5 ml; 231 mmol) in Dimethylformamid (120 ml) werden nach 17 Stunden bei 85°C Badtemperatur 15 g (74 %) 5-(2-Benzyloxy-6-ethoxy-4'-methoxymethoxy-biphenyl-4-ylmethyl)-pyrimidin-2,4-diamin als gelber Schaum erhalten.
MS ISP): 487,3 $(M+H)^+$

Stufe b) 4-(2,4-Diamino-pyrimidin-5-ylmethyl)-6-ethoxy-4'-methoxymethoxy-biphenyl-2-ol

5-(2-Benzyloxy-6-ethoxy-4'-methoxymethoxy-biphenyl-4-ylmethyl)-pyrimidin-2,4-diamin (15 g; 30,8 mmol) wird in Methanol (2000 ml) über Pd/C 10% (7,5 g) 6 Stunden hydriert. Der Katalysator wird abfiltriert, zweimal mit einem Gemisch aus Methanol (300 ml) und Dimethylformamid (50 ml) ausgekocht, und die vereinigten Mutterlaugen eingeengt. Der Rückstand wird mit Diethylether verrührt, abgenutscht und am Hochvakuum getrocknet.
Ausbeute: 12,1 g (99 %) 4-(2,4-Diamino-pyrimidin-5-ylmethyl)-6-ethoxy-4'-methoxymethoxy-biphenyl-2-ol als hellbraunes Pulver.
MS (ISP): 397,2$(M+H)^+$

Beispiel 9:

(9a) 5-[6-Ethoxy-4'-methoxy-2-(3-methoxy-2,2-dimethyl-propoxy)-biphenyl-4-ylmethyl]-pyrimidin-2,4-diamin

Wird in Analogie zu Beispiel 7, aber ausgehend von 4-(2,4-Diamino-pyrimidin-5-ylmethyl)-6-ethoxy-4'-methoxy-biphenyl-2-ol hergestellt: Schema 1, (VI) -> (VII)
5- [2-Ethoxy-4'-methoxy-6-[(1-methoxymethyl-cyclopropyl)-methoxy]-biphenyl-4-ylmethyl]-pyrimidin-2,4-diamin (255 mg; 0,549 mmol) wird in Ethanol/Essigsäure konz./Wasser (4/1/0,1) (12 ml) übec $PtO_2$ (3 x 35 mg) bei 100°C hydriert. Das Reaktionsgemisch wird auf Raumtemperatur abgekühlt, vom Katalysator abgenutscht und eingeengt. Der Rückstand wird in Wasser verrührt, das pH durch zugabe von $NH_4OH$ konz auf pH 10 eingestellt und das Ganze abgenutscht. Das Rohprodukt wird über Kieselgel (25 g) mit Diethylether chromatographiert. Es werden 80 mg (31 %)

5-[6-Ethoxy-4'-methoxy-2-(3-methoxy-2,2-dimethyl-propoxy)-biphenyl-4-ylmethyl]-pyrimidin-2,4-diamin als hellbraunes Pulver erhalten.
MS (ISP): 467,3 $(M+H)^+$

Das eingesetzte 5-[2-Ethoxy-4'-methoxy-6-[-(1-methoxymethyl-cyclopropyl)-methoxy]-biphenyl-4-ylmethyl]-pyrimidin-2,4-diamin wird ausgehend von 4-(2,4-Diamino-pyrimidin-5-ylmethyl)-6-ethoxy-4'-methoxy-biphenyl-2-ol wie folgt hergestellt:

4-(2,4-Diamino-pyrimidin-5-ylmethyl)-6-ethoxy-4'-methoxy-biphenyl-2-ol (544 mg; 1,48 mmol) wird in Dimethylformamid (15 ml; über Molekularsieb getrocknet) unter Argonbegasung gelöst und mit Kalium-tert-butylat (254 mg; 2,22 mmol) bei Raumtemperatur versetzt. Nach einstündigem Rühren wird 1-Brommethyl-1-methoxymethylcyclopropan (330 mg; 1,93 mmol) hinzugefügt und das Ganze etwa 4 Stunden bei einer Badtemperatur von 80°C weitergerührt. Anschliessend wird das Reaktionsgemisch auf Raumtemperatur abgekühlt und eingeengt. Der Rückstand wird mit Wasser versetzt und mit Ethylacetat extrahiert. Die Wasserphase wird abgetrennt und einmal mit Ethylacetat nachextrahiert. Die vereinigten organischen Phasen werden zweimal mit einer gesättigten wässrigen Kochsalz-Lösung gewaschen, über Magnesiumsulfat getrocknet und eingeengt. Der Rückstand wird über Kieselgel (154 g) mit Methylenchlorid/Methanol/$NH_4OH$ konz. (90/10/1) chromatographiert. Die reinen Fraktionen werden eingengt, mit Diethylether/n-Hexan 1:2 (18 ml) verrührt, abgenutscht, mit etwas Diethylether gewaschen und am Hochvakuum getrocknet.
Ausbeute: 412 mg (60 %) 5-[2-Ethoxy-4'-methoxy-6-[(1-methoxymethyl-cyclopropyl)-methoxy]-biphenyl-4-ylmethyl]-pyrimidin-2,4-diamin als beiges Pulver.
MS(ISP): 465,3 $(M+H)^+$

(9b) [1-[4-(2,4-Diamino-pyrimidin-5-ylmethyl)-6-ethoxy-4'-methoxy-biphenyl-2-yloxymethyl]-cyclopropyl]-acetonitril

Wird analog zu Beispiel 8c hergestellt.

Ausgehend von 4-(2,4-Diamino-pyrimidin-5-ylmethyl)-6-ethoxy-4'-methoxybiphenyl-2-ol (183 mg; 0,5 mmol) werden 146,5 mg (64 %)[1-(4-(2,4-Diaminopyrimidin-5-ylmethyl)-6-ethoxy-4'-methoxy-biphenyl-2-yloxymethyl]-cyclopropyl]-acetonitril als hellgelbes Pulver erhalten.
MS (ISP): 460,4 $(M+H)^+$

(9c) 3-{1-[4-(2,4-Diamino-pyrimidin-5-ylmethyl)-6-ethoxy-4'-methoxy-biphenyl-2-yloxymethyl]-cyclopropyl}-propionitril

Wird durch folgende Sequenz (Stufen a-d), ausgehend von 4-(2,4-Diaminopyrimidin-5-ylmethyl)-6-ethoxy-4'-methoxy-biphenyl-2-ol hergestellt:

Stufe a) 5-[6-(1-Benzyloxymethyl-cyclopropylmethoxy)-2-ethoxy-4'-methoxy-biphenyl-4-ylmethyl)-pyrimidin-2,4-diamin

4-(2,4-Diamino-pyrimidin-5-ylmethyl)-6-ethoxy-4'-methoxy-biphenyl-2-ol (476 mg; 1,3 mmol) wird in Dimethylformamid (16 ml; über Molekularsieb getrocknet) vorgelegt und mit Kalium-tert-butylat (224 mg; 1,95 mmol) bei Raumtemperatur versetzt. Nach einstündigem Rühren wird [(1-Brommethyl-cyclopropyl)-methoxymethyl]-benzol (431 mg; 1,69 mmol) zugegeben und anschliessend 3 Stunden bei einer Badtemperatur von 80°C gerührt. Danach wird auf Raumtemperatur abgekühlt und erneut Kalium-tert-butylat (112 mg; 0,975 mmol) und [(1-Brommethyl-ryclopropyl)-methoxymethyl]-benzol (215 mg; 0,845 mmol) hinzugefügt. Das Reaktionsgemisch wird erneut 1 Stunde bei 80°C gerührt, auf Raumtemperatur abgekühlt und eingeengt. Der Rückstand wird mit Wasser versetzt und mit Ethylacetat extrahiert, mit einer wässrigen gesättigten Kochsalz-lösung gewaschen, über Magnesiumsulfat getrocknet, abgenutscht und eingeengt. Das Rohprodukt wird über Kieselgel (100 g) mit Methylenchlorid/Methanol/$NH_4OH$ konz. (19/1/0,05) chromatographiert. Die reinen Fraktionen werden vereinigt und eingeengt, und anschliessend mit n-Hexan/Diethylether 3/2 (20 ml) verrührt, abgenutscht, mit n-Hexan gewaschen und am Hochvakuum getrocknet. Es werden 360 mg (48 %) 5-[6-[(1-Benzyloxymethyl-cyclopropyl)-methoxy]-2-ethoxy-4'-methoxy-biphenyl-4-ylmethyl]-pyrimidin-2,4-diamin als beiges Pulver erhalten.
MS (ISP): 541,3 $(M+H)^+$

Stufe b) [1-[4-(2,4-Diamino-pyrimidin-5-ylmethyl)-6-ethoxy-4'-methoxy-biphenyl-2-yloxymethyl]-cyclopropyl}-methanol

5-[6-[(1-Benzyloxymethyl-cyclopropyl)-methoxy]-2-ethoxy-4'-methoxy-biphenyl-4-ylmethyl]-pyrimidin-2,4-diamin (350 mg; 0,647 mmol) wird in Essigsäure konz (8ml) und Ethanol (2,7 ml) über Pd/C 10 % (100 mg) 4 ½ Stunden hydriert. Der Katalysator wird abgenutscht und mit Ethanol gewaschen. Die erhaltene Lösung wird eingedampft, mit Wasser (15 ml) versetzt und das pH durch Zugabe von $NH_4OH$ konz. auf ca 10 eingestellt. Das ausgefallene

Material wird abgenutscht, in Ethylacetat gelöst und mit Wasser gewaschen, über Magnesiumsulfat getrocknet und eingedampft. Der Rückstand wird mit n-Hexan/Diethylether 2/1 (15 ml) verrührt, abgenutscht, mit n-Hexan gewaschen und am Hochvakuum getrocknet. Es werden 278 mg (95 %) {1-[4-(2,4-Diamino-pyrimidin-5-ylmethyl)-6-ethoxy-4'-methoxy-biphenyl-2-yloxymethyl]-cyclopropyl}-methanol als farbloses Pulver erhalten.
MS (ISP): 451,2 $(M+H)^+$

Stufe c) (E)-3-[1-[4-(2,4-Diamino-pyrimidin-5-ylmethyl)-6-ethoxy-4'-methoxy-biphenyl-2-yloxymethyl]-cyclopropyl]-acrylnitril

{1-[4-(2,4-Diamino-pyrimidin-5-ylmethyl)-6-ethoxy-4'-methoxy-biphenyl-2-yloxymethyl]-cyclopropyl}-methanol (225 mg; 0,5 mmol) wird in Methylenchlorid (40 ml) und Dimethylformamid (10 ml) mit $MnO_2$ (1,5 g) bei einer Badtemperatur von 40°C 20 Stunden gerührt. Die Suspension wird abgenutscht, gut mit Methanol nachgewaschen und die Mutterlaugen eingeengt. Das erhaltene gelbe Öl (ca. 200 mg) wird in Acetonitril (5 ml) gelöst, mit (Triphenylphosphoranyliden)acetonitril (200 mg; 0,75 mmol) versetzt und 3 Tage bei Raumtemperatur gerührt. Das Reaktionsgemisch wird eingeengt und über Kieselgel (60 g) mit Methylenchlorid/Methanol/$NH_4OH$ konz. (19/1/0,05) chromatographiert. Es werden nach Verrühren mit n-Hexan/Diethylether (2/1) 110 mg (47 %) (E)-3-[1-[4-(2,4-Diamino-pyrimidin-5-ylmethyl)-6-ethoxy-4'-methoxy-biphenyl-2-yloxymethyl]-cyclopropyl]-acrylnitril als farbloses Pulver erhalten.
MS (ISP): 472,3 $(M+H)^+$

Stufe d) 3-{1-[4-(2,4-Diamino-pyrimidin-5-ylmethyl)-6-ethoxy-4'-methoxy-biphenyl-2-yloxymethyl]-cyclopropyl}-propionitril

(E)-3-[1-[4-(2,4-Diamino-pyrimidin-5-ylmethyl)-6-ethoxy-4'-methoxy-biphenyl-2-yloxymethyl]-cyclopropyl]-acrylnitril (220 mg; 0,467 mmol) wird in Isopropanol (25 ml) mit Natriumborhydrid (45 mg; 1,2 mmol) versetzt und 19 Stunden unter Rückfluss gerührt. Das Reaktionsgemsich wird eingeengt, mit Wasser (15 ml) verrührt, mit 1N wässriger Salzsäure auf pH 2 eingestellt, mit konz. $NH_4OH$ auf pH 9 zurückgestellt, abgenutscht und am Hochvakuum getrocknet. Das Rohprodukt wird über Kieselgel (20 g) mit Methylenchlorid/Methanol/$NH_4OH$ konz. (19/1/0,05) chromatographiert. Die reinen Fraktionen werden zusammen eingeengt, mit Diethylether verrührt, abgenutscht und am Hochvakuum getrocknet. Es werden 75 mg (34 %) 3-{1-[4-(2,4-Diamino-pyrimidin-5-ylmethyl)-6-ethoxy-4'-methoxy-biphenyl-2-yloxymethyl]-cyclopropyl}-propionitril als farbloses Pulver erhalten.
MS (ISP): 474,3 $(M+H)^+$

(9d) 5-(2-Cyclopropylmethoxy-6-ethoxy-4'-methoxy-biphenyl-4-ylmethyl)-pyrimidin-2,4-diamin

Wird analog zu Beispiel 7a hergestellt:
Ausgehend von 4-(2,4-Diamino-pyrimidin-5-ylmethyl)-6-ethoxy-4'-methoxybiphenyl-2-ol (200 mg; 0,545 mmol) werden 119 mg (48 %) 5-(2-Cyclopropylmethoxy-6-ethoxy-4'-methoxy-biphenyl-4-ylmethyl)-pyrimidin-2,4-diamin als farbloses Pulver erhalten.
MS (ISP): 421,3 $(M+H)^+$

(9e) 5-(6-Ethoxy-4'-methoxy-2-propoxy-biphenyl-4-ylmethyl)-pyrimidin-2,4-diamin

Ausgehend von 4-(2,4-Diamino-pyrimidin-5-ylmethyl)-6-ethoxy-4'-methoxybiphenyl-2-ol (200 mg; 0,545 mmol) und 1-Brompropan (0,060 ml; 0,654 mmol) werden 121 mg (454 %) 5-(6-Ethoxy-4'-methoxy-2-propoxy-biphenyl-4-ylmethyl)-pyrimidin-2,4-diamin als farbloses Pulver erhalten.
MS (ISP): 409,4 $(M+H)^+$

(9f) 5-(2-Ethoxy-6-isopropoxy-4'-methoxy-biphenyl-4-ylmethyl)-pyrimidin-2,4-diamin

Wird analog zu Beispiel 8m (Stufe a)) hergestellt:
Ausgehend von 4-(2,4-Diamino-pyrimidin-5-ylmethyl)-6-ethoxy-4'-methoxybiphenyl-2-ol (200 mg; 0,545 mmol) werden 139 mg (62 %) 5-(2-Ethoxy-6-isopropoxy-4'-methoxy-biphenyl-4-ylmethyl)-pyrimidin-2,4-diamin als beiges Pulver erhalten.
MS (ISP): 409,4 $(M+H)^+$

(9g) 5-(2-Ethoxy-6-isobutoxy-4'-methoxy-biphenyl-4-ylmethyl)-pyrimidin-2,4-diamin

Wird analog zu Beispiel 8n (Stufe a)) hergestellt:

Ausgehend von 4-(2,4-Diamino-pyrimidin-5-ylmethyl)-6-ethoxy-4'-methoxybiphenyl-2-ol (200 mg; 0,545 mmol) werden 147 mg (64 %) 5-(2-Ethoxy-6-isobutoxy-4'-methoxy-biphenyl-4-ylmethyl)-pyrimidin-2,4-diamin als beiges Pulver erhalten.
MS (ISP): 423,3 $(M+H)^+$

Die obige Ausgangsverbindung 4-(2,4-Diamino-pyrimidin-5-ylmethyl)-6-ethoxy-4'methoxy-biphenyl-2-ol wird wie folgt hergestellt:
Schema 1, (IV) -> (V) -> (VI)

Stufe a) 5-(2-Benzyloxy-6-ethoxy-4'-methoxy-biphenyl-4-ylmethyl)-pyrimidin-2,4-diamin Wird in Analogie zu Beispiel (2a) (Suzuki-kopplung mit Boronsäure) hergestellt:

Ausgehend von 5-(3-Benzyloxy-5-ethoxy-4-iod-benzyl)-pyrimidin-2,4-diamin (Beispiel 6) (474 mg; 1 mmol) und 4-Methoxy-phenylboronsäure (304 mg; 2 mmol) werden 254 g (56 %) 5-(2-Benzyloxy-6-ethoxy-4'-methoxy-biphenyl-4-ylmethyl)-pyrimidin-2,4-diamin als beiges Pulver erhalten.
MS: 456 (M)

Stufe b) 4-(2,4-Diamino-pyrimidin-5-ylmethyl)-6-ethoxy-4'-methoxy-biphenyl-2-ol

5-(2-Benzyloxy-6-ethoxy-4'-methoxy-biphenyl-4-ylmethyl)-pyrimidin-2,4-diamin (100 mg; 0,22 mmol) wird in Essigsäure konz. (3 ml) und Ethanol (1 ml) über Pd/C 10 % (25 mg) hydriert. Nach 1 Stunde wird der Katalysator abgenutscht, mit Ethanol nachgewaschen und eingeengt. Der Rückstand wird mit Wasser (10 ml) verrührt, wonach man durch Zugabe einer wässrigen gesättigten Natriumhydrogencarbonat-Lösung der pH-Wert auf 8 einstellt. Die Suspension wird abgenutscht, mit Wasser gewaschen und am Hochvakuum getrocknet. Es werden 51 mg (63 %) 4-(2,4-Diamino-pyrimidin-5-ylmethyl)-6-ethoxy-4'-methoxy-biphenyl-2-ol als leicht graues Pulver erhalten.
MS (ISP): 367,2 $(M+H)^+$

Beispiel 10:

In Analogie zu dem Beispiel 7 und 8 werden hergestellt; Schema 1, (VI) -> (VII)

(10a) 5-(4-Benzo[1,3]dioxol-5-yl-3-cyclobutylmethoxy-5-ethoxy-benzyl)-pyrimidin-2,4-diamin wird analog Beispiel 8i (Stufe a))hergestellt:

Ausgehend von 2-Benzo[1,3]dioxol-5-yl-5-(2,4-diamino-pyrimidin-5-ylmethyl)-3-ethoxy-phenol (247 mg; 0,65 mmol) werden 198 mg (68 %) 5-(4-Benzo[1,3]dioxol-5-yl-3-cyclobutylmethoxy-5-ethoxy-benzyl)-pyrimidin-2,4-diamin als gelbes Pulver erhalten.
MS (ISP): 449,3 $(M+H)^+$

(10b) 5-(4-Benzo[1,3]dioxol-5-yl-3-cyclopropylmethoxy-5-ethoxy-benzyl)-pyrimidin-2,4-diamin wird analog Beispiel 7a (Stufe a))hergestellt:

Ausgehend von 2-Benzo[1,3]dioxol-5-yl-5-(2,4-diamino-pyrimidin-5-ylmethyl)-3-ethoxy-phenol (247 mg; 0,65 mmol) werden 195 mg (69 %) 5-(4-Benzo[1,3]dioxol-5-yl-3-cyclopropylmethoxy-5-ethoxy-benzyl)-pyrimidin-2,4-diamin als gelbes Pulver erhalten.
MS : 434 (M)

(10c) 5-(4-Benzo[1,3]dioxol-5-yl-3-ethoxy-5-isobutoxy-benzyl)-pyrimidin-2,4-diamin wird analog Beispiel 8n (Stufe a)) hergestellt.

Ausgehend von 2-Benzo[1,3]dioxol-5-yl-5-(2,4-diamino-pyrimidin-5-ylmethyl)-3-ethoxy-phenol (247 mg; 0,65 mmol) werden 123 mg (43 %) 5-(4-Benzo[1,3]dioxol-5-yl-3-ethoxy-5-isobutoxy-benzyl)-pyrimidin-2,4-diamin als gelbes Pulver erhalten.
MS (ISP) : 437,3 $(M+H)^+$

(10d) 5-(4-Benzo[1,3]dioxol-5-yl-3-cyclopentyloxy-5-ethoxy-benzyl)-pyrimidin-2,4-diamin wird analog Beispiel 7d hergestellt:

Ausgehend von 2-Benzo[1,3]dioxol-5-yl-5-(2,4-diamino-pyrimidin-5-ylmethyl)-3-ethoxy-phenol (247 mg; 0,65

mmol) werden 136 mg (47 %) 5-(4-Benzo[1,3]dioxol-5-yl-3-cyclopentyloxy-5-ethoxy-benzyl)-pyrimidin-2,4-diamin als gelbes Pulver erhalten.
MS (ISP) : 449,3 $(M+H)^+$

(10e) 5-(4-Benzo[1,3]dioxol-5-yl-3-cyclobutoxy-5-ethoxy-benzyl)-pyrimidin-2,4-diamin wird analog Beispiel 7e hergestellt:

Ausgehend von 2-Benzo[1,3]dioxol-5-yl-5-(2,4-diamino-pyrimidin-5-ylmethyl)-3-ethoxy-phenol (247 mg; 0,65 mmol) werden 113 mg (40 %) 5-(4-Benzo[1,3]dioxol-5-yl-3-cyclobutoxy-5-ethoxy-benzyl)-pyrimidin-2,4-diamin als gelbes Pulver erhalten.
MS (ISP) : 435,3 $(M+H)^+$

(10f) 5-[4-Benzo[1,3]dioxol-5-yl-3-ethoxy-5-(2-methyl-allyloxy)-benzyl]-pyrimidin-2,4-diamin wird analog Beispiel 7a hergestellt:

Ausgehend von 2-Benzo[1,3]dioxol-5-yl-5-(2,4-diamino-pyrimidin-5-ylmethyl)-3-ethoxy-phenol (247 mg; 0,65 mmol) und 3-Brom-2-methylpropen (0,101 ml; 0,975 mml) werden 223 mg (79 %) 5-[4-Benzo[1,3]dioxol-5-yl-3-ethoxy-5-(2-methyl-allyloxy)-benzyl]-pyrimidin-2,4-diamin als farbloses Pulver erhalten.
MS (ISP) : 435,2 $(M+H)^+$

Die obige Ausgangsverbindung 2-Benzo[1,3]dioxol-5-yl-5-(2,4-diamino-pyrimidin-5-ylmethyl)-3-ethoxy-phenol wird wie folgt hergestellt:
Schema 1, (IV) -> (V) -> (VI)

Stufe a) 5-(4-Benzo[1,3]dioxol-5-yl-3-benzyloxy-5-ethoxy-benzyl)-pyrimidin-2,4-diamin wird in Analogie zu Beispiel (2a) (Suzuki-Kopplung mit Boronsäure) hergestellt:

Ausgehend von 5-(3-Benzyloxy-5-ethoxy-4-iod-benzyl)-pyrimidin-2,4-diamin (Beispiel 6) (10 g; 21 mmol), (1,3-Benzodioxol-5-yl)-boronsäure (7,0 g; 42 mmol), Tetrakis-triphenylphosphin-palladium (1,17 g; 1 mmol) in Dimethylformamid (140 ml), Ethanol (35 ml) und einer 2M $K_3PO_4$-Lösung in Wasser (93 ml) 2 Stunden bei 86°C Badtemperatur werden 9,7 g (98 %) 5-(4-Benzo[1,3]dioxol-5-yl-3-benzyloxy-5-ethoxybenzyl)-pyrimidin-2,4-diamin als gelber fester Schaum erhalten.
MS(ISP): 471,2 $(M+H)^+$

Stufe b) 2-Benzo[1,3]dioxol-5-yl-5-(2,4-diamino-pyrimidin-5-ylmethyl)-3-ethoxy-phenol

5-(4-Benzo[1,3]dioxol-5-yl-3-benzyloxy-5-ethoxy-benzyl)-pyrimidin-2,4-diamin (900 mg; 1,9 mmol) wird in Essigsäure konz. (24 ml) und Ethanol (8 ml) über Pd/C 10 % (300 mg) hydriert. Nach 3 Stunden wird der Katalysator abgenutscht, mit Ethanol nachgewaschen und eingeengt. Der Rückstand wird mit Wasser (20 ml) verrührt und durch Zugabe von $NH_4OH$ konz. das pH auf 9 eingestellt. Die Suspension wird abgenutscht, mit Wasser gewaschen und am Hochvakuum getrocknet. Es werden 533 mg (74 %) 2-Benzo[1,3]dioxol-5-yl-5-(2,4-diamino-pyrimidin-5-ylmethyl)-3-ethoxy-phenol als farbloses Pulver erhalten.
MS (ISP): 381,2 $(M+H)^+$

Beispiel 11:

In Analogie zu Beispiel 7 werden hergestellt: Schema 1, (VI) -> (VII)

(11a) 5-(4'-Amino-2-cyclopropylmethoxy-6-ethoxy-3'-methyl-biphenyl-4-ylmethyl)-pyrimidin-2,4-diamin

Ausgehend von 5-(4'-Amino-6-ethoxy-2-hydroxy-3'-methyl-biphenyl-4-ylmethyl)-pyrimidin-2,4-diamin (250 mg; 0.68 mmol) und Brommethylcyclopropan (0.085 ml; 0.89 mmol) werden 143 mg (49%) 5-(4'-Amino-2-cyclopropylmethoxy-6-ethoxy-3'-methylbiphenyl-4-ylmethyl)-pyrimidin-2,4-diamin als gelber Schaum erhalten.
MS (ISP): 420.3 $(M+H)^+$

(11b) 5-{4'-Amino-2-[(2-tetrahydropyran-4-yl)-ethoxy]-6-ethoxy-4'-methyl-biphenyl-4-ylmethyl}-pyrimidin-2,4-diamin

Ausgehend von 5-(4'-Amino-6-ethoxy-2-hydroxy-4'-methyl-biphenyl-4-ylmethyl)-pyrimidin-2,4-diamin (250 mg; 0.68 mmol) und (2-tetrahydropyran-4-yl)-ethyl-1-bromid (308 mg; 1.71 mmol) werden 240 mg (73%) 5-{4'-Amino-2-[(2-tetrahydropyran-4-yl)-ethoxy]-6-ethoxy-4'-methyl-biphenyl-4-ylmethyl}-pyrimidin-2,4-diamin als gelber Feststoff erhalten.
MS (ISP): 478.4 $(M+H)^+$

Die obige Ausgangsverbindung 5-(4'-Amino-6-ethoxy-2-hydroxy-4'-methylbiphenyl-4-ylmethyl)-pyrimidin-2,4-diamin wird wie folgt hergestellt in Analogie zu Beispiel 4a (Suzuki-Kopplung mit *in sit*u Generierung von $R^2$-B $(OH)_2$)) und anschliessend Abspaltung der Benzylgruppe durch katalytische Hydrierung :
Schema 1, (IV) -> (V) -> (VI)

Ausgehend von N-(4-Iod-2-methyl-phenyl)-2,2,2-trifluoracetamid (6 g; 18.2 mmol), (Bis-pinacolato)diboron (6.61 ml; 45.57 mmol), Triethylamin (7.62 ml; 54.7 mmol) und (Diphenylphosphino)-dichlor-palladium (II) (716 mg; 1.02 mmol) in Dioxan und danach 5-(3-Benzyloxy-5-ethoxy-4-iod-benzyl)-pyrimidin-2,4-diamin (Beispiel 6) (4.21 g; 8.83 mmol), Tetrakis-triphenylphosphin-palladium (786 mg; 0.68 mmol), wässriger 2M Natriumcarbonat-Lösung (136 ml; 272 mmol) in 5/1 Dimethoxyethan/Ethanol (184 ml), werden 4.4 g (98 %) 5-(4'-Amino-2-benzyloxy-6-ethoxy-4'-methyl-biphenyl-4-ylmethyl)-pyrimidin-2,4-diamin als braune Flüssigkeit erhalten. Das gesamte Zwischenprodukt 5-(4'-Amino-2-benzyloxy-6-ethoxy-4'-methyl-biphenyl-4-ylmethyl)-pyrimidin-2,4-diamin (10.3 g; 12.57 mmol) wird in Dimethylformamid/Methanol 1/3 gelöst und über Pd/C 10 % (1.66 g) hydriert.
Ausbeute: 4.06 g (88%)5-(4'-Amino-6-ethoxy-2-hydroxy-4'-methyl-biphenyl-4-ylmethyl)-pyrimidin-2,4-diamin als brauner Feststoff.
MS (ISP): 366.2 $(M+H)^+$

Beispiel 12:

In Analogie zu Beispiel 7 wird hergestellt: Schema 1, (VI) -> (VII)

5-(3'-Amino-2-cyclopropylmethoxy-6-ethoxy-4'-methoxy-biphenyl-4-ylmethyl)-pyrimidin-2,4-diamin

Ausgehend von 5-(4'-Amino-6-ethoxy-2-hydroxy-4'-methoxy-biphenyl-4-ylmethyl)-pyrimidin-2,4-diamin (220 mg; 0.68 mmol) und Brommethylcyclopropan (0.138 ml; 1.44 mmol) werden 170 mg (65%) 5-(3'-Amino-2-cyclopropylmethoxy-6-ethoxy-4'-methoxybiphenyl-4-ylmethyl)-pyrimidin-2,4-diamin als gelber Schaum erhalten.
MS (ISP): 436.4 $(M+H)^+$

Die obige Ausgangsverbindung 5-(4'-Amino-6-ethoxy-2-hydroxy-4'-methoxybiphenyl-4-ylmethyl)-pyrimidin-2,4-diamin wird wie folgt hergestellt (in Analogie zu Beispiel 4a; Suzuki-Kopplung mit *in situ* Generierung von $R^2$-B $(OH)_2$ und anschliessende Abspaltung der Benzylgruppe durch katalytische Hydrierung) :
Schema 1, (IV) -> (V) -> (VI)

Ausgehend von N-(3-Brom-6-methoxy-phenyl)-2,2,2-trifluoracetamid (5 g; 16.8 mmol), (Bis-pinacolato)diboron (6.09 ml; 41.94 mmol), Triethylamin (7.0 ml; 50.3 mmol) und (Diphenylphosphino)-dichlor-palladium (II) (768 mg; 0.67 mmol) in Dioxan, danach 5-(3-Benzyloxy-5-ethoxy-4-iod-benzyl)-pyrimidin-2,4-diamin (Beispiel 6) (3.83 g; 8.04 mmol), Tetra-(triphenyl-phosphin)-palladium (620 mg; 0.54 mmol) und wässeriger 2M Natriumcarbonat-Lösung (81 ml; 162 mmol) in 5/1 Dimethoxyethan/Ethanol (72 ml), werden 2.71g (72 %) 5-(3'-Amino-2-benzyloxy-6-ethoxy-4'-methoxy-biphenyl-4-ylmethyl)-pyrimidin-2,4-diamin als leicht grüner Schaum erhalten. Das gesamte Zwischenprodukt wird in Dimethylformamid/Methanol 1/3 gelöst und über Pd/C 10 % (0.66 g) hydriert.
Ausbeute: 0.97g (53%) 5-(4'-Amino-6-ethoxy-2-hydroxy-4'-methoxy-biphenyl-4-ylmethyl)-pyrimidin-2,4-diamin als gelbe Kristalle.
MS (ISP): 382.2 $(M+H)^+$

Beispiel 13:

In Analogie zu Beispiel 7 wird hergestellt: Schema 1, (VI) -> (VII)

5-(4'-Amino-2-cyclopropylmethoxy-6-ethoxy-biphenyl-4-ylmethyl)-pyrimidin-2,4-diamin

Wird analog Beispiel 7a (Stufe a))hergestellt.

Ausgehend von 4'-Amino-4-(2,4-diamino-pyrimidin-5-ylmethyl)-6-ethoxybiphenyl-2-ol (176 mg; 0,5 mmol) werden 110 mg (54 %) 5-(4'-Amino-2-cyclopropylmethoxy-6-ethoxy-biphenyl-4-ylmethyl)-pyrirnidin-2,4-diamin als gelbes Pulver erhalten.
MS(ISP) : 406,4 $(M+H)^+$

Die Ausgangsverbindung wird wie folgt hergestellt:
Schema 1, (IV) -> (V) -> (VI)

Stufe a) 5-(4'-Amino-2-benzyloxy-6-ethoxy-biphenyl-4-ylmethyl)-pyrimidin-2,4-diamin

Wird in Analogie zu Beispiel 4a (Suzuki-Kopplung mit *in situ* Generierung von $R^2$-$B(OH)_2$)) hergestellt.

Trifluoraceto-*p*-bromanilid (4,02 g; 15 mmol), Bis(pinacolato)diboron (5,0 g; 19,6 mmol), Kaliumacetat (4,41 g; 45 mmol) und 1,1'-Bis(Diphenylphosphino)ferrocendichlorpalladium (II) ($PdCl_2$(dppf)) (730 mg; 0,95 mmol) werden unter Argonbegasung in Dimethoxyethan (150 ml; über Molekularsieb getrocknet) bei 80°C Badtemperatur während 24 Stunden gerührt. Nach Abkühlen auf Raumtemperatur wird das Reaktionsgemisch mit Dimethylformamid (300 ml), Tetrakis-triphenylphosphin-palladium (1,35 g; 1,17 mmol), 5-(3-Benzyloxy-5-ethoxy-4-iod-benzyl)-pyrimidin-2,4-diamin (5,7 g; 12 mmol) und einer 2M wässrigen Kaliumphosphat-Lösung (60 ml) versetzt. Das Reaktionsgemisch wird ca. 15 Stunden bei 77°C gerührt, auf Raumtemperatur abgekühlt und eingeengt. Der erhaltene Rückstand wird in Ethanol (100 ml) aufgenommen, mit einer 1N wässrigen NaOH-Lösung (40 ml) versetzt und 3 Stunden unter Rückfluss gerührt. Nach Abkühlen wird das Ethanol eingedampft, Wasser (50 ml) hinzugefügt, mit Methylenchlorid (200 ml + 100 ml) extrahiert, nacheinander mit Wasser (50 ml) und einer gesättigten wässrigen Kochsalzlösung gewaschen, über Magnesiumsulfat getrocknet, abgenutscht und eingeengt. Der Rückstand wird über Kieselgel (600 g) mit Methylenchlorid/Methanol/$NH_4OH$ (19/1/0,05) chromatographiert. Es werden 2,85 g (54 %) 5-(4'-Amino-2-benzyloxy-6-ethoxy-biphenyl-4-ylmethyl)-pyrimidin-2,4-diamin als beiges Pulver erhalten.
MS(ISP): 442,3 $(M+H)^+$

Stufe b) 4'-Amino-4-(2,4-diamino-pyrimidin-5-ylmethyl)-6-ethoxy-biphenyl-2-ol

5-(4'-Amino-2-benzyloxy-6-ethoxy-biphenyl-4-ylmethyl)-pyrimidin-2,4-diamin (2,78 g; 6,3 mmol) wird in Ethanol (150 ml) und 1N wässriger Salzsäure (18,9 ml) über Pd/C 10 % (1,4 g) hydriert. Nach ca 20 Stunden wird der Katalysator abgenutscht, mit Ethanol nachgewaschen und eingeengt. Der Rückstand wird mit Wasser (100 ml) verrührt. Durch Zugabe von $NH_4OH$ konz. wird das pH auf 9-10 eingestellt. Die Suspension wird abgenutscht, mit Wasser gewaschen und am Hochvakuum getrocknet. Das erhaltene Rohprodukt wird in Methanol (30 ml) ca 30 Minuten gerührt, abgenutscht, mit Methanol gewaschen und am Hochvakuum getrocknet. Es werden 1,02 g (46 %) 4'-Amino-4-(2,4-diamino-pyrimidin-5-ylmethyl)-6-ethoxy-biphenyl-2-ol als hellgraues Pulver erhalten.
MS (ISP): 352,3 $(M+H)^+$

Beispiel 14:

Herstellung von Verbindungen der Formel VIII, Schema 1, aus Verbindungen der Formel VI.

(14a) Methansulfonsäure-3'-amino-4-(2,4-diamino-parrimidin-5-ylmethyl)-6-ethoxybiphenyl-2-ylester (Methode 1: Sulfonierung mit 2,2,2-Trifluorethylester)

3'-Amino-4-(2,4-diamino-pyrimidin-5-ylmethyl)-6-ethoxy-biphenyl-2-ol (527 mg; 1,5 mmol) wird in Acetonitril (60 ml; über Molekularsieb getrocknet) suspendiert, mit Kaliumcarbonat (621 mg ; 4,5 mmol) versetzt und auf 100°C Badtemperatur erhitzt. Es wird 2,2,2-Trifluorethylmethansulfonat (0,353 ml; 3,0 mmol) hinzugefügt und 3 Stunden am Rückfluss gekocht. Nach dieser Zeit werden erneut Kaliumcarbonat (414 mg; 3 mmol) und 2,2,2-Trifluorethylmethansulfonat (0,353 ml; 3,0 mmol) zugegeben und weitere 2 Stunden am Rückfluss gerührt. Dasselbe wird noch viermal mit zweistündigem Abstand wiederholt. Anschliessend wird die Suspension abgenutscht und eingeengt. Der Rückstand wird über Kieselgel (120 g) mit Methylenchlorid/Methanol/$NH_4OH$ (1/1 Gemisch von 90/10/1 und 19/1/0,05) chromatographiert.
Ausbeute: 33 mg (5 %) Methansulfonsäure-3'-amino-4-(2,4-diamino-pyrimidin-5-ylmethyl)-6-ethoxy-biphenyl-2-ylester als farbloses Pulver.
MS ISP): 430,4 $(M+H)^+$

Bei diesem Chromatographieverfahren erhält man auch als zweite Komponente das Endprodukt von Beispiel 7i.

(14b) Ethansulfonsäure-3'-amino-4-(2,4-diamino-pyrimidin-5-ylmethyl)-6-ethoxybiphenyl-2-yl-ester (Methode 2: Sulfonylierung mit Chlorid)

3'-Amino-4-(2,4-diamino-pyrimidin-5-ylmethyl)-6-ethoxy-biphenyl-2-ol (176 mg; 0,5 mmol) wird in Dimethylformamid (11 ml; über Molekularsieb getrocknet) unter Argon gelöst und mit Kalium-tert-butylat (69 mg; 0,6 mmol) versetzt. Nach einstündigem Rühren bei Raumtemperatur wird auf 0-5°C abgekühlt und mit Ethansulfonylchlorid (0,058 ml; 0,6 mmol) versetzt. Nach 2 ½ Stunden bei dieser Temperatur wird das Reaktionsgemisch eingeengt und über Kieselgel (20 g) mit Methylenchlorid/Methanol/$NH_4OH$ (19/1/0,05) chromatographiert.

Ausbeute: 81 mg (36 %) Ethansulfonsäure-3'-amino-4-(2,4-diamino-pyrimidin-5-ylmethyl)-6-ethoxy-biphenyl-2-yl-ester als gelber fester Schaum.
MS (ISP): 444,3 $(M+H)^+$

In Analogie hierzu werden hergestellt:

(14c) Propan-2-sulfonsäure-3'-amino-4-(2,4-diamino-pyrimidin-5-ylmethyl)-6-ethoxybiphenyl-2-yl-ester (Methode 2)

Ausgehend von 3'-Amino-4-(2,4-diamino-pyrimidin-5-ylmethyl)-6-ethoxy-biphenyl-2-ol (176 mg; 0,5 mmol) und 2-Propansulfonylchlorid (0,068 ml; 0,6 mmol) werden 74 mg (32%) Propan-2-sulfonsäure-3'-amino-4-(2,4-diamino-pyrimidin-5-ylmethyl)-6-ethoxybiphenyl-2-yl-ester als gelbes Pulver erhalten.
MS (ISP): 458,4 $(M+H)^+$
NMR $^1H$ (250 MHz, $\delta$, TMS, DMSO): 1.01 (d; J=6.8; 6H); 1.16 (t; J=6.9; 3H); 3.03 (p; J=6.8; 1H); 3.65 (s; 2H); 3.94 (q; J=6.9; 2H); 5.03 (s; 2H); 5.73 (s; 2H); 6.16 (s; 2H); 6.3-6.55 (m; 3H); 6.84 (s; 1H); 6.9-7.1 (m; 2H); 7.58 (s; 1H).

(14d) Propan-1-sulfonsäure-3'-amino-4-(2,4-diamino-pyrimidin-5-ylmethyl)-6-ethoxybiphenyl-2-yl-ester (Methode 2)

Ausgehend von 3'-Amino-4-(2,4-diamino-pyrimidin-5-ylmethyl)-6-ethoxybiphenyl-2-ol (211 mg; 0,6 mmol) und 1-Propan-sulfonylchlorid (0,082 ml; 0,72 mmol) werden 87 mg (32 %) Propan-1-sulfonsäure-3'-amino-4-(2,4-diamino-pyrimidin-5-ylmethyl)-6-ethoxy-biphenyl-2-yl-ester als gelber fester Schaum erhalten.
MS (ISP): 458,4 $(M+H)^+$

(14e) 3-Fluor-4-methoxy-benzolsulfonsäure-3' -amino-4-(2,4-diamino-pyrimidin-5-ylmethyl)-6-ethoxy-biphenyl-2-yl-ester (Methode 2)

Ausgehend von 3'-Amino-4-(2,4-diamino-pyrimidin-5-ylmethyl)-6-ethoxybiphenyl-2-ol (100 mg; 0,28 mmol) und 3-Fluor-4-methoxy-benzolsulfonylchlorid (115 mg; 0,51 mmol) werden 115 mg der Titelverbindung als blass gelber Feststoff erhalten.
MS (ISP): 540,3 $(M+H)^+$

(14f) Thiophen-2-sulfonsäure-3'-amino-4-(2,4-diamino-pyrimidin-5-ylmethyl)-6-ethoxybiphenyl-2-yl-ester (Methode 2)

Ausgehend von 3'-Amino-4-(2,4-diamino-pyrimidin-5-ylmethyl)-6-ethoxybiphenyl-2-ol (100 mg; 0,28 mmol) und 2-Thienylsulfonylchlorid (94 mg; 0,51 mmol) werden 72 mg der Titelverbindung als gelber Schaum isoliert.
MS (ISP): 498,1 $(M+H)^+$

(14g) Butan-2-sulfonsäure-3'-amino-4-(2,4-diamino-pyrimidin-5-ylmethyl)-6-ethoxybiphenyl-2-yl-ester (Methode 2)

Ausgehend von 3'-Amino-4-(2,4-diamino-pyrimidin-5-ylmethyl)-6-ethoxybiphenyl-2-ol (176 mg; 0,50 mmol) und sec-Butyl-sulfonylchlorid (94 mg; 0,60 mmol) werden 159 mg der Titelverbindung als weisser Feststoff isoliert.
MS (ISP): 472,3 $(M+H)^+$
NMR $^1H$ (250 MHz, $\delta$, TMS, DMSO): 0.78 (t, J=7.5, 3H), 0.99 (d, J=7,3H), 1.16 (t, J=7, 3H), 1.25 (m, 1H), 1.63 (m, 1H), 2.73 (m, 1H), 3.65 (s, 2H), 3.96 (q, J=7,2H), 5.04 (br s, 2H), 5.73 (br s, 2H), 6.17 (br s, 2H), 6.35 (d, J=7,1H), 6.43 (s, 1H), 6.52 (br d, J=8.5, 1H), 6.82 (s, 1H), 6.94 (s, 1H), 7.01 (t, J=8,1H), 7.59 (s, 1H).

(14h) 2-Methyl-propan-1-sulfonsäure-3'-amino-4-(2,4-diamino-pyrimidin-5-ylmethyl)-6-ethoxy-biphenyl-2-yl-ester (Methode 2)

Ausgehend von 3'-Amino-4-(2,4-diamino-pyrimidin-5-ylmethyl)-6-ethoxybiphenyl-2-ol (176 mg; 0,50 mmol) und iso-Butyl-sulfonylchlorid (94 mg; 0,60 mmol) werden 129 mg der Titelverbindung als blass-gelbe Kristalle isoliert.
MS (ISP): 472,3 $(M+H)^+$

(14i) 2,2-Dimethyl-propan-1-sulfonsäure-3'-amino-4-(2,4-diamino-pyrimidin-5-ylmethyl)-6-ethoxy-biphenyl-2-yl-ester

3'-Amino-4-(2,4-diamino-pyrimidin-5-ylmethyl)-6-ethoxy-biphenyl-2-ol (176 mg, 0,50 mmol) werden in 3 ml abs. Dimethylformamid vorgelegt und mit 500 mg pulverisiertem Molekularsieb 4 A versetzt. Man rührt 15 Min., kühlt auf 0°, gibt 69 mg (0,60 mmol) KOtBu zu und versetzt nach weiteren 5 Min. mit 2,2-Dimethyl-propan-1-sulfonylchlorid (102 mg; 0,50 mmol). Man lässt 5/4 h bei 0° und 2 h bei Raumtemperatur reagieren. Abfiltrieren, Eindampfen unter vermindertem Druck , gefolgt von Flash-Chromatographie mit $SiO_2$ ($CH_2Cl_2$/MeOH/25% $NH_3$(19/1/0,05)) liefert, nach

Verrühren mit EtOEt, 106 mg der Titelverbindung als farblose Kristalle.
Smp: 103° dec.
MS (ISP): 486,4 $(M+H)^+$

Die obige Ausgangsverbindung 3'-Amino-4-(2,4-diamino-pyrimidin-5-ylmethyl)-6-ethoxy-biphenyl-2-ol wird wie in Beispiel 7 beschrieben hergestellt.

Beispiel 15:

In Analogie zu Beispiel 14 (Methode 1 Sulfonieerung mit 2,2,2-Trifluorethylester oder 2 Sulfonierung mit Chlorid nach Angabe) werden Verbindungen der Formel VIII, Schema 1, aus Verbindungen der Formel VI hergestellt.

(15a) Methansulfonsäure-4-(2,4-diamino-pyrimidin-5-ylmethyl)-6-ethoxy-4'-hydroxy-biphenyl-2-yl-ester (Methode 1)

Ausgehend von 4-(2,4-Diamino-pyrimidin-5-ylmethyl)-6-ethoxy-4'methoxymethoxy-biphenyl-2-ol (594 mg; 1,5 mmol) erhält man nach den Angaben von Beispiel 8s 68 mg (19 %) Methanesulfonsäure-4-(2,4-diamino-pyrimidin-5-ylmethyl)-6-ethoxy-4'-hydroxy-biphenyl-2-yl-ester als farbloses Pulver.
MS (ISP): 429,3 $(M+H)^+$

Bei der abschliessenden Chromatographie erhält man auch als zweite Komponente das Endprodukt von Beispiel 8s.

(15b) Ethansulfonsäure-4-(2,4-diamino-pyrimidin-5-ylmethyl)-6-ethoxy-4'-hydroxybiphenyl-2-yl-ester (Methode 2)

Ausgehend von 4-(2,4-Diamino-pyrimidin-5-ylmethyl)-6-ethoxy-4'methoxymethoxy-biphenyl-2-ol (248 mg; 0,63 mmol) werden durch Reaktion mit Ethansulfonylchlorid (0,073 ml; 0,75 mmol) und anschliessende saure Abspaltung der Methoxymethyl-Gruppe (Beispiel 8a, Stufe b) 99 mg (36 %) Ethansulfonsäure-4-(2,4-diamino-pyrimidin-5-ylmethyl)-6-ethoxy-4'-hydroxy-biphenyl-2-yl-ester als farbloses Pulver erhalten.
MS (ISP): 445,3 $(M+H)^+$

(15c) Propan-2-sulfonsäure-4-(2,4-diamino-pyrimidin-5-ylmethyl)-6-ethoxy-4'-hydroxybiphenyl-2-yl-ester (Methode 2)

Ausgehend von 4-(2,4-Diamino-pyrimidin-5-ylmethyl)-6-ethoxy-4'-methoxymethoxy-biphenyl-2-ol (238 mg; 0,60 mmol) werden durch Reaktion mit 2-Propansulfonylchlorid (0,082 ml; 0,72 mmol) und anschliessende saure Abspaltung der Methoxymethyl-Gruppe (Beispiel 8a, Stufe b) 125 mg (45 %) Propan-2-sulfonsäure-4-(2,4-diamino-pyrimidin-5-ylmethyl)-6-ethoxy-4'-hydroxy-biphenyl-2-yl-ester als farbloses Pulver erhalten.
MS (ISP): 459,4 $(M+H)^+$

(15d) Propan-1-sulfonsäure-4-(2,4-diamino-pyrimidin-5-ylmethyl)-6-ethoxy-4'-hydroxybiphenyl-2-yl-ester (Methode 2)

Ausgehend von 4-(2,4-Diamino-pyrimidin-5-ylmethyl)-6-ethoxy-4'-methoxymethoxy-biphenyl-2-ol (238 mg; 0,60 mmol) werden durch Reaktion mit 1-Propansulfonylchlorid (0,164 ml; 1,44 mmol in zwei Portionen) und anschliessende saure Abspaltung der Methoxymethyl-Gruppe (Beispiel 8a, Stufe b) 150 mg (54 %) Propan-1-sulfonsäure-4-(2,4-diamino-pyrimidin-5-ylmethyl)-6-ethoxy-4'-hydroxy-biphenyl-2-ylester als hellgelbes Pulver erhalten.
MS (ISP): 459,4 $(M+H)^+$

(15e) Cyclopropansulfonsäure-4-(2,4-diamino-pyrimidin-5-ylmethyl)-6-ethoxy-4'-hydroxy-biphenyl-2-yl-ester (Methode 2)

Ausgehend von 4-(2,4-Diamino-pyrimidin-5-ylmethyl)-6-ethoxy-4'-methoxymethoxy-biphenyl-2-ol (792 mg; 2,0 mmol) werden durch Reaktion mit Cyclopropylsulfonylchlorid (453 mg; 3,0 mmol in zwei Portionen) und Kaliumcarbonat (480 mg; 3,0 mmol) in Dimethylformamid (20 ml) bei Raumtemperatur und anschliessende saure Abspaltung der Methoxymethyl-Gruppe (Beispiel 8a, Stufe b) 70 mg (8 %) Cyclopropansulfonsäure-4-(2,4-diamino-pyrimidin-5-ylmethyl)-6-ethoxy-4'-hydroxybiphenyl-2-yl-ester als farbloses Pulver erhalten.
MS (ISP): 457,3 $(M+H)^+$

(15f) 2-Methyl-propan-1-sulfonsäure-4-(2,4-diamino-pyrimidin-5-ylmethyl)-6-ethoxy-4'-hydroxy-biphenyl-2-yl-ester (Methode 2)

Ausgehend von 4-(2,4-Diamino-pyrirnidin-5-ylmethyl)-6-ethoxy-4'-methoxymethoxy-biphenyl-2-ol (198 mg;

0,50 mmol) werden durch Reaktion mit iso-Butylsulfonylchlorid (94 mg; 0,60 mmol) und anschliessende saure Abspaltung der Methoxymethyl-Gruppe 151 mg der Titelverbindung als blass-gelbe Kristalle erhalten.
MS (ISP): 473,2 $(M+H)^+$

(15g) Butan-2-sulfonsäure-4-(2,4-diamino-pyrimidin-5-ylmethyl)-6-ethoxy-4'-hydroxybiphenyl-2-yl-ester (Methode 2)

Ausgehend von 4-(2,4-Diamino-pyrimidin-5-ylmethyl)-6-ethoxy-4'-methoxymethoxy-biphenyl-2-ol (198 mg; 0,50 mmol) werden durch Reaktion mit sec-Butyl-sulfonylchlorid (94 mg; 0,60 mmol) und anschliessende saure Abspaltung der Methoxymethyl-Gruppe 112 mg der Titelverbindung als nahezu farblose Kristalle erhalten.
MS (ISP): 473,3 $(M+H)^+$

(15h) 2,2-Dimethyl-propan-1-sulfonsäure-4-(2,4-diamino-pyrimidin-5-ylmethyl)-6-ethoxy-4'-hydroxy-biphenyl-2-yl-ester (Methode 2)

Ausgehend von 4-(2,4-Diamino-pyrimidin-5-ylmethyl)-6-ethoxy-4'methoxymethoxy-biphenyl-2-ol (158 mg ; 0,40 mmol) werden durch Reaktion mit Neopentyl-sulfonylchlorid (82 mg; 0,48 mmol) und anschliessende saure Abspaltung der Methoxymethyl-Gruppe 115 mg der Titelverbindung als nahezu farblose Kristalle erhalten.
MS (ISP): 487,3 $(M+H)^+$

(15i) 2-Methyl-propan-2-sulfinsäure-4-(2,4-diamino-pyrimidin-5-ylmethyl)-6-ethoxy-4'-hydroxy-biphenyl-2-yl-ester (Methode 2)

Ausgehend von 4-(2,4-Diamino-pyrimidin-5-ylmethyl)-6-ethoxy-4'-methoxymethoxy-biphenyl-2-ol (238 mg; 0,60 mmol) werden durch Reaktion mit tert-Butylsulfinylchlorid (101 mg; 0,72 mmol) und anschliessende saure Abspaltung der Methoxymethyl-Gruppe (Beispiel 8a, Stufe b) 51 mg (53 %) 2-Methyl-propan-2-sulfinsäure-4-(2,4-diamino-pyrimidin-5-ylmethyl)-6-ethoxy-4'-hydroxy-biphenyl-2-ylester als farbloses Pulver erhalten.
MS (ISP): 457,5 $(M+H)^+$

(15j) 3-Fluor-4-methoxy-benzolsulfonsäure-4-(2,4-diamino-pyrimidin-5-ylmethyl)-6-ethoxy-4'-hydroxy-biphenyl-2-yl-ester (Methode 2)

Ausgehend von 4-(2,4-Diamino-pyrimidin-5-ylmethyl)-6-ethoxy-4'-methoxymethoxy-biphenyl-2-ol (200 mg ; 0,40 mmol) werden durch Reaktion mit 3-Fluor-4-methoxy-benzolsulfonylchlorid (204 mg; 0,91 mmol) und anschliessende saure Abspaltung der Methoxymethyl-Gruppe 145 mg der Titelverbindung als blass-gelbe Kristalle erhalten.
MS (ISP): 541,1 $(M+H)^+$

Die obige Ausgangsverbindung 4-(2,4-Diamino-pyrimidin-5-ylmethyl)-6-ethoxy-4'methoxymethoxy-biphenyl-2-ol wird wie in Beispiel 8 beschrieben hergestellt.

Beispiel 16:

In Analogie zu Beispiel 14, Methode 2 dh Sulfonierung mit Chlorid, werden Verbindungen der Forel VIII, Schema 1, aus Verbindungen der Formel VI hergestellt.

(16a) Methansulfonsäure-4-(2,4-diamino-pyrimidin-5-ylmethyl)-6-ethoxy-4'-methoxybiphenyl-2-yl-ester

Ausgehend von 4-(2,4-Diamino-pyrimidin-5-ylmethyl)-6-ethoxy-4'-methoxybiphenyl-2-01 (366 mg; 1 mmol) werden durch Reaktion mit Methansulfonylchlorid (0,100 ml; 1,3 mmol) 121 mg (27 %) ) Methansulfonsäure-4-(2,4-diamino-pyrimidin-5-ylmethyl)-6-ethoxy-4'-methoxy-biphenyl-2-yl-ester als farbloses Pulver erhalten.
MS (ISP): 445,2 $(M+H)^+$

(16b) Ethansulfonsäure-4-(2,4-diamino-pyrimidin-5-ylmethyl)-6-ethoxy-4'-methoxybiphenyl-2-yl-ester

Ausgehend von 4-(2,4-Diamino-pyrimidin-5-ylmethyl)-6-ethoxy-4'-methoxybiphenyl-2-ol (183 mg; 0,5 mmol) werden durch Reaktion mit Ethansulfonylchlorid (0,087 ml; 0,9 mmol) 98 mg (43 %) ) Ethansulfonsäure-4-(2,4-diamino-pyrimidin-5-ylmethyl)-6-ethoxy-4'-methoxy-biphenyl-2-yl-ester als hellgelber fester Schaum erhalten. MS (ISP): 459,3 $(M+H)^+$

(16c) Propan-2-sulfonsäure-4-(2,4-diamino-pyrimidin-5-ylmethyl)-6-ethoxy-4'-methoxybiphenyl-2-yl-ester

Ausgehend von 4-(2,4-Diamino-pyrimidin-5-ylmethyl)-6-ethoxy-4'-methoxybiphenyl-2-ol (183 mg; 0,5 mmol) werden durch Reaktion mit 2-Propylsulfonylchlorid (0,136 ml; 1,2 mmol in 2 Portionen) 58 mg (24 %) ) Propan-2-sulfonsäure-4-(2,4-diamino-pyrimidin-5-ylmethyl)-6-ethoxy-4'-methoxy-biphenyl-2-yl-ester als hellgelbes Pulver erhalten.
MS (ISP): 473,3 $(M+H)^+$

Die obige Ausgangsverbindung 4-(2,4-Diamino-pyrimidin-5-ylmethyl)-6-ethoxy-4'methoxy-biphenyl-2-ol wird wie in Beispiel 9 beschrieben hergestellt.

Beispiel 17:

In Analogie zu Beispiel 14, Methode 2 dh Sulfonierung mit Chlorid, werden Verbindungen der Formel VIII, Schema 1, aus Verbindungen der Formel VI hergestellt.

(17a) Methansulfonsäure-2-benzo[1,3]dioxol-5-yl-5-(2,4-diamino-pyrimidin-5-ylmethyl)-3-ethoxy-phenyl-ester

Ausgehend von 2-Benzo[1,3]dioxol-5-yl-5-(2,4-diamino-pyrimidin-5-ylmethyl)-3-ethoxy-phenol (380 mg; 1,0 mmol) werden durch Reaktion mit Methansulfonylchlorid (0,100 ml; 1,3 mmol) 93 mg (20 %) ) Methansulfonsäure-2-benzo[1,3]dioxol-5-yl-5-(2,4-diamino-pyrimidin-5-ylmethyl)-3-ethoxy-phenyl-ester als hellgelbes Pulver erhalten.
MS (ISP): 459,3 $(M+H)^+$

(17b) Ethansulfonsäure-2-benzo[1,3]dioxol-5-yl-5-(2,4-diamino-pyrimidin-5-ylmethyl)-3-ethoxy-phenyl-ester

Ausgehend von 2-Benzo[1,3]dioxol-5-yl-5-(2,4-diamino-pyrimidin-5-ylmethyl)-3-ethoxy-phenol (228 mg; 0,6 mmol) werden durch Reaktion mit Ethansulfonylchlorid (0,140ml; 1,44 mmol in 2 Portionen) 182 mg (64 %) ) Ethansulfonsäure-2-benzo-[1,3]dioxol-5-yl-5-(2,4-diamino-pyrimidin-5-ylmethyl)-3-ethoxy-phenyl-ester als hellgelber fester Schaum erhalten.
MS (ISP): 473,2 $(M+H)^+$

(17c) Propan-2-sulfonsäure-2-benzo[1,3]dioxol-5-yl-5-(2,4-diamino-pyrimidin-5-ylmethyl)-3-ethoxy-phenyl-ester

Ausgehend von 2-Benzo[1,3]dioxol-5-yl-5-(2,4-diamino-pyrimidin-5-ylmethyl)-3-ethoxy-phenol (228 mg; 0,6 mmol) werden durch Reaktion mit 2-Propansulfonylchlorid (0,164 ml; 1,44 mmol in 2 Portionen) 100 mg (34 %) Propan-2-sulfonsäure-2-benzo-[1,3]dioxol-5-yl-5-(2,4-diamino-pyrimidin-5-ylmethyl)-3-ethoxy-phenyl-ester als hellgelber fester Schaum erhalten.
MS (ISP): 487,2 $(M+H)^+$

(17d) Propan-1-sulfonsäure-2-benzo[1,3]dioxol-5-yl-5-(2,4-diamino-pyrimidin-5-ylmethyl)-3-ethoxy-phenyl-ester

Ausgehend von 2-Benzo[1,3]dioxol-5-yl-5-(2,4-diamino-pyrimidin-5-ylmethyl)-3-ethoxy-phenol (228 mg; 0,6 mmol) werden durch Reaktion mit 1-Propansulfonylchlorid (0,161 ml; 1,44 mmol in 2 Portionen) 100 mg (29 %) Propan-1-sulfonsäure-2-benzo[1,3]dioxol-5-yl-5-(2,4-diamino-pyrimidin-5-ylmethyl)-3-ethoxy-phenyl-ester als hellgelber fester Schaum erhalten.
MS (ISP): 487,2 $(M+H)^+$

Die obige Ausgangsverbindung 2-Benzo[1,3]dioxol-5-yl-5-(2,4-diamino-pyrimidin-5-ylmethyl)-3-ethoxy-phenol wird wie in Beispiel 10 beschrieben hergestellt.

Beispiel 18:

In Analogie zu Beispiel 14, Methode 2 dh Sulfonierung mit Chlorid, wie in Schema 1, (VI) -> (VIII) folgende Verbindung hergestellt.

Propan-2-sulfonsäure-4'-amino-4-(2,4-diamino-pyrimidin-5-ylmethyl)-6-ethoxybiphenyl-2-yl-ester

Ausgehend von 4'-Amino-4-(2,4-diamino-pyrimidin-5-ylmethyl)-6-ethoxybiphenyl-2-ol (246 mg; 0,7 mmol) werden durch Reaktion mit 2-Propansulfonylchlorid (0,192 ml; 1,68 mmol in 2 Portionen) 49 mg (12 %) Propan-2-sulfonsäure-4'-amino-4-(2,4-diamino-pyrimidin-5-ylmethyl)-6-ethoxy-biphenyl-2-yl-ester als hellgelber fester Schaum erhalten.

MS (ISP): 458,4 (M+H)+
NMR $^1$H(250 MHz, δ, TMS, DMSO): 1.04 (d; J=6.8; 6H); 1.17 (t; J=6.8; 3H); 3.08 (p; J=6.8; 1H); 3.63 (s; 2H); 3.94 (q; J=6.8; 2H); 5.10 (s; 2H); 5.73 (s; 2H); 6.16 (s; 2H); 6.56 (d; J=8.5; 2H); 6.8-6.9 (m; 4H); 7.58 (s; 1H).
Die obige Ausgangsverbindung 4'-Amino-4-(2,4-diamino-pyrimidin-5-ylmethyl)-6-ethoxy-biphenyl-2-öl wird wie in Beispiel 13 beschrieben hergestellt.

Beispiel 19:

Herstellung von Verbindungen der Formel IX, Schema 1, aus Verbindungen der Formel VI.

(19a) Dimethyl-sulfaminsäure-3'-amino-4-(2,4-diamino-pyrimidin-5-ylmethyl)-6-ethoxybiphenyl-2-yl-ester

3'-Amino-4-(2,4-diamino-pyrimidin-5-ylmethyl)-6-ethoxy-biphenyl-2-ol (200 mg; 0,57 mmol) wird in Dimethylformamid (11 ml; über Molekularsieb getrocknet) unter Argon gelöst und mit Kalium-tert-butylat (77 mg; 0,68 mmol) versetzt. Nach einstündigem Rühren bei Raumtemperatur wird mit N,N-Dimethyl-sulfamoylchlorid (0,073 ml; 0,68 mmol) versetzt. Nach 3 Stunden bei dieser Temperatur wird das Reaktionsgemisch eingeengt und über Kieselgel (38 g) mit Methylenchlorid/Methanol/$NH_4OH$ (Gemisch 1/1 von 19/1/0,05 und 90/10/1) chromatographiert.
Ausbeute:144 mg (55 %) Dimethyl-sulfaminsäure-3'-amino-4-(2,4-diamino-pyrimidin-5-ylmethyl)-6-ethoxy-biphenyl-2-yl-ester als gelber fester Schaum.
MS (ISP): 459,5 (M+H)+
In Analogie hierzu werden hergestellt:

(19b) Piperidin-1-sulfonsäure-3'-amino-4-(2,4-diamino-pyrimidin-5-ylmethyl)-6-ethoxybiphenyl-2-yl-ester

Ausgehend von 3'-Amino-4-(2,4-diamino-pyrimidin-5-ylmethyl)-6-ethoxybiphenyl-2-ol (200 mg; 0,57 mmol) werden durch Reaktion mit 1-Piperidinsulfonylchlorid (136 mg; 0,74 mmol) 131 mg (46 %) Piperidin-1-sulfonsäure-3'-amino-4-(2,4-diamino-pyrimidin-5-ylmethyl)-6-ethoxy-biphenyl-2-yl-ester als gelbliches Pulver erhalten.
MS (ISP): 499,3 (M+H)+

(19c) Pyrrolidin-1-sulfonsäure-3'-amino-4-(2,4-diamino-pyrimidin-5-ylmethyl)-6-ethoxy-biphenyl-2-yl-ester

Ausgehend von 3'-Amino-4-(2,4-diamino-pyrimidin-5-ylmethyl)-6-ethoxybiphenyl-2-ol (200 mg; 0,57 mmol) werden durch Reaktion mit 1-Pyrrolidinsulfonylchlorid (157 mg; 0,925 mmol) 174 mg (63 %) Pyrrolidin-1-sulfonsäure-3'-amino-4-(2,4-diamino-pyrimidin-5-ylmethyl)-6-ethoxy-biphenyl-2-yl-ester als gelbliches Pulver erhalten.
MS (ISP): 485,4 (M+H)+

(19d) N-Isopropyl-N-methyl-sulfaminsäure-3'-amino-4-(2,4-diamino-pyrimidin-5-ylmethyl)-6-ethoxy-biphenyl-2-yl-ester

Ausgehend von 3'-Amino-4-(2,4-diamino-pyrimidin-5-ylmethyl)-6-ethoxybiphenyl-2-ol (200 mg; 0,57 mmol) werden durch Reaktion mit N-Isopropyl-N-methyl-sulfamoylchlorid (127 mg; 0,74 mmol) 116 mg (35 %) N-Isopropyl-N-methyl-sulfaminsäure-3'-amino-4-(2,4-diamino-pyrimidin-5-ylmethyl)-6-ethoxy-biphenyl-2-yl-ester als oranges Pulver erhalten.
MS (ISP): 487,3 (M+H)+
Das eingesetzte N-Isopropyt-N-methyl-sulfamoylchlorid wird wie folgt hergestellt:
Sulfurylchlorid (8,1 ml, 100 mmol) wird in Toluol (80 ml) gelöst und bei 0-5°C mit einer Lösung von N-Isopropyl-N-methylamin (10,4 ml; 100 mmol) in Toluol (10 ml) innerhalb 30 Minuten versetzt. Das Reaktionsgemisch wird anschliessend 30 Minuten bei der gleichen Temperatur und 2 Stunden bei Raumtemperatur gerührt. Danach wird das Reaktionsgemisch auf Eiswasser (ca 150 ml) unter heftigem Rühren gegossen. Die organische Phase wird abgetrennt, mit 1N wässriger Salzsäure-Lösung gewaschen, über Magnesiumsulfat getrocknet,abgenutscht und eingeengt (40°C 70-30 mBar). Das Rohprodukt wird anschliessend destilliert.
Ausbeute: 3,95 g (23 %) N-Isopropyl-N-methyl-sulfamoylchlorid als gelbes Öl. Siedepunkt: 80°C bei 13 mBar

(19e) Morpholin-4-sulfonsäure-3'-amino-4-(2,4-diamino-pyrimidin-5-ylmethyl)-6-ethoxy-biphenyl-2-yl-ester

Ausgehend von 3'-Amino-4-(2,4-diamino-pyrimidin-5-ylmethyl)-6-ethoxybiphenyl-2-ol (200 mg; 0,57 mmol) werden durch Reaktion mit 4-Morpholinsulfonylchlorid (137 mg; 0,74 mmol) 159 mg (56 %) Morpholin-4-sulfonsäure-3'-amino-4-(2,4-diamino-pyrimidin-5-ylmethyl)-6-ethoxy-biphenyl-2-yl-ester als gelbes Pulver erhalten.

MS (ISP): 501,3 $(M+H)^+$

(19f) N-Ethyl-N-methyl-sulfaminsäure-3'-amino-4-(2,4-diamino-pyrimidin-5-ylmethyl)-6-ethoxy-biphenyl-2-yl-ester

Ausgehend von 3'-Amino-4-(2,4-diamino-pyrimidin-5-ylmethyl)-6-ethoxybiphenyl-2-ol (176 mg; 0,5 mmol) werden durch Reaktion mit N-Ethyl-N-methylsulfamoylchlorid (95 mg; 0,6 mmol) 155 mg (65 %) N-Ethyl-N-methylsulfaminsäure-3'-amino-4-(2,4-diamino-pyrimidin-5-ylmethyl)-6-ethoxy-biphenyl-2-yl-ester als farbloses Pulver erhalten.
MS (ISP): 473,3 $(M+H)^+$

(19g) N-Cyclopropyl-N-methyl-sulfaminsäure-3'-amino-4-(2,4-diamino-pyrimidin-5-ylmethyl)-6-ethoxy-biphenyl-2-yl-ester

Ausgehend von 3'-Amino-4-(2,4-diamino-pyrimidin-5-ylmethyl)-6-ethoxybiphenyl-2-ol (176 mg; 0,5 mmol) werden durch Reaktion mit N-Cyclopropyl-N-methylsulfamoylchlorid (102 mg; 0,6 mmol) 154 mg (61 %) N-Cyclopropyl-N-methylsulfaminsäure-3'-amino-4-(2,4-diamino-pyrimidin-5-ylmethyl)-6-ethoxy-biphenyl-2-ylester als farbloses Pulver erhalten.
MS (ISP): 485,3 $(M+H)^+$
Das eingesetzte N-Cyclopropyl-N-methyl-sulfamoylchlorid wird in Analogie zu N-Isopropyl-N-methyl-sulfamoylchlorid (in Beispiel 9d beschrieben) hergestellt:
Ausgehend von N-Cyclopropyl-N-methylamin (4,48 g; 63 mmol) werden 1,29 g (12 %) N-Cyclopropyl-N-methyl-sulfamoylchlorid als gelbes Öl erhalten.
Siedepunkt: 75-100°C bei 9 mBar.

(19h) Diethyl-sulfaminsäure-3'-amino-4-(2,4-diamino-pyrimidin-5-ylmethyl)-6-ethoxybiphenyl-2-yl-ester

Ausgehend von 3'-Amino-4-(2,4-diamino-pyrimidin-5-ylmethyl)-6-ethoxybiphenyl-2-ol (176 mg; 0,5 mmol) werden durch Reaktion mit N,N-Diethylsulfamoylchlorid (103 mg; 0,6 mmol) 150 mg (55 %) Diethyl-sulfaminsäure-3'-amino-4-(2,4-diamino-pyrimidin-5-ylmethyl)-6-ethoxy-biphenyl-2-yl-ester als farbloses Pulver erhalten.
MS (ISP): 487,3 (M+H)+
Die obige Ausgangsverbindung 3'-Amino-4-(2,4-diamino-pyrimidin-5-ylmethyl)-6-ethoxy-biphenyl-2-ol wird wie in Beispiel 7 beschrieben hergestellt.

Beispiel 20:

In Analogie zu Beispiel 19 werden Verbindungen der Formel IX, Schema 1, aus Verbindungen der Formel VI hergestellt.

(20a) Dimethyl-sulfaminsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'methoxymethoxy-biphenyl-2-ol (200 mg; 0,5 mmol) werden durch Reaktion mit N,N-Dimethylsulfamoylchlorid (0,084 ml; 0,5 mmol) und anschliessende saure Abspaltung der Methoxymethyl-Gruppe (Analog zu Beispiel 8a, Stufe b) 79 mg (31 %) Dimethylsulfaminsäure-4-(2,4-diamino-pyrimidin-5-ylmethyl)-6-ethoxy-4'-hydroxy-biphenyl-2-yl-ester als beiges Pulver erhalten.
MS (ISP): 460,5 $(M+H)^+$

(20b) Piperidin-1-sulfonsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'-methoxymethoxy-biphenyl-2-ol (200 mg; 0,5 mmol) werden durch Reaktion mit 1-Piperidinsulfonylchlorid (120 mg; 0,66 mmol) und anschliessende saure Abspaltung der Methoxymethyl-Gruppe (Analog zu Beispiel 8a, Stufe b) 106 mg (43 %) Piperidin-1-sulfonsäure-4-(2,4-diamino-pyrimidin-5-ylmethyl)-6-ethoxy-4'-hydroxy-biphenyl-2-ylester als farbloses Pulver erhalten.
MS (ISP): 500,3 $(M+H)^+$

(20c) Pyrrolidin-1-sulfonsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'methoxymethoxy-biphenyl-2-ol (200 mg; 0,5 mmol) werden durch Reaktion mit 1-Pyrrolidinsulfonylchlorid (148 mg; 0,87 mmol) und anschliessende saure Abspaltung der Methoxymethyl-Gruppe (Analog zu Beispiel 8a, Stufe b) 149 mg (62 %) Pyrrolidin-1-sulfonsäure-4-(2,4-diamino-pyrimidin-5-ylmethyl)-6-ethoxy-4'-hydroxy-biphenyl-2-ylester als farbloses Pulver erhalten.
MS (ISP): 486,3 $(M+H)^+$

(20d) Morpholin-4-sulfonsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'methoxymethoxy-biphenyl-2-ol (200 mg; 0,5 mmol) werden durch Reaktion mit 4-Morpholinsulfonylchlorid (162 mg; 0,88 mmol) und anschliessende saure Abspaltung der Methoxymethyl-Gruppe (Analog zu Beispiel 8a, Stufe b) 85 mg (41 %) Morpholin-4-sulfonsäure-4-(2,4-diamino-pyrimidin-5-ylmethyl)-6-ethoxy-4'-hydroxy-biphenyl-2-ylester als farbloses Pulver erhalten.
MS (ISP): 502,2 $(M+H)^+$

(20e) N-Isopropyl-N-methyl-sulfaminsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'methoxymethoxy-biphenyl-2-ol (300 mg; 0,76 mmol) werden durch Reaktion mit N-Isopropyl-N-methyl-sulfamoylchlorid (169 mg; 0,98 mmol) ) und anschliessende saure Abspaltung der Methoxymethyl-Gruppe (Analog zu Beispiel 8a, Stufe b) 105 mg (28 %) N-Isopropyl-N-methyl-sulfaminsäure-4-(2,4-diamino-pyrimidin-5-ylmethyl)-6-ethoxy-4'hydroxy-biphenyl-2-yl-ester als farbloses Pulver erhalten.
MS (ISP): 488,3 $(M+H)^+$
Das eingesetzte N-Isopropyl-N-methyl-sulfamoylchlorid wird wie in Beispiel 19d beschrieben hergestellt.

(20f) N-Cyclopropyl-N-methyl-sulfaminsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'methoxymethoxy-biphenyl-2-ol (300 mg; 0,76 mmol) werden durch Reaktion mit N-Cyclopropyl-N-methylsulfamoylchlorid (153 mg; 0,98 mmol) und anschliessende saure Abspaltung der Methoxymethyl-Gruppe (Analog zu Beispiel 8a, Stufe b) 205 mg (56 %) N-Cyclopropyl-N-methyl-sulfaminsäure-4-(2,4-diamino-pyrimidin-5-ylmethyl)-6-ethoxy-4'hydroxy-biphenyl-2-yl-ester als farbloses Pulver erhalten.
MS (ISP): 486,3 $(M+H)^+$
Das eingesetzte N-Cyclopropyl-N-methylsulfamoylchlorid wird wie in Beispiel 19g beschrieben hergestellt.

(20g) N-Ethyl-N-methyl-sulfaminsäure-4-(2,4-diamino-pyrimidin-5-ylmethyl)-6-ethoxy-4'-hydroxy-biphenyl-2-yl-ester

Ausgehend von 4-(2,4-Diamino-pyrimidin-5-ylmethyl)-6-ethoxy-4'-methoxymethoxy-biphenyl-2-ol (300 mg; 0,76 mmol) werden durch Reaktion mit N-Ethyl-N-methylsulfamoylchlorid (207 mg; 1,3 mmol) und anschliessende saure Abspaltung der Methoxymethyl-Gruppe (Analog zu Beispiel 8a, Stufe b) 174 mg (48 %) N-Ethyl-N-methyl-sulfaminsäure-4-(2,4-diamino-pyrimidin-5-ylmethyl)-6-ethoxy-4'-hydroxybiphenyl-2-yl-ester als farbloses Pulver erhalten.
MS (ISP): 474,3 $(M+H)^+$

(20h) Diethyl-sulfaminsäure-4-(2,4-diamino-pyrimidin-5-ylmethyl)-6-ethoxy-4'-hydroxybiphenyl-2-yl-ester

Ausgehend von 4-(2,4-Diamino-pyrimidin-5-ylmethyl)-6-ethoxy-4'-methoxymethoxy-biphenyl-2-ol (200 mg; 0,5 mmol) werden durch Reaktion mit N,N-Diethylsulfamoylchlorid (250 mg; 0,87 mmol) und anschliessende saure Abspaltung der Methoxymethyl-Gruppe (Analog zu Beispiel 8a, Stufe b) 100 mg (41 %) Diethylsulfaminsäure-4-(2,4-diamino-pyrimidin-5-ylmethyl)-6-ethoxy-4'-hydroxy-biphenyl-2-yl-ester als farbloses Pulver erhalten.
MS (ISP): 488,3 $(M+H)^+$
Die obige Ausgangsverbindung 4-(2,4-Diamino-pyrimidin-5-ylmethyl)-6-ethoxy-4'methoxymethoxy-biphenyl-2-ol wird wie in Beispiel 8 beschrieben hergestellt.

Beispiel 21:

In Analogie zu Beispiel 19 wird hergestellt: folgende Verbindung der Formel 1X Schema 1, aus der entsprechenden Verbindung der Formel VI hergestellt.

Dimethylsulfaminsäure-4-(2,4-diamino-pyrimidin-5-ylmethyl)-6-ethoxy-4'-methoxybiphenyl-2-yl-ester

Ausgehend von 4-(2,4-Diamino-pyrimidin-5-ylmethyl)-6-ethoxy-4'-methoxybiphenyl-2-ol (200 mg; 0,55 mmol) werden durch Reaktion mit N,N-Dimethylsulfamoylchlorid (0,105 ml; 0,97 mmol) 54 mg (21 %) Dimethylsulfaminsäure-4-(2,4-diamino-pyrimidin-5-ylmethyl)-6-ethoxy-4'-methoxy-biphenyl-2-yl ester als farbloses Pulver erhalten.
MS (ISP): 474,3 $(M+H)^+$

Die obige Ausgangsverbindung 4-(2,4-Diamino-pyrimidin-5-ylmethyl)-6-ethoxy-4'methoxymethoxy-biphenyl-2-ol wird wie in Beispiel 9 beschrieben hergestellt.

Beispiel 22:

In Analogie zu Beispiel 19 werden Verbindungen der Formel IX, Schema 1, aus den entsprechenden Verbindungen der Formel VI hergestellt:

(22a) Dimethylsulfaminsäure-4'-amino-4-(2,4-diamino-pyrimidin-5-ylmethyl)-6-ethoxybiphenyl-2-yl-ester

Ausgehend von 4'-Amino-4-(2,4-diamino-pyrimidin-5-ylmethyl)-6-ethoxybiphenyl-2-ol (211 mg; 0,6 mmol) werden durch Reaktion mit N,N-Dimethylsulfamoylchlorid (0,078 ml; 0,72 mmol) 180 mg (66 %) Dimethylsulfaminsäure-4'-amino-4-(2,4-diamino-pyrimidin-5-ylmethyl)-6-ethoxy-biphenyl-2-yl-ester als leicht gelbes Pulver erhalten.
MS (ISP): 459,5 $(M+H)^+$

(22b) N-Ethyl-N-methyl-sulfaminsäure-4'-amino-4-(2,4-diamino-pyrimidin-5-ylmethyl)-6-ethoxy-biphenyl-2-yl-ester

Ausgehend von 4'-Amino-4-(2,4-diamino-pyrimidin-5-ylmethyl)-6-ethoxybiphenyl-2-ol (176 mg; 0,5 mmol) werden durch Reaktion mit N-Ethyl-N-methylsulfamoylchlorid (95 mg; 0,6 mmol) 148 mg (58 %) N-Ethyl-N-methylsulfaminsäure-4'-amino-4-(2,4-diamino-pyrimidin-5-ylmethyl)-6-ethoxy-biphenyl-2-yl-ester als leicht gelbes Pulver erhalten.
MS (ISP): 473,3 $(M+H)^+$

(22c) Pyrrolidin-1-sulfonsäure-4'-amino-4-(2,4-diamino-pyrimidin-5-ylmethyl)-6-ethoxy-biphenyl-2-yl-ester

Ausgehend von 4'-Amino-4-(2,4-diamino-pyrimidin-5-ylmethyl)-6-ethoxybiphenyl-2-ol (176 mg; 0,5 mmol) werden durch Reaktion mit 1-Pyrrolidinsulfonylchlorid (110 mg; 0,65 mmol) 130 mg (54 %) Pyrrolidin-1-sulfonsäure-4'-amino-4-(2,4-diamino-pyrimidin-5-ylmethyl)-6-ethoxy-biphenyl-2-yl-ester als gelbes Pulver erhalten.
MS (ISP): 485,3 $(M+H)^+$

(22d) Morpholin-4-sulfonsäure-4'-amino-4-(2,4-diamino-pyrimidin-5-ylmethyl)-6-ethoxy-biphenyl-2-yl-ester

Ausgehend von 4'-Amino-4-(2,4-diamino-pyrimidin-5-ylmethyl)-6-ethoxybiphenyl-2-ol (200 mg; 0,57 mmol) werden durch Reaktion mit 4-Morpholinsulfonylchlorid (127 mg; 0,68 mmol) 128 mg (40 %) Morpholin-4-sulfonsäure-4'-amino-4-(2,4-diamino-pyrimidin-5-ylmethyl)-6-ethoxy-biphenyl-2-yl-ester als leicht gelbes Pulver erhalten.
MS (ISP): 501,2 $(M+H)^+$

(22e) N-Cyclopropyl-N-methyl-sulfaminsäure-4'-amino-4-(2,4-diamino-pyrimidin-5-ylmethyl)-6-ethoxy-biphenyl-2-yl-ester

Ausgehend von 4'-Amino-4-(2,4-diamino-pyrimidin-5-ylmethyl)-6-ethoxybiphenyl-2-ol (176 mg; 0,5 mmol) werden durch Reaktion mit N-Cyclopropyl-N-methylsulfamoylchlorid (101 mg; 0,6 mmol) 119 mg (47 %) N-Cyclopropyl-N-methylsulfaminsäure-4'-amino-4-(2,4-diamino-pyrimidin-5-ylmethyl)-6-ethoxy-biphenyl-2-ylester als leicht gelbes Pulver erhalten.
MS (ISP): 485,4 $(M+H)^+$
NMR $^1$H (250 MHz, $\delta$, TMS, DMSO): 0.54 (m; 4H); 1.18 (t; J=6.9; 3H); 2.04 (p; J=4.9; 1H); 3.63 (s; 2H); 3.95 (q; J=6.9; 2H); 5.07 (s; 2H); 5.72 (s; 2H); 6.14 (s; 2H); 6.54 (d; J=8.4; 2H); 6.84 (s; 1H); 6.91 (s; 1H); 6.93 (d; J=8.4; 2H); 7.57 (s; 1H).

Das eingesetzte N-Cyclopropyl-N-methylsulfamoylchlorid wird wie in Beispiel 19g beschrieben hergestellt.

(22f) Diethylsulfaminsäure-4'-amino-4-(2,4-diamino-pyrimidin-5-ylmethyl)-6-ethoxybiphenyl-2-yl-ester

Ausgehend von 4'-Amino-4-(2,4-diamino-pyrimidin-5-ylmethyl)-6-ethoxybiphenyl-2-ol (176 mg; 0,5 mmol) werden durch Reaktion mit N,N-Diethylsulfamoylchlorid (103 mg; 0,6 mmol) 113 mg (45 %) Diethylsulfaminsäure-4'-amino-4-(2,4-diamino-pyrimidin-5-ylmethyl)-6-ethoxy-biphenyl-2-yl-ester als leicht gelbes Pulver erhalten.
MS (ISP): 487,3 $(M+H)^+$
Die obige Ausgangsverbindung 4'-Amino-4-(2,4-diamino-pyrimidin-5-ylmethyl)-6-ethoxy-biphenyl-2-ol wird wie in Beispiel 13 beschrieben hergestellt.

Beispiel 23:

In Analogie zu Beispiel 7 wird die Verbindung der Formel VII, Schema 1, aus der entsprechenden Verbindung der Formel VI hergestellt.

5-[3-Cyclopropylmethoxy-5-ethoxy-4-(5-morpholin-4-ylmethyl-thiophen-2-yl)-benzyl]-pyrimidin-2,4-diamin

In Analogie zu Beispiel 7 erhält man ausgehend von 5-(2,4-Diamino-pyrimidin-5-ylmethyl)-3-ethoxy-2-(5-morpholin-4-ylmethyl-thiophen-2-yl)-phenol (88 mg; 0,2 mmol), Kalium-tert-butylat (25 mg; 0,22 mmol) und Brommethylcyclopropan (24 mg; 0,22 mmol) in Dimethylformamid (4 ml, über Molekularsieb getrocknet) 50 mg (34 %) 5-[3-Cyclopropylmethoxy-5-ethoxy-4-(5-morpholin-4-ylmethyl-thiophen-2-yl)-benzyl]-pyrimidin-2,4-diamin als farbloses Pulver.
MS (ISP): 496,2 $(M+H)^+$
Die obige Ausgangsverbindung 5-(2,4-Diamino-pyrimidin-5-ylmethyl)-3-ethoxy-2-(5-morpholin-4-ylmethyl-thiophen-2-yl)-phenol wird durch folgende Sequenz (a-b) ausgehend von 5-(3-Benzyloxy-5-ethoxy-4-iod-benzyl)-pyrimidin-2,4-diamin (Verbindung der Formel IV, Schema 1, Beispiel 6) hergestellt: Schema 1, (IV) -> (V) -> (VI)

Stufe a) 5-[3-Benzyloxy-5-ethoxy-4-(5-morpholin-4-ylmethyl-thiophen-2-yl)-benzyl]-pyrimidin-2,4-diamin

Wird in Analogie zu Beispiel (2ac) ausgehend von 5-(3-Benzyloxy-5-ethoxy-4-iodbenzyl)-pyrimidin-2,4-diamin (Beispiel 6) (1,429 g; 3 mmol) und (5-Morpholin-4-ylmethyl-thiophen-2-yl)-boronsäure (681 mg; 3 mmol) hergestellt.
Ausbeute: 1,15 g (74 %) 5-[3-Benzyloxy-5-ethoxy-4-(5-morpholin-4-ylmethyl-thiophen-2-yl)-benzyl]-pyrimidin-2,4-diamin als beiges Pulver.
Smp: >145°C Zersetzung
MS(ISP): 532,3 $(M+H)^+$

Stufe b) 5-(2,4-Diamino-pyrimidin-5-ylmethyl)-3-ethoxy-2-(5-morpholin-4-ylmethylthiophen-2-yl)-phenol

5-[3-Benzyloxy-5-ethoxy-4-(5-morpholin-4-ylmethyl-thiophen-2-yl)-benzyl]-pyrimidin-2,4-diamin (532 mg;1 mmol) wird in Trifluoressigsäure (32 ml; 400 mmol) unter Argon gelöst, mit Thioanisol (1,18 ml; 10 mmol) versetzt und 17 Stunden bei Raumtemperatur verrührt. Das Reaktionsgemisch wird auf ein Gemisch von Eiswasser (300 ml) und einer gesättigten wässrigen Natriumbicarbonatlösung (300 ml) vorsichtig gegossen. Anschliessend wird mit Methylenchlorid dreimal extrahiert, mit einer gesättigten wässrigen Kochsalzlösung gewaschen, über Natriumcarbonat getrocknet, abgenutscht und eingeengt. Das erhaltene Rohprodukt wird über Kieselgel mit Methylenchlorid/Methanol/$NH_4OH$ konz. (90/10/1) chromatographiert.
Ausbeute: 350 mg (79 %) 5-(2,4-Diamino-pyrimidin-5-ylmethyl)-3-ethoxy-2-(5-morpholin-4-ylmethyl-thiophen-2-yl)-phenol als farbloses Pulver.
Smp: >180°C
MS(ISP): 442,3 $(M+H)^+$

Beispiel 24:

In Analogie zu Beispiel 14b (Methode 2, Sulfonierung mit Chlorid) wird folgende Verbinung der Formel VIII, Schema 1, aus der entsprechenden Verbindung der Formel VI hergestellt.

Cyclopropansulfonsäure-5-(2,4-diamino-pyrimidin-5-ylmethyl)-3-ethoxy-2-(5-morpholin-4-ylmethyl-thiophen-2-yl)-phenyl-ester

Ausgehend von 5-(2,4-Diamino-pyrimidin-5-ylmethyl)-3-ethoxy-2-(5-morpholin-4-ylmethyl-thiophen-2-yl)-phenol (120 mg; 0,27 mmol) werden durch Reaktion mit Cyclopropylsulfonylchlorid (0,036 ml; 0,328 mmol) 34 mg (23 %) Cyclopropansulfonsäure-5-(2,4-diamino-pyrimidin-5-ylmethyl)-3-ethoxy-2-(5-morpholin-4-ylmethylthiophen-2-yl)-phenyl-ester als leicht gelbes Pulver erhalten.
MS (ISP): 546,2 $(M+H)^+$

Die obige Ausgangsverbindung 5-(2,4-Diamino-pyrimidin-5-ylmethyl)-3-ethoxy-2-(5-morpholin-4-ylmethyl-thiophen-2-yl)-phenol wird wie in Beispiel 23 beschrieben hergestellt.

Beispiel 25

Herstellung von 5-(3-Ethoxy-4-iod-5-methoxymethoxy-benzyl)-pyrimidin-2,4-diamin (Schlüsselzwischenprodukt):

Schema 2, Verbindung (X) mit $R^1$ = Ethyl.

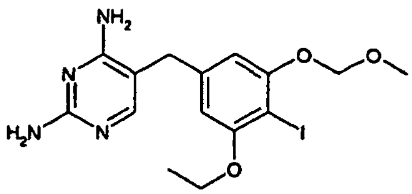

Diese Verbindung wird durch folgende Reaktionssequenz (Stufen a)-e)) hergestellt:

Stufe a) 3-Ethoxy-5-hydroxy-4-iod-benzoesäure-methylester:

3,5-Dihydroxy-4-iod-benzoesäure-methylester (hergestellt wie in Beispiel 1 ) (60,0 g; 0,204 Mol) wird in Dimethylformamid (600 ml) gelöst und bei 0°C mit Ethylbromid (16,25 ml; 0,224 Mol) und Kalium-tert-butylat (50,38 g; 0,449 Mol) versetzt. Anschliessend wird eine Stunde bei Raumtemperatur gerührt. Dann wird Aether (600 ml) hinzugefügt und das Gemisch auf Wasser (600 ml) gegossen. Die organische Phase wird abgetrennt (sie enthält hauptsächlich 3,5-Dihydroxy-4-iod-benzoesäure-methylester). Die wässrige Phase wird unter Kühlung mit Salzsäure (25%ig) sauer gestellt und mit Aether zweimal extrahiert (2-mal 300 ml). Die vereinigten organischen Phasen werden anschliessend über Natriumsulfat (400 g) getrocknet und eingeengt. Der Rückstand wird in einem 4/1 n-Hexan/Ethylacetat-Gemisch (250 ml) verrührt und die Kristalle abgenutscht. Man erhält 46 g (70%) gelbliche Kristalle. Die Mutterlauge wird eingedampft und der Rückstand über Kieselgel mit n-Hexan/Ethylacetat (7/3) chromatographiert. Die reinen Fraktionen werden vereinigt und das Lösungsmittel abgedampft. Man erhält zusätzlich 12.1 g (18%).
Total Ausbeute: 58.1 g (88 %) 3-Ethoxy-5-hydroxy-4-iod-benzoesäure-methylester als gelbliche Kristalle.
MS: 322 (M)

Stufe b) 3-Ethoxy-4-iod-5-methoxymethoxy-benzoesäure-methylester:

3-Ethoxy-5-hydroxy-4-iod-benzoesäure-methylester (58.1 g; 0.18 mmol) wird in Tetrahydrofuran (600 ml) gelöst und mit Natriumhydrid (15.74 g; 55%ig; 0.361 mmol) versetzt. Die Suspension wird eine Stunde bei Raumtemperatur gerührt und anschliessend auf -10°C abgekühlt. Danach wird Methoxymethylchlorid (27.4 ml; 0.361 Mol) innerhalb 5 Minuten zugetropft, und eine halbe Stunde bei Raumtemperatur nachgerührt. Das Reaktionsgemisch wird auf Wasser (600 ml) gegossen und zweimal mit Aether extrahiert (2 x 300 ml). Die vereinigten organischen Phasen werden anschliessend über Natriumsulfat (400 g) getrocknet, filtriert und eingeengt. Der Rückstand wird in n-Hexan (100 ml) verrührt und abgenutscht.
Ausbeute: 49.1g (75 %) 3-Ethoxy-4-iod-5-methoxymethoxy-benzoesäure-methylester als weisse Kristalle.
MS: 366 (M)

Stufe c) (3-Ethoxy-4-iod-5-methoxymethoxy-phenyl)-methanol:

Wird in Analogie zu Beispiel 1 Stufe d) hergestellt.

Ausgehend von 3-Ethoxy-4-iod-5-methoxymethoxy-benzoesäure-methylester (16.49 g; 0.045 Mol), Diisobutylaluminiumhydrid 1,2 M in Toluol (132 ml; 0.158 Mol) werden 15.6 g (3-Ethoxy-4-iod-5-methoxymethoxy-phenyl)-methanol als farblose feste Masse, die ohne weitere Reinigung in die nächste Stufe eingesetzt wird, erhalten.
MS (EI): 338 (M)

Stufe d) 3-Ethoxy-4-iod-5-methoxymethoxy-benzaldehyd:

Wird in Analogie zu Beispiel 1(e) ausgehend von (3-Ethoxy-4-iod-5-methoxymethoxy-phenyl)-methanol (15.6 g; 0.045 Mol) und Mangandioxid (40.16 g; 0.462 Mol) hergestellt.
Ausbeute: 13,76 g rot-gelbliche Kristalle (89 % über 2 Stufen).
NMR (1H, 250 MHz in DMSO) in ppm: 1,40 (t, 3H); 3,41 (s, 3H); 4,18 (q, 2H); 5,36 (s, 2H); 7,15 (s, 1H); 7,23 (s 1H); 9,94 (s, 1H)

Stufe e) 5-(3-Ethoxy-4-iod-5-methoxymethoxy-benzyl)-pyrimidin-2,4-diamin

Kalium-tert-butylat (3.33 g; 29.77 mmol) wird unter Argon und Rühren in tert-Butanol (33 ml) heiss gelöst. Die Lösung wird dann auf 40°C abgekühlt und innerhalb 25 Minuten zu einer Lösung bestehend aus 3-Ethoxy-4-iod-5-methoxymethoxy-benzaldehyd (8,34g; 24,81 mmol) und 3-Anilinopropionitril (3.44 g; 23.57 mmol) in Dimethylsulfoxid (33 ml) bei 38-40°C (20 Minuten) zugetropft. Anschliessend wird das Reaktionsgemisch während 5 Stunden bei Raumtemperatur gerührt. Danach werden 33 ml Lösungsmittel abdestilliert. Das Reaktionsgemisch wird auf Wasser (100 ml) gegossen und zweimal mit Ethylacetat extrahiert (2 x 50 ml). Die vereinigten organischen Phasen werden anschliessend über Natriumsulfat (100 g) getrocknet, filtriert und eingeengt. Das erhaltene rohe Zwischenprodukt wird direkt weiterverarbeitet.
Guanidinhydrochlorid (7.81 g; 81.9 mmol) wird in Ethanol (1500 ml) gelöst und mit Kalium-tert-butylat (10.03 g; 89,8 mmol) versetzt. Die Temperatur des Reaktionsgemisches wird durch leichte Kühlung während einer Stunde unterhalb 38°C gehalten. Anschliessend wird das so hergestellte rohe Zwischenprodukt zugegeben und das Reaktionsgemisch 20 Stunden auf 67-69°C erwärmt. Die Reaktionslösung wird auf Wasser (100 ml) gegossen und zweimal mit Ethylacetat extrahiert (2 x 50 ml). Die vereinigten organischen Phasen werden anschliessend über Natriumsulfat (100 g) getrocknet, filtriert und eingeengt. Der Rückstand wird in Aether (80 ml) verrührt, die Kristalle abgenutscht, mit Aether gewaschen und am Hochvakuum getrocknet.
Ausbeute: 6.82 g (64 %)5-(3-Ethoxy-4-iod-5-methoxymethoxy-benzyl)-pyrimidin-2,4-diamin als gelbe Kristalle.
MS (ISP): 431.2 $(M+H)^+$

Beispiel 26:

Ausgehend von 4-(2,4-Diamino-pyrimidin-5-ylmethyl)-6-ethoxy-4'-methylaminobiphenyl-2-ol werden in Analogie zu Beispiel 19 die Verbindungen der Formel IX, Schema 2aus den entsprechenden Verbindungen der Formel VI hergestellt.

(26a) Dimethylsulfaminsäure-4-(2,4-diamino-pyrimidin-5-ylmethyl)-6-ethoxy-4'methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit N,N-Dimethylsulfamoylchlorid (0,065 ml; 0,6 mmol) 123 mg (48 %) Dimethylsulfaminsäure-4-(2,4-diaminopyrimidin-5-ylmethyl)-6-ethoxy-4'-methylamino-biphenyl-2-yl-ester als leicht gelbes Pulver erhalten.
MS (ISP): 473,3 $(M+H)^+$

(26b) N-Ethyl-N-methyl-sulfaminsäure-4-(2,4-diamino-pyrimidin-5-ylmethyl)-6-ethoxy-4'-methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit N-Ethyl-N-methylsulfamoylchlorid (95 mg; 0,6 mmol) 130 mg (49 %) N-Ethyl-N-methyl-sulfaminsäure-4-(2,4-diamino-pyrimidin-5-ylmethyl)-6-ethoxy-4'-methylamino-biphenyl-2-yl-ester als farbloses Pulver erhalten.
MS (ISP): 483,3 $(M+H)^+$

(26c) Morpholin-4-sulfonsäure-4-(2,4-diamino-pyrimidin-5-ylmethyl)-6-ethoxy-4'-methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit 4-Morpholinsulfonylchlorid (111 mg; 0,6 mmol) 128 mg (42 %) Morpholin-4-sulfonsäure-4-(2,4-diaminopyrimidin-5-ylmethyl)-6-ethoxy-4'-methylamino-biphenyl-2-yl-ester als farbloses Pulver erhalten.
MS (ISP): 515,3 $(M+H)^+$
NMR $^1H$: (250 MHz, $\delta$, TMS, DMSO): 1.18 (t; J=6.9; 3H; 2.68 (d; J=5; 3H); 2.81 (m; 4H); 3.44 (m; 4H); 3.64 (s; 2H);

3.95 (q; J=6.9; 2H); 5.66 (q; J=5; 1H); 5.73 (br s; 2H); 6.15 (br s; 2H); 6.54 (d; J=8.5; 2H); 6.86 (s; 1H); 6.93 (s; 1H); 6.98 (d; J=8.5; 2H); 7.58 (s; 1H).

(26d) N-Cyclopropyl-N-methyl-sulfaminsäure-4-(2,4-diamino-pyrimidin-5-ylmethyl)-6-ethoxy-4'-methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit N-Cyclopropyl-N-methylsulfamoylchlorid (101 mg; 0,6 mmol) 76 mg (24 %) N-Cyclopropyl-N-methylsulfaminsäure-4-(2,4-diamino-pyrimidin-5-ylmethyl)-6-ethoxy-4'-methylaminobiphenyl-2-yl-ester als beiges Pulver erhalten.
MS (ISP): 499,3 $(M+H)^+$

Das eingesetzte N-Cyclopropyl-N-methylsulfamoylchlorid wird wie in Beispiel 19g beschrieben hergestellt.

(26e) Pyrrolidin-1-sulfonsäure-4-(2,4-diamino-pyrimidin-5-ylmethyl)-6-ethoxy-4'-methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit 1-Pyrrolidinsulfonylchlorid (101 mg; 0,6 mmol) 120 mg (37 %) Pyrrolidin-1-sulfonsäure-4-(2,4-diamino-pyrimidin-5-ylmethyl)-6-ethoxy-4'-methylamino-biphenyl-2-yl-ester als farbloses Pulver erhalten.
MS (ISP): 499,2 $(M+H)^+$

(26f) Diethylsulfaminsäure-4-(2,4-diamino-pyrimidin-5-ylmethyl)-6-ethoxy-4'-methylamino-biphenyl-2-yl-ester

Ausgehend von 4-(2,4-Diamino-pyrimidin-5-ylmethyl)-6-ethoxy-4'-methylaminobiphenyl-2-ol (183 mg; 0,5 mmol) werden durch Reaktion mit N,N-Diethylsulfamoylchlorid (103 mg; 0,6 mmol) 92 mg (31 %) Diethylsulfaminsäure-4-(2,4-diaminopyrimidin-5-ylmethyl)-6-ethoxy-4'-methylamino-biphenyl-2-yl-ester als farbloses Pulver erhalten.
MS (ISP): 501,3 $(M+H)^+$

Das eingesetzte 4-(2,4-Diamino-pyrimidin-5-ylmethyl)-6-ethoxy-4'-methylaminobiphenyl-2-ol wird ausgehend von 5-(3-Benzyloxy-5-ethoxy-4-iod-benzyl)-pyrimidin-2,4-diamin in Analogie zu Beispiel 4a (Suzuki-Kopplung mit in situ Generierung von $R^2$-$B(OH)_2$) und anschliessend Abspaltung der Benzyl-Schutzgruppe hergestellt:

N-(4-Brom-phenyl)-2,2,2-trifluor-N-methyl-acetamid (2,68 g; 9 mmol), Bis(pinacolato)diboron (2,51 g; 9,9 mmol), Kaliumacetat (2,65 g; 27 mmol) und 1,1'-Bis(Diphenylphosphino)ferrocendichlorpalladium (II) ($PdCl_2$(dppf)) (936 mg; 0,81 mmol) werden in Dimethoxyethan (90 ml; über Molekularsieb getrocknet) suspendiert, viermal unter heftigem Rühren zur Entfernung von Sauerstoffester evakuiert und mit Argon belüftet. Anschliessend wird bei 80°C Badtemperatur während 24 Stunden gerührt. Nach Abkühlen auf Raumtemperatur wird das Reaktionsgemisch mit Dimethylformamid (170 ml), Tetrakis-triphenylphosphin-palladium (936 mg; 0,81 mmol), 5-(3-Benzyloxy-5-ethoxy-4-iod-benzyl)-pyrimidin-2,4-diamin (3,42 g; 7,2 mmol) und einer 2M wässrigen Kaliumphosphatlösung (36 ml) versetzt. Das Reaktionsgemisch wird ca. 17 Stunden unter Argon bei 90°C (Badtemperatur) gerührt, auf Raumtemperatur abgekühlt, abgenutscht und eingeengt. Der erhaltene Rückstand wird mit Wasser (100 ml) versetzt und mit Methylenchlorid (150 ml) extrahiert. Die organische Phase wird nacheinander mit Wasser (100 ml) und einer gesättigten wässrigen Kochsalzlösung (100 ml) gewaschen, über Magnesiumsulfat getrocknet, abgenutscht und eingeengt. Der Rückstand wird über Kieselgel (600 g) mit Methylenchlorid/Methanol/$NH_4OH$ 19/1/0,05 chromatographiert. Es werden 2,30 g (47 %) 5-(6-Benzyloxy-2-ethoxy-4'-methylamino-biphenyl-4-ylmethyl)-pyrimidin-2,4-diamin als leicht braunes Pulver, die direkt in die nächste Stufe eingesetzt werden, erhalten.

Dieses Produkt wird in Methanol (100 ml) gelöst und nach Zugabe einer 1N wässrigen Salzsäure-Lösung (12,8 ml) über Pd/C 10 % (0,5 g) hydriert. Nach ca 1,5 Stunden wird der Katalysator abgenutscht, mit Methanol nachgewaschen und eingeengt. Der Rückstand wird mit Wasser (70 ml) verrührt, wonach durch Zugabe von $NH_4OH$ konz. das pH auf 9-10 eingestellt wird. Die Suspension wird abgenutscht, mit Wasser gewaschen und am Hochvakuum getrocknet. Es werden 1,32 g (78 %) 4-(2,4-Diaminopyrimidin-5-ylmethyl)-6-ethoxy-4'-methylamino-biphenyl-2-ol als graues Pulver erhalten.
MS (ISP): 366,3 $(M+H)^+$

Beispiel 27:

In Analogie zu Beispiel 19 werden die Verbindungen der Formel IX, Schema 2, aus den ensprechenden Verbindungen der Formel VI hergestellt.

(27a) Dimethylsulfaminsäure-4'-amine-4-(2,4-diamino-pyrimidin-5-ylmethyl)-6-ethoxy-3'-methyl-biphenyl-2-yl-ester

Ausgehend von 4'-Amino-4-(2,4-diamino-pyrimidin-5-ylmethyl)-6-ethoxy-3'methyl-biphenyl-2-ol (182 mg; 0,5 mmol) werden durch Reaktion mit N,N-Dimethylsulfamoylchlorid (0,065 ml; 0,6 mmol) 140 mg (55 %) Dimethylsulfaminsäure-4'-amino-4-(2,4-diamino-pyrimidin-5-ylmethyl)-6-ethoxy-3'-methyl-biphenyl-2-yl-ester als farbloses Pulver erhalten.
MS (ISP): 473,3 $(M+H)^+$

(27b) N-Ethyl-N-methyl-sulfaminsäure-4'-amino-4-(2,4-diamino-pyrimidin-5-ylmethyl)-6-ethoxy-3'-methyl-biphenyl-2-yl-ester

Ausgehend von 4'-Amino-4-(2,4-diamino-pyrimidin-5-ylmethyl)-6-ethoxy-3'methyl-biphenyl-2-ol (182 mg; 0,5 mmol) werden durch Reaktion mit N-Ethyl-N-methylsulfamoylchlorid (95 mg; 0,6 mmol) 122 mg (47 %) N-Ethyl-N-methyl-sulfaminsäure-4'-amino-4-(2,4-diamino-pyrimidin-5-ylmethyl)-6-ethoxy-3'-methyl-biphenyl-2-ylester als farbloses Pulver erhalten.
MS (ISP): 487,3 $(M+H)^+$

(27c) N-Cyclopropyl-N-methyl-sulfaminsäure-4'-amino-4-(2,4-diamino-pyrimidin-5-ylmethyl)-6-ethoxy-3'-methyl-biphenyl-2-yl-ester

Ausgehend von 4'-Amino-4-(2,4-diamino-pyrimidin-5-ylmethyl)-6-ethoxy-3'methyl-biphenyl-2-ol (182 mg; 0,5 mmol) werden durch Reaktion mit N-Cyclopropyl-N-methylsulfamoylchlorid (101 mg; 0,6 mmol) 145 mg (55 %) N-Cyclopropyl-N-methylsulfaminsäure-4'-amino-4-(2,4-diamino-pyrimidin-5-ylmethyl)-6-ethoxy-3'-methylbiphenyl-2-yl-ester als leicht gelbes Pulver erhalten.
MS (ISP): 499,3 $(M+H)^+$
Das eingesetzte N-Cyclopropyl-N-methylsulfamoylchlorid wird wie in Beispiel 19g beschrieben hergestellt.
Die obige Ausgangsverbindung 4'-Amino-4-(2,4-diamino-pyrimidin-5-ylmethyl)-6-ethoxy-3'-methyl-biphenyl-2-ol wird wie in Schema 2, ((X) -> (XI) -> (VI)) aufgezeichnet, hergestellt, dh in Analogie zu Beispiel 4a (Suzuki-Kopplung mit *in sit*u Generierung von $R^2$-$B(OH)_2$)) und durch anschliessende Abspaltung der Methoxymethyl-Schutzgruppe.
N-(4-Brom-2-methyl-phenyl)-2,2,2-trifluor-acetamid (in Beispiel 4f beschrieben) (4,9 g; 17,5 mmol), Bis(pinacolato)diboron (6,66 g; 26,25 mmol), Kaliumacetat (5,15 g; 52,5 mmol) und Bis(triphenylphosphin)palladium(II)dichlorid ($PdCl_2(PPh_3)_2$) (737 mg; 1,05 mmol) werden in Dioxan (175 ml; über Molekularsieb getrocknet) suspendiert, viermal unter heftigem Rühren evakuiert und mit Argon belüftet. Anschliessend wird bei 85°C Badtemperatur während 4 Stunden gerührt. Nach Abkühlen auf Raumtemperatur wird das Reaktionsgemisch eingeengt, mit Dimethoxyethan (10 ml), Tetrakis-triphenylphosphin-palladium (1,21 g; 1,05 mmol), 5-(3-Ethoxy-4-iod-5-methoxymethoxybenzyl)-pyrimidin-2,4-diamin (4,51 g; 10,5 mmol) und einer 2M wässrigen Natriumcarbonat-Lösung (70 ml) versetzt. Das Reaktionsgemisch wird ca. 25 Stunden unter Argon bei 85°C (Badtemperatur) gerührt, auf Raumtemperatur abgekühlt, abgenutscht und eingeengt. Der erhaltene Rückstand wird in Ethanol (210 ml) gelöst, mit einer 1N wässriger Natronlauge versetzt und 4 ½ Stunden im Ölbad von 50°C gerührt. Das Gemisch wird auf Eiswasser gegossen und zweimal mit Ethylacetat extrahiert. Die organischen Phase werden mit einer gesättigten wässrigen Kochsalzlösung gewaschen, über Magnesiumsulfat getrocknet, abgenutscht und eingeengt. Der Rückstand wird über Kieselgel (810 g) mit Methylenchlorid/Methanol/$NH_4OH$ 19/1/0,05 chromatographiert. Das erhaltene rote Pulver (2,6 g) wird in Methanol (100 ml) suspendiert, mit einer 4,5 N wässriger Salzsäure (30 ml; 137 mmol) versetzt und 45 Minuten in einem Ölbad bei 60°C gerührt. Das Methanol wird eingedampft, der Rückstand in Wasser (200 ml) gelöst und das pH unter Rühren mit $NH_4OH$ konz. auf ca. 10 eingestellt. Nach 30-minütigem Rühren bei Raumtemperatur wird die Suspension abgenutscht, mit Wasser gewaschen und am Hochvakuum getrocknet. Es werden 2,20 g (33 %) 4'-Amino-4-(2,4-diamino-pyrimidin-5-ylmethyl)-6-ethoxy-3'-methyl-biphenyl-2-ol als leicht rotes Pulver erhalten.
MS (ISP): 366,3 $(M+H)^+$
Diese Verbindung kann auch wie in Beispiel 11 beschrieben hergestellt werden.

Beispiel 28:

In Analogie zu Beispiel 19 werden die Verbindungen der Formel IX, Schema 2, aus den entsprechenden Verbindungen der Formel VI hergestellt.

(28a) Dimethylsulfaminsäure-4'-amino-4-(2,4-diamino-pyrimidin-5-ylmethyl)-6-ethoxy-3'-fluor-biphenyl-2-yl-ester

Ausgehend von 4'-Amino-4-(2,4-diamino-pyrimidin-5-ylmethyl)-6-ethoxy-3'-fluorbiphenyl-2-ol (185 mg; 0,5

mmol) werden durch Reaktion mit N,N-Dimethylsulfamoylchlorid (0,065 ml; 0,6 mmol) 145 mg (59 %) Dimethyl-sulfaminsäure-4'-amine-4-(2,4-diamino-pyrimidin-5-ylmethyl)-6-ethoxy-3'-fluor-biphenyl-2-yl-ester als farbloses Pulver erhalten.
MS (ISP): 477,3 $(M+H)^+$

(28b) N-Ethyl-N-methyl-sulfaminsäure-4'-amino-4-(2,4-diamino-pyrimidin-5-ylmethyl)-6-ethoxy-3'-fluor-biphenyl-2-yl-ester

Ausgehend von 4'-Amino-4-(2,4-diamino-pyrimidin-5-ylmethyl)-6-ethoxy-3'-fluorbiphenyl-2-ol (185 mg; 0,5 mmol) werden durch Reaktion mit N-Ethyl-N-methylsulfamoylchlorid (95 mg; 0,6 mmol) 139 mg (56 %) N-Ethyl-N-methyl-sulfaminsäure- 4'-amino-4-(2,4-diamino-pyrimidin-5-ylmethyl)-6-ethoxy-3'-fluor-biphenyl-2-yl-ester als leicht gelbes Pulver erhalten.
MS (ISP): 491,2 $(M+H)^+$

(28c) N-Cyclopropyl-N-methyl-sulfaminsäure-4'-amino-4-(2,4-diamino-pyrimidin-5-ylmethyl)-6-ethoxy-3'-fluor-biphenyl-2-yl-ester

Ausgehend von 4'-Amino-4-(2,4-diamino-pyrimidin-5-ylmethyl)-6-ethoxy-3'-fluorbiphenyl-2-ol (185 mg; 0,5 mmol) werden durch Reaktion mit N-Cyclopropyl-N-methylsulfamoylchlorid (102 mg; 0,6 mmol) 126 mg (47 %) N-Cyclopropyl-N-methylsulfaminsäure-4'-amino-4-(2,4-diamino-pyrimidin-5-ylmethyl)-6-ethoxy-3'-fluorbiphenyl-2-yl-ester als farbloses Pulver erhalten.
MS (ISP): 503,3 $(M+H)^+$

Das eingesetzte N-Cyclopropyl-N-methylsulfamoylchlorid wird wie in Beispiel 19g beschrieben hergestellt.

Die obige Ausgangsverbindung 4'-Amino-4-(2,4-diamino-pyrimidin-5-ylmethyl)-6-ethoxy-3'-fluor-biphenyl-2-ol wird in Analogie zu 4'-Amino-4-(2,4-diamino-pyrimidin-5-ylmethyl)-6-ethoxy-3'-methyl-biphenyl-2-ol (Vorstufe in Beispiel 27) hergestellt vgl. Schema 2, (X) -> (XI) -> (VI)

Ausgehend von 5-(3-Ethoxy-4-iod-5-methoxymethoxy-benzyl)-pyrimidin-2,4-diamin (4,51 g; 10,5 mmol) und N-(4-Brom-2-fluor-phenyl)-2,2,2-trifluor-acetamid (in Beispiel 4r beschrieben) (5,0 g; 26,25 mmol) werden nach Suzuki-Kopplung (*in situ* Generierung von $R^2$-$B(OH)_2$)) und saurer Abspaltung der Schutzgruppe 2,0 g (29 %) 4'-Amino-4-(2,4-diamino-pyrimidin-5-ylmethyl)-6-ethoxy-3'-fluor-biphenyl-2-ol als rotes Pulver erhalten.
MS (ISP): 370,3 $(M+H)^+$

Beispiel 29:

In Analogie zu Beispiel 19 werden die Verbindungen der Formel IX, Schema 2, aus den entsprechenden Verbindungen der Formel VI hergestellt:

(29a) N-Cyclopropyl-N-methyl-sulfaminsäure-3'-amino-4-(2,4-diamino-pyrimidin-5-ylmethyl)-6-ethoxy-5'-fluor-biphenyl-2-yl-ester

Ausgehend von 3'-Amino-4-(2,4-diamino-pyrimidin-5-ylmethyl)-6-ethoxy-5'-fluorbiphenyl-2-ol (150 mg; 0,39 mmol) werden durch Reaktion mit N-Cydopropyl-N-methylsulfamoylchlorid (80 mg; 0,47 mmol) 119 mg (59 %) N-Cyclopropyl-N-methylsulfaminsäure-3'-amino-4-(2,4-diamino-pyrimidin-5-ylmethyl)-6-ethoxy-5'-fluorbiphenyl-2-yl-ester als leicht gelbes Pulver erhalten.
MS (ISP): 503,2 $(M+H)^+$

Das eingesetzte N-Cyclopropyl-N-methylsulfamoylchlorid wird wie in Beispiel 19g beschrieben hergestellt.

(29b) Dimethylsulfaminsäure-3'-amino-4-(2,4-diamino-pyrimidin-5-ylmethyl)-6-ethoxy-5'-fluor-biphenyl-2-yl-ester

Ausgehend von 3'-Amino-4-(2,4-diamino-pyrimidin-5-ylmethyl)-6-ethoxy-5'-fluorbiphenyl-2-ol (150 mg; 0,39 mmol) werden durch Reaktion mit N,N-Dimethylsulfamoylchlorid (0,051 ml; 0,47 mmol) 120 mg (62 %) Dimethylsulfaminsäure-3'-amino-4-(2,4-diamino-pyrimidin-5-ylmethyl)-6-ethoxy-5'-fluor-biphenyl-2-yl-ester als farbloses Pulver erhalten.
MS (ISP): 477,2 $(M+H)^+$

(29c) N-Ethyl-N-methyl-sulfaminsäure-3'-amino-4-(2,4-diamino-pyrimidin-5-ylmethyl)-6-ethoxy-5'-fluor-biphenyl-2-yl-ester

Ausgehend von 3'-Amino-4-(2,4-diamino-pyrimidin-5-ylmethyl)-6-ethoxy-5'-fluorbiphenyl-2-ol (150 mg; 0,39 mmol) werden durch Reaktion mit N-Ethyl-N-methylsulfamoylchlorid (73 mg; 0,47 mmol) 50 mg (24 %) N-Ethyl-N-methyl-sulfaminsäure-3'-amino-4-(2,4-diamino-pyrimidin-5-ylmethyl)-6-ethoxy-5'-fluor-biphenyl-2-ylester als farbloses Pulver erhalten.
MS (ISP): 491,2 $(M+H)^+$

Die obige Ausgangsverbindung 3'-Amino-4-(2,4-diamino-pyrimidin-5-ylmethyl)-6-ethoxy-5'-fluor-biphenyl-2-ol wird in Analogie zu 4'-Amino-4-(2,4-diamino-pyrimidin-5-ylmethyl)-6-ethoxy-3'-methyl-biphenyl-2-ol (Vorstufe in Beispiel 27) hergestellt, vgl. Schema 2, (X) -> (XI) -> (VI)

Ausgehend von 5-(3-Ethoxy-4-iod-5-methoxymethoxy-benzyl)-pyrimidin-2,4-diamin (1,18 g; 2,76 mmol) und N-(5-Iod-3-fluor-phenyl)-2,2,2-trifluoracetamid (in Beispiel 4t beschrieben) (1,53 g; 4,6 mmol) werden nach Suzuki -Kopplung (*In situ* Generierung von $R^2$-$B(OH)_2$)) und saurer Abspaltung der Schutzgruppe 0,554 g (42 %) 3'-Amino-4-(2,4-diamino-pyrimidin-5-ylmethyl)-6-ethoxy-5'-fluor-biphenyl-2-ol als leicht braunes Pulver erhalten.
MS (ISP): 370,3 $(M+H)^+$

Beispiel 30:

In Analogie zu Beispiel 19 werden die Verbindungen der Formel IX, Schema 2, aus den entsprechenden Verbindungen der Formel VI hergestellt.

(30a) Dimethylsulfaminsäure-3'-amino-4-(2,4-diamino-pyrimidin-5-ylmethyl)-6-ethoxy-4'-morpholin-4-ylmethyl-biphenyl-2-yl-ester

Ausgehend von 3'-Amino-4-(2,4-diamino-pyrimidin-5-ylmethyl)-6-ethoxy-4'morpholin-4-ylmethyl-biphenyl-2-ol (185 mg; 0,4 mmol) werden durch Reaktion mit N,N-Dimethylsulfamoylchlorid (0,052 ml; 0,48 mmol) 113 mg (50 %) Dimethylsulfaminsäure-3'-amino-4-(2,4-diamino-pyrimidin-5-ylmethyl)-6-ethoxy-4'-morpholin-4-ylmethyl-biphenyl-2-yl-ester als farbloses Pulver erhalten.
MS (ISP): 558,3 $(M+H)^+$

(30b) N-Cyclopropyl-N-methyl-sulfaminsäure-3'-amino-4-(2,4-diamino-pyrimidin-5-ylmethyl)-6-ethoxy-4'-morpholin-4-ylmethyl-biphenyl-2-yl-ester

Ausgehend von 3'-Amino-4-(2,4-diamino-pyrimidin-5-ylmethyl)-6-ethoxy-4'morpholin-4-ylmethyl-biphenyl-2-ol (185 mg; 0,4 mmol) werden durch Reaktion mit N-Cyclopropyl-N-methylsulfamoylchlorid (81 mg; 0,48 mmol) 96 mg (40 %) N-Cyclopropyl-N-methyl-sulfaminsäure-3'-amino-4-(2,4-diamino-pyrimidin-5-ylmethyl)-6-ethoxy-4'-morpholin-4-ylmethyl-biphenyl-2-yl-ester als leicht gelbes Pulver erhalten.
MS (ISP): 584,2$(M+H)^+$

Das eingesetzte N-Cyclopropyl-N-methylsulfamoylchlorid wird wie in Beispiel 19g beschrieben hergestellt.

(30c) N-Ethyl-N-methyl-sulfaminsäure-3'-amino-4-(2,4-diamino-pyrimidin-5-ylmethyl)-6-ethoxy-4'-morpholin-4-ylmethyl-biphenyl-2-yl-ester

Ausgehend von 3'-Amino-4-(2,4-diamino-pyrimidin-5-ylmethyl)-6-ethoxy-4'morpholin-4-ylmethyl-biphenyl-2-ol (278 mg; 0,6 mmol) werden durch Reaktion mit N-Ethyl-N-methylsulfamoylchlorid (113 mg; 0,72 mmol) 210 mg (61 %) N-Ethyl-N-methyl-sulfaminsäure-3'-amino-4-(2,4-diamino-pyrimidin-5-ylmethyl)-6-ethoxy-4'morpholin-4-ylmethyl-biphenyl-2-yl-ester als leicht gelbes Pulver erhalten.
MS (ISP): 572,2$(M+H)^+$

Die obige Ausgangsverbindung 3'-Amino-4-(2,4-diamino-pyrimidin-5-ylmethyl)-6-ethoxy-4'-morpholin-4-ylmethyl-biphenyl-2-ol wird in Analogie zu 4'-Amino-4-(2,4-diamino-pyrimidin-5-ylmethyl)-6-ethoxy-3'-methyl-biphenyl-2-ol (Vorstufe in Beispiel 27) hergestellt, vgl. Schema 2, (X) -> (XI) -> (VI)

Ausgehend von 5-(3-Ethoxy-4-iod-5-methoxymethoxy-benzyl)-pyrimidin-2,4-diamin (2,11 g; 4,9 mmol) und N-(5-Brom-2-morpholin-4-ylmethyl-phenyl)-2,2,2-trifluor-acetamid (in Beispiel 4j beschrieben) (3,0 g; 8,17 mmol) werden nach Suzuki - Kopplung (in situ Generierung von $R^2$-$B(OH)_2$)) und saurer Abspaltung der Schutzgruppe 1,36 g (36 %) 3'-Amino-4-(2,4-diamino-pyrimidin-5-ylmethyl)-6-ethoxy-4'-morpholin-4-ylmethyl-biphenyl-2-ol als beiges Pulver erhalten.
MS (ISP): 451,4 $(M+H)^+$

Beispiel 31:

In Analogie zu Beispiel 19 werden die Verbindungen der Formel IX, Schema 2, aus den entsprechenden Verbindungen der Formel VI hergestellt.

(31a) Dimethylsulfaminsäure-3'-amino-4-(2,4-diamino-pyrimidin-5-ylmethyl)-6-ethoxy-4'-methyl-biphenyl-2-yl-ester

Ausgehend von 3'-Amino-4-(2,4-diamino-pyrimidin-5-ylmethyl)-6-ethoxy-4'methyl-biphenyl-2-ol (183 mg; 0,5 mmol) werden durch Reaktion mit N,N-Dimethylsulfamoylchlorid (0,064 ml; 0,6 mmol) 128 mg (52 %) Dimethylsulfaminsäure-3'-amino-4-(2,4-diamino-pyrimidin-5-ylmethyl)-6-ethoxy-4'-methyl-biphenyl-2-yl-ester als farbloses Pulver erhalten.
MS (ISP): 473,3 $(M+H)^+$
NMR $^1H$: (400 MHz, $\delta$, TMS, DMSO): 1.17 (t; J=6.8; 3H); 2.06 (s; 3H); 2.46 (s; 6H); 3.64 (s; 2H); 3.95 (q; J=6.8; 2H); 4.74 (s; 2H); 5.69 (s; 2H); 6.11 (s; 2H); 6.37 (d; J=7.2; 1H); 6.52 (s; 1H); 6.84 (s; 1H); 6.90 (m; 2H); 7.57 (s; 1H).

(31b) N-Ethyl-N-methyl-sulfaminsäure-3'-amino-4-(2,4-diamino-pyrimidin-5-ylmethyl)-6-ethoxy-4'-methyl-biphenyl-2-yl-ester

Ausgehend von 3'-Amino-4-(2,4-diamino-pyrimidin-5-ylmethyl)-6-ethoxy-4'methyl-biphenyl-2-ol (183 mg; 0,5 mmol) werden durch Reaktion mit N-Ethyl-N-methylsulfamoylchlorid (95 mg; 0,6 mmol) 149 mg (59 %) N-Ethyl-N-methyl-sulfaminsäure-3'-amino-4-(2,4-diamino-pyrimidin-5-ylmethyl)-6-ethoxy-4'-methyl-biphenyl-2-ylester als farbloses Pulver erhalten.
MS (ISP): 487,3 $(M+H)^+$

Die obige Ausgangsverbindung 3'-Amino-4-(2,4-diamino-pyrimidin-5-ylmethyl)-6-ethoxy-4'-methyl-biphenyl-2-ol wird in Analogie zu 4'-Amino-4-(2,4-diamino-pyrimidin-5-ylmethyl)-6-ethoxy-3'-methyl-biphenyl-2-ol (Vorstufe in Beispiel 27) hergestellt, vgl.
Schema 2, (X) -> (XI) -> (VI)

Ausgehend von 5-(3-Ethoxy-4-iod-5-methoxymethoxy-benzyl)-pyrimidin-2,4-diamin (2,11 g; 4,9 mmol) und N-(5-Brom-2-methyl-phenyl)-2,2,2-trifluor-acetamid (aus 5-Brom-2-methyl-anilin hergestellt in Analogie zu Beispiel 41) (2,3 g; 8,17 mmol) werden nach Suzuki-Kopplung (*In-situ* Generierung von $R^2$-$B(OH)_2$) und saurer abspaltung der Schutzgruppe 0,963 g (30 %) 3'-Amino-4-(2,4-diamino-pyrimidin-5-ylmethyl)-6-ethoxy-4'-methyl-biphenyl-2-ol als beiges Pulver erhalten.
MS (ISP): 366,2 $(M+H)^+$

Beispiel 32:

Analog Beispiel 4 werden die Verbindungen der Formel VIII, Schema 2, aus den entsprechenden Verbindungen der Formel XIII nach Methode A dh Suzuki-Kupplung mit $R^2$-$B(OH)_2$ hergestellt.

(32a) Propan-1-sulfonsäure-3'-amino-4-(2,4-diamino-pyrimidin-5-ylmethyl)-6-ethoxy-4'-morpholinmethyl-4-ylmethyl-biphenyl-2-yl ester

Ausgehend von N-(5-Brom-2-morpholinmethyl-4-ylmethyl-phenyl)-2,2,2-trifluoracetamid (400 mg; 1.09 mmol), Bis (pinacolato) diboron (414 mg; 1.63 mmol), Kalium-acetat (321 mg; 3,27 mmol) und 1,1'-Bis(diphenylphosphino)ferrocen-dichlor-palladium (II) ($PdCl_2$(dppf)) (48 mg; 0,06 mmol) in 20 ml Dimethylformamid, danach Propan-1-sulfonsäure-5-(2,4-diamino-pyrimidin-5-ylmethyl)-3-ethoxy-2-iod-phenyl-ester (375 mg; 0,76 mmol), Tetrakis-triphenylphosphin-palladium (76 mg; 0,07 mmol), wässriger 2M Kaliumphosphatlösung (3,3 ml; 6,6 mmol) und 20 ml Dimethylformamid werden 97 mg (23 %) Propan-1-sulfonsäure-3'-amino-4-(2,4-diamino-pyrimidin-5-ylmethyl)-6-ethoxy-4'-morpholinmethyl-4-ylmethyl-biphenyl-2-yl-ester als brauner Schaum erhalten.
MS (ISP): 557.2 $(M+H)^+$

(32b) Propan-1-sulfonsäure-4'-amino-4-(2,4-diamino-pyrimidin-5-ylmethyl)-6-ethoxy-3'-methyl-4-ylmethyl-biphenyl-2-yl-ester

Ausgehend von N-(4-Brom-2-methyl)-2,2,2-trifluor-acetamid (516 mg; 1.83 mmol), Bis (pinacolato) diboron (697 mg; 2,74 mmol), Kaliumacetat (539 mg; 5,5 mmol) und 1,1'-Bis(diphenylphosphino)ferrocen-dichlor-palladium (II) ($PdCl_2$(dppf)) (80 mg; 0,11 mmol) in Dimethylformamid, danach Propan-1-sulfonsäure-5-(2,4-diaminopyrimidin-5-ylmethyl)-3-ethoxy-2-iod-phenyl-ester (613 mg; 1,28 mmol), Tetrakis-triphenylphosphin-palladium (127 mg; 0.11 mmol),

wässriger 2M Kaliumphosphatlösung (5.5 ml; 11 mmol) und 20 ml Dimethylformamid werden 106 mg (17 %) Propan-1-sulfonsäure-4'-amino-4-(2,4-diamino-pyrimidin-5-ylmethyl)-6-ethoxy-3'-methyl-4-ylmethyl-biphenyl-2-yl-ester als brauner Schaum erhalten.
MS (ISP): 472.2 $(M+H)^+$

(32c) Propan-1-sulfonsäure-3'-amino-4-(2,4-diamino-pyrimidin-5-ylmethyl)-6-ethoxy-4'-fluor-4-ylmethyl-biphenyl-2-yl-ester

Ausgehend von N-(3-Brom-4-fluor-phenyl)-2,2,2-trifluoracetamid (350 mg; 1,22 mmol), Bis (pinacolato)diboron (466 mg; 1,84 mmol), Kaliumacetat (360 mg; 3,67 mmol) und 1,1'-Bis(diphenylphosphino)ferrocen-dichlor-palladium (II) ($PdCl_2$(dppf)) (54 mg; 0,07 mmol) in 20 ml Dimethylformamid, danach Propan-1-sulfonsäure-5-(2,4-diamino-pyrimidin-5-ylmethyl)-3-ethoxy-2-iod-phenyl-ester (402 mg; 0.82 mmol), Tetrakis-triphenylphosphin-palladium (85 mg; 0,07 mmol), wässriger 2M Kaliumphosphatlösung (3,7 ml; 7,4 mmol) und 20 ml Dimethylformamid werden 92 mg (24 %) Propan-1-sulfonsäure-3'-amino-4-(2,4-diamino-pyrimidin-5-ylmethyl)-6-ethoxy-4'-fluor-4-ylmethyl-biphenyl-2-yl-ester als brauner Feststoff erhalten.
MS (ISP): 476.2 $(M+H)^+$

(32d) Propan-1-sulfonsäure-3'-amino-4-(2,4-diamino-pyrimidin-5-ylmethyl)-6-ethoxy-4'-methoxy-4-ylmethyl-biphenyl-2-yl-ester

N-(3-Brom-6-methoxy-phenyl)-2,2,2-trifluoracetamid (894 mg; 3 mmol), Bis(pinacolato)diboron (1143 mg; 4.5 mmol), Kaliumacetat (883 mg; 9 mmol) und 1,1'-Bis(diphenylphosphino)-dichlor-palladium (II) (126 mg; 0,18 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und einegeengt.
Das so erhaltene Produkt wird mit Propan-1-sulfonsäure-5-(2,4-diaminopyrimidin-5-ylmethyl)-3-ethoxy-2-iod-phenyl-ester (370 mg; 0.75 mmol), Tetrakis-triphenylphosphin-palladium (52 mg; 0.05 mmol) und wässriger 2M Natriumcarbonatösung (5.6 ml; 11.2 mmol) in einem 4/1 Dimethoxyethan/Ethanol-Gemisch (15 ml) behandelt. Man erhält 300 mg (82 %) Propan-1-sulfonsäure-3'-amino-4-(2,4-diaminopyrimidin-5-ylmethyl)-6-ethoxy-4'-methoxy-4-ylmethyl-biphenyl-2-yl-ester als braunen Schaum.
MS (ISP): 488.3 $(M+H)^+$

(32e) Propan-1-sulfonsäure-4'-amino-4-(2,4-diamino-pyrimidin-5-ylmethyl)-6-ethoxy-3'-fluoro-4-ylmethyl-biphenyl-2-yl-ester

N-(4-Brom-3-fluor-phenyl)-2,2,2-trifluoracetamid (858 mg; 3 mmol), Bis-(pinacolato)diboron (1143 mg; 4.5 mmol), Kaliumacetat (883 mg; 9 mmol) und (Diphenylphosphino)-dichlor-palladium (II) (126 mg; 0,18 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und einegeengt.
Das so erhaltene Produkt wird mit Propan-1-sulfonsäure-5-(2,4-diaminopyrimidin-5-ylmethyl)-3-ethoxy-2-iod-phenyl-ester (332 mg; 0,67 mmol), Tetrakis-triphenylphosphin-palladium (47 mg; 0,04 mmol) und wässriger 2M Natriumcarbonatlösung (5 ml; 10 mmol) in einem 4/1 Dimethoxyethan/Ethanol-Gemisch (15 ml) behandelt. Man erhält 174 mg (54 %) Propan-1-sulfonsäure-4'-amino-4-(2,4-diaminopyrimidin-5-ylmethyl)-6-ethoxy-3'-fluoro-4-ylmethyl-biphenyl-2-yl-ester als braunen Schaum.
MS (ISP): 476.2 $(M+H)^+$

(32f) Propan-1-sulfonsäure-3'-amino-4-(2,4-diamino-pyrimidin-5-ylmethyl)-6-ethoxy-5'-fluoro-4-ylmethyl-biphenyl-2-yl-ester

N-(5-Iod-3-fluor-phenyl)-2,2,2-trifluoracetamid (hergestellt wie in Beispiel 4t)(999 mg; 3 mmol), Bis(pinacolato)diboron (1143 mg; 4.5 mmol), Kaliumacetat (883 mg; 9 mmol) und (Diphenylphosphino)-dichlor-palladium (II) (126 mg; 0,18 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und einegeengt.
Das so erhaltene Produkt wird mit Propan-1-sulfonsäure-5-(2,4-diaminopyrimidin-5-ylmethyl)-3-ethoxy-2-iod-phenyl-ester (332 mg; 0,67 mmol), Tetrakis-triphenylphosphin-palladium (47 mg; 0.04 mmol) und wässriger 2M Natriumcarbonatlösung (5 ml; 10 mmol) in einem 4/1 Dimethoxyethan/Ethanol-Gemisch (15 ml) behandelt. Man erhält 202

mg (63 %) Propan-1-sulfonsäure-3'-amino-4-(2,4-diaminopyrimidin-5-ylmethyl)-6-ethoxy-5'-fluor-4-ylmethyl-biphenyl-2-yl ester als leicht gelben Schaum.
MS (ISP): 476.2 $(M+H)^+$

(32g) Propan-1-sulfonsäure-3'-amino-4-(2,4-diamino-pyrimidin-5-ylmethyl)-6-ethoxy-4'-dimethylaminomethyl-4-ylmethyl-biphenyl-2-yl-ester

N-(5-Brom-2- dimethylaminomethyl-4-ylmethyl-phenyl)-2,2,2-trifluor-acetamid (1,16 g; 3,57 mmol), Bis(pinacolato)diboron (1360 mg; 5,35 mmol), Kaliumacetat (1050 mg; 10,7 mmol) und (Diphenylphosphino)-dichlor-palladium (II) (150 mg; 0,21 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und einegeengt.

Das so erhaltene Produkt wird mit Propan-1-sulfonsäure-5-(2,4-diaminopyrimidin-5-ylmethyl)-3-ethoxy-2-iod-phenyl-ester (395 mg; 0,80 mmol), Tetrakis-triphenylphosphin-palladium (56 mg; 0.05 mmol) und wässriger 2M Natriumcarbonatlösung (6 ml; 12 mmol) in einem 4/1 Dimethoxyethan/Ethanol-Gemisch (15 ml) behandelt. Man erhält 166 mg (40 %) Propan-1-sulfonsäure-3'-amino-4-(2,4-diaminopyrimidin-5-ylmethyl)-6-ethoxy-5'-fluor-4-ylmethyl-biphenyl-2-yl-ester als orangen Schaum erhalten.
MS (ISP): 515.3 $(M+H)^+$

Die obige Ausgangsverbindung Propan-1-sulfonsäure-5-(2,4-diamino-pyrimidin-5-ylmethyl)-3-ethoxy-2-iod-phenyl-ester wird wie folgt (Stufen a-b) hergestellt, vgl. Schema 2, (X) -> (XII) -> (XIII)

Stufe a) 5-(3-Ethoxy-5-hydroxy-4-iod-benzyl)-pyrimidin-2,4-diamin

5-(3-Ethoxy-4-iod-5-methoxymethoxy-benzyl)-pyrimidin-2,4-diamin (20 g; 46,48 mmol) wird in Methanol (900 ml) suspendiert und unter Rühren und Argonbegasung auf 50°C erwärmt. Die Suspension wird mit 4,5 M Salzsäure in Methanol (62 ml; 279 mmol) versetzt, wobei eine Lösung entsteht. Nach 30 Minuten wird das Reaktionsgemisch mit einem Eisbad auf 5°C abgekühlt, das pH mit $NH_4OH$ konz. auf 10 eingestellt und am Rollverdampfer das Volumen auf ca 1/10 reduziert. Die erhaltene Suspension wird im Eisbad abgekühlt, mit Wasser (350 ml) versetzt und 30 Minuten gerührt. Anschliessend wird die Suspension abgenutscht, mit Wasser gewaschen und am Hochvakuum getrocknet. Ausbeute: 16,74 g (93 %) 5-(3-Ethoxy-5-hydroxy-4-iod-benzyl)-pyrimidin-2,4-diamin als farblose Kristalle erhalten.
MS (ISP): 387,1 $(M+H)^+$

Stufe b) Propan-1-sulfonsäure-5-(2,4-diamino-pyrimidin-5-ylmethyl)-3-ethoxy-2-iodphenyl-ester

5-(3-Ethoxy-5-hydroxy-4-iod-benzyl)-pyrimidin-2,4-diamin (907 mg; 2.35 mmol) wird in Dimethylformamid (25 ml) gelöst, mit Kalium-tert-butylat (395 mg; 3.52 mmol) versetzt und bei 0°C mit n-Propansulfonylchlorid (0.395 ml; 3.52 mmol) versetzt. Anschliessend wird eine Stunde bei Raumtemperatur gerührt. Das Reaktionsgemisch wird eingedampft und der Rückstand über Kieselgel mit Methylenchlorid /Methanol (19/1) und 0.5% Ammoniak chromatographiert . Die reinen Fraktionen werden vereinigt und das Lösungsmittel abgedampft.
Ausbeute: 813 mg (49 %) Propan-1-sulfonsäure-5-(2,4-diamino-pyrimidin-5-ylmethyl)-3-ethoxy-2-iod-phenyl-ester als gelbliches Wachs.
MS (ISP): 493.1 $(M+H)^+$

Beispiel 33:

Analog Beispiel 4) werden Verbindungen der Formel VIII, Schema 2, aus den entsprechenden Verbindungen der Formel III hergestellt.

(33a) Propan-2-sulfonsäure-3'-amino-4-(2,4-diamino-pyrimidin-5-ylmethyl)-6-ethoxy-4'-methoxy-4-ylmethyl-biphenyl-2-yl-ester

N-(3-Brom-6-methoxy-phenyl)-2,2,2-trifluoracetamid (894 mg; 3 mmol), Bis-(pinacolato)diboron (1143 mg; 4,5 mmol), Kaliumacetat (883 mg; 9 mmol) und 1,1'-Bis(diphenylphosphino)-dichlor-palladium (II) (126 mg; 0,18 mmol) in 60 ml Dioxan werden bei 80°C 3 Stunden gerührt. Die Häfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit Propan-2-sulfonsäure-5-(2,4-diaminopyrimidin-5-ylmethyl)-3-ethoxy-2-iod-phenyl-ester (370 mg; 0,75 mmol), Tetrakis-triphenylphosphin-palladium (52 mg; 0.05 mmol), wässriger 2M Natriumcarbonatlösung (5,6 ml; 11,2 mmol) in einem 4/1 Dimethoxyethan/Ethanol-Gemisch (15 ml) behandelt. Man erhält 59 mg (16 %) Propan-2-sulfonsäure-3'-amino-4-(2,4-diamino-pyrimidin-5-ylmethyl)-6-ethoxy-4'-methoxy-4-ylmethyl-biphenyl-2-yl-ester als leicht grünen Schaum.
MS (ISP): 488.3 $(M+H)^+$

(33b) Propan-2-sulfonsäure-4'-amino-4-(2,4-diamino-pyrimidin-5-ylmethyl)-6-ethoxy-3'-fluor-4-ylmethyl-biphenyl-2-yl-ester

Ausgehend von N-(4-Brom-3-fluor-phenyl)-2,2,2-trifluoracetamid (858 mg; 3 mmol), Bis(pinacolato)diboron (1143 mg; 4.5 mmol), Kaliumacetat (883 mg; 9 mmol) und (Diphenylphosphino)-dichlor-palladium (II) (126 mg; 0,18 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit Propan-2-sulfonsäure-5-(2,4-diaminopyrimidin-5-ylmethyl)-3-ethoxy-2-iod-phenyl-ester (406 mg; 0.82 mmol), Tetrakis-triphenylphosphin-palladium (58 mg; 0.05 mmol), wässriger 2M Natriumcarbonatlösung (6.2 ml; 12.4 mmol) in einem 4/1 Dimethoxyethan/Ethanol-Gemisch (15 ml) behandelt. Man erhält 195 mg (56 %) Propan-2-sulfonsäure-4'-amino-4-(2,4-diamino-pyrimidin-5-ylmethyl)-6-ethoxy-3'-fluor-4-ylmethyl-biphenyl-2-yl-ester als weisse Kristalle.
MS (ISP): 476.2 $(M+H)^+$

(33c) Propan-2-sulfonsäure-3'-amino-4-(2,4-diamino-pyrimidin-5-ylmethyl)-6-ethoxy-5'-fluor-4-ylmethyl-biphenyl-2-yl-ester

Ausgehend von N-(5-Iod-3-fluor-phenyl)-2,2,2-trifluoracetamid (hergestellt wie in Beispiel 4t)(999 mg; 3 mmol), Bis(pinacolato)diboron (1143 mg; 4,5 mmol), Kaliumacetat (883 mg; 9 mmol) und (Diphenylphosphino)-dichlor-palladium (II) (126 mg; 0,18 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit Propan-2-sulfonsäure-5-(2,4-diaminopyrimidin-5-ylmethyl)-3-ethoxy-2-iod-phenyl-ester (406 mg; 0,82 mmol),Tetrakistriphenylphosphin-palladium (58 mg; 0.05 mmol), wässriger 2M Natriumcarbonatlösung (6,2 ml; 12.4 mmol) in einem 4/1 Dimethoxyethan/Ethanol-Gemisch (15 ml) behandelt. Man erhält 197 mg (50 %) Propan-2-sulfonsäure-3'-amino-4-(2,4-diamino-pyrimidin-5-ylmethyl)-6-ethoxy-5'-fluor-4-ylmethyl-biphenyl-2-yl-ester als weissen Feststoff.
MS (ISP): 476.2 $(M+H)^+$

(33d) Propan-2-sulfonsäure-3-amino-4-(2,4-diamino-pyrimidin-5-ylmethyl)-6-ethoxy-4'-morpholinmethyl-4-ylmethyl-biphenyl-2-yl-ester

N-(5-Brom-2-morpholinmethyl-4-ylmethyl-phenyl)-2,2,2-trifluor-acetamid (500 mg; 1,36 mmol), Bis(pinacolato)diboron (519 mg; 2,04 mmol), Kaliumacetat (401 mg; 4,09 mmol) und (Diphenylphosphino)-dichlor-palladium (II) (88 mg; 0,08 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Propan-2-sulfonsäure-5-(2,4-diamino-pyrimidin-5-ylmethyl)-3-ethoxy-2-iod-phenyl-ester (336 mg; 0,68 mmol), Tetrakis-triphenylphosphin-palladium (94 mg; 0.08 mmol) und wässriger 2M Natriumcarbonatlösung (10 ml; 20 mmol) in einem 4/1 Dimethoxyethan/Ethanol-Gemisch (15 ml) werden 104 mg (27 %) Propan-2-sulfonsäure-3'-amino-4-(2,4-diaminopyrimidin-5-ylmethyl)-6-ethoxy-4'-morpholinmethyl-4-ylmethyl-biphenyl-2-yl-ester als brauner Schaum erhalten.
MS (ISP): 557.3 $(M+H)^+$

(33e) Propan-2-sulfonsäure-4'-amino-4-(2,4-diamino-pyrimidin-5-ylmethyl)-6-ethoxy-3'-methyl-biphenyl-2-yl-ester

N-(4-Brom-2-methyl)-2,2,2-trifluor-acetamid (600 mg; 2.13 mmol), Bis-(pinacolato)diboron (810 mg; 3,20 mmol), Kaliumacetat (626 mg; 6,38 mmol) und (Diphenylphosphino)-dichlor-palladium (II) (89 mg; 0,13 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Propan-2-sulfonsäure-5-(2,4-diaminopyrimidin-5-ylmethyl)-3-ethoxy-2-iod-phenyl-ester (524 mg; 1,06 mmol), Tetrakis-triphenylphosphin-palladium (147 mg; 0,13 mmol) und wässriger 2M Natriumcarbonatlösung (16 ml; 32 mmol) in einem 4/1 Dimethoxyethan/Ethanol-Gemisch (15 ml) werden 229 mg (60 %) Propan-2-sulfonsäure-4'-amino-4-(2,4-diamino-pyrimidin-5-ylmethyl)-6-ethoxy-3'-methyl-biphenyl-2-yl-ester als leicht braune Kristalle erhalten.
MS (ISP): 472.3 $(M+H)^+$
NMR $^1H$: (250 MHz, $\delta$, TMS, DMSO): 1.13 (d; J=6.8; 6H); 1.16 (t; J=6.9; 3H);); 2.04 (s; 3H); 3.03 (Sept.; J=6.8, 1 H); 3.63 (s; 2H); 3.94 (q; J=6.9; 2H); 4.88 (s(br); 2H); 5.72 (s(br); 2H); 6.15 (s(br); 2H); 6.60 (d; J=7.6; 1H); 6.78 (d; J=8.0; 1H); 6.81 (s; 1H); 6.84 (s; 1H); 6.90 (s; 1H); 7.57 (s; 1H).

(33f) Propan-2-sulfonsäure-3'-amino-4-(2,4-diamino-pyrimidin-5-ylmethyl)-6-ethoxy-4'-dimethylaminomethyl-biphenyl-2-yl-ester

N-(5-Brom-2- dimethylaminomethyl-4-ylmethyl-phenyl)-2,2,2-trifluor-acetamid (1.16 g; 3.57 mmol), Bis(pinacolato)diboron (1360 mg; 5.35 mmol), Kaliumacetat (1050 mg; 10.7 mmol) und (Diphenylphosphino)-dichlor-palladium (II) (150 mg; 0,21 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.
Das so erhaltene Produkt wird mit Propan-2-sulfonsäure-5-(2,4-diaminopyrimidin-5-ylmethyl)-3-ethoxy-2-iod-phenyl-ester (483 mg; 0,98 mmol), Tetrakis-triphenylphosphin-palladium (68 mg; 0.06 mmol) und wässriger 2M Natriumcarbonatlösung (7,4 ml; 14,8 mmol) in einem 4/1 Dimethoxyethan/Ethanol-Gemisch (15 ml) behandelt. Man erhält 123 mg (24 %) Propan-2-sulfonsäure-3'-amino-4-(2,4-diaminopyrimidin-5-ylmethyl)-6-ethoxy-4'-dimethylaminomethyl-biphenyl-2-yl-ester als weissen Schaum.
MS (ISP): 515.3 $(M+H)^+$
In Analogie zu Beispiel 2a (Suzuki-Kopplung mit Boronsäure) wird hergestellt:

(33g) Propan-2-sulfonsäure 5-(2,4-diamino-pyrimidin-5-ylmethyl)-3-ethoxy-2-(6-methyl-pyridin-3-yl)-phenyl-ester

Ausgehend von Propan-2-sulfonsäure 5-(2,4-diamino-pyrimidin-5-ylmethyl)-3-ethoxy-2-iod-phenyl-ester (246 mg; 0.5 mmol) und 2-Methylpyridinyl-5-boronsäure (205 mg; 1,5 mmol) werden 146 mg (64%) Propan-2-sulfonsäure 5-(2,4-diamino-pyrimidin-5-ylmethyl)-3-ethoxy-2-(6-methyl-pyridin-3-yl)-phenyl-ester als leicht gelbes Pulver erhalten.
MS (ISP): 458.5 $(M+H)^+$
Die obige Ausgangsverbindung Propan-2-sulfonsäure-5-(2,4-diamino-pyrimidin-5-ylmethyl)-3-ethoxy-2-iod-phenyl-ester wird wie folgt hergestellt, vgl. Schema 2, (XII) -> (XIII)
5-(3-Ethoxy-5-hydroxy-4-iod-benzyl)-pyrimidin-2,4-diamin (siehe Beispiel 32 Stufe a)) (5,9 g; 15,28 mmol) wird in Dimethylformamid (150 ml) gelöst und.mit Kalium-tert-butylat (2,57 g; 22,92 mmol) versetzt . Bei 0°C wird Propan-2-sulfonylchlorid (2,56 ml; 22,92 mmol) hinzugefügt. Anschliessend wird eine Stunde bei Raumtemperatur gerührt. Das Reaktionsgemisch wird eingedampft und der Rückstand über Kieselgel mit Methylenchlorid/Methanol (19/1) und 0.5% Ammoniak chromatographiert . Die reinen Fraktionen werden vereinigt und das Lösungsmittel abgedampft.
Ausbeute: 4,16 g (55 %) Propan-2-sulfonsäure-5-(2,4-diamino-pyrimidin-5-ylmethyl)-3-ethoxy-2-iod-phenyl-ester als oranger Feststoff.
MS (ISP): 493.1 $(M+H)^+$

Beispiel 34:

In Analogie zu Beispiel 2a wird die Verbindung der Formel VIII, Schema 2, aus den entsprechenden Verbindungen der Formel XIII hergestellt.

(34a) Cyclopropansulfonsäure-3'-amino-4-(2,4-diamino-pyrimidin-5-ylmethyl)-6-ethoxy-biphenyl-2-yl-ester

Ausgehend von Cyclopropansulfonsäure-5-(2,4-diamino-pyrimidin-5-ylmethyl)-3-ethoxy-2-iod-phenyl-ester (190 mg; 0,387 mmol) und 3-Amino-phenylboronsäure (87 mg; 0,564 mmol) werden 69 mg (39 %) Cyclopropansulfonsäure-3'-amino-4-(2,4-diaminopyrimidin-5-ylmethyl)-6-ethoxy-biphenyl-2-yl-ester als farbloses Pulver erhalten.
MS (ISP): 456,5 $(M+H)^+$
In Analogie zu Beispiel 4 werden hergestellt:

(34b) Cyclopropansulfonsäure-4-(2,4-diamino-pyrimidin-5-ylmethyl)-6-ethoxy-4'-methylamino-biphenyl-2-yl-ester

Ausgehend von Cyclopropansulfonsäure-5-(2,4-diamino-pyrimidin-5-ylmethyl)-3-ethoxy-2-iod-phenyl-ester (450 mg; 0,920 mmol) und N-(4-Brom-phenyl)-2,2,2-trifluor-N-methyl-acetamid (405 mg; 1,15 mmol) (erster Schritt in 11,5 ml Dimethoxyethan anstatt Dimethylformamid) werden 114 mg (16 %) Cyclopropansulfonsäure-4-(2,4-diamino-pyrimidin-5-ylmethyl)-6-ethoxy-4'-methylamino-biphenyl-2-yl-ester als farbloses Pulver erhalten.
MS (ISP): 470,2 $(M+H)^+$

(34c) Cyclopropansulfonsäure-3'-amino-4-(2,4-diamino-pyrimidin-5-ylmethyl)-6-ethoxy-5'-fluor-biphenyl-2-yl ester

Ausgehend von Cyclopropansulfonsäure-5-(2,4-diamino-pyrimidin-5-ylmethyl)-3-ethoxy-2-iod-phenyl-ester (294 mg; 0,60 mmol) und N-(5-Iod-3-fluor-phenyl)-2,2,2-trifluoracetamid (Herstellung in Beispiel 4t beschrieben) (333 mg; 1,0 mmol) (erster Schritt in 12 ml Dioxan anstatt Dimethylformamid mit Bis-triphenylphosphin-palladium-dichlorid (56 mg; 0,08 mmol) und zweiter Schritt in Dimethoxyethan (8 ml) und Ethanol (2 ml) mit Tetrakis-triphenylphosphin-palladium (69 mg; 0,06 mmol)) werden 147 mg (30%) Cyclopropansulfonsäure-3'-amino-4-(2,4-diamino-pyrimidin-5-ylmethyl)-6-ethoxy-5'-fluor-biphenyl-2-yl-ester als farbloses Pulver erhalten.
MS (ISP): 474,2 $(M+H)^+$

(34d) Cyclopropansulfonsäure-4'-amino-4-(2,4-diamino-pyrimidin-5-ylmethyl)-6-ethoxy-3'-methyl-biphenyl-2-yl-ester

Ausgehend von Cyclopropansulfonsäure-5-(2,4-diamino-pyrimidin-5-ylmethyl)-3-ethoxy-2-iod-phenyl-ester (294 mg; 0,6 mmol) und N-(4-Brom-2-methyl-phenyl)-2,2,2-trifluoracetamid (282 mg; 1,0 mmol) (erster Schritt in 12 ml Dioxan anstatt Dimethylformamid mit Bis-triphenylphosphin-palladium-dichlorid (56 mg; 0,08 mmol) und zweiter Schritt in Dimethoxyethan (8 ml) und Ethanol (2 ml) mit Tetrakis-triphenylphosphin-palladium (69 mg; 0,06 mmol)) werden 151 mg (30 %) Cyclopropansulfonsäure-4'-amino-4-(2,4-diamino-pyrimidin-5-ylmethyl)-6-ethoxy-3'methyl-biphenyl-2-yl-ester als farbloses Pulver erhalten.
MS (ISP): 470,3 $(M+H)^+$
N-(4-Brom-2-methyl-phenyl)-2,2,2-trifluoracetamid wird aus 4-Brom-2-methyl-anilin hergestellt (in Analogie zu Beispiel 4l).

(34e) Cyclopropansulfonsäure-3'-amino-4-(2,4-diamino-pyrimidin-5-ylmethyl)-6-ethoxy-4'-morpholin-4-ylmethyl-biphenyl-2-yl-ester

Ausgehend von Cyclopropansulfonsäure-5-(2,4-diamino-pyrimidin-5-ylmethyl)-3-ethoxy-2-iod-phenyl-ester (294 mg; 0,6 mmol) und N-(5-Brom-2-methylmorpholinphenyl)-2,2,2-trifluor-acetamid (367 mg; 1,0 mmol) (erster Schritt in 10 ml Dioxan und zweiter Schritt in Dimethoxyethan (10 ml) und Ethanol (2 ml)) werden 262 mg (46 %) Cyclopropansulfonsäure-3'-amino-4-(2,4-diamino-pyrimidin-5-ylmethyl)-6-ethoxy-4'morpholin-4-ylmethyl-biphenyl-2-yl-ester als farbloses Pulver erhalten.
MS (ISP): 555,2 $(M+H)^+$
N-(5-Brom-2-methylmorpholin-phenyl)-2,2,2-trifluor-acetamid wird aus 5-Brom-2-methylmorpholin-anilin hergestellt (in Analogie zu Beispiel 41).

(34f) Cyclopropansulfonsäure-3'-amino-4-(2,4-diamino-pyrimidin-5-ylmethyl)-6-ethoxy-4'-methyl-biphenyl-2-yl-ester

Ausgehend von Cyclopropansulfonsäure-5-(2,4-diamino-pyrimidin-5-ylmethyl)-3-ethoxy-2-iod-phenyl-ester (294 mg; 0,6 mmol) und N-(5-Brom-2-methyl-phenyl)-2,2,2-trifluor-acetamid (aus 5-Brom-2-methyl-anilin in Analogie zu Beispiel 41 hergestellt) (367 mg; 1,0 mmol) (erster Schritt in 10 ml Dimethoxyethan und zweiter Schritt in Dimethoxyethan (10 ml) und Ethanol (2 ml)) werden 187 mg (38 %) Cyclopropansulfonsäure-3'-amino-4-(2,4-diamino-pyrimidin-5-ylmethyl)-6-ethoxy-4'-methylbiphenyl-2-yl-ester beiges Pulver erhalten.
MS (ISP): 470,3 $(M+H)^+$

Die obige Ausgangsverbindung Cyclopropansulfonsäure-5-(2,4-diamino-pyrimidin-5-ylmethyl)-3-ethoxy-2-iod-phenyl-ester wird wie folgt hergestellt, vgl. Schema 2, (XII) -> (XIII)

5-(3-Ethoxy-5-hydroxy-4-iod-benzyl)-pyrimidin-2,4-diamin (siehe Beispiel 32 Stufe a)) (360 mg; 0,932 mmol) wird in Dimethylformamid (30 ml) gelöst und mit Kalium-tert-butylat (125 mg; 1,12 mmol) bei Raumtemperatur versetzt. Bei 0°C wird Cyclopropansulfonylchlorid (2.56 ml; 22.92 mmol) hinzugegeben. Anschliessend wird 4½ Stunden bei 0-5°C gerührt. Das Reaktionsgemisch wird eingedampft und der Rückstand über Kieselgel (45 g) mit Methylenchlorid /Methanol/$NH_4OH$ konz. (1/1 Gemisch aus 19/1/0,05 und 90/10/1) chromatographiert . Die reinen Fraktionen werden vereinigt und das Lösungsmittel abgedampft.

Ausbeute: 210 mg (46 %) Cyclopropansulfonsäure-5-(2,4-diamino-pyrimidin-5-ylmethyl)-3-ethoxy-2-iod-phenyl-ester als farbloser Feststoff.
MS (ISP): 491.1 $(M+H)^+$

Beispiel 35:

Analog Beispiel 4, bzw. wie in Beispiel 35(a) beschrieben, werden Verbindungen der Formel VIII, Schema 2, aus den entsprechenden Verbindungen der Formel III hergestellt.

(35a) Butan-2-sulfonsäure-4'-amino-4-(2,4-diamino-pyrimidin-5-ylmethyl)-6-ethoxybiphenyl-2-yl-ester

Bis(pinacolato)diboron (322 mg, 1,26 mmol), N-(4-Brom-phenyl)-2,2,2-trifluoracetamid (225 mg, 0,84 mmol), Kaliumacetat (249 mg, 0,254 mmol) und Tetrakis-triphenylphosphin-palladium (59 mg, 0,051 mmol) werden in 12 ml abs. Dioxan gelöst, viermal evakuiert und mit Argon begast und anschliessend während 2 h bei 80°C gehalten. Eine HPLC-Analyse zeigt den vollständigen Umsatz des aromatischen Bromids an. Man dampft unter vermindertem Druck ein, trocknet 1 Stunde unter stark vermindertem Druck und versetzt den Rückstand mit Butan-2-sulfonsäure-5-(2,4-diamino-pyrimidin-5-ylmethyl)-3-ethoxy-2-iod-phenyl-ester (214 mg, 0,422 mmol), 59 mg frischem Tetrakis-triphenylphosphin-palladium, 7.5 ml Dimethoxyethan, 1,75 ml EtOH, 6 ml 2N wässrigem $Na_2CO_3$ und lässt 2 Stunden bei 75°C reagieren. Man kühlt ab, giesst auf Eis, extrahiert mit Ethylacetat, wäscht mit gesättigter wässriger Kochsalzlösung, trocknet über $MgSO_4$ und dampft zur Trockne ein. Flash-Chromatographie über $SiO_2$ ($CH_2Cl_2$/MeOH/25% $NH_3$ (19/1/0,05) liefert 114 mg der Titelverbindung als bräunlichen Schaum.
MS (ISP): 472,2 $(M+H)^+$
NMR $^1$H(250 MHz, δ, TMS, DMSO): 0.79 (t, J=7, 3H), 1.01 (d, J=7, 3H), 1.17 (t, J=7, 3H), 1.27 (m, 1H), 1.63 (m, 1H), 2.74 (m, 1H), 3.63 (s, 2H), 3.93 (q, J=7, 2H), 5.08 (br s, 2H), 5.71 (br s, 2H), 6.13 (br s, 2H), 6.55 (d, J=9, 2H), 6.81 (s, 1H), 6.89 (d, J=9, 2H), 6.91 (s, 1H), 7.58 (s, 1H).

(35b) Butan-2-sulfonsäure-4'-amino-4-(2,4-diamino-pyrimidin-5-ylmethyl)-6-ethoxy-3'-fluor-biphenyl-2-yl-ester

Ausgehend von 241 mg (0,84 mmol) N-(4-Brom-2-fluor-phenyl)-2,2,2-trifluoracetamid und 214 mg (0,422 mmol) Butan-2-sulfonsäure-5-(2,4-diamino-pyrimidin-5-ylmethyl)-3-ethoxy-2-iod-phenyl-ester werden 85 mg der Titelverbindung als hellbraunes Pulver erhalten.
MS (ISP): 490,3 $(M+H)^+$

(35c) Butan-2-sulfonsäure-3'-amino-4-(2,4-diamino-pyrimidin-5-ylmethyl)-6-ethoxy-4'-morpholin-4-ylmethyl-biphenyl-2-yl-ester

Ausgehend von 308 mg (0,84 mmol) N-(5-Brom-2-morpholin-4-ylmethyl-phenyl)-2,2,2-trifluor-acetamid und 214 mg (0,422 mmol) Butan-2-sulfonsäure-5-(2,4-diaminopyrimidin-5-ylmethyl)-3-ethoxy-2-iod-phenyl-ester werden 139 mg der Titelverbindung als hellbraune Kristalle erhalten.
MS (ISP): 571,2 $(M+H)^+$

(35d) Butan-2-sulfonsäure-3'-amino-4-(2,4-diamino-pyrimidin-5-ylmethyl)-6-ethoxy-4'-methoxy-biphenyl-2-yl-ester

Ausgehend von 197 mg (0,66 mmol) N-(5-Brom-2-methoxy-phenyl)-2,2,2-trifluoracetamid und 168 mg (0,33 mmol) Butan-2-sulfonsäure-5-(2,4-diamino-pyrimidin-5-ylmethyl)-3-ethoxy-2-iod-phenyl-ester werden 41 mg der Titelverbindung als hellbraune Kristalle erhalten.
MS (ISP): 502,3 $(M+H)^+$

(35e) Butan-2-sulfonsäure-4'-amino-4-(2,4-diamino-pyrimidin-5-ylmethyl)-6-ethoxy-3'-methyl-biphenyl-2-yl-ester

Ausgehend von 191 mg (0,68 mmol) N-(4-Brom-2-methyl-phenyl)-2,2,2-trifluoracetamid und 173 mg (0,34 mmol) Butan-2-sulfonsäure-5-(2,4-diamino-pyrimidin-5-ylmethyl)-3-ethoxy-2-iod-phenyl-ester werden nach zweimaliger Chromatographie 53 mg der Titelverbindung als hellbrauner Schaum erhalten.
MS (ISP): 486,3 $(M+H)^+$
NMR $^1$H (250 MHz, δ, TMS, DMSO): 0.77 (t, J=7,3H), 1.00 (d, J=7,3H), 1.16 (t, J=7, 3H), 1.25 (m, 1H), 1.62 (m, 1H), 2.04 (s, 3H), 2.70 (m, 1H), 3.62 (s, 2H), 3.93 (q, J=7, 2H), 4.9 (br d, 2H), 5.73 (br s, 2H), 6.15 (br s, 2H), 6.60 (d, J=9, 1H), 6.78 (d, J=9, 1H), 6.80 (s, 1H), 6.80 (s, 1H), 6.92 (s, 1H), 7.58 (s, 1H).

(35f) Butan-2-sulfonsäure-4-(2,4-diamino-pyrimidin-5-ylmethyl)-6-ethoxy-4'-(2-hydroxy-ethylamino)-biphenyl-2-yl-ester

Ausgehend von 370 mg (0,87 mmol) N-(4-Brom-phenyl)-N-[2-(tert-butyldimethyl-silanyloxy)-ethyl]-2,2,2-trifluor-acetamid und 220 mg (0,43 mmol) Butan-2-sulfonsäure-5-(2,4-diamino-pyrimidin-5-ylmethyl)-3-ethoxy-2-iod-phenyl-ester werden, nach saurer Abspaltung der tert-Butyl-dimethylsilanyl-Schutzgruppe ((4,5 N HCl [MeOH]) 35 mg der Titelverbindung als farbloser Schaum erhalten.
MS (ISP): 516,3 $(M+H)^+$

Das hierzu benötigte N-(4-Brom-phenyl)-N-[2-(tert-butyl-dimethyl-silanyloxy)-ethyl]-2,2,2-trifluor-acetamid wird wie folgt hergestellt:

N-(4-Brom-phenyl)-2,2,2-trifluor-acetamid (1,12 g, 4,18 mmol) werden in 8,5 ml abs. Dimethylformamid vorgelegt und mit 1,2 g pulverisiertem Molekularsieb 4 A versetzt. Man rührt 15 Min., gibt 612 mg (5,45 mmol) KOtBu zu und gibt nach weiteren 5 Min. 1,44 g (5,02 mmol) tert-Butyl-(2-iod-ethoxy)-dimethyl-silan zu. Man lässt 36 Stunden bei 70°C reagieren und kühlt ab. Abfiltrieren, Extrahieren mit Ethylacetat, Waschen mit Wasser, Trocknen über $MgSO_4$, Eindampfen unter vermindertem Druck, gefolgt von Flash-Chromatographie mit $SiO_2$ (n-Hexan/Ethylacetat 9/1) liefert 887 mg der Titelverbindung als farbloses Öl.
MS (EI): 410, 412 $(M-CH_3)^+$

Die obige Ausgangsverbindung Butan-2-sulfonsäure-5-(2,4-diamino-pyrimidin-5-ylmethyl)-3-ethoxy-2-iod-phenyl-ester wird wie folgt hergestellt, vgl. Schema 2, (XII) -> (XIII)

5-(2,4-Diamino-pyrimidin-5-ylmethyl)-3-ethoxy-2-iod-phenol (Beispiel 32 Stufe a)) (40 mg; 0,104 mmol) werden in 2,1 ml abs. Tetrahydrofuran gelöst, mit 160 mg Molekularsieb 4A und 14 mg (0,125 mmol) KOtBu versetzt und während 20 Min. gerührt. Man kühlt anschliessend auf -20°C und gibt 20 mg (0,127 mmol) sec.Butylsulfonylchlorid zu. Nach 30 Min. erhöht man die Temperatur auf +10°C und lässt noch 2 Stunden reagieren. Man verdünnt das Reaktionsgemisch mit einer Mischung aus $CH_2Cl_2$/MeOH/25% $NH_3$(90/10/1), filtriert, dampft ein und reinigt das Rohprodukt mittels Flash-Chromatographie auf $SiO_2$ ($CH_2Cl_2$/MeOH/25% $NH_3$(19/1/0,05). Dabei werden 27 mg der Titelverbindung als farbloses Pulver erhalten.
MS (ISP): 507,2 $(M+H)^+$

Beispiel 36:

Analog Beispiel 4) werden Verbindungen der Formel VIII, Schema 2, aus den entsprechenden Verbindungen der Formel III hergestellt.

(36a) 2-Methyl-propan-1-sulfonsäure-4'-amine-4-(2,4-diamino-pyrimidin-5-ylmethyl)-6-ethoxy-3'-methyl-biphenyl-2-yl-ester

Ausgehend von 191 mg (0,68 mmol) N-(4-Brom-2-methyl-phenyl)-2,2,2-trifluoracetamid und 173 mg (0,34 mmol) 2-Methyl-propan-1-sulfonsäure-5-(2,4-diaminopyrimidin-5-ylmethyl)-3-ethoxy-2-iod-phenyl-ester werden nach Chromatographie und Verrühren in 5 ml Ether 109 mg der Titelverbindung als brauner Feststoff erhalten.
MS (ISP): 486,4 $(M+H)^+$

(36b) 2-Methyl-propan-1-sulfonsäure-4'-amino-4-(2,4-diamino-pyrimidin-5-ylmethyl)-6-ethoxy-3'-fluor-biphenyl-2-yl-ester

Ausgehend von 241 mg (0,84 mmol) N-(4-Brom-2-fluor-phenyl)-2,2,2-trifluoracetamid und 215 mg (0,42 mmol) 2-Methyl-propan-1-sulfonsäure-5-(2,4-diaminopyrimidin-5-ylmethyl)-3-ethoxy-2-iod-phenyl-ester werden 164 mg der Titelverbindung als hellbrauner Schaum erhalten.
MS (ISP): 490,3 $(M+H)^+$

(36c) 2-Methyl-propan-1-sulfonsäure-4'-amino-4-(2,4-diamino-pyrimidin-5-ylmethyl)-6-ethoxy-biphenyl-2-yl-ester

Ausgehend von 225 mg (0,84 mmol) N-(4-Brom-phenyl)-2,2,2-trifluor-acetamid und 214 mg (0,42 mmol) 2-Methyl-propan-1-sulfonsäure-5-(2,4-diamino-pyrimidin-5-ylmethyl)-3-ethoxy-2-iod-phenyl-ester werden nach zweimaliger Chromatographie 78 mg der Titelverbindung als brauner Schaum erhalten.
MS (ISP): 472,2 $(M+H)^+$

(36d) 2-Methyl-propan-1-sulfonsäure-3'-amino-4-(2,4-diamino-pyrimidin-5-ylmethyl)-6-ethoxy-4'-methoxy-biphenyl-2-yl-ester

Ausgehend von 250 mg (0,84 mmol) N-(5-Brom-2-methoxy-phenyl)-2,2,2-trifluoracetamid und 214 mg (0,42 mmol) 2-Methyl-propan-1-sulfonsäure-5-(2,4-diaminopyrimidin-5-ylmethyl)-3-ethoxy-2-iod-phenyl-ester werden 56 mg der Titelverbindung als hellbrauner Schaum erhalten.
MS (ISP): 502,3 $(M+H)^+$

(36e) 2-Methyl-propan-1-sulfonsäure-4-(2,4-diamino-pyrimidin-5-ylmethyl)-6-ethoxy-4'-(2-hydroxy-ethylamino)-biphenyl-2-yl-ester

Ausgehend von 397 mg (0,93 mmol) N-(4-Brom-phenyl)-N-[2-(tert-butyldimethyl-silanyloxy)-ethyl]-2,2,2-trifluor-acetamid und 220 mg (0,47 mmol) 2-Methylpropan-1-sulfonsäure-5-(2,4-diamino-pyrimidin-5-ylmethyl)-3-ethoxy-2-iod-phenylester werden nach saurer Abspaltung der tert-Butyl-dimethylsilanyl-Schutzgruppe (HCl/MeOH 165 mg der Titelverbindung als hellbeiger Schaum erhalten.
MS (ISP): 516,3 $(M+H)^+$

Die obige Ausgangsverbindung 2-Methyl-propan-1-sulfonsäure-5-(2,4-diaminopyrimidin-5-ylmethyl)-3-ethoxy-2-iod-phenyl-ester wird wie folgt hergestellt:

5-(2,4-Diamino-pyrimidin-5-ylmethyl)-3-ethoxy-2-iod-phenol (579 mg, 1,5 mmol) werden in 15 ml abs. Dimethylformamid vorgelegt und mit 2,4 g pulverisiertem Molekularsieb 4A und 202 mg (1,8 mmol) KOtBu versetzt. Man rührt 10 Min. bei Raumtemperatur und gibt dann bei 0°C 480 mg (3 mmol) Isobutylsulfonylchlorid zu. Nach 2 Stunden wird abfiltriert und sorgfältig mit Ethylacetat nachgewaschen. Die vereinigten Filtrate werden unter vermindertem Druck eingedampft. Flash-Chromatographie auf $SiO_2$ ($CH_2Cl_2$/MeOH/25% $NH_3$(19/1/0,05)) liefert 521 mg der Titelverbindung als blass-gelbe Kristalle; MS (ISP): 507,2 $(M+H)^+$

Beispiel 37:

Analog Beispiel 4 bzw. wie in Beispiel 37(a) beschrieben, werden Verbindungen der Formel VIII, Schema 2, aus den entsprechenden Verbindungen der Formel XIII hergestellt.

(37a) 2,2-Dimethyl-propan-1-sulfonsäure-4'-amino-4-(2,4-diamino-pyrimidin-5-ylmethyl)-6-ethoxy-3'-fluor-biphenyl-2-yl-ester

Bis-(pinacolato)diboron (318 mg, 1,25 mmol), N-(4-Brom-2-fluor-phenyl)-2,2,2-trifluor-acetamid (238 mg, 0,83 mmol), Kaliumacetat (246 mg, 0,251 mmol) und Tetrakis-triphenylphosphin-palladium (94 mg, 0,08 mmol) werden in 12 ml abs. Dioxan gelöst, viermal evakuiert und mit Argon begast und anschliessend über Nacht bei 80°C gehalten. Eine HPLC-Analyse zeigt den vollständigen Umsatz des aromatischen Bromids an. Man dampft unter vermindertem Druck ein, trocknet unter stark vermindertem Druck, versetzt den Rückstand mit 2,2-Dimethyl-propan-1-sulfonsäure-5-(2,4-diamino-pyrimidin-5-ylmethyl)-3-ethoxy-2-iod-phenyl-ester (217 mg, 0,42 mmol), 58 mg frischem Tetrakis-triphenylphosphin-palladium, 7.5 ml Dimethoxyethan, 1,75 ml EtOH, 6 ml 2N wässriger Natriumcarbonatlösung, entgast nochmals und lässt über Nacht bei 75°C reagieren. Man kühlt ab, giesst auf Eis, extrahiert mit Ethylacetat, wäscht mit gesättigtem NaCl, trocknet über $MgSO_4$ und dampft zur Trockne ein. Flash-Chromatographie auf $SiO_2$ ($CH_2Cl_2$/MeOH/25% $NH_3$(19/1/0,05) liefert, nach Verrühren mit Diethylether, 69 mg der Titelverbindung als hellbraunen Schaum.
MS (ISP): 504,3 $(M+H)^+$;

(37b) 2,2-Dimethyl-propan-1-sulfonsäure-3'-amino-4-(2,4-diamino-pyrimidin-5-ylmethyl)-6-ethoxy-4'-methoxy-biphenyl-2-yl-ester

Ausgehend von 197 mg (0,66 mmol) N-(5-Brom-2-methoxy-phenyl)-2,2,2-trifluoracetamid und 173 mg (0,33 mmol) 2,2-Dimethyl-propan-1-sulfonsäure-5-(2,4-diaminopyrimidin-5-ylmethyl)-3-ethoxy-2-iod-phenyl-ester werden nach zweimaliger Chromatographie 46 mg der Titelverbindung als bräunlicher Feststoff erhalten.
MS (ISP): 516,3 $(M+H)^+$

(37c) 2,2-Dimethyl-propan-1-sulfonsäure-4'-amino-4-(2,4-diamino-pyrimidin-5-ylmethyl)-6-ethoxy-biphenyl-2-yl-ester

Ausgehend von 272 mg (1,01 mmol) N-(4-Brom-phenyl)-2,2,2-trifluor-acetamid und 265 mg (0,51 mmol) 2,2-Dimethyl-propan-1-sulfonsäure-5-(2,4-diamino-pyrimidin-5-ylmethyl)-3-ethoxy-2-iod-phenyl-ester werden nach zweimaliger Chromatographie 56 mg der Titelverbindung als gelblicher Schaum isoliert.

MS (ISP): 486,4 $(M+H)^+$

(37d) 2,2-Dimethyl-propan-1-sulfonsäure-4-(2,4-diamino-pyrimidin-5-ylmethyl)-6-ethoxy-4'-(2-hxdroxy-ethylamino)-biphenyl-2-yl-ester

Bis-(pinacolato)diboron (336 mg, 1,32 mmol), N-(4-Brom-phenyl)-N-[2-(tertbutyl-dimethyl-silanyloxy)-ethyl]-2,2,2-trifluor-acetamid (451 mg, 1,06 mmol), Kalium-acetat (311 mg, 3,17 mmol) und $PdCl_2$(dppf) (39 mg, 0,05 mmol) werden in 7 ml abs. Dimethylformamid gelöst, zweimal evakuiert, mit Argon begast und anschliessend über Nacht bei 80°C gehalten. Man versetzt mit 275 mg (0,53 mmol) 2,2-Dimethyl-propan-1-sulfonsäure-5-(2,4-diamino-pyrimidin-5-ylmethyl)-3-ethoxy-2-iod-phenyl-ester, 61 mg Tetrakis-triphenylphosphin-palladium und 1,75 ml 2M $K_3PO_4$, entgast nochmals und lässt über Nacht bei 80°C reagieren. Man kühlt ab, giesst auf Eis, extrahiert mit Ethylacetat, wäscht mit gesättiger wässriger Kochsalzlösung, trocknet über $MgSO_4$ und dampft zur Trockne ein. Zweimalige Flash-Chromatographie auf SiO2 ($CH_2Cl_2$/MeOH/25% $NH_3$(19/1/0,05) liefert 152 mg Produkt, das wie folgt zum freien Alkohol gespalten wird:

Es wird in 1 ml Tetrahydrofuran gelöst und mit 1 ml 4,5 M HCl (MeOH) versetzt. Nach 45 Min. wird mit 10% wässriger Natriumcarbonatlösung neutralisiert, mit Ethylacetat extrahiert, mit konz. wässriger Kochsalzlösung gewaschen, über $MgSO_4$ getrocknet und eingedampft. Flash-Chromatographie auf SiO2 ($CH_2Cl_2$/MeOH/25% $NH_3$ (19/1/0,05) liefert, nach Verrühren mit Diethylether, 37 mg der Titelverbindung als nahezu weisse Kristalle.
MS (ISP): 530,2 $(M+H)^+$;

Die obige Ausgangsverbindung Dimethyl-propan-1-sulfonsäure-5-(2,4-diaminopyrimidin-5-ylmethyl)-3-ethoxy-2-iod-phenyl-ester wird wie folgt hergestellt:

5-(2,4-Diamino-pyrimidin-5-ylmethyl)-3-ethoxy-2-iod-phenol (1000 mg; 2,59 mmol) werden in 50 ml abs. Tetrahydrofuran vorgelegt, mit 4,0 g pulverisiertem Molekularsieb 4A und 407 mg (3,63 mmol) KOtBu versetzt und während 45 Min. gerührt. Man kühlt anschliessend auf -20°C und gibt 618 mg (3,62 mmol) 2,2-Dimethyl-propan-1-sulfonylchlorid zu. Nach 60 Min. bei -20°C, 4 Stunden bei 0°C und 12 Stunden bei +8°C verdünnt man das Reaktionsgemisch mit einer Mischung aus $CH_2Cl_2$/MeOH/25% $NH_3$(90/10/1), filtriert, dampft ein und reinigt das Rohprodukt mittels Flash-Chromatographie auf SiO2 ($CH_2Cl_2$/MeOH/25% $NH_3$(19/1/0,05). Dabei werden 552 mg der Titelverbindung als farbloses Pulver erhalten.
MS (ISP): 521,1 $(M+H)^+$

Beispiel 38:

Analog Beispiel 2 wird die Verbindung der Formel VIII, Schema 2, worin $R^2$ Amino ist aus der entsprechenden Verbindung der Formel XIII hergestellt.

(38a) Cyclopropylmethan-sulfonsäure-3'-amino-4-(2,4-diamino-pyrimidin-5-ylmethyl)-6-ethoxy-biphenyl-2-yl ester

Ausgehend von Cyclopropylmethan-sulfonsäure-5-(2,4-diamino-pyrimidin-5-ylmethyl)-3-ethoxy-2-iod-phenyl-ester (250 mg; 0,5 mmol), 3-Aminophenylboronsäure (108 mg; 0,7 mmol). Tetrakis-triphenylphosphin-palladium (34 mg; 0,03 mmol) und wässriger 2M Natriumcarbonatlösung (3,7 ml; 7,4 mmol) in einem 4/1 Dimethoxyethan/Ethanol-Gemisch (10 ml) werden 205 mg (88 %) Cyclopropylmethan-sulfonsäure-3'-amino-4-(2,4-diamino-pyrimidin-5-ylmethyl)-6-ethoxy-biphenyl-2-yl-ester als gelber Schaum erhalten.
MS (ISP): 470.2 $(M+H)^+$

In Analogie zu Beispiel 4 werden hergestellt:

(38b) Cyclopropylmethan-sulfonsäure-4'-amino-4-(2,4-diamino-pyrimidin-5-ylmethyl)-6-ethoxy-3'-fluor-4-ylmethyl-biphenyl-2-yl-ester

N-(4-Brom-3-fluor-phenyl)-2,2,2-trifluoracetamid (1,0 g; 3,5 mmol), Bis-(pinacolato)diboron (1332 mg; 5,2 mmol), Kaliumacetat (1030 mg; 10,5 mmol) und (Diphenylphosphino)-dichlor-palladium (II) (147 mg; 0,21 mmol) in 60 ml Dioxan werden bei 80°C 3 Stunden gerührt. Die Hälfte dieses Ansatzes wird eingeengt, mit Wasser versetzt, mit Ethylacetat extrahiert, die vereinigten organischen Phasen mit einer gesättigten wässrigen Kochsalzlösung gewaschen, über Magnesiumsulfat getrocknet, abgenutscht und eingeengt.

Das so erhaltene Produkt wird mit Cyclopropylmethan-sulfonsäure-5-(2,4-diaminopyrimidin-5-ylmethyl)-3-ethoxy-2-iod-phenyl-ester (441 mg; 0,87 mmol), Tetra-(triphenyl-phosphin)-palladium (121 mg; 0.10 mmol), wässriger 2M Natriumcarbonatlösung (13 ml; 26 mmol) in einem 4/1 Dimethoxyethan/Ethanol-Gemisch (15 ml) behandelt. Man erhält 249 mg (58 %) Cyclopropylmethan-sulfonsäure-4'-amino-4-(2,4-diamino-pyrimidin-5-ylmethyl)-6-ethoxy-3'-fluor-4-ylmethyl-biphenyl-2-yl-ester als violetten Schaum.

MS (ISP): 488.3 $(M+H)^+$

(38c) Cyclopropylmethan-sulfonsäure-4'-amino-4-(2,4-diamino-pyrimidin-5-ylmethyl)-6-ethoxy-3'-methyl-biphenyl-2-yl-ester

N-(4-Brom-2-methyl)-2,2,2-trifluor-acetamid (600 mg; 2.13 mmol), Bis(pinacolato)diboron (810 mg; 3,20 mmol), Kaliumacetat (626 mg; 638 mmol) und (Diphenylphosphino)-dichlor-palladium (II) (89 mg; 0,13 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Cyclopropylmethan-sulfonsäure-5-(2,4-diamino-pyrimidin-5-ylmethyl)-3-ethoxy-2-iod-phenyl-ester (469 mg; 0,93 mmol), Tetrakis-triphenylphosphin-palladium (129 mg; 0.11 mmol) und wässriger 2M Natriumcarbonatlösung (14 ml; 28 mmol) in einem 4/1 Dimethoxyethan/Ethanol-Gemisch (15 ml) werden 315 mg (70 %) Cyclopropylmethan-sulfonsäure-4'-amino-4-(2,4-diamino-pyrimidin-5-ylmethyl)-6-ethoxy-3'-methyl-biphenyl-2-yl-ester als brauner Schaum erhalten.
MS (ISP): 484.3 $(M+H)^+$

(38d) Cyclopropylmethan-sulfonsäure-3'-amino-4-(2,4-diamino-pyrimidin-5-ylmethyl)-6-ethoxy-4'-morpholinmethyl-biphenyl-2-yl-ester

N-(5-Brom-2-morpholinmethyl-4-ylmethyl-phenyl)-2,2,2-trifluor-acetamid (500 mg; 1,36 mmol), Bis(pinacolato)diboron (519 mg; 2,04 mmol), Kaliumacetat (401 mg; 4,09 mmol) und (Diphenylphosphino)-dichlor-palladium (II) (88 mg; 0,08 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Cyclopropylmethansulfonsäure-5-(2,4-diamino-pyrimidin-5-ylmethyl)-3-ethoxy-2-iod-phenyl-ester (308 mg; 0,61 mmol), Tetra-(triphenyl-phosphin)-palladium (85 mg; 0.07 mmol), wässriger 2M Natriumcarbonatlösung (9 ml; 18 mmol) in einem 4/1 Dimethoxyethan/Ethanol-Gemisch (15 ml) werden 254 mg (73 %) Cyclopropylmethan-sulfonsäure-3'-amino-4-(2,4-diaminopyrimidin-5-ylmethyl)-6-ethoxy-4'-morpholinmethyl-biphenyl-2-yl-ester als weisser Schaum erhalten.
MS (ISP): 569.3 $(M+H)^+$

Die obige Ausgangsverbindung Cyclopropylmethan-sulfonsäure-5-(2,4-diaminopyrimidin-5-ylmethyl)-3-ethoxy-2-iod-phenyl-ester wird wie folgt hergestellt, vgl. Schema 2, (XII) -> (XIII)

5-(3-Ethoxy-5-hydroxy-4-iod-benzyl)-pyrimidin-2,4-diamin (siehe Beispiel 32 Stufe a) (250 mg; 0,67 mmol) wird in Tetrahydrofuran (6 ml) gelöst und mit Kalium-tert-butylat (109 mg; 0,97 mmol) versetzt. Bei 0°C wird Cyclopropylmethan-sulfonyl chlorid (200 mg; 1,29 mmol) hinzugegeben. Anschliessend wird eine Stunde bei Raumtemperatur gerührt. Das Reaktionsgemisch wird eingedampft und der Rückstand über Kieselgel mit Methylenchlorid /Methanol (19/1) und 0.5% Ammoniak chromatographiert. Die reinen Fraktionen werden vereinigt und das Lösungsmittel abgedampft.
Ausbeute: 185 mg (57 %) Cyclopropylmethan-sulfonsäure-5-(2,4-diamino-pyrimidin-5-ylmethyl)-3-ethoxy-2-iod-phenyl-ester als weisser Feststoff.
MS (ISP): 504 $(M+H)^+$

Beispiel 39:

In Analogie zu Beispiel 2a wird die Verbindung der Formel VIII, Schema 2, worin $R^2$ Amino ist aus der entsprechenden Verbindung der Formel XIII hergestellt.

(39a) (2-Methoxy-2-methyl-propan)-1-sulfonsäure-3'-amino-4-(2,4-diamino-pyrimidin-5-ylmethyl)-6-ethoxy-biphenyl-2-yl-ester

Ausgehend von (2-Methoxy-2-methyl-propan)-1-sulfonsäure-5-(2,4-diaminopyrimidin-5-ylmethyl)-3-ethoxy-2-iod-phenyl-ester (250 mg; 0,5 mmol), 3-Aminophenylboronsäure (108 mg; 0,7 mmol), Tetrakis-triphenylphosphin-palladium (34 mg; 0,03 mmol) und wässriger 2M Natriumcarbonatlösung (3.5 ml; 7.0 mmol) in einem 4/1 Dimethoxyethan/Ethanol-Gemisch (15 ml) werden 108 mg (46 %) (2-Methoxy-2-methylpropan)-1-sulfonsäure-3'-amino-4-(2,4-diamino-pyrimidin-5-ylmethyl)-6-ethoxybiphenyl-2-yl-ester als gelber Schaum erhalten.
MS (ISP): 502,3 $(M+H)^+$

In Analogie zu Beispiel 4 werden hergestellt:

(39b) (2-Methoxy-2-methyl-propan)-1-sulfonsäure-4'-amino-4-(2,4-diamino-pyrimidin-5-ylmethyl)-6-ethoxy-3'-methyl-biphenyl-2-yl-ester

Ausgehend von N-(4-Brom-3-methyl-phenyl)-2,2,2-trifluoracetamid (1,05 g; 3,7 mmol), Bis(pinacolato)diboron (1,42 g; 5,58 mmol), Kaliumacetat (1,09 g; 11,2 mmol) und (Diphenylphosphino)-dichlor-palladium (II) (157 mg; 0,22 mmol) in in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit (2-Methoxy-2-methyl-propan)-1-sulfonsäure-5-(2,4-diamino-pyrimidin-5-ylmethyl)-3-ethoxy-2-iod-phenyl-ester (499 mg; 0,93 mmol), Tetrakis-triphenylphosphin-palladium (64 mg; 0,06 mmol) und wässriger 2M Natriumcarbonatlösung (6,9 ml; 13,8 mmol) in einem 4/1 Dimethoxyethan/Ethanol-Gemisch (15 ml) behandelt. Man erhält 243 mg (51 %) (2-Methoxy-2-methyl-propan)-1-sulfonsäure-4'-amino-4-(2,4-diamino-pyrimidin-5-ylmethyl)-6-ethoxy-3'-methylbiphenyl-2-yl-ester als braunen Schaum.
MS (ISP): 516,3 $(M+H)^+$

(39c) (2-Methoxy-2-methyl-propan)-1-sulfonsäure-4'-amino-4-(2,4-diamino-pyrimidin-5-ylmethyl)-6-ethoxy-3'-fluor-4-ylmethyl-biphenyl-2-yl-ester

N-(4-Brom-3-fluor-phenyl)-2,2,2-trifluoracetamid (1.0 g; 3.5 mmol), Bis(pinacolato)diboron (1332 mg; 5.2 mmol), Kaliumacetat (1030 mg; 10.5 mmol) und (Diphenylphosphino)-dichlor-palladium (II) (147 mg; 0,21 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit (2-Methoxy-2-methyl-propan)-1-sulfonsäure-5-(2,4-diamino-pyrimidin-5-ylmethyl)-3-ethoxy-2-iod-phenyl-ester (320 mg; 0,60 mmol), Tetrakis-triphenylphosphin-palladium (42 mg; 0,04 mmol) und wässriger 2M Natriumcarbonatlösung (4,5 ml; 9 mmol) in einem 4/1 Dimethoxyethan/Ethanol-Gemisch (15 ml) behandelt. Man erhält 167 mg (54 %) (2-Methoxy-2-methyl-propan)-1-sulfonsäure-4'-amino-4-(2,4-diamino-pyrimidin-5-ylmethyl)-6-ethoxy-3'-fluor-4-ylmethyl-biphenyl-2-yl-ester als weissen Schaum.
MS (ISP): 520,2 $(M+H)^+$

(39d) (2-Methoxy-2-methyl-propan)-1-sulfonsäure-3'-amino-4-(2,4-diamino-pyrimidin-5-ylmethyl)-6-ethoxy-4'-morpholinmethyl-biphenyl-2-yl-ester

N-(5-Brom-2-morpholinmethyl-4-ylmethyl-phenyl)-2,2,2-trifluor-acetamid (894 mg; 2,43 mmol), Bis (Pinacolato) diboron (927 mg; 3,65 mmol), Kaliumacetat (717 mg; 7,3 mmol) und (Diphenylphosphino)-dichlor-palladium (II) (103 mg; 0,146 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit (2-Methoxy-2-methyl-propan)-1-sulfonsäure-5-(2,4-diamino-pyrimidin-5-ylmethyl)-3-ethoxy-2-iod-phenyl-ester (326 mg; 0,61 mmol), Tetrakis-triphenylphosphin-palladium (42 mg; 0,04 mmol) und wässriger 2M Natriumcarbonatlösung (4,5 ml; 9 mmol) in einem 4/1 Dimethoxyethan/Ethanol-Gemisch (15 ml) behandelt. Man erhält 158 mg (43 %) 2-Methoxy-2-methyl-propan)-1-sulfonsäure-3'-amino-4-(2,4-diamino-pyrimidin-5-ylmethyl)-6-ethoxy-4'morpholinmethyl-biphenyl-2-yl-ester als weissen Feststoff.
MS (ISP): 601,1 $(M+H)^+$

Die obige Ausgangsverbindung (2-Methoxy-2-methyl-propan)-1-sulfonsäure-5-(2,4-diamino-pyrimidin-5-ylmethyl)-3-ethoxy-2-iod-phenyl-ester wird wie folgt hergestellt, vgl. Schema 2, (XII) -> (XIII)

5-(3-Ethoxy-5-hydroxy-4-iod-benzyl)-pyrimidin-2,4-diamin (siehe Beispiel 32 Stufe a) (2170 mg; 5,63 mmol) wird in Tetrahydrofuran (60 ml) gelöst und bei Raumtemperatur mit Kalium-tert-butylat (947 mg; 8,44 mmol) versetzt. Anschliessend wird auf 0°C gekühlt und 2-Methoxy-2-methyl-propan-1-sulfonylchlorid (2,1 g; 11,25 mmol) hinzugefügt. Nach einer Stunde bei Raumtemperatur wird das Reaktionsgemisch eingedampft und der Rückstand über Kieselgel mit Methylenchlorid /Methanol (19/1) und 0.5% Ammoniak chromatographiert . Die reinen Fraktionen werden vereinigt und das Lösungsmittel abgedampft.
Ausbeute: 1710 mg (57 %) (2-Methoxy-2-methyl-propan)-1-sulfonsäure-5-(2,4-diaminopyrimidin-5-ylmethyl)-3-ethoxy-2-iod-phenyl-ester als beiger Feststoff.
MS (ISP): 537,1 $(M+H)^+$

Das eingesetzte(2-Methoxy-2-methyl-propan)-1-sulfonylchlorid wird wie folgt hergestellt (Stufen a-b):

Stufe a) 2, 2'-Dithiobis-(1,1-dimethylethyl methyl ether)

1,1-Dimethyl-thiiran (1,00 g; 10,2 mmol) wird in Methanol (50 ml) gelöst, mit Dichlor-dicyan-benzochinon (2,32 g; 10,2 mmol) versetzt und 6 Stunden am Rückfluss erhitzt. Das Reaktionsgemisch wird eingedampft und der Rückstand über Kieselgel mit n-Hexan/Ethylacetat (8/2) chromatographiert. Die reinen Fraktionen werden vereinigt und das Lösungsmittel abgedampft.
Ausbeute: 870 mg (71%) 2, 2'-Dithiobis-(1,1-dimethylethyl-methylether) als gelbes Oel.
MS (EI): 238 (M)

Stufe b) (2-Methoxy-2-methyl-propan)-1-sulfonylchlorid

2, 2'-Dithiobis-(1,1-dimethylethyl methyl ether) (2.00 g; 8.4 mmol) wird in Aether (250 ml) gelöst, mit Iodosobenzol (10.8 g; 33.6 mmol) und mit Salzsäure (14 ml; 37%ig) bei Raumtemperatur versetzt. Nach einer Stunde wird das Reaktionsgemisch auf eine gesättigte Natriumcarbonatlösung gegossen und mit Ether zweimal extrahiert. Die organische Phase wird über Natriumsulfat getrocknet, die Salze werden abfiltriert und das Lösungsmittel abgedampft. Der Rückstand wird über Kieselgel mit n-Hexan/Ethylacetat (8/2) chromatographiert. Die reinen Fraktionen werden vereinigt und das Lösungsmittel abgedampft.
Ausbeute: 2.44 g (78 %) (2-Methoxy-2-methyl-propan)-1-sulfonylchlorid als leicht gelbe Flüssigkeit.
MS (EI): 171 ($M-CH_3$)

Beispiel 40:

In Analogie zu Beispiel 2 werden die Verbindung der Formel VIII, Schema 2, aus den entsprechenden Verbindungen der Formel XIII hergestellt.

(40a) Cyclobutan-sulfonsäure-3'-amino-4-(2,4-diamino-pyrimidin-5-ylmethyl)-6-ethoxybiphenyl-2-yl-ester

Ausgehend von Cyclobutan-sulfonsäure-5-(2,4-diamino-pyrimidin-5-ylmethyl)-3-ethoxy-2-iod-phenyl-ester (250 mg; 0,5 mmol), 3-Aminophenylboronsäure (115 mg; 0,7 mmol) Tetrakis-triphenylphosphin-palladium (34 mg; 0,03 mmol) und wässriger 2M Natriumcarbonatlösung (3,7 ml; 7,4 mmol) in einem 4/1 Dimethoxyethan/Ethanol-Gemisch (10 ml) werden 157 mg (67 %) Cyclobutan-sulfonsäure-3'-amino-4-(2,4-diamino-pyrimidin-5-ylmethyl)-6-ethoxy-biphenyl-2-yl-ester als gelber Schaum erhalten.
MS (ISP): 470,3 $(M+H)^+$

(40b) Cyclobutan-sulfonsäure-4'-amino-4-(2,4-diamino-pyrimidin-5-ylmethyl)-6-ethoxy-3'-fluor-4-ylmethyl-biphenyl-2-yl-ester

N-(4-Brom-3-fluor-phenyl)-2,2,2-trifluoracetamid (900 mg; 3.15 mmol), Bis-(pinacolato)diboron (1198 mg; 4.7 mmol), Kaliumacetat (926 mg; 9.44 mmol) und(Diphenylphosphino)-dichloro-palladium (II) (132 mg; 0,19 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Da so erhaltene Produkt wird mit Cyclobutan-sulfonsäure-5-(2,4-diaminopyrimidin-5-ylmethyl)-3-ethoxy-2-iod-phenyl-ester (397 mg; 0,79 mmol), Tetrakis-triphenylphosphin-palladium (109 mg; 0,09 mmol) und wässriger 2M Natriumcarbonatlösung (12 ml; 24 mmol) in einem 4/1 Dimethoxyethan/Ethanol-Gemisch (15 ml) behandelt. Man erhält 314 mg (81,8 %) Cyclobutan-sulfonsäure-4'-amino-4-(2,4-diamino-pyrimidin-5-ylmethyl)-6-ethoxy-3'-fluor-4-ylmethyl-biphenyl-2-yl-ester als leicht braunen Schaum.
MS (ISP): 488,3 $(M+H)^+$

(40c) Cyclobutan-sulfonsäure-4'-amino-4-(2,4-diamino-pyrimidin-5-ylmethyl)-6-ethoxy-3'-methyl-biphenyl-2-yl-ester

N-(4-Brom-2-methyl)-2,2,2-trifluor-acetamid (600 mg; 2.13 mmol), Bis(pinacolato) diboron (810 mg; 3.20 mmol), Kaliumacetat (626 mg; 6.38 mmol) und (Diphenylphosphino)-dichlor-palladium (II) (89 mg; 0,13 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Cyclobutan-sulfonsäure-5-(2,4-diamino-pyrimidin-5-ylmethyl)-3-ethoxy-2-iod-phenyl-ester (358 mg; 0,71 mmol),Tetrakis-triphenylphosphinpalladium (98 mg; 0,09 mmol) und wässriger 2M Natriumcarbonatlösung (10,6 ml; 21,2 mmol) in einem 4/1 Dimethoxyethan/Ethanol-Gemisch (15 ml) werden 153 mg (44,6 %) Cyclobutan-sulfonsäure-4'-amino-4-(2,4-diamino-pyrimidin-5-ylmethyl)-6-ethoxy-3'methyl-biphenyl-2-yl-ester als brauner Schaum erhalten.
MS (ISP): 484,4 $(M+H)^+$
NMR $^1H$: (250 MHz, $\delta$, TMS, DMSO): 1.17 (t; J=6.6; 3H); 2.05 (s; 3H); 1.65-2.16 (m; 6H); 3.62 (s; 2H); 3.65 (m, 1H); 3.94 (q; J=6.6; 2H); 4.90 (s(br); 2H); 5.72 (s(br); 2H); 6.15 (s(br); 2H); 6.61 (d; J=7.6; 1H); 6.79 (s; 1H); 6.81 (d; J=7.6; 1H); 6.83 (s; 1H); 1H); 6.91 (s; 1H); 7.57 (s; 1H).

(40d) Cyclobutan-sulfonsäure-3'-amino-4-(2,4-diamino-pyrimidin-5-ylmethyl)-6-ethoxy-4'-morpholinomethyl-biphenyl-2-yl-ester

N-(5-Brom-2-morpholinmethyl-phenyl)-2,2,2-trifluor-acetamid (450 mg; 1,23 mmol), Bis(pinacolato)diboron (466 mg; 1,84 mmol), Kaliumacetat (361 mg; 3,68 mmol) und (Diphenylphosphino)-dichlor-palladium (II) (52 mg; 0,07 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Cyclobutan-sulfonsäure-5-(2,4-diamino-pyrimidin-5-ylmethyl)-3-ethoxy-2-iod-phenyl-ester (310 mg; 0,61 mmol), Tetrakis-triphenylphosphin-palladium (43 mg; 0,04 mmol) und wässriger 2M Natriumcarbonatlösung (4,5 ml; 9 mmol) in einem 4/1 Dimethoxyethan/Ethanol-Gemisch (15 ml) werden 259 mg (74 %) Cyclobutan-sulfonsäure-3'-amino-4-(2,4-diaminopyrimidin-5-ylmethyl)-6-ethoxy-4'-morpholinomethyl-biphenyl-2-yl-ester als weisser Schaum erhalten.
MS (ISP): 569,3 $(M+H)^+$

(40e) Cyclobutan-sulfonsäure-3'-amino-4-(2,4-diamino-pyrimidin-5-ylmethyl)-6-ethoxy-5'-fluor-biphenyl-2-yl-ester

N-(5-Iod-3-fluor-phenyl)-2,2,2-trifluoracetamid (hergestellt wie in Beispiel 4t)(326 mg; 0,98 mmol), Bis(pinacolato)diboron (373 mg; 1,47 mmol), Kaliumacetat (288 mg; 2,94 mmol), und (Diphenylphosphino)-dichlor-palladium (II) ($PdCl_2(PPh_3)_2$) (41 mg; 0,06 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Cyclobutan-sulfonsäure-5-(2,4-diamino-pyrimidin-5-ylmethyl)-3-ethoxy-2-iod-phenylester (247 mg; 0,49 mmol) ,Tetrakis-triphenylphosphin-palladium (34 mg; 0,03 mmol) und wässriger 2M Natriumcarbonatlösung (3,65 ml; 7,3 mmol) in einem 4/1 Dimethoxyethan/Ethanol-Gemisch (15 ml) werden 154 mg (65 %) Cyclobutansulfonsäuce-3'-amino-4-(2,4-diamino-pyrimidin-5-ylmethyl)-6-ethoxy-5'-fluor-biphenyl-2-yl-ester als weisser Schaum erhalten.
MS (ISP): 488,3 $(M+H)^+$
Die obige Ausgangsverbindung Cyclobutan-sulfonsäure-5-(2,4-diamino-pyrimidin-5-ylmethyl)-3-ethoxy-2-iod-phenylester wird wie folgt hergestellt, vgl. Schema 2, (XII) -> (XIII)
5-(3-Ethoxy-5-hydroxy-4-iod-benzyl)-pyrimidin-2,4-diamin (siehe Beispiel 32 Stufe a) (2800 mg; 7.25 mmol) wird in Tetrahydrofuran (50 ml) gelöst, mit Kalium-tert-butylat (1220 mg; 10,8 mmol) versetzt, und auf -20°C gekühlt. Danach wird Cyclobutan-sulfonylchlorid (2,24 g; 14,5 mmol) zugegeben und anschliessend eine Stunde bei -20°C gerührt. Das Reaktionsgemisch wird auf Eiswasser gegossen und mit Ethylacetat zweimal extrahiert. Die organische Phase wird über Natriumsulfat getrocknet, die Salze werden abfiltriert und das Lösungsmittel abgedampft. Der Rückstand wird über Kieselgel mit Methylenchlorid /Methanol (19/1) und 0,5% Ammoniak chromatographiert . Die reinen Fraktionen werden vereinigt und das Lösungsmittel abgedampft.
Ausbeute: 2180 mg (59,6 %) Cyclobutan-sulfonsäure-5-(2,4-diamino-pyrimidin-5-ylmethyl)-3-ethoxy-2-iod-phenylester als gelbe Kristalle.
MS (ISP): 505,1 $(M+H)^+$
Die eingesetzte Cyclobutan-sulfonylchlorid wird wie folgt hergestellt (Stufen a-b-c):

Stufe a) Cyclobutan-sulfonsäure-n-butylester

4-Chlor-n-butan-1-sulfonsäure-n-butylester (68,10 g; 298 mmol) wird in Tetrahydrofuran (1,5 L) gelöst und bei -70°C mit n-Butyllithium (205 ml; 1,6 M in Tetrahydrofuran; 327 mmol) innerhalb 1,5 Stunden versetzt. Nach halbstündigem Rühren bei Raumtemperatur wird Wasser (15 ml) hinzugefügt und das Reaktionsgemisch eingeengt. Der Rückstand wird mit einer gesättigten wässrigen Kochsalzlösung verdünnt und mit Ethylacetat zweimal extrahiert. Die organische Phase wird über Natriumsulfat getrocknet, die Salze werden abfiltriert und das Lösungsmittel abgedampft. Der Rückstand wird über Kieselgel mit n-Hexan/Ethylacetat (8/2) chromatographiert. Die reinen Fraktionen werden vereinigt und das Lösungsmittel abgedampft.
Ausbeute: 14,81 g (25,9 %) Cyclobutan-sulfonsäure-n-butylester als gelbes Oel.

MS (ISP): 136 $(M-C_4H_8)^+$
NMR (250 MHz; J in Hz; $CDCl_3$)in ppm 4,21(t; J=6,5; 2H); 3,90(quint.; J=8,0; 1H); 2,54 (m; 2H); 2,34 (m; 2H); 2,02 (m; 2H); 1,68 (m; 2H); 1,4 (sext.; J=7,2; 2H); 0,95 (t; J=7,2; 3H).

Stufe b) Kalium-cyclobutan-sulfonat

Cyclobutan-sulfonsäure-n-butylester (14,81 g; 77 mmol) wird in einem 1/1 Dimethoxyethan/Wasser Gemisch (220 ml) gelöst, mit Kaliumthiocyanat (7,78 g; 80 mmol) versetzt und am Rückfluss 20 Stunden gekocht. Das Reaktionsgemisch wird auf die Hälfte eingeengt. Die wässrige Phase wird abgetrennt, zweimal mit Aether gewaschen und eingedampft. Der Rückstand wird mit Ethanol (200 ml) bei -10°C verrührt und die weissen Kristalle abgenutscht.
Ausbeute: 10,77 g (80,2 %) Kalium-cyclobutan-sulfonat als weisse Kristalle.
MS (ISN): 135,2 $(M-K)^-$

Stufe c) Cyclobutan-sulfonylchlorid

Kalium-cyclobutan-sulfonat (10,76 g; 62 mmol) wird in Methylenchlorid (50 ml) suspendiert, mit Dimethylformamid (0,5 ml) als Katalysator und mit Thionylchlorid (49,3 ml; 68 mmol) versetzt und 20 Stunden am Rückfluss gekocht. Das Reaktionsgemisch wird eingeengt, mit Wasser versetzt und mit Ether extrahiert. Die organische Phase wird mit einer gesättigten wässrigen Kochsalzlösung gewaschen, über Natriumsulfat getrocknet, abgenutscht und eingeengt.
Ausbeute: 8,44 g (88 %) Cyclobutan-sulfonylchlorid als leicht braune Flüssigkeit.
GC-MS (ISP): 55 $(M-SO_2Cl)^+$
NMR (250 MHz; J in Hz; $CDCl_3$)in ppm 4,43 (Quint.; J=7,7; 1H); 2,69 (m; 2H); 2,50 (m; 2H); 2,09 (m; 2H).

Beispiel 41:

Analog Beispiel 4 werden wie in Schema 2 aufgezeichnet Verbindungen der Formel XIII aus Verbindungen der Formel VIII hergestellt.
Schema 2, (XIII) -> (VIII)

(41a) Tetrahydropyran-4-sulfonsäure-4'-amino-4-(2,4-diamino-pyrimidin-5-ylmethyl)-6-ethoxy-3'-methyl-biphenyl-2-yl-ester

N-(4-Brom-2-methyl)-2,2,2-trifluor-acetamid (800 mg; 2.84 mmol), Bis(pinacolato) diboron (1.08 g; 4.25 mmol), Kaliumacetat (835 mg; 8.51 mmol) und (Diphenylphosphino)-dichlor-palladium (II) (119 mg; 0,17 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit Tetrahydropyran-4-sulfonsäure-5-(2,4-diaminopyrimidin-5-ylmethyl)-3-ethoxy-2-iod-phenyl-ester (379 mg; 0,71 mmol), Tetrakis-triphenylphosphin-palladium (49 mg; 0,04 mmol) und wässriger 2M Natriumcarbonatlösung (5,3 ml; 10,6 mmol) in einem 4/1 Dimethoxyethan/Ethanol-Gemisch (15 ml) behandelt. Man erhält 261 mg (72 %) Tetrahydropyran-4-sulfonsäure-4'-amino-4-(2,4-diamino-pyrimidin-5-ylmethyl)-6-ethoxy-3'-methyl-biphenyl-2-yl-ester als braunen Schaum erhalten:
MS (ISP): 514,3 $(M+H)^+$

(41b) Tetrahydropyran-4-sulfonsäure-4'-amino-4-(2,4-diamino-pyrimidin-5-ylmethyl)-6-ethoxy-3'-fluor-biphenyl-2-yl-ester

N-(4-Brom-3-fluor-phenyl)-2,2,2-trifluoracetamid (900 mg; 3,15 mmol), Bis-(pinacolato)diboron (1198 mg; 4,7 mmol), Kaliumacetat (926 mg; 9,44 mmol) und(Diphenylphosphino)-dichlor-palladium (II) (132 mg; 0,19 mmol) in 60 ml Dioxan werden bei 80°C 3 Stunden gerührt. Die Hälfte dieses Ansatzes wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt.

Das so erhaltene Produkt wird mit Tetrahydropyran-4-sulfonsäure-5-(2,4-diaminopyrimidin-5-ylmethyl)-3-ethoxy-2-iod-phenyl-ester (421 mg; 0,79 mmol), Tetrakis-triphenylphosphin-palladium (55 mg; 0,05 mmol) und wässriger 2M Natriumcarbonatlösung (6 ml; 12 mmol) in einem 4/1 Dimethoxyethan/Ethanol-Gemisch (15 ml) behandelt. Man erhält 245 mg (60 %) Tetrahydropyran-4-sulfonsäure-4'-amino-4-(2,4-diamino-pyrimidin-5-ylmethyl)-6-ethoxy-3'-fluor-biphenyl-2-yl-ester als braunen Schaum erhalten.

MS (ISP): 518,3 $(M+H)^+$

In Analogie zu Beispiel 2a wird hergestellt:

(41c) Tetrahydropyran-4-sulfonsäure-3'-amino-4-(2,4-diamino-pyrimidin-5-ylmethyl)-6-ethoxy-biphenyl-2-yl-ester

Ausgehend von Tetrahydropyran-4-sulfonsäure-5-(2,4-diamino-pyrimidin-5-ylmethyl)-3-ethoxy-2-iod-phenylester (250 mg; 0,5 mmol), 3-Aminophenylboronsäure (109 mg; 0,7 mmol) Tetrakis-triphenylphosphin-palladium (34 mg; 0,03 mmol) und wässriger 2M Natriumcarbonatlösung (3,5 ml; 7,0 mmol) in einem 4/1 Dimethoxyethan/Ethanol-Gemisch (10 ml) werden 167 mg (71 %) Tetrahydropyran-4-sulfonsäure-3'-arnino-4-(2,4-diamino-pyrimidin-5-ylmethyl)-6-ethoxy-biphenyl-2-yl-ester als weisser Schaum erhalten.
MS (ISP): 500,3 $(M+H)^+$

Die obige Ausgangsverbindung Tetrahydropyran-4-sulfonsäure-5-(2,4-diaminopyrimidin-5-ylmethyl)-3-ethoxy-2-iod-phenyl-ester wird wie folgt hergestellt, vgl. Schema 2, (XII) -> (XIII)

5-(3-Ethoxy-5-hydroxy-4-iod-benzyl)-pyrimidin-2,4-diamin (siehe Beispiel 32 Stufe a) (2300 mg; 5,96 mmol) wird in Tetrahydrofuran (70 ml) gelöst, mit Kalium-tert-butylat (1000 mg; 8,9 mmol) versetzt und auf -20°C gekühlt. Bei dieser Temperatur wird Tetrahydropyran-4-sulfonylchlorid (2,2 g; 11,9 mmol) zugegeben und anschliessend eine Stunde bei -20°C gerührt. Das Reaktionsgemisch wird auf Eiswasser gegossen und mit Ethylacetat zweimal extrahiert. Die organische Phase wird über Natriumsulfat getrocknet, die Salze werden abfiltriert und das Lösungsmittel abgedampft. Der Rückstand wird über Kieselgel mit Methylenchlorid /Methanol (19/1) und 0,5% Ammoniak chromatographiert. Die reinen Fraktionen werden vereinigt und das Lösungsmittel abgedampft.
Ausbeute: 1370 mg (43 %) Tetrahydropyran-4-sulfonsäure-5-(2,4-diamino-pyrimidin-5-ylmethyl)-3-ethoxy-2-iod-phenyl-ester als weisser Feststoff.
MS (ISP): 535,2 $(M+H)^+$

Das eingesetzte Tetrahydropyran-4-sulfonylchlorid wird wie folgt hergestellt (Stufen a-b):

Stufe a) 4,4'-Dithiobis-tetrahydro-pyran

Natriumsulfid (42,2 g; 35%ig; 188,8 mmol) und Schwefel (3,33 g; 104 mmol) werden in Wasser (120 ml) suspendiert und 1,5 Stunden bei 60°C gerührt. Benzol (250 ml), gefolgt von Tetrabutylammonium bromid (0,61 g; 1,89 mmol) und 4-Brom-tetrahydropyran (7,79 g; 47,2 mmol) werden zugegeben und das Ganze bei 60°C vier Stunden gerührt. Das Reaktionsgemisch wird auf Eiswasser gegossen und mit Aether zweimal extrahiert. Die organische Phase wird über Natriumsulfat getrocknet, die Salze werden abfiltriert und das Lösungsmittel abgedampft.
Ausbeute: 2,41 g (44 %) 4,4'-Dithiobis-tetrahydropyran als gelbes Oel.
MS (ISP): 234 $(M)^+$

Stufe b) Tetrahydropyran-4-sulfonylchlorid

4,4'-Dithiobis-tetrahydropyran (2,41 g; 10,3 mmol) wird in Aether (250 ml) gelöst, mit Iodosobenzol (13,25 g; 41,1 mmol) und mit Salzsäure (117ml; 37%ig) bei Raumtemperatur versetzt und eine Stunde gerührt. Das Reaktionsgemisch wird auf eine gesättigte wässrige Natriumcarbonatlösung gegossen und mit Aether zweimal extrahiert. Die vereinigten organischen Phasen werden über Natriumsulfat getrocknet, die Salze werden abfiltriert und das Lösungsmittel abgedampft. Der Rückstand wird über Kieselgel mit n-Hexan/Ethylacetat (7/3) chromatographiert . Die reinen Fraktionen werden vereinigt und das Lösungsmittel abgedampft.
Ausbeute: 2,47 g (65 %) Tetrahydropyran-4-sulfonylchlorid als leicht gelbe Flüssigkeit.
MS (ISP): 149 $(M-Cl)^+$

Beispiel 42:

Analog Beispiel 2 werden die Verbindungen der Formel VIII, Schema 2, aus den entsprechenden Verbindungen der Formel XIII hergestellt.

(42a) Tetrahydrofuran-2-yl-methansulfonsäure-3'-amino-4-(2,4-diamino-pyrimidin-5-ylmethyl)-6-ethoxy-biphenyl-2-yl-ester

Ausgehend von Tetrahydrofuran-2-yl)-methansulfonsäure-5-(2,4-diaminopyrimidin-5-ylmethyl)-3-ethoxy-2-iod-phenyl-ester (220 mg; 0,5 mmol), 3-Aminophenylboronsäure (98 mg; 0,63 mmol), Tetrakis-triphenylphosphin-palladium (29 mg; 0,03 mmol) und wässriger 2M Natriumcarbonatlösung (3,2 ml; 6,4 mmol) in einem 4/1 Dimethoxyethan/Ethanol-Gemisch (10 ml) werden 107 mg (52 %) Tetrahydrofuran-2-ylmethansulfonsäure-3'-amino-4-(2,4-diamino-pyrimidin-5-ylmethyl)-6-ethoxy-biphenyl-2-yl-ester als gelber Schaum erhalten.
MS (ISP): 500,3 $(M+H)^+$

(42b) Tetrahydrofuran-2-yl-methansulfonsäure-4'-amino-4-(2,4-diamino-pyrimidin-5-ylmethyl)-6-ethoxy-3'-fluor-biphenyl-2-yl-ester

N-(4-Brom-3-fluor-phenyl)-2,2,2-trifluoracetamid (471 mg; 1.65 mmol), Bis-(pinacolato)diboron (628 mg; 2.47 mmol), Kaliumacetat (485 mg; 4.9 mmol) und (Diphenylphosphino)-dichloro-palladium (II) (69 mg; 0,10 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Tetrahydrofuran-2-yl-methansulfonsäure-5-(2,4-diamino-pyrimidin-5-ylmethyl)-3-ethoxy-2-iod-phenyl-ester (220 mg; 0,41 mmol), Tetrakis-triphenylphosphin-palladium (57 mg; 0,05 mmol) und wässriger 2M Natriumcarbonatlösung (4.1 ml; 8,2 mmol) in einem 4/1 Dimethoxyethan/Ethanol-Gemisch (10 ml) werden 170 mg (80 %) Tetrahydrofuran-2-yl-methansulfonsäure-4'-amino-4-(2,4-diamino-pyrimidin-5-ylmethyl)-6-ethoxy-3'-fluor-biphenyl-2-yl-ester als brauner Schaum erhalten.
MS (ISP): 518,3 $(M+H)^+$

(42c) Tetrahydrofuran-2-yl-methansulfonsäure-4'-amine-4-(2,4-diamino-pyrimidin-5-ylmethyl)-6-ethoxy-3'-methyl-biphenyl-2-yl-ester

N-(4-Brom-2-methyl)-2,2,2-trifluor-acetamid (465 mg; 1.65 mmol), Bis-(pinacolato)diboron (627 mg; 2,47 mmol), Kaliumacetat (485 mg; 4,94 mmol) und (Diphenylphosphino)-dichlor-palladium (II) (69 mg; 0,10 mmol) in 60 ml Dioxan werden bei 80°C 3 Stunden gerührt. Das Reaktionsgemisch wird eingeengt, mit Wasser versetzt und mit Ethylacetat extrahiert. Die vereinigten organischen Phasen werden mit einer gesättigten wässrigen Kochsalzlösung gewaschen, über Magnesiumsulfat getrocknet, abgenutscht und eingeengt. Nach Umsetzung mit Tetrahydrofuran-2-yl-methansulfonsäure-5-(2,4-diamino-pyrimidin-5-ylmethyl)-3-ethoxy-2-iod-phenyl-ester (220 mg; 0,71 mmol), Tetrakis-triphenylphosphin-palladium (57 mg; 0,05 mmol) und wässriger 2M Natriumcarbonatlösung (4,1 ml; 8,2 mmol) in 4/1 Dimethoxyethan/Ethanol (20 ml) werden 66 mg (31%) Tetrahydrofuran-2-yl-methansulfonsäure-4'-amino-4-(2,4-diaminopyrimidin-5-ylmethyl)-6-ethoxy-3'-methyl-biphenyl-2-yl-ester als brauner Schaum erhalten.
MS (ISP): 514,3 $(M+H)^+$

Die obige Ausgangsverbindung Tetrahydrofuran-2-yl-methansulfonsäure-5-(2,4-diamino-pyrimidin-5-ylmethyl)-3-ethoxy-2-iod-phenyl-ester wird wie folgt hergestellt, vgl. Schema 2, (XII) -> (XIII)

5-(3-Ethoxy-5-hydroxy-4-iod-benzyl)-pyrimidin-2,4-diamin (siehe Beispiel 32 Stufe **a**) (3,00 g; 7,77 mmol) wird in Tetrahydrofuran (50 ml) gelöst, mit Kalium-tert-butylat (1,31 g; 11,65 mmol) versetzt und auf -20°C gekühlt. Bei dieser Temperatur wird Tetrahydrofuran-2-yl-methansulfonylchlorid (4,3 g; 23,3 mmol) zugegeben und eine Stunde bei -20°C gerührt. Das Reaktionsgemisch wird auf Eiswasser gegossen und mit Ethylacetat zweimal extrahiert. Die vereinigten organischen Phasen werden über Natriumsulfat getrocknet, die Salze werden abfiltriert und das Lösungsmittel abgedampft. Der Rückstand wird über Kieselgel mit Methylenchlorid /Methanol (19/1) und 0,5% Ammoniak chromatographiert . Die reinen Fraktionen werden vereinigt und das Lösungsmittel abgedampft.
Ausbeute: 1690 mg (41 %) Tetrahydrofuran-2-yl-methansulfonsäure-5-(2,4-diaminopyrimidin-5-ylmethyl)-3-ethoxy-2-iod-phenyl-ester als gelber Schaum.
MS (ISP): 535,2 $(M+H)^+$

Das eingesetzte Tetrahydrofuran-2-yl-methansulfonylchlorid wird wie folgt hergestellt (Stufen a,b):

Stufe a) Dithio-bis-(tetrahydrofuran-2-yl)-methan-yl

Natriumsulfid (72,9 g; 35%ig; 327 mmol) und Swefel (5,77 g; 180 mmol) werden in Wasser (130 ml) suspendiert und 1,5 Stunden bei 60°C gerührt. Benzol (300 ml), gefolgt von Tetrabutylammonium-bromid (1,06 g; 3,27 mmol) und Tetrahydrofuran-2-yl-methyl bromid (15 g; 81,8 mmol) werden hinzugefügt und das Gemisch bei 60°C vier Stunden gerührt. Das Reaktionsgemisch wird auf Eiswasser gegossen und mit Aether zweimal extrahiert. Die organische Phase wird auf Natriumsulfat getrocknet, die Salze werden abfiltriert und das Lösungsmittel abgedampft.
Ausbeute: 10,2 g (100 %) Dithio-bis-(tetrahydrofuran-2-yl)-methan-yl als hellbraune Flüssigkeit.
MS (ISP): 234 $(M)^+$

Stufe b) Tetrahydrofuran-2-yl-methansulfonylchlorid

Dithiobis-(tetrahydrofuran-2-yl)-methan-yl (4,07 g; 32,6 mmol) wird in Aether (300 ml) gelöst, mit Iodosobenzol (42 g; 130 mmol) und Salzsäure (55 ml; 37%ig) bei Raumtemperatur versetzt, und das Ganze eine Stunde gerührt.

Das Reaktionsgemisch wird auf eine gesättigte Natriumcarbonatlösung gegossen und mit Aether zweimal extrahiert. Die organischen Phasen werden über Natriumsulfat getrocknet, die Salze werden abfiltriert und das Lösungsmittel abgedampft. Der Rückstand wird über Kieselgel mit n-Hexan/Ethylacetat (7/3) chromatographiert . Die reinen Fraktionen werden vereinigt und das Lösungsmittel abgedampft.
Ausbeute: 6,76 g (56 %) Tetrahydrofuran-2-yl-methansulfonylchlorid als leicht hellbraune Flüssigkeit.
NMR (250 MHz; J in Hz; $CDCl_3$)in ppm: 4,58 (m; 1H); 4,02-3,75 (m; 4H); 2,25 (m; 1H); 1,99 (m; 2H); 1,76 (m; 1H).

Beispiel 43:

Analog Beispiel 2 (Nos. 43a, 43b, 43d) bzw. Beispiel 4 (No. 43c) werden Verbindungen der Formel IX aus den entsprechenden Verbindungen der Formel XIV hergestellt, vgl. Schema 2.

(43a) Dimethylsulfaminsäure-4-(2,4-diamino-pyrimidin-5-ylmethyl)-6-ethoxy-4'-methylsulfanyl-biphenyl-2-yl-ester

Ausgehend von Dimethylsulfaminsäure-5-(2,4-diamino-pyrimidin-5-ylmethyl)-3-ethoxy-2-iod-phenyl-ester (509 mg; 1,0 mmol) und 4-(Methylthio)phenylboronsäure (269 mg; 1,55 mmol) werden 410 mg (71%) Dimethylsulfaminsäure-4-(2,4-diaminopyrimidin-5-ylmethyl)-6-ethoxy-4'-methylsulfanyl-biphenyl-2-yl-ester als farbloses Pulver erhalten.
MS (ISP): 490,3 $(M+H)^+$

(43b) Dimethylsulfaminsäure 4-(2,4-diamino-pyrimidin-5-ylmethyl)-6-ethoxy-biphenyl-2-yl-ester

Ausgehend von Dimethylsulfaminsäure-5-(2,4-diamino-pyrimidin-5-ylmethyl)-3-ethoxy-2-iod-phenyl-ester (280 mg; 0.57 mmol) und Phenylboronsäure (118 mg; 0,96 mMol) werden 206 mg (82%) Dimethylsulfaminsäure 4-(2,4-diamino-pyrimidin-5-ylmethyl)-6-ethoxy-biphenyl-2-yl-ester als farbloses Pulver erhalten.
MS (ISP): 444,4 $(M+H)^+$

(43c) Dimethylsulfaminsäure-4'-amino-3'-cyan-4-(2,4-diamino-pyrimidin-5-ylmethyl)-6-ethoxy-biphenyl-2-yl-ester

Ausgehend von Dimethylsulfaminsäure-5-(2,4-diamino-pyrimidin-5-ylmethyl)-3-ethoxy-2-iod-phenyl-ester (148 mg; 0,3 mmol) und 2-Amino-5-iod-benzonitril (143 mg; 0,59 mmol) werden 12 mg (4 %) Dimethyl-sulfaminsäure-4'-amino-3'-cyan-4-(2,4-diamino-pyrimidin-5-ylmethyl)-6-ethoxy-biphenyl-2-yl-ester als farbloses Pulver erhalten.
MS (ISP): 484,4 $(M+H)^+$

(43d) Dimethylsulfaminsäure 5-(2,4-diamino-pyrimidin-5-ylmethyl)-3-ethoxy-2-(1H-indol-5-yl)-phenyl-ester

Ausgehend von Dimethylsulfaminsäure-5-(2,4-diamino-pyrimidin-5-ylmethyl)-3-ethoxy-2-iod-phenyl-ester ( 470 mg; 0,95 mmol) und 5-(1H-Indolyl)-boronsäure (230 mg; 1,43 mmol) werden 373 mg (81%) Dimethylsulfaminsäure 5-(2,4-diamino-pyrimidin-5-ylmethyl)-3-ethoxy-2-(1H-indol-5-yl)-phenyl-ester als farbloses Pulver erhalten.
MS (ISP): 483,3 $(M+H)^+$
Die obige Ausgangsverbindung Dimethylsulfaminsäure-5-(2,4-diamino-pyrimidin-5-ylmethyl)-3-ethoxy-2-iod-phenyl-ester wird wie folgt hergestellt, vgl. Schema 2, (XII) -> (XIII)
5-(3-Ethoxy-5-hydroxy-4-iod-benzyl)-pyrimidin-2,4-diamin (siehe Beispiel 32) (386 mg; 1,0 mmol) wird in Dimethylformamid (31 ml) gelöst, mit Kalium-tert-butylat (137 mg; 1,2 mmol) bei Raumtemperatur versetzt und eine Stunde gerührt. Danach wird bei 0°C mit N,N-Dimethylsulfamoylchlorid (0,129 ml; 1,2 mmol) versetzt und 3 Stunden bei 0-5°C gerührt. Das Reaktionsgemisch wird eingedampft und der Rückstand über Kieselgel (43 g) mit Methylenchlorid /Methanol/$NH_4OH$ konz. (19/1/0,05) chromatographiert. Die reinen Fraktionen werden vereinigt und das Lösungsmittel abgedampft. Der erhaltene Rückstand wird mit Diethylether verrührt, abgenutscht und am Hochvakuum getrocknet. Ausbeute: 229 mg (46 %) Dimethylsulfaminsäure-5-(2,4-diamino-pyrimidin-5-ylmethyl)-3-ethoxy-2-iod-phenyl-ester als farbloser Feststoff.
MS (ISP): 494,1 $(M+H)^+$

Beispiel 44:

N-[4'-(2,4-Diamino-pyrimidin-5-ylmethyl)-2',6'-diethoxy-biphenyl-3-yl]-methansulfonamid

Diese Verbindung wird ausgehend von 5-(3'-Amino-2,6-diethoxy-biphenyl-4-ylmethyl)-pyrimidin-2,4-diamin (Beispiel (2a)) hergestellt:

5-(3'-Amino-2,6-diethoxy-biphenyl-4-ylmethyl)-pyrimidin-2,4-diamin (228 mg; 0,6 mmol) wird in Dimethylformamid (6 ml; über Molekularsieb getrocknet) gelöst, mit Triethylamin (0,090 ml; 0,66 mmol) bei Raumtemperatur versetzt, auf 0°C abgekühlt und innerhalb 5 Minuten mit einer Lösung von Methansulfonylchlorid (0,051 ml; 0,66 mmol) in Dimethylformamid (1,0 ml) versetzt. Nach 1 Stunde bei 0-5°C wird das Reaktionsgemisch eingeengt und der Rückstand in Wasser aufgenommen, worauf durch Zugabe von $NH_4OH$ konz das pH auf 10 eingestellt wird. Nach 15-minütigem Rühren wird abgenutscht , am Hochvakuum getrocknet und über Kieselgel (60 g) mit Methylenchlorid/Methanol/$NH_4OH$ konz. (19/1/0,05) chromatographiert.
Ausbeute: 161 mg (58 %) N-[4'-(2,4-Diamino-pyrimidin-5-ylmethyl)-2',6'-diethoxybiphenyl-3-yl]-methansulfonamid als farbloses Pulver.
MS (ISP): 458,2 $(M+H)^+$

Beispiel 45:

Methansulfonsäure-4'-(2,4-diamino-pyrimidin-5-ylmethyl)-2',6'-diethoxy-biphenyl-4-yl-ester

Diese Verbindung wird ausgehend von 4'-(2,4-Diamino-pyrimidin-5-ylmethyl)-2',6'-diethoxy-biphenyl-4-ol (Beispiel (2t)) in Analogie zu Beispiel 44 hergestellt: Ausgehend von 4'-(2,4-Diamino-pyrimidin-5-ylmethyl)-2',6'-diethoxy-biphenyl-4-ol (300 mg; 0,79 mmol), Kalium-tert-butylat (anstatt Triethylamin; 133 mg; 1,18 mmol) in Dimethylformamid (10 ml), und von Methansulfonylchlorid (0,080 ml; 1,03 mmol) in Dimethylformamid (5,0 ml) bei Raumtemperatur erhält man 102 mg (22 %) Methansulfonsäure-4'-(2,4-diamino-pyrimidin-5-ylmethyl)-2',6'-diethoxy-biphenyl-4-ylester als farbloses Pulver.
MS (ISP): 459,4 $(M+H)^+$

Beispiel 46:

2-[4'-(2,4-Diamino-pyrimidin-5-ylmethyl)-2',6'-diethoxy-biphenyl-4-yloxy]-acetamid

Diese Verbindung wird ausgehend von 4'-(2,4-Diamino-pyrimidin-5-ylmethyl)-2',6'-diethoxy-biphenyl-4-ol (Beispiel (2t) hergestellt:

4'-(2,4-Diamino-pyrimidin-5-ylmethyl)-2',6'-diethoxy-biphenyl-4-ol (380 mg; 1,0 mmol) wird in Dimethylformamid (10 ml) mit Molekularsieb (0,5 g) und anschliessend Kalium-tert-butylat (180 mg; 1,6 mmol) bei Raumtemperatur versetzt. Nach 15-minütigem Rühren wird 2-Bromacetamid (193 mg; 1,4 mmol) zugegeben. Nach 70 Minuten wird das Reaktionsgemisch eingeengt und über Kieselgel (60 g) mit Methylenchlorid/Methanol/$NH_4OH$ konz. (90/10/1) chromatographiert.
Ausbeute: 190 mg (43 %) 2-[4'-(2,4-Diamino-pyrimidin-5-ylmethyl)-2',6'-diethoxybiphenyl-4-yloxy]-acetamid als farbloses Pulver.
MS (ISP): 438,3 $(M+H)^+$

Beispiel 47:

3-{1-[4-(2,4-Diamino-pyrimidin-5-ylmethyl)-6-ethoxy-4'-hydroxy-biphenyl-2-yloxymethyl]-cyclopropyl}-propionitril

Diese Verbindung wird ausgehend von (E)-3-[1-[4-(2,4-Diamino-pyrimidin-5-ylmethyl)-6-ethoxy-4'-hydroxy-biphenyl-2-yloxymethyl]-cyclopropyl]-acrylnitril (Beispiel 8d) hergestellt:

(E)-3-[1-[4-(2,4-Diamino-pyrimidin-5-ylmethyl)-6-ethoxy-4'-hydroxy-biphenyl-2-yloxymethyl]-cyclopropyl]-acrylnitril (Beispiel 8d) (190 mg; 0,415 mmol) wird in Isopropylalkohol (20 ml) suspendiert und innerhalb ca. 10 Minuten portionenweise mit Natriumborhydrid (103 mg; 2,69 mmol) versetzt. Danach wird unter Argon während 23 Stunden am Rückfluss gekocht, auf Raumtemperatur abgekühlt und eingeengt. Der Rückstand wird mit Wasser (27 ml) verrührt und das pH mit 1 N wässriger Salzsäure auf 2 eingestellt. Anschliessend wird das pH durch Zugabe von $NH_4OH$ konz. auf 9 eingestellt und die erhaltene Suspension eine Stunde bei Raumtemperatur gerührt und abgenutscht. Der Feststoff wird mit etwas Wasser gewaschen und am Hochvakuum getrocknet. Ausbeute: 180 mg (94 %) 3-{1-[4-(2,4-Diamino-pyrimidin-5-ylmethyl)-6-ethoxy-4'-hydroxy-biphenyl-2-yloxymethyl]-cyclopropyl}-propionitril.
MS (ISP): 460,4 $(M+H)^+$

Beispiel 48:

5-[4-(2,4-Diamino-pyrimidin-5-ylmethyl)-6-ethoxy-4'-hydroxy-biphenyl-2-yloxy]-4,4-dimethyl-pentannitril

Diese Verbindung wird ausgehend von (E)-5-[4-(2,4-Diamino-pyrimidin-5-ylmethyl)-6-ethoxy-4'-hydroxy-biphenyl-2-yloxy]-4,4-dimethyl-pent-2-ennitril (Beispiel (8b)) in Analogie zu Beispiel 47 hergestellt:
Ausgehend von (E)-5-[4-(2,4-Diamino-pyrimidin-5-ylmethyl)-6-ethoxy-4'-hydroxybiphenyl-2-yloxy]-4,4-dimethyl-pent-2-ennitril (110 mg; 0,239 mmol) werden 85 mg (77 %) 5-[4-(2,4-Diamino-pyrimidin-5-ylmethyl)-6-ethoxy-4'-hydroxy-biphenyl-2-yloxy]-4,4-dimethyl-pentannitril als hellbraunes Pulver erhalten.
MS (ISP): 462,3 $(M+H)^+$

Beispiel 49:

5-[3,5-Diethoxy-4-(1,2,3,4-tetrahydro-chinolin-6-yl)-benzyl]-pyrimidin-2,4-diamin

Eine Lösung von 5-(3,5-Diethoxy-4-chinolin-6-yl-benzyl)-pyrimidin-2,4-diamin (Beispiel (4m)) (200 mg; 0,48 mmol) in 5,5 ml MeOH wird mit Nickel(II)chloridhexahydrat (200 mg; 0,84 mmol) unter Argonbegasung versetzt. Natriumborhydrid (73 mg, 1,93 mmol) wird portionenweise zugegeben. Das Gemisch wird 3 Stunden bei Raumtemperatur gerührt. Nach Eindampfen des Reaktionsgemisches wird der Rückstand in 20 ml 10% wässriger Salzsäure 10 Minuten verrührt. Die Lösung wird mit 25% wässriger Ammoniaklösung auf pH 10 eingestellt und dreimal mit jeweils 25 ml Methylenchlorid extrahiert. Die vereinigten organischen Phasen werden über Magnesiumsulfat getrocknet und eingeengt. Das rohe Produkt wird über Kieselgel mit Methylenchlorid/Methanol (19/1) chromatographiert. Man erhält 125 mg (62%) 5-[3,5-Diethoxy-4-(1,2,3,4-tetrahydro-chinolin-6-yl)-benzyl]-pyrimidin-2,4-diamin als farbloses Pulver.
MS (ISP): 420,3 $(M+H)^+$.

Beispiel 50:

5-[2,6-Diethoxy-3'-(2-methoxy-ethoxy)-biphenyl-4-ylmethyl]-pyrimidin-2,4-diamin

Diese Verbindung wird ausgehend von 4'-(2,4-Diamino-pyrimidin-5-ylmethyl)-2',6'-diethoxy-biphenyl-3-ol (Beispiel (5d)) hergestellt:
4'-(2,4-Diamino-pyrimidin-5-ylmethyl)-2',6'-diethoxy-biphenyl-3-ol (200 mg; 0,526 mmol) wird in Dimethylformamid (5 ml; über Molekularsieb getrocknet) suspendiert und bei Raumtemperatur mit Kalium-tert-butylat (83 mg; 0,736 mmol) versetzt. Die entstandene Lösung wird innerhalb 35 Minuten mit 2-Bromethyl-methylether (0,059 ml; 0,63 mmol) in Dimethylformamid (5 ml ; über Molekularsieb getrocknet) versetzt. Nach 12-stündigem Rühren bei Raumtemperatur wird das Reaktionsgemisch eingeengt, nacheinander mit Wasser (zweimal) und Diethylether verrührt, abgenutscht und am Hochvakuum getrocknet.
Ausbeute: 180 mg (78 %) 5-[2,6-Diethoxy-3'-(2-methoxy-ethoxy)-biphenyl-4-ylmethyl]-pyrimidin-2,4-diamin als leicht beiges Pulver.
MS (ISP): 439,4 $(M+H)^+$

Beispiel 51:

5-(3'-Amino-2,6-di-n-propoxy-biphenyl-4-ylmethyl)-pyrimidin-2,4-diamin

Wird in Analogie zu Beispiel (2a) (Suzuki-Kopplung mit Boronsäure) hergestellt:
Ausgehend von 5-(4-Iod-3,5-di-n-propoxy-benzyl)-pyrimidin-2,4-diamin (200 mg; 0,45 mmol) und 3-Aminophenyl-boronsäure (175 mg; 1,13 mmol) werden 110 mg (60 %) 5-(3'-Amino-2,6-di-n-propoxy-biphenyl-4-ylmethyl)-pyrimidin-2,4-diamin als farbloses Pulver erhalten.
MS (ISP): 408,4 $(M+H)^+$
Die obige Ausgangsverbindung, 5-(4-Iod-3,5-di-n-propoxy-benzyl)-pyrimidin-2,4-diamin wird durch folgende Sequenz (Stufen a)-f)) hergestellt:

Stufe a) 4-Iod-3,5-di-n-propoxy-benzoesäure-methylester

3,5-Dihydroxy-4-iod-benzoesäure-methylester (Beispiel 1, Stufe b) (5 g; 17 mmol) wird in Dimethylformamid (125 ml) gelöst und mit Kalium-tert-butylat (3,81 g; 34 mmol) bei Raumtemperatur versetzt. Nach einstündigem Rühren wird n-Propylbromid (5,0 g; 40 mmol) in Dimethylformamid (5 ml) innerhalb einer Stunde zugetropft. Das Reaktionsgemisch wird anschliessend auf 40°C erwärmt und 48 Stunden bei dieser Temperatur gerührt. Das Reaktionsgemisch wird auf Raumtemperatur abgekühlt, das Lösungsmittel eingedampft, der Rückstand mit Wasser und 1 N wässriger Salzsäure versetzt und mit Ethylacetat extrahiert. Die organischen Phasen werden mit einer wässrigen gesättigten Kochsalzlösung gewaschen, über Magnesiumsulfat getrocknet und eingeengt.
Ausbeute: 4,2 g (68 %) 4-Iod-3,5-dipropoxy-benzoesäure-methylester als gelbes Pulver.
MS: 378 (M)

Stufe b) (4-Iod-3,5-di-n-propoxy-phenyl)-methanol

Wird analog Beispiel 6, Stufe c) hergestellt:
Ausgehend von 4-Iod-3,5-di-n-propoxy-benzoesäure-methylester (4,2 g; 11 mmol) werden nach Kristallisation aus n-Hexan, 3,8 g (99 %) (4-Iod-3,5-di-n-propoxy-phenyl)-methanol als farbloses Pulver erhalten.
MS: 350 (M)

Stufe c) 4-Iod-3,5-di-n-propoxy-benzaldehyd

Wird analog Beispiel 6, Stufe d) hergestellt:
Ausgehend von (4-Iod-3,5-di-n-propoxy-phenyl)-methanol (3,8 g; 11 mmol) werden 3,36 g (87 %) 4-Iod-3,5-di-n-propoxy-benzaldehyd als farbloses Pulver erhalten.
MS: 348 (M)

Stufe d) (E/Z)- 2-(4-Iod-3,5-di-n-propoxy-benzyl)-3-phenylamino-acrylnitril

Wird Analog Beispiel 6, Stufe e) hergestellt:
Ausgehend von 4-Iod-3,5-di-n-propoxy-benzaldehyd (3,36 g; 9,6 mmol) werden nach Chromatographie über Kieselgel mit Ethylacetat/n-Hexan 7/3; 3,0 g (65 %) (E/Z)- 2-(4-Iod-3,5-di-n-propoxy-benzyl)-3-phenylamino-acrylnitril als braunes sehr viskoses Öl erhalten.
MS (ISP); 477,2 $(M+H)^+$

Stufe e) 5-(4-Iod-3,5-di-n-propoxy-benzyl)-pyrimidin-2,4-diamin

Wird analog zu Beispiel 6, Stufe f) hergestellt:
Ausgehend von (E/Z)- 2-(4-Iod-3,5-di-n-propoxy-benzyl)-3-phenylaminoacrylnitril (3,0 g; 6,2 mmol) werden nach Chromatographie über Kieselgel mit Methylenchlorid/Methanol/NH4OH konz. (90/10/1) 1,8 g (65 %) 5-(4-Iod-3,5-di-n-propoxy-benzyl)-pyrimidin-2,4-diamin als beiges Pulver erhalten.
MS (ISP): 443,2 $(M+H)^+$

Beispiel 52:

4'-(2,4-Diamino-pyrimidin-5-ylmethyl)-2',6'-dipropoxy-biphenyl-3-ol

Wird in Analogie zu Beispiel (2a) (Methode A Suzuki-Kopplung mit Bororlsäure)hergestellt:
Ausgehend von 5-(4-Iod-3,5-di-n-propoxy-benzyl)-pyrimidin-2,4-diamin (100 mg; 0,22 mmol) und 3-Hydroxyphenyl-boronsäure (78 mg; 0,56 mmol) werden 153 mg (73 %) 4'-(2,4-Diamino-pyrimidin-5-ylmethyl)-2',6'-dipropoxy-biphenyl-3-ol als farbloses Pulver erhalten.
MS (ISP): 409,3 $(M+H)^+$
Die obige Ausgangsverbindung, 5-(4-Iod-3,5-dipropoxy-benzyl)-pyrimidin-2,4-diamin wird wie in Beispiel 51 beschrieben hergestellt.

Beispiel 53

4'-(2,4-Diamino-pyrimidin-5-ylmethyl)-6'-ethoxy-2'-n-propoxy-biphenyl-3-ol

Wird in Analogie zu Beispiel (7a) hergestellt:
Ausgehend 4'-(2,4-Diamino-pyrimidin-5-ylmethyl)-6'-ethoxy-2'-hydroxy-biphenyl-3-yl -acetat (300 mg; 0,76 mmol) und 1-Brom-n-propan (0,07 ml; 0,912 mmol) werden nach Behandlung mit wässriger gesättigter Natriumbicarbonatlösung (2 ml) und Methanol (5 ml) 2 Stunden bei Raumtemperatur 70 mg (23 %) 4'-(2,4-Diamino-pyrimidin-5-ylmethyl)-6'-ethoxy-2'-propoxy-biphenyl-3-ol als farbloses Pulver erhalten.

MS (ISP): 395,3 $(M+H)^+$

Die obige Ausgangsverbindung, 4'-(2,4-Diamino-pyrimidin-5-ylmethyl)-6'-ethoxy-2'-hydroxy-biphenyl-3-yl - acetat, wird durch folgende Sequenz (Stufen a)-c)) hergestellt:

Stufe a) 2'-Benzyloxy-4'-(2,4-diamino-pyrimidin-5-ylmethyl)-6'-ethoxy-biphenyl-3-ol

Wird in Analogie zu Beispiel 2a (Suzuki-Kopplung mit Boronsäure) hergestellt:

Ausgehend von 5-(3-Benzyloxy-5-ethoxy-4-iod-benzyl)-pyrimidin-2,4-diamin (Beispiel 6) (3,4 g; 7,12 mmol) und 3-Hydroxyphenyl-boronsäure (1,97 g; 14,3 mmol) werden 2,34 g (74 %) 2'-Benzyloxy-4'-(2,4-diamino-pyrimidin-5-ylmethyl)-6'-ethoxybiphenyl-3-ol als farbloses Pulver erhalten.
MS (ISP): 443,3 $(M+H)^+$

Stufe b) 2'-Benzyloxy-4'-(2,4-diamino-pyrimidin-5-ylmethyl)-6'-ethoxy-biphenyl-3-yl-acetat

2'-Benzyloxy-4'-(2,4-diamino-pyrimidin-5-ylmethyl)-6'-ethoxy-biphenyl-3-ol (1,9 g; 4,3 mmol) in Dimethylformamid (40 ml) wird bei 0-5°C mit Kalium-tert-butylat (602 mg; 5,36 mmol) in Dimethylformamid (40 ml) versetzt. Nach 15-minütigem Rühren bei dieser Temperatur wird Acetanhydrid (0,406 ml; 4,3 mmol) zugegeben. Nach 3 Stunden bei 0-5°C werden erneut Kalium-tert-butylat (60 mg; 0,53 mmol) und danach Acetanhydrid (0,08 ml; 0,85 mmol) hinzugefügt und 16 Stunden bei Raumtemperatur weitergerührt. Das Reaktionsgemisch wird eingeengt, der Rückstand mit Wasser verrührt und abgenutscht. Das Rohprodukt wird aus Isopropanol umkristallisiert.
Ausbeute: 1,57 g (75 %) 2'-Benzyloxy-4'-(2,4-diamino-pyrimidin-5-ylrnethyl)-6'-ethoxybiphenyl-3-yl-acetat als farbloses Pulver.
MS (ISP): 485,3 $(M+H)^+$

Stufe c) 4'-(2,4-Diamino-pyrimidin-5-ylmethyl)-6'-ethoxy-2'-hydroxy-biphenyl-3-yl-acetat

2'-Benzyloxy-4'-(2,4-diamino-pyrimidin-5-ylmethyl)-6'-ethoxy-biphenyl-3-yl-acetat (1,5 g; 3,09 mmol) wird in Essigsäure konz. (45 ml) und Ethanol (15 ml) über Pd/C 10% (380 mg) 2 Stunden hydriert. Der Katalysator wird abgenutscht, mit Ethanol nachgewaschen und das Lösungsmittel abgedampft. Der Rückstand wird mit Wasser verrührt und das pH durch Zugabe wässriger gesättigter Natriumbicarbonatlösung auf 7 eingestellt. Die entstandene Suspension wird abgenutscht und am Hochvakuum getrocknet.
Ausbeute: 1,05 g (86 %) 4'-(2,4-Diamino-pyrimidin-5-ylmethyl)-6'-ethoxy-2'-hydroxybiphenyl-3-yl-acetat als farbloses Pulver.
MS (ISP): 495,2 $(M+H)^+$

Beispiel 54

2'-Cyclopropylmethoxy-4'-(2,4-diamino-pyrimidin-5-ylmethyl)-6'-ethoxy-biphenyl-3-ol

Wird in Analogie zu Beispiel 55 hergestellt:

Ausgehend von 4'-(2,4-Diamino-pyrimidin-5-ylmethyl)-6'-ethoxy-2'-hydroxybiphenyl-3-yl-acetat (300 mg; 0,76 mmol) und Brommethyl-cyclopropan (123 mg; 0,91 mmol) werden 101 mg (31 %) 2'-Cyclopropylmethoxy-4'-(2,4-diamino-pyrimidin-5-ylmethyl)-6'-ethoxy-biphenyl-3-ol als farbloses Pulver erhalten.
MS (ISP): 407,3 $(M+H)^+$

Die obige Ausgangsverbindung, 4'-(2,4-Diamino-pyrimidin-5-ylmethyl)-6'-ethoxy-2'-hydroxy-biphenyl-3-yl-acetat wird wie in Beispiel 53 beschrieben hergestellt.

Beispiel 55

2-[2'-Cyclopropylmethoxy-4'-(2,4-diamino-pyrimidin-5-ylmethyl)-6'-ethoxy-biphenyl-4-yloxy]-ethanol

Wird in Analogie zu Beispiel 46 hergestellt:

Ausgehend von 2'-Cyclopropylmethoxy-4'-(2,4-diamino-pyrimidin-5-ylmethyl)-6'ethoxy-biphenyl-4-ol (Beispiel 8h) (406 mg; 1 mmol) und 2-(2-Chlorethoxy)-tetrahydro-2H-pyran (0,47 ml; 3,2 mmol) werden nach saurer Abspaltung der Schutzgruppe (HCl 3 N in Methanol bei Raumtemperatur) 95 mg (21 %) 2-[2'-Cyclopropylmethoxy-4'-(2,4-diamino-pyrimidin-5-ylmethyl)-6'-ethoxy-biphenyl-4-yloxy]-ethanol als leicht gelbes Pulver erhalten.
MS (ISP): 451,3 $(M+H)^+$

Beispiel 56

5-[2-Cyclopropylmethoxy-6-ethoxy-4'-(2-morpholin-4-yl-ethoxy)-biphenyl-4-ylmethyl]-pyrimidin-2,4-diamin

Wird in Analogie zu Beispiel 46 hergestellt:

Ausgehend von 2'-Cyclopropylmethoxy-4'-(2,4-diamino-pyrimidin-5-ylmethyl)-6'ethoxy-biphenyl-4-ol (Beispiel 8h) (300 mg; 0,74 mmol) und 4-(2-Chlorethyl)-morpholinhydrochlorid(170 mg; 0,89 mmol) erhält man 129 mg (25 %) 5-[2-Cyclopropylmethoxy-6-ethoxy-4'-(2-morpholin-4-yl-ethoxy)-biphenyl-4-ylmethyl]-pyrimidin-2,4-diamin als farbloses Pulver.
MS (ISP): 520,3 $(M+H)^+$

Beispiel 57

Methansulfonsäure-2'-cyclopropylmethoxy-4'-(2,4-diamino-pyrimidin-5-ylmethyl)-6'-ethoxy-biphenyl-4-yl-ester

Wird in Analogie zu Beispiel 45 hergestellt:

Ausgehend von 2'-Cyclopropylmethoxy-4'-(2,4-diamino-pyrimidin-5-ylmethyl)-6'ethoxy-biphenyl-4-ol (Beispiel 8h) (300 mg; 0,74 mmol) und Methansulfonylchlorid (0,063 ml; 0,81 mmol) werden 152 mg (42 %) Methansulfonsäure-2'-cyclopropylmethoxy-4'-(2,4-diamino-pyrimidin-5-ylmethyl)-6'-ethoxy-biphenyl-4-yl-ester als farbloses Pulver erhalten.
MS (ISP): 485,2 $(M+H)^+$

Beispiel 58

4'-(2,4-Diamino-pyrimidin-5-ylmethyl)-6'-ethoxy-2'-(propan-2-sulfonyloxy)-biphenyl-4-yl-acetat

Wird in Analogie zu Beispiel 45 hergestellt:

Ausgehend von Propan-2-sulfonsäure-4-(2,4-diamino-pyrimidin-5-ylmethyl)-6-ethoxy-4'-hydroxy-biphenyl-2-yl-ester (Beispiel 15c) (137 mg; 0,30 mmol), Kalium-tert-butylat (42 mg; 0,37 mmol) und Acetanhydrid (0,028 ml; 0,30 mmol) in Dimethylformamid (3,5 ml) bei 0-5°C (15 Minuten) und anschliessend Raumtemperatur (3 Stunden) werden 39 mg (27 %) 4'-(2,4-Diamino-pyrimidin-5-ylmethyl)-6'-ethoxy-2'-(propan-2-sulfonyloxy)-biphenyl-4-yl-acetat als farbloses Pulver erhalten.
MS (ISP): 501,3 $(M+H)^+$

Beispiel 59

[2'-Cyclopropylmethoxy-4'-(2,4-diamino-pyrimidin-5-ylmethyl)-6'-ethoxy-biphenyl-4-yloxy]-acetonitril

Wird in Analogie zu Beispiel 46 hergestellt:

Ausgehend von 2'-Cyclopropylmethoxy-4'-(2,4-diamino-pyrimidin-5-ylmethyl)-6'ethoxy-biphenyl-4-ol (Beispiel 8h) (203 mg; 0,5 mmol) und Bromacetonitril (0,06 ml; 0,9 mmol) werden 85 mg (38 %) [2'-Cyclopropylmethoxy-4'-(2,4-diamino-pyrimidin-5-ylmethyl)-6'-ethoxy-biphenyl-4-yloxy]-acetonitril als beiges Pulver erhalten.
MS (ISP): 446,3 $(M+H)^+$

Beispiel 60

Dimethylsulfaminsäure-4-(2,4-diamino-pyrimidin-5-ylmethyl)-6-ethoxy-4'-methansulfonyl-biphenyl-2-yl-ester

Wird in Analogie zu Beispiel 2b hergestellt:

Ausgehend von Dimethylsulfaminsäure-4-(2,4-diamino-pyrimidin-5-ylmethyl)-6-ethoxy-4'-methylsulfanyl-biphenyl-2-yl-ester (Beispiel 43a) (342 mg; 0,7 mmol) und m-Chlorperbenzoesäure (403 mg; 2,12 mmol) werden 340 mg (84 %) Dimethylsulfaminsäure- 4-(2,4-diamino-pyrimidin-5-ylmethyl)-6-ethoxy-4'-methansulfonyl-biphenyl-2-yl ester als beiges Pulver erhalten.
MS (ISP): 522,1 $(M+H)^+$

Beispiel 61

(61a) 2-Methyl-propan-1-sulfonsäure-4-(2,4-diamino-pyrimidin-5-ylmethyl)-6-ethoxy-4'-(2-hydroxy-ethoxy)-biphenyl-2-yl-ester

2-Methyl-propan-1-sulfonsäure-4-(2,4-diamino-pyrimidin-5-ylmethyl)-6-ethoxy-4'-hydroxy-biphenyl-2-yl ester (Beispiel 15f) (85 mg, 0,18 mmol) werden in 2 ml absolutem Dimethylformamid vorgelegt, mit 280 mg pulverisiertem Molekularsieb (4A) und 37 mg (0,32 mmol) Kalium-tert-butylat versetzt und 10 Min. gerührt. Man kühlt anschliessend auf 0°C und gibt 93 mg (0,32 mmol) tert-Butyl-(2-iod-ethoxy)-dimethylsilan zu. Nach 10 Min. bei 0°C und 1 h bei Raumtemperatur verdünnt man das Reaktionsgemisch mit einer Mischung aus $CH_2Cl_2$/MeOH/25% $NH_3$ (90/10/1), filtriert, dampft ein und reinigt das Rohprodukt mittels Flash-Chromatographie auf $SiO_2$ ($CH_2Cl_2$/MeOH/25% $NH_3$(19/1/0,05). Dabei werden 66 mg des Silylethers obiger Verbindung als farbloses Öl erhalten, der wie folgt hydrolysiert wird:

Man löst ihn in 1,2 ml Tetrahydrofuran und gibt 1,2 ml 3N HCl in Methanol zu. Nach 15 Min. wird mit 10% Natriumcarbonatlösung neutralisiert, mit Ethylacetat extrahiert, mit konzentrierter wässriger Kochsalzlösung gewaschen, über Magnesiumsulfat getrocknet und eingedampft. Verrühren in Diethylether liefert 42 mg der Titelverbindung als weissen Feststoff.
MS (ISP): 517,4 $(M+H)^+$

In Analogie hierzu wird hergestellt:

(61b) Butan-2-sulfonsäure-4-(2,4-diamino-pyrimidin-5-ylmethyl)-6-ethoxy-4'-(2-hydroxy-ethoxy)-biphenyl-2-yl-ester

Ausgehend von 105 mg (0,22 mmol) 2-Methyl-propan-1-sulfonsäure-4-(2,4-diamino-pyrimidin-5-ylmethyl)-6-ethoxy-4'-hydroxy-biphenyl-2-yl ester und 115 mg (0,40 mmol) tert-Butyl-(2-iod-ethoxy)-dimethyl-silan werden nach saurer Abspaltung der Silyl-Schutzgruppe 56 mg der Titelverbindung als farbloser Schaum erhalten.
MS (ISP): 517,3 $(M+H)^+$

Beispiel 62

4'-(2,4-Diamino-pyrimidin-5-ylmethyl)-2'-dimethylsulfamoyloxy-6'-ethoxy-biphenyl-4-yl-acetat

Wird in Analogie zu Beispiel 45 hergestellt:

Ausgehend von Dimethylsulfaminsäure-4-(2,4-diamino-pyrimidin-5-ylmethyl)-6-ethoxy-4'-hydroxy-biphenyl-2-yl-ester (Beispiel 20a) (250 mg; 0,54 mmol), Kalium-tert-butylat (91 mg; 0,81 mmol) und Acetanhydrid (0,061 ml; 0,65 mmol) in Dimethylformamid (6 ml) bei 0-5°C (30 Minuten) und anschliessend Raumtemperatur (65 Stunden) werden 133 mg (49 %) als farbloses Pulver erhalten.
MS (ISP): 502,2 $(M+H)^+$

Beispiel 63

2-Methyl-propan-1-sulfonsäure-4-(2,4-diamino-pyrimidin-5-ylmethyl)-6-ethoxy-4'-(2-methoxy-ethoxy)-biphenyl-2-yl-ester

2-Methyl-propan-1-sulfonsäure-4-(2,4-diamino-pyrimidin-5-ylmethyl)-6-ethoxy-4'-hydroxy-biphenyl-2-yl-ester (100 mg, 0,21 mmol) werden in 2,1 ml abs. Dimethylformamid vorgelegt, mit 320 mg pulverisiertem Molekularsieb 4A und nach 15 Min. mit 43 mg (0,38 mmol) Kalium-tert-butylat versetzt und während weiteren 5 Minuten gerührt. Man kühlt anschliessend auf 0°C und gibt dann 55 mg (0,30 mmol) 1-Iod-2-methoxyethan zu. Nach 1,5 h bei 0°C verdünnt man das Reaktionsgemisch mit einer Mischung aus $CH_2Cl_2$/MeOH/25% $NH_3$(90/10/1), filtriert, dampft ein und reinigt das Rohprodukt mittels Flash-Chromatographie auf $SiO_2$ ($CH_2Cl_2$/MeOH/25% $NH_3$(19/1/0,05)). Dabei werden 70 mg der Titelverbindung als farbloser Schaum erhalten.
MS (ISP): 531,3 $(M+H)^+$

Beispiel 64

Herstellung von Verbindungen der Formel VIII, Schema 2, aus den entsprechenden Verbindungen der Formel VI.

(64a) Methansulfonsäure-4-(2,4-diamino-pyrimidin-5-ylmethyl)-6-ethoxy-3'-fluor-4'-morpholin-4-ylmethyl-biphenyl-2-yl-ester

4-(2,4-Diamino-pyrimidin-5-ylmethyl)-6-ethoxy-3'-fluor-4'-morpholin-4-ylmethylbiphenyl-2-ol (250 mg, 0.55 mmol) wird in 11 ml Tetrahydrofuran suspendiert, mit 0,5 g pulverisiertem Molekularsieb versetzt und 15 Min. bei Raumtemperatur verrührt. Kalium-tert-Butylat (93 mg; 0,83 mmol) wird zugegeben und die Suspension nochmals 1 Stunde bei Raumtemperatur gerührt. Danach wird die beige Suspension auf -20°C abgekühlt und mit Methansulfonylchlorid (126 mg; 1,1 mmol) versetzt. Das Reaktionsgemisch wird 4 Stunden bei 0°C gerührt und anschliessend eingeengt. Der Rückstand wird an Kieselgel mit Methylenchlorid/MeOH (99:1) chromatographiert. Man erhält 99 mg (33%) Methansulfonsäure-4-(2,4-diamino-pyrimidin-5-ylmethyl)-6-ethoxy-3'-fluor-4'morpholin-4-ylmethyl-biphenyl-2-yl-ester als leicht gelbes Pulver.
MS (ISP): 523,3 $(M+H)^+$.

(64b) Dimethylsulfaminsäure-4-(2,4-diamino-pyrimidin-5-ylmethyl)-6-ethoxy-3'-fluor-4'-morpholin-4-ylmethyl-biphenyl-2-yl-ester, Schema 2, (VI) -> (IX)

4-(2,4-Diamino-pyrimidin-5-ylmethyl)-6-ethoxy-3'-fluor-4'-morpholin-4-ylmethylbiphenyl-2-ol (250 mg, 0.55 mMol) werden in 11 ml Tetrahydrofuran suspendiert, mit 0,5 g pulverisiertem Molekularsieb versetzt und 15 Minuten bei Raumtemperatur verrührt. Kalium-tert-Butylat (93 mg; 0,83 mmol) wird zugegeben und die Suspension nochmals 1 Stunde bei Raumtemperatur gerührt. Danach wird die beige Suspension auf -20°C abgekühlt und mit N,N-Dimethylsulfamoylchlorid (158 mg; 1,1 mmol) versetzt. Das Reaktionsgemisch wird 4 Stunden bei 0°C und 2 Stunden bei 10°C gerührt und anschliessend eingeengt. Der Rückstand wird über Kieselgel mit Methylenchlorid/MeOH (99:1) chromatographiert. Man erhält 151 mg (49%) Dimethylsulfaminsäure-4-(2,4-diamino-pyrimidin-5-ylmethyl)-6-ethoxy-3'-fluor-4'-morpholin-4-ylmethyl-biphenyl-2-yl-ester als leichtgelbes Pulver.
MS (ISP): 561,3 $(M+H)^+$.

Die obige Ausgangsverbindung 4-(2,4-Diamino-pyrimidin-5-ylmethyl)-6-ethoxy-3'fluor-4'-morpholin-4-ylmethyl-biphenyl-2-ol wird durch folgende Sequenz (Stufen a)-c))hergestellt:

Stufe a) 4'-(2,4-Diamino-pyrimidin-5-ylmethyl)-6'-ethoxy-3-fluor-2'-methoxymethoxybiphenyl-4-carbaldehyd

In Analogie zu Beispiel (4a) erhält man ausgehend von 4-Brom-2-fluor-benzaldehyd (1,015 g; 5 mmol), ), Bis (pinacolato)diboron (1,395 g ; 5,5 mmol), Kaliumacetat (1,08 g; 11 mmol), Bis(Triphenylphosphin) palladium (II)dichlorid (150 mg; 0,21 mmol) und von 5-(3-Ethoxy-4-iod-5-methoxymethoxy-benzyl)-pyrimidin-2,4-diamin (Beispiel 25) (1,075 g; 2,5 mmol) 980 mg (92 %) 4'-(2,4-Diamino-pyrimidin-5-ylmethyl)-6'-ethoxy-3-fluor-2'-methoxymethoxy-biphenyl-4-carbaldehyd als hellgelbes Pulver.
MS (ISP): 427,4 $(M+H)^+$.

Stufe b) 5-(6-Ethoxy-3'-fluor-2-methoxymethoxy-4'-morpholin-4-ylmethyl-biphenyl-4-ylmethyl)-pyrimidin-2,4-diamin

In Analogie zu Beispiel (4v) erhält man aus 4'-(2,4-Diamino-pyrimidin-5-ylmethyl)-6'-ethoxy-3-fluor-2'-methoxymethoxy-biphenyl-4-carbaldehyd (900 mg; 2,11 mmol) und Morpholin (1,1 g; 12,6 mMol) 699 mg (66%) 5-(6-Ethoxy-3'-fluor-2-methoxymethoxy-4'-morpholin-4-ylmethyl-biphenyl-4-ylmethyl)-pyrimidin-2,4-diamin als leicht gelbes Pulver.
MS (ISP): 498,4 $(M+H)^+$.

Stufe c) 4-(2,4-Diamino-pyrimidin-5-ylmethyl)-6-ethoxy-3'-fluor-4'-morpholin-4-ylmethyl-biphenyl-2-ol

Eine Lösung von 5-(6-Ethoxy-3'-fluor-2-methoxymethoxy-4'-morpholin-4-ylmethyl-biphenyl-4-ylmethyl)-pyrimidin-2,4-diamin (120 mg; 0,24 mmol) in 20 ml Methanol wird auf 50°C erwärmt und anschliessend mit 2,7 ml 4,5 M Salzsäure in Methanol versetzt und 1 Stunde bei dieser Temperatur gerührt. Die hellgelbe Lösung wird auf 0°C abgekühlt, mit 25% wässriger Ammoniak Lösung auf pH 10 eingestellt und auf ein Volumen von 2 ml eingeengt. Der Rückstand wird mit 20 ml Wasser versetzt und dreimal mit jeweils 50 ml Methylenchlorid extrahiert. Die vereinigten organischen Phasen werden über Magnesiumsulfat getrocknet und eingeengt. Man erhält 57 mg (52%) von 4-(2,4-Diamino-pyrimidin-5-ylmethyl)-6-ethoxy-3'-fluor-4'-morpholin-4-ylmethyl-biphenyl-2-ol als farbloses Pulver.
MS (ISP): 454,5 $(M+H)^+$.

Beispiel 65

Dimethylsulfaminsäure-4-(2,4-diamino-pyrimidin-5-ylmethyl)-6-ethoxy-4'-morpholin-4-ylmethyl-biphenyl-2-yl-ester

In Analogie zu Beispiel (64b) erhält man ausgehend von 4-(2,4-Diamino-pyrimidin-5-ylmethyl)-6-ethoxy-4'-morpholin-4-ylmethyl-biphenyl-2-ol (290 mg; 0,67 mmol) und N,N-Dimethylsulfamoylchlorid (191 mg; 1,33 mmol) 282 mg (78%) Dimethylsulfaminsäure 4-(2,4-diamino-pyrimidin-5-ylmethyl)-6-ethoxy-4'-morpholin-4-ylmethylbiphenyl-2-yl-ester als gelber Schaum.
MS (ISP): 543,3 $(M+H)^+$.

Die obige Ausgangsverbindung 4-(2,4-Diamino-pyrimidin-5-ylmethyl)-6-ethoxy-4'morpholin-4-ylmethyl-biphenyl-2-ol wird durch folgende Sequenz (Stufen a)-c)) hergestellt:

Stufe a) 4'-(2,4-Diamino-pyrimidin-5-ylmethyl)-6'-ethoxy-2'-methoxymethoxy-biphenyl-4-carbaldehyd

In Analogie zu Beispiel (2ag) ausgehend von 1,075 g (2,5 mmol) 5-(3-Ethoxy-4-iod-5-methoxymethoxy-benzyl)-pyrimidin-2,4-diamin (Beispiel 25) und 4-Formyl-phenylboronsäure (562 mg; 3,75 mmol) werden 956 mg (93 %) 4'-(2,4-Diaminopyrimidin-5-ylmethyl)-6'-ethoxy-2'-methoxymethoxy-biphenyl-4-carbaldehyd als beiger Schaum erhalten.
MS (ISP): 409,4 $(M+H)^+$.

Stufe b) 5-(6-Ethoxy-2-methoxymethoxy-4'-morpholin-4-ylmethyl-biphenyl-4-ylmethyl)-pyrimidin-2,4-diamin

In Analogie zu Beispiel (4v) erhält man aus 4'-(2,4-Diamino-pyrimidin-5-ylmethyl)-6'-ethoxy-2'-methoxymethoxy-biphenyl-4-carbaldehyd (950 mg; 2,33 mmol) und Morpholin (1,21 g; 13,95 mmol) 746 mg (67%)5-(6-Ethoxy-2-methoxymethoxy-4'-morpholin-4-ylmethyl-biphenyl-4-ylmethyl)-pyrimidin-2,4-diamin als weisses Pulver.
MS (ISP): 480,4 $(M+H)^+$.

Stufe c) 4-(2,4-Diamino-pyrimidin-5-ylmethyl)-6-ethoxy-4'-morpholin-4-ylmethylbiphenyl-2-ol

In Analogie zu Beispiel (64b) (Herstellung der Ausgangsverbindung, Stufe c)) erhält man aus 5-(6-Ethoxy-2-methoxymethoxy-4'-morpholin-4-ylmethyl-biphenyl-4-ylmethyl)-pyrimidin-2,4-diamin (0,74g; 1,54 mmol) und Salzsäure in Methanol 0,587 g (87%) 4-(2,4-Diamino-pyrimidin-5-ylmethyl)-6-ethoxy-4'-morpholin-4-ylmethyl-biphenyl-2-ol als weisses Pulver.
MS (ISP): 436,5 $(M+H)^+$.

Beispiel 66

Dimethylsulfaminsäure-4-(2,4-diamino-pyrimidin-5-ylmethyl)-6-ethoxy-4'-{[N-(2-fluorethyl)-N-methyl-amino]-methyl}-biphenyl-2-yl-ester, vgl. Schema 2, (X) -> (XI) -> (VI) - > (IX).

In Analogie zu Beispiel (64b) erhält man ausgehend von 4-(2,4-Diamino-pyrimidin-5-ylmethyl)-6-ethoxy-4'-{[N-(2-fluor-ethyl)-N-methyl-amino]-methyl}-biphenyl-2-ol (150 mg; 0,35 mmol) und N,N-Dimethylsulfamoylchlorid (101 mg; 0,71 mmol) 44 mg (23%) Dimethylsulfaminsäure-4-(2,4-diamino-pyrimidin-5-ylmethyl)-6-ethoxy-4'-{[N-(2-fluorethyl)-N-methyl-amino]-methyl}-biphenyl-2-yl-ester als gelben Schaum.
MS (ISP): 533,3 $(M+H)^+$.

Die obige Ausgangsverbindung 4-(2,4-Diamino-pyrimidin-5-ylmethyl)-6-ethoxy-4'-{[N-(2-fluor-ethyl)-N-methyl-amino]-methyl}-biphenyl-2-ol wird durch folgende Sequenz (Stufen a)-c)) hergestellt:

Stufe a) 5-{6-Ethoxy-4'-[(2-fluor-ethylamino)-methyl]-2-methoxymethoxy-biphenyl-4-ylmethyl}-pyrimidin-2,4-diamin

In Analogie zu Beispiel (4v) erhält man aus 4'-(2,4-Diamino-pyrimidin-5-ylmethyl)-6'-ethoxy-2'-methoxymethoxy-biphenyl-4-carbaldehyd (Beispiel 65, Stufe a)) (1 g; 2,45 mmol) und 2-Fluorethylamin-hydrochlorid (1,46 g; 14,69 mmol) 1,059 g (95%) 5-{6-Ethoxy-4'-[(2-fluor-ethylamino)-methyl]-2-methoxymethoxy-biphenyl-4-ylmethyl}pyrimidin-2,4-diamin als weisses Pulver.
MS (ISP): 456,4 $(M+H)^+$.

Stufe b) 5-(6-Ethoxy-4'-{[N-(2-fluor-ethyl)-N-methyl-amino]-methyl}-2-methoxymethoxy-biphenyl-4-ylmethyl)-pyrimidin-2,4-diamin

In Analogie zu Beispiel (4ad) erhält man aus 5-{6-Ethoxy-4'-{(2-fluor-ethylamino)-methyl]-2-methoxymethoxy-biphenyl-4-ylmethyl}-pyrimidin-2,4-diamin (0,835g; 1,83 mmol) und 0,61 ml 35% wässrigem Formaldehyd durch Reduktion mit Natriumborhydrid (138 mg; 3,67 mmol) 0,422g (49%) 5-(6-Ethoxy-4'-{[N-(2-fluor-ethyl)-N-methyl-amino]-methyl}-2-methoxymethoxy-biphenyl-4-ylmethyl)-pyrimidin-2,4-diamin als weissen Schaum.
MS (ISP): 470,4$(M+H)^+$.

Stufe c) 4-(2,4-Diamino-pyrimidin-5-ylmethyl)-6-ethoxy-4'-{[N-(2-fluor-ethyl)-N-methyl-amino]-methyl}-biphenyl-2-ol

In Analogie zu Beispiel (64b) (Herstellung der Ausgangsverbindung, Stufe c)) erhält man aus 5-(6-Ethoxy-4'-{[N-(2-fluor-ethyl)-N-methyl-amino]-methyl}-2-methoxymethoxy-biphenyl-4-ylmethyl)-pyrimidin-2,4-diamin ( 0,46g; 0,98 mmol) und Salzsäure in Methanol 305 mg (73%) 4-(2,4-Diamino-pyrimidin-5-ylmethyl)-6-ethoxy-4'-{[N-(2-fluor-ethyl)-N-methyl-amino]-methyl}-biphenyl-2-ol als weisses Pulver.
MS (ISP): 426,5 $(M+H)^+$.

| Beispiel A | |
|---|---|
| Tablette | |
| Sulfamethoxazol | 400 mg |
| Verbindung der Formel A als Dihydrochlorid | 80 mg |
| PRIMOJEL (Stärke-Derivat) | 6 mg |
| POVIDONE K30 (Polyvinylpyrrolidon) | 8 mg |
| Magnesiumstearat | 6 mg |
| Totalgewicht | 500 mg |

| Beispiel B | |
|---|---|
| Verbindung der Formel A, z.B. als Hydrochlorid | 100 mg |
| Maisstärke | 15 mg |
| Talkum | 3 mg |
| Magnesiumstearat | 2 mg |
| | 120 mg |

**Patentansprüche**

**1.** Substituierte 5-Benzyl-2,4-diaminopyrimidine der allgemeinen Formel

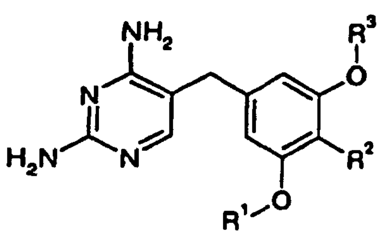

A

in der

$R^1$ C2-C3 Alkyl ist;
$R^2$ Phenyl, Naphthyl oder über eines seiner C-Atome gebundenes Heterocyclyl bedeutet, wobei Phenyl, Naphthyl oder Heterocyclyl einfach oder mehrfach substituiert sein können; und
$R^3$ C2-C6 Alkyl, C2-C6 Alkenyl, Cycloalkyl, Cycloalkylalkyl, Heterocyclylalkyl, Alkylsulfonyl, Phenylsulfonyl, Cycloalkylsulfonyl, Cycloalkylalkylsulfonyl, Cycloalkylalkylsulfamoyl, Heterocyclylsulfonyl, Heterocyclylalkylsulfonyl oder Dialkylsulfamoyl darstellt, wobei diese Gruppen unsubstituiert oder substituiert sein können;

sowie Säureadditionssalze dieser Verbindungen;

wobei die Heterocyclyl-und Phenylgruppen $R^2$ unsubstituiert oder einfach oder mehrfach durch Halogen, Cyan, Alkyl, Alkoxy, Hydroxy, Nitro, Amino, Alkylamino, Dialkylamino, Alkanoylamino, Formyl, Alkanoyloxy, Cyanalkyl, Cyanalkoxy, Hydroxyalkyl, Alkoxyalkyl, Hydroxyalkoxy, Hydroxyalkylamino, Alkoxyalkoxy, Carbamoylalkoxy, Alkylaminoalkyl, Dialkylaminoalkyl, Halogenalkylaminoalkyl, N-Alkyl-N-halogenalkyl-aminoalkyl, Alkylsulfanyl (Alkylthio), Alkylsulfinyl, Alkylsulfonyl, Alkylsulfonyloxy, Alkylsulfonylamino, $R^7$, $R^7$-alkyl, $R^7$-alkoxy, $R^7$- carbonylalkoxyalkyl oder $R^7$-alkanoylamino substituiert sind, oder auch durch zwei benachbarte, zusammen einen ankondensierten 5- oder 6-gliedrigen Heterocyclus bildende Substituenten substituiert sind; wobei $R^7$ ggfs. substituiertes Heterocyclyl darstellt und Alkyl allein oder in Zusammensetzungen bis zu 6 Kohlenstoffatome tragen kann; die Naphthylgruppe $R^2$ unsubstituiert oder durch Hydroxy substituiert ist;

die Heterocyclylgruppen $R^7$ unsubstituiert oder durch Halogen, Alkyl, Alkoxyalkyl, Hydroxyalkyl, Alkanoyl, Alkanoylaminoalkyl oder Oxo substituiert sind; wobei Alkyl, Alkoxy und Alkanoyl allein oder in Zusammensetzungen bis zu 6 Kohlenstoffatome tragen können;

$R^3$, wenn dieses C2-C6 Alkyl, Alkenyl, Alkylsulfonyl oder Dialkylsulfamoyl bedeutet, unsubstituiert oder durch Halogen, Cyan, Hydroxy oder Alkoxy mit bis zu 6 Kohlenstoffatomen substituiert ist; und

$R^3$, wenn dieses Cycloalkyl, Cycloalkylalkyl, Heterocyclylalkyl, Cycloalkylsulfonyl, Cycloalkylalkylsulfonyl, Heterocyclylsulfonyl oder Heterocyclylalkylsulfonyl bedeutet, unsubstituiert oder durch Alkyl, Alkoxy, Hydroxyalkyl, Alkoxyalkyl, Cyan, Cyanalkyl oder Cyanalkenyl substituiert ist; wobei Alkyl, Alkoxy und Alkenyl allein oder in Zusammensetzungen bis zu 6 Kohlenstoffatome tragen können;

und wobei weiterhin

"Halogen" Fluor, Chlor, Brom oder Iod bedeutet;

"Alkyl" bzw. "Alkoxy" geradkettige oder verzweigte Kohlenwasserstoffgruppen mit höchstens 6 Kohlenstoffatomen, falls nicht anders definiert, bedeuten;

"Alkenyl" eine ungesättigte Kohlenwasserstoffgruppe bedeutet und höchstens 6 Kohlenstoffatome trägt;

"Cycloalkyl" einen cyclischen Kohlenwasserstoff mit 3 bis 6 Kohlenstoffatomen bedeutet;

"Alkanoyloxy" bzw. "Alkanoylamino" Alkyl-COO- bzw. Alkyl-CONH-Gruppen mit höchstens 6 Kohlenstoffen, worin Alkyl die oben erläuterte Bedeutung hat, bedeuten; und

"Heterocyclyl" bzw. "Heterocyclus" ungesättigte oder gesättigte, unsubstituierte oder substituierte 5-oder 6-gliedrige heterocyclische Ringe mit mindestens einem Heteroatom aus Stickstoff, Sauerstoff und Schwefel bedeuten, welche unsubstituiert oder durch Halogen, Hydroxy, Alkyl, Alkoxy, Hydroxyalkyl, Alkoxyalkyl, Cyan, Cyanalkyl oder Cyanalkenyl substituiert sind.

2. Verbindungen nach Anspruch 1 der allgemeinen Formel

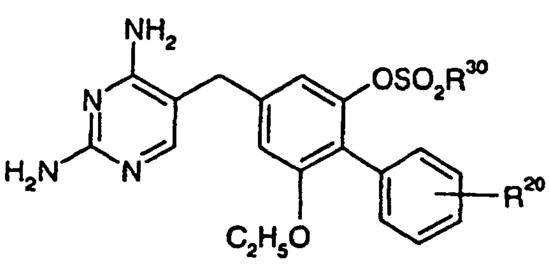

A1

in der $R^{20}$ C1-C6 Alkyl, C1-C6 Alkoxy, Amino, C1-C6 Alkylamino, Fluor oder Chlor und $R^{30}$ C1-C6 Alkyl, C3-C6 Cydoalkyl; di-(C1-C6 Alkyl)amino, N-(C3-C6 Cycloalkyl)-N-(C1-C6 Alkyl)amino oder einen 5- oder 6-gliedrigen, gesättigten, am Stickstoff verknüpften N-Heterocyclus darstellt,

sowie Säureadditionssalze dieser Verbindungen.

3. Verbindungen gemäss Anspruch 2, **dadurch gekennzeichnet, dass** $R^{20}$ Methyl, Methoxy, Amino, Methylamino oder Fluor darstellt.

4. Verbindungen gemäss Anspruch 2 oder 3, **dadurch gekennzeichnet, dass** $R^{30}$ Isopropyl, sec-Butyl, Cyclobutyl, Dimethylamino, N-Cyclopropyl-N-methyl-amino oder Morpholino darstellt.

**5.** Verbindungen nach Anspruch 1 der allgemeinen Formel

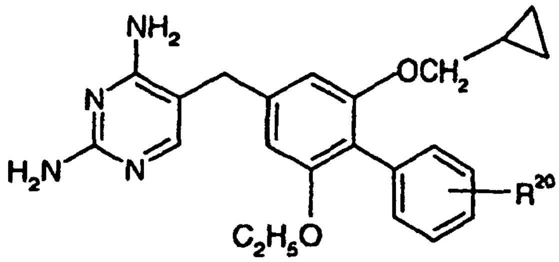

A2

in der $R^{20}$ C1-C6 Alkyl, C1-C6 Alkoxy, Amino, C1-C6 Alkylamino, Fluor oder Chlor bedeutet, sowie Säureadditionssalze dieser Verbindungen.

**6.** Verbindungen gemäss Anspruch 5, **dadurch gekennzeichnet, dass** $R^{20}$ Methyl, Methoxy, Amino, Methylamino oder Fluor darstellt.

**7.** Verbindungen nach Anspruch 1 der allgemeinen Formel

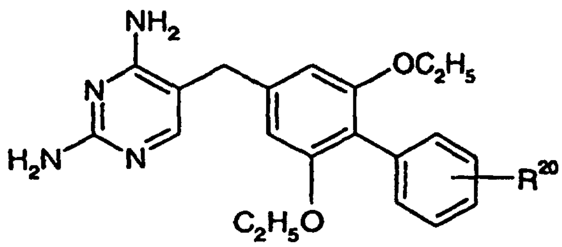

A3

in der $R^{20}$ C1-C6 Alkyl, C1-C6 Alkoxy, Amino, C1-C6 Alkylamino, Fluor oder Chlor bedeutet, sowie Säureadditionssalze dieser Verbindungen.

**8.** Verbindungen gemäss Anspruch 7, **dadurch gekennzeichnet, dass** $R^{20}$ Methyl, Methoxy, Amino, Methylamino oder Fluor darstellt.

**9.** Butan-2-sulfonsäure-4'-amino-4-(2,4-diamino-pyrimidin-5-ylmethyl)-6-ethoxy-biphenyl-2-yl-ester sowie Säureadditionssalze dieser Verbindung.

**10.** Cyclobutan-sulfonsäure-4'-amino-4-(2,4-diamino-pyrimidin-5-ylmethyl)-6-ethoxy-3'-methyl-biphenyl-2-yl-ester sowie Säureadditionssalze dieser Verbindung.

**11.** Morpholino-4-sulfonsäure-4-(2,4-diamino-pyrimidin-5-ylmethyl)-6-ethoxy-4'-methylamino-biphenyl-2-yl-ester sowie Säureadditionssalze dieser Verbindung.

**12.** Dimethyl-sulfaminsäure-3'-amino-4-(2,4-diamino-pyrimidin-5-ylmethyl)-6-ethoxy-4'-methyl-biphenyl-2-yl-ester sowie Säureadditionssalze dieser Verbindung.

**13.** Propan-2-sulfonsäure-4'-amino-4-(2,4-diamino-pyrimidin-5-ylmethyl)-6-ethoxy-biphenyl-2-yl-ester sowie Säureadditionssalze dieser Verbindung.

**14.** Butan-2-sulfonsäure-3'-amino-4-(2,4-diamino-pyrimidin-5-ylmethyl)-6-ethoxy-biphenyl-2-yl-ester sowie Säureadditionssalze dieser Verbindung.

**15.** N-Cyclopropyl-N-methyl-sulfaminsäure-4'-amino-4-(2,4-diamino-pyrimidin-5-ylmethyl)-6-ethoxy-biphenyl-2-yl-ester sowie Säureadditionssalze dieser Verbindung.

**16.** Propan-2-sulfonsäure-3'-amino-4-(2,4-diamino-pyrimidin-5-ylmethyl)-6-ethoxy-biphenyl-2-yl-ester sowie Säureadditionssalze dieser Verbindung.

**17.** Propan-2-sulfonsäure-4'-amino-4-(2,4-diamino-pyrimidin-5-ylmethyl)-6-ethoxy-3'-methyl-biphenyl-2-yl-ester sowie Säureadditionssalze dieser Verbindung.

**18.** 5-(3'-Amino-6-cyclopropylmethoxy-2-ethoxy-biphenyl-4-ylmethyl)-pyrimidin-2,4-diamin sowie Säureadditionssalze dieser Verbindung.

**19.** 5-(3'-Amino-2,6-diethoxy-biphenyl-4-ylmethyl)-pyrimidin-2,4-diamin sowie Säureadditionssalze dieser Verbindung.

**20.** Verbindungen der allgemeinen Formel

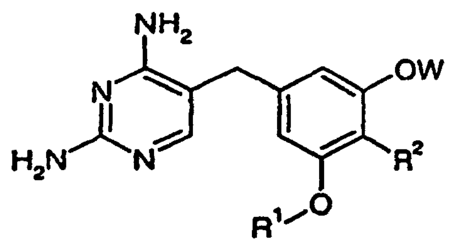

in der $R^1$ und $R^2$ die in Anspruch 1 angegebene Bedeutung haben und W Wasserstoff, Benzyl oder Methoxymethyl darstellt.

**21.** Verbindungen nach einem der Ansprüche 1-19 zur Anwendung als Heilmittel, bevorzugt als Antiinfektiva.

**22.** Verfahren zur Herstellung der Verbindungen gemäss einem der Ansprüche 1-19, **dadurch gekennzeichnet, dass** man

a) eine Verbindung der allgemeinen Formel

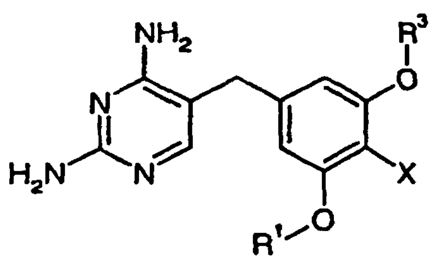

**B**

mit einer Verbindung der allgemeinen Formel

$R^2Y$ C

umsetzt,

in denen $R^1$, $R^2$ und $R^3$ die obige Bedeutung haben, wobei darin ggfs. vorhandene phenolische Hydroxygruppen und Amino/Alkylaminogruppen geschützt sind, eines der Symbole X und Y eine Abgangsgruppe und das andere eine mit dieser Abgangsgruppe austretende Gruppe darstellt,
vorhandene Schutzgruppen abspaltet und gewünschtenfalls aromatische Substituenten an $R^2$/$R^3$ derivatisiert, oder dass man
b) eine Verbindung der allgemeinen Formel

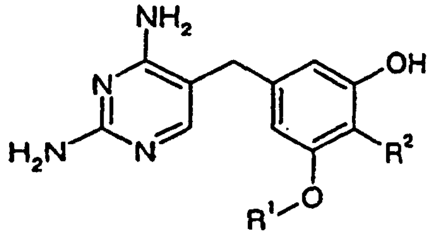

D

mit einer Verbindung der allgemeinen Formel

$R^3Z$ E

in Gegenwart einer Base umsetzt,

in denen $R^1$, $R^2$ und $R^3$ die obige Bedeutung haben, wobei darin ggfs. vorhandene phenolische Hydroxygruppen und Amino/Alkylaminogruppen geschützt sind und Z eine Abgangsgruppe darstellt,
vorhandene Schutzgruppen abspaltet und gewünschtenfalls aromatische Substituenten an $R^2$/$R^3$ derivatisiert, oder dass man
c) zur Herstellung von Verbindungen der Formel A, worin $R^2$ und/oder $R^3$ eine Sulfonylgruppe —$SO_2$- enthält, eine entsprechende Verbindung, worin $R^2$ und/oder $R^3$ eine entsprechende Sulfanylgruppe —S- oder Sulfinylgruppe —SO- enthält, einer Oxidation unterwirft, oder dass man
d) eine Verbindung der Formel A in ein pharmazeutisch annehmbares Säureadditionssalz überführt.

**23.** Arzneimittel, enthaltend eine Verbindung nach einem der Ansprüche 1-19 und einen therapeutisch inerten Träger.

**24.** Verwendung von Verbindungen nach einem der Ansprüche 1-19 zur Herstellung von antibiotisch wirksamen Arzneimitteln, welche diese Verbindungen enthalten.

## Claims

**1.** Substituted 5-benzyl-2,4-diaminopyrimidines of the general formula

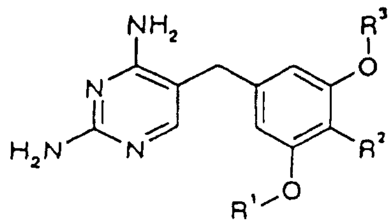

A

in which
$R^1$ is C2-C3 alkyl;
$R^2$ denotes phenyl, naphthyl or heterocyclyl bonded via one of its C atoms, wherein phenyl, naphthyl or heterocyclyl can be mono- or polysubstituted; and
$R^3$ represents C2-C6 alkyl, C2-C6 alkenyl, cycloalkyl, cycloalkylalkyl, heterocyclylalkyl, alkylsulphonyl, phenylsulphonyl, cycloalkylsulphonyl, cycloalkylalkylsulphonyl, cycloalkylalkylsulphamoyl, heterocyclylsulphonyl, heterocyclylalkylsulphonyl or dialkylsulphamoyl, wherein these groups can be unsubstituted or substituted;
and acid addition salts of these compounds;
wherein the heterocyclyl and phenyl groups $R^2$ are unsubstituted or mono- or polysubstituted by halogen, cyano, alkyl, alkoxy, hydroxyl, nitro, amino, alkylamino, dialkylamino, alkanoylamino, formyl, alkonyloxy, cyanoalkyl, cyanoalkoxy, hydroxyalkyl, alkoxyalkyl, hydroxyalkoxy, hydroxyalkylamino, alkoxyalkoxy, carbamoylalkoxy, alkylaminoalkyl, dialkylaminoalkyl, halogenoalkylaminoalkyl, N-alkyl-N-halogenoalkyl-aminoalkyl, alkylsulphanyl (alkylthio), alkylsulphinyl, alkylsulphonyl, alkylsulphonyloxy, alkylsulphonylamino, $R^7$, $R^7$-alkyl, $R^7$-alkoxy, $R^7$-carbonylalkoxyalkyl or $R^7$-alkanoylamino, or are also substituted by two adjacent substituents which together form a fused-on 5- or 6-membered heterocyclic radical; wherein $R^7$ represents optionally substituted heterocyclyl and alkyl, by itself or in combinations, can carry up to 6 carbon atoms;

the naphthyl group $R^2$ is unsubstituted or substituted by hydroxyl;

the heterocyclyl groups $R^7$ are unsubstituted or substituted by halogen, alkyl, alkoxyalkyl, hydroxyalkyl, alkanoyl, alkanoylaminoalkyl or oxo; wherein alkyl, alkoxy and alkanoyl, by themselves or in combinations, can carry up to 6 carbon atoms;

$R^3$, if this denotes C2-C6 alkyl, alkenyl, alkylsulphonyl or dialkylsulphamoyl, is unsubstituted or substituted by halogen, cyano, hydroxyl or alkoxy with up to 6 carbon atoms; and

$R^3$, if this denotes cycloalkyl, cycloalkylalkyl, heterocyclylalkyl, cycloalkylsulphonyl, cycloalkylalkylsulphonyl, heterocyclylsulphonyl or heterocyclylalkylsulphonyl, is unsubstituted or substituted by alkyl, alkoxy, hydroxyalkyl, alkoxyalkyl, cyano, cyanoalkyl or cyanoalkenyl; wherein alkyl, alkoxy and alkenyl, by themselves or in combinations, can carry up to 6 carbon atoms;

and further wherein

"halogen" denotes fluorine, chlorine, bromine or iodine;

"alkyl" or "alkoxy" denote straight chain or branched chain hydrocarbon groups having at most 6 carbon atoms, unless defined otherwise;

"alkenyl" denotes an unsaturated hydrocarbon group and carries at most 6 carbon atoms;

"cycloalkyl" denotes a cyclic hydrocarbon having 3 to 6 carbon atoms;

"alkanoyloxy" or "alkanoylamino" denote alkyl-COO- or alkyl-CONH- groups having at most 6 carbon atoms, wherein alkyl has the meaning explained above; and

"heterocyclyl" or "heterocyclic radical" denote unsaturated or saturated, unsubstituted or substituted 5- or 6-membered heterocyclic rings with at least one heteroatom from nitrogen, oxygen and sulphur, which are unsubstituted or substituted by halogen, hydroxyl, alkyl, alkoxy, hydroxyalkyl, alkoxyalkyl, cyano, cyanoalkyl or cyanoalkenyl.

2. Compounds according to claim 1 of the general formula

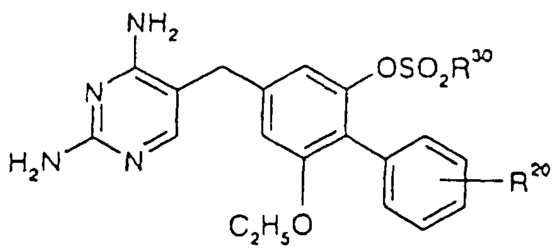

A1

in which $R^{20}$ represents C1-C6 alkyl, C1-C6 alkoxy, amino, C1-C6 alkylamino, fluorine or chlorine and $R^{30}$ represents C1-C6 alkyl, C3-C6 cycloalkyl; di-(C1-C6 alkyl)amino, N-(C3-C6 cycloalkyl)-N-(C1-C6 alkyl)amino or a 5- or 6- membered, saturated N-heterocyclic radical linked on the nitrogen,

and acid addition salts of these compounds.

3. Compounds according to claim 2, **characterised in that** $R^{20}$ represents methyl, methoxy, amino, methylamino or fluorine.

4. Compounds according to claim 2 or 3, **characterised in that** $R^{30}$ denotes isopropyl, sec-butyl, cyclobutyl, dimethylamino, N-cyclopropyl-N-methyl-amino or morpholino.

5. Compounds according to claim 1 of the general formula

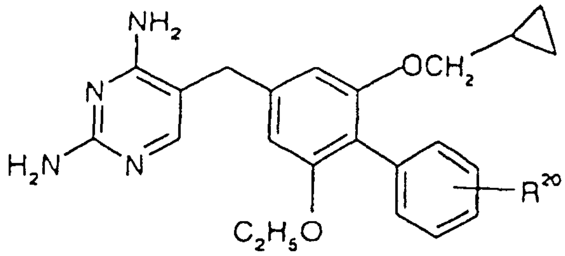

A2

in which $R^{20}$ denotes C1-C6 alkyl, C1-C6 alkoxy, amino, C1-C6 alkylamino, fluorine or chlorine, and acid addition salts of these compounds.

**6.** Compounds according to claim 5, **characterised in that** $R^{20}$ represents methyl, methoxy, amino, methylamino or fluorine.

**7.** Compounds according to claim 1 of the general formula

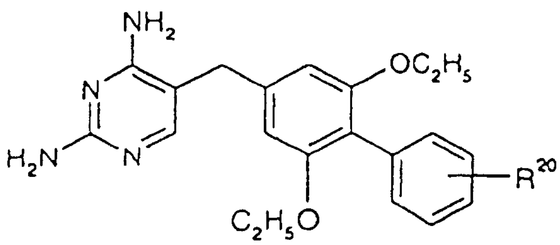

A3

in which $R^{20}$ denotes C1-C6 alkyl, C1-C6 alkoxy, amino, C1-C6 alkylamino, fluorine or chlorine, and acid addition salts of these compounds.

**8.** Compounds according to claim 7, **characterised in that** $R^{20}$ represents methyl, methoxy, amino, methylamino or fluorine.

**9.** Butane-2-sulphonic acid 4'-amino-4-(2,4-diamino-pyrimidin-5-ylmethyl)-6-ethoxy-biphenyl-2-yl ester and acid addition salts of this compound.

**10.** Cyclobutane-sulphonic acid 4'-amino-4-(2,4-diamino-pyrimidin-5-ylmethyl)-6-ethoxy-3'-methyl-biphenyl-2-yl ester and acid addition salts of this compound.

**11.** Morpholino-4-sulphonic acid 4-(2,4-diamino-pyrimidin-5-ylmethyl)-6-ethoxy-4'-methylamino-biphenyl-2-yl ester and acid addition salts of this compound.

**12.** Dimethyl-sulphamic acid 3'-amino-4-(2,4-diamino-pyrimidin-5-ylmethyl)-6-ethoxy-4'-methyl-biphenyl-2-yl ester and acid addition salts of this compound.

**13.** Propane-2-sulphonic acid 4'-amino-4-(2,4-diamino-pyrimidin-5-ylmethyl)-6-ethoxy-biphenyl-2-yl ester and acid addition salts of this compound.

**14.** Butane-2-sulphonic acid 3'-amino-4-(2,4-diamino-pyrimidin-5-ylmethyl)-6-ethoxy-biphenyl-2-yl ester and acid addition salts of this compound.

**15.** N-Cyclopropyl-N-methyl-sulphamic acid 4'-amino-4-(2,4-diamino-pyrimidin-5-ylmethyl)-6-ethoxy-biphenyl-2-yl ester and acid addition salts of this compound.

**16.** Propane-2-sulphonic acid 3'-amino-4-(2,4-diamino-pyrimidin-5-ylmethyl)-6-ethoxy-biphenyl-2-yl ester and acid

addition salts of this compound.

**17.** Propane-2-sulphonic acid 4'-amino-4-(2,4-diamino-pyrimidin-5-ylmethyl)-6-ethoxy-3'-methyl-biphenyl-2-yl ester and acid addition salts of this compound.

**18.** 5-(3'-Amino-6-cyclopropylmethoxy-2-ethoxy-biphenyl-4-ylmethyl)-pyrimidin-2,4-diamine and acid addition salts of this compound.

**19.** 5-(3'-amino-2,6-diethoxy-biphenyl-4-ylmethyl)-pyrimidin-2,4-diamine and acid addition salts of this compound.

**20.** Compounds of the general formula

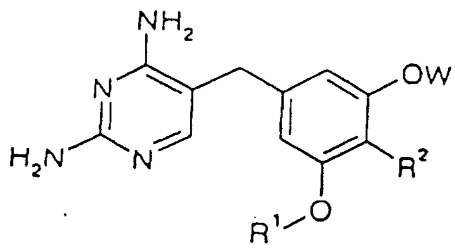

in which $R^1$ and $R^2$ have the meaning given in claim 1 and W represents hydrogen, benzyl or methoxymethyl.

**21.** Compounds according to any one of claims 1-19 for use as medicines, preferably as anti-infectious agents.

**22.** Process for the preparation of the compounds according to any one of claims 1-19, **characterised in that**

a) a compound of the general formula

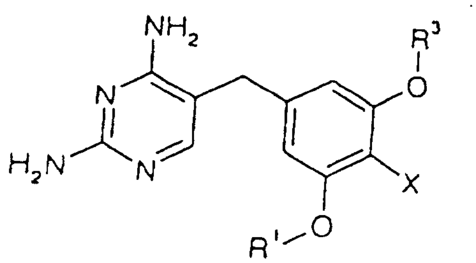

B

is reacted with a compound of the general formula

$R^2Y$ C

in which $R^1$, $R^2$ and $R^3$ have the above meaning, wherein phenolic hydroxyl groups and amino/alkylamino groups optionally present therein are protected, one of the symbols X and Y represents a leaving group and the other represents a group which withdraws with this leaving group,

protective groups present are split off and, if desired, aromatic substituents on $R^2$/$R^3$ are derivatized, or **in that**

b) a compound of the general formula

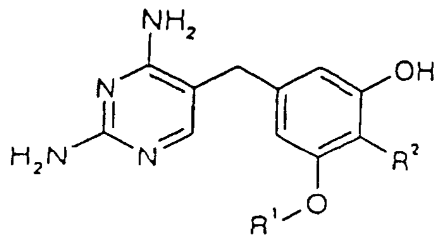

D

is reacted with a compound of the general formula

$R^3Z$ E

in the presence of a base,

in which $R^1$, $R^2$ and $R^3$ have the above meaning, wherein phenolic hydroxyl groups and amino/alkylamino groups optionally present therein are protected and Z represents a leaving group,

protective groups present are split off and, if desired, aromatic substituents on $R^2$/$R^3$ are derivatized, or **in that**

c) to prepare compounds of the formula A, wherein $R^2$ and/or $R^3$ contains a sulphonyl group $-SO_2-$, a corresponding compound wherein $R^2$ and/or $R^3$ contains a corresponding sulphanyl group -S- or sulphinyl group -SO- is subjected to an oxidation, or **in that**

d) a compound of the formula A is converted into a pharmaceutically acceptable acid addition salt.

**23.** Medicament comprising a compound according to any one of claims 1-19 and a therapeutically inert carrier.

**24.** Use of compounds according to any one of claims 1-19 for the preparation of antibiotically active medicaments which comprise these compounds.

## Revendications

**1.** 5-benzyl-2,4-diaminopyrimidines substituées de formule générale :

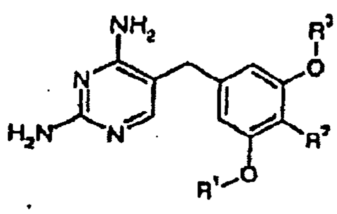

A

dans laquelle

$R^1$ est un alkyle en C2-C3,

$R^2$ signifie phényle, naphtyle ou hétérocyclyle lié par un de ses atomes de C, le phényle, naphtyle ou hétérocyclyle pouvant être mono- ou polysubstitué, et

$R^3$ représente un alkyle en C2-C6, alcényle en C2-C6, cycloalkyle, cycloalkylalkyle, hétérocyclylalkyle, alkylsulfonyle, phénylsulfonyle, cycloalkylsulfonyle, cycloalkylalkylsulfonyle, cycloalkylalkylsulfamoyle, hétérocyclylsulfonyle, hétérocyclylalkylsulfonyle ou dialkylsulfamoyle, ces groupes pouvant être non substitués ou substitués, ainsi que les sels d'addition acide de ces composés,

dans lesquels
les groupes hétérocyclyle et phényle $R^2$ sont non substitués ou sont mono- ou polysubstitués par un halogène, cyano, alkyle, alkoxy, hydroxy, nitro, amino, alkylamino, dialkylamino, alcanoylamino, formyle, alcanoylloxy, cyanalkyle, cyanalkoxy, hydroxyalkyle, alkoxyalkyle, hydroxyalkoxy, hydroxyalkylamino, alkoxyalkoxy, carbamoylalkoxy, alkylaminoalkyle, dialkylaminoalkyle, halogénoalkylaminoalkyle, N-alkyl-N-halogénoalkyl-aminoalkyle, alkylsulfanyle (alkylthio), alkylsulfinyle, alkylsulfonyle, alkylsulfonyloxy, alkylsulfonylamino, $R^7$, $R^7$-alkyle, $R^7$-alkoxy, $R^7$-carbonylalkoxyalkyle ou $R^7$-alcanoylamino, ou sont également substitués par deux substituants contigus formant ensemble un hétérocycle à 5 ou 6 éléments condensé, dans lesquels $R^7$ représente un hétérocyclyle éventuellement substitué et l'alkyle peut comporter seul ou dans les groupements jusqu'à 6 atomes de carbone,
le groupe $R^2$ naphtyle est non substitué ou est substitué par un hydroxy,
les groupes hétérocyclyle $R^7$ ne sont pas substitués ou sont substitués par un halogène, alkyle, alkoxyalkyle, hydroxyalkyle, alcanoyle, alcanoylaminoalkyle ou oxo, dans lesquels l'alkyle, l'alkoxy ou l'alcanoyle peuvent comporter seuls ou dans les groupements jusqu'à 6 atomes de carbone,
$R^3$, quand celui-ci représente un alkyle en C2-C6, alcényle, alkylsulfonyle ou dialkylsulfamoyle, est non substitué ou est substitué par un halogène, cyano, hydroxy ou alkoxy comportant jusqu'à 6 atomes de carbone,
$R^3$, quand celui-ci représente un cycloalkyle, cycloalkylalkyle, hétérocyclylalkyle, cycloalkylsulfonyle, cycloalkylalkylsulfonyle, hétérocyclylsulfonyle ou hétérocyclylalkylsulfonyle, est non substitué ou est substitué par un alkyle, alkyloxy, hydroxyalkyle, alkoxyalkyle, cyano, cyanoalkyle ou cyanoalcényle, l'alkyle, l'alkoxy et l'alcényle pouvant comporter seuls ou dans les groupements jusqu'à 6 atomes de carbone,
et dans lesquels, en outre,
'halogène' signifie fluor, chlore, brome ou iode,
'alkyle' ou 'alkoxy' représentent des groupes d'hydrocarbures à chaîne droite ou ramifiés comportant au plus 6 atomes de carbone, àmoins qu'il n'en soit spécifié autrement,
'alcényle' signifie un groupe hydrocarbure insaturé comportant au maximum 6 atomes de carbone,
'cycloalkyle' signifie un hydrocarbure cyclique comportant 3 à 6 atomes de carbone,
'alcanoyloxy' et 'alcanoylamino' signifient respectivement des groupes alkyl-COO ou alkyl-CONH comportant au plus 6 atomes de carbone, dans lesquels alkyle a la signification mentionnée ci-dessus,
'hétérocyclyl' et 'hétérocycle' signifient des noyaux hétérocycliques à 5 ou 6 éléments, insaturés ou saturés, non substitués ou substitués, comprenant au moins un hétéroatome d'azote, d'oxygène ou de soufre, qui sont non substitués ou substitués par un halogène, hydroxy, alkyle, alkoxy, hydroxyalkyle, alkoxyalkyle, cyano, cyanoalkyle ou cyanoalcényle.

**2.** Composés selon la revendication 1 de formule générale :

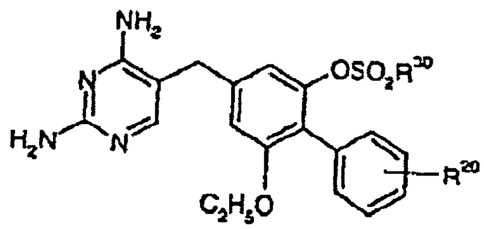

A1

dans laquelle $R^{20}$ représente un alkyle en C1-C6, alkoxy en C1-C6, amino, alkylamino en C1-C6, fluor ou chlore et $R^{30}$ représente un alkyle en C1-C6, cycloalkyle en C3-C6, di-(C1-C6 alkyl)amino, N-(C3-C6 cycloalkyl) -N-(C1-C6 alkyl)-amino ou un N-hétérocycle à 5 ou 6 éléments, saturé, lié par l'atome d'azote,
ainsi que des sels d'addition acide de ces composés.

**3.** Composés selon la revendication 2, **caractérisés en ce que** $R^{20}$ représente un méthyle, méthoxy, amino, méthylamino ou fluor.

**4.** Composés selon la revendication 2 ou 3, **caractérisés en ce que** $R^{30}$ représente isopropyl, sec-butyl, cyclobutyl, di-méthylamino, N-cyclopropyl-N-méthyl-amino ou morpholino.

**5.** Composés selon la revendication 1 de formule générale

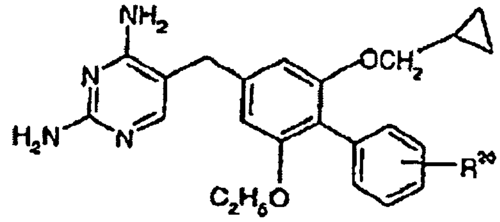

A2

dans laquelle $R^{20}$ signifie alkyle en C1-C6, alkoxy en C1-C6, amino, alkylamino en C1-C6, fluor ou chlore, ainsi que les sels d'addition acide de ces composés.

**6.** Composés selon la revendication 5, **caractérisés en ce que** $R^{20}$ représente un méthyle, méthoxy, amino, méthylamino ou fluor.

**7.** Composés selon la revendication 1 de formule générale

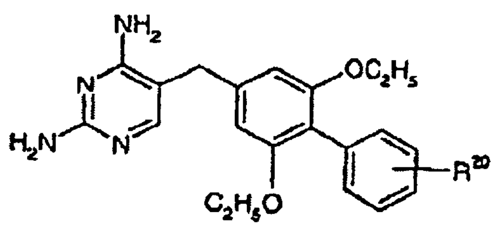

A3

dans laquelle $R^{20}$ représente un alkyle en C1-C6, alkoxy en C1-C6, amino, alkylamino en C1-C6, fluor ou chlore, ainsi que les sels d'addition acide de ces composés.

**8.** Composés selon la revendication 7, **caractérisés en ce que** $R^{20}$ représente un méthyle, méthoxy, amino, méthylamino ou fluor.

**9.** 4'-amino-4-(2,4-diamino-pyrimidin-5-ylméthyl)-6-éthoxy-biphényl-2-yl-ester d'acide butane-2-sulfonique ainsi que les sels d'addition acide de ce composé.

**10.** 4'-amino-4-(2,4-diamino-pyrimidin-5-ylméthyl)-6-éthoxy-3'-méthyl-biphényl-2-yl-ester d'acide cyclobutane sulfonique ainsi que les sels d'addition acide de ce composé.

**11.** 4-(2,4-diamino-pyrimidin-5-ylméthyl)-6-éthoxy-4'-méthylamino-biphényl-2-yl-ester d'acide morpholino-4-sulfonique ainsi que les sels d'addition acide de ce composé.

**12.** 3'-amino-4-(2,4-diamino-pyrimidin-5-ylméthyl)-6-éthoxy-4'-méthyl-biphényl-2-yl-ester d'acide diméthylsulfamique ainsi que les sels d'addition acide de ce composé.

**13.** 4'-amino-4-(2,4-diamino-pyrimidin-5-ylméthyl)-6-éthoxy-biphényl-2-yl-ester d'acide propane-2-sulfonique ainsi que les sels d'addition acide de ce composé.

**14.** 3'-amino-4-(2,4-diamino-pyrimidin-5-ylméthyl)-6-éthoxy-biphényl-2-yl-ester d'acide butane-2-sulfonique ainsi que les sels d'addition acide de ce composé.

**15.** 4'-amino-4-(2,4-diamino-pyrimidin-5-ylméthyl)-6-éthoxy-biphényl-2-yl-ester d'acide N-cyclopropyl-N-méthylsulfamique ainsi que les sels d'addition acide de ce composé.

**16.** 3'-amino-4-(2,4-diamino-pyrimidin-5-ylméthyl)-6-éthoxy-biphényl-2-yl-ester d'acide propane-2-sulfonique ainsi que les sels d'addition acide de ce composé.

**17.** 4'-amino-4-(2,4-diamino-pyrimidin-5-ylméthyl)-6-éthoxy-3'-méthyl-biphényl-2-yl-ester d'acide propane-2-sulfonique ainsi que les sels d'addition acide de ce composé.

**18.** 5-(3'-amino-6-cyclopropylméthoxy-2-éthoxy-biphényl-4-yl-méthyl)pyrimidino-2,4-diamine ainsi que les sels d'addition acide de ce composé.

**19.** 5 - (3'-amino-2,6-diéthoxy-biphényl-4-ylméthyl)-pyrimidino-2,4-diamine ainsi que les sels d'addition acide de ce composé.

**20.** Composés de formule générale

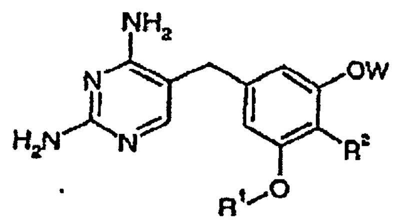

dans laquelle $R^1$ et $R^2$ ont la signification indiquée dans la revendication 1 et W représente hydrogène, benzyle ou méth

**21.** Composés selon une des revendications 1 à 19 destinés à être utilisés en tant que médicaments, de préférence en tant qu'agents anti-infectieux. oxyméthyle.

**22.** Procédé de préparation de composés selon une des revendications 1 à 19, caractérisé en ce

a) qu'on met en réaction un composé de formule générale

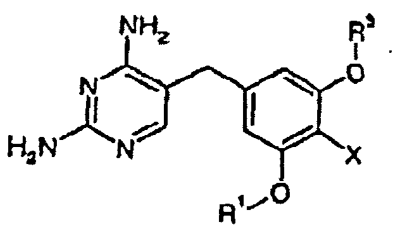

B

avec un composé de formule générale

$$R^2Y \qquad c$$

dans lesquelles $R^1$, $R^2$ et $R^3$ ont la signification indiquée ci-dessus, moyennant quoi les groupes hydroxy phénoliques et les groupes amino/alkylamino éventuellement présents à l'intérieur de ceux-ci sont protégés, l'un des symboles X et Y représente un groupe labile et l'autre un groupe mobile avec ce groupe,
on élimine les groupes de protection présents et on dérivatise si on le souhaite les substituants aromatiques à $R^2/R^3$, ou on

b) fait réagir un composé de formule générale

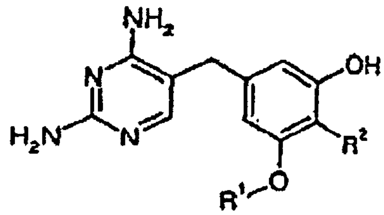

D

avec un composé de formule générale

$$R^3Z \qquad E$$

en présence d'une base,
dans lesquelles $R^1$, $R^2$ et $R^3$ ont la signification indiquée ci-dessus, moyennant quoi les groupes hydroxy phénoliques et les groupes amino/alkylamino éventuellement présents à l'intérieur de ceux-ci sont protégés, et Z représente un groupe labile,
on élimine les groupes de protection présents et on dérivatise si on le souhaite les substituants aromatiques à $R^2$/$R^3$, ou on

c) soumet, pour préparer des composés de formule A dans lesquelles $R^2$ et/ou $R^3$ contien(nent) un groupe sulfonyle - $SO_2$-, un composé correspondant, dans lequel lesquelles $R^2$ et/ou $R^3$ contien(nent) un groupe sulfanyle -S- ou sulfinyle - SO- correspondant, à une oxydation, ou on

d) transforme un composé de formule A en un sel d'addition acide pharmaceutiquement acceptable.

**23.** Médicaments contenant un composé selon une des revendications 1 à 19 et un support inerte du point de vue thérapeutique.

**24.** Utilisation de composés selon une des revendications 1 à 19 pour préparer des médicaments ayant une action antibiotique qui contiennent ces composés.